(19) Europäisches Patentamt
European Patent Office
Office européen des brevets

(11) Veröffentlichungsnummer : **0 358 290 B1**

# (12) EUROPÄISCHE PATENTSCHRIFT

(45) Veröffentlichungstag der Patentschrift : **03.02.93 Patentblatt 93/05**

(21) Anmeldenummer : **89250031.5**

(22) Anmeldetag : **07.09.89**

(51) Int. Cl.$^5$ : **C07D 307/00,** C07D 405/00, C07D 407/00, C07D 413/00, C07F 7/18, A61K 31/34, // C07C405/00, A61K31/557

## (54) 2-Oxa-bicyclo[2.2.1]heptanderivate und diese Verbindungen enthaltende Arzneimittel.

(30) Priorität : **07.09.88 DE 3830878**

(43) Veröffentlichungstag der Anmeldung : **14.03.90 Patentblatt 90/11**

(45) Bekanntmachung des Hinweises auf die Patenterteilung : **03.02.93 Patentblatt 93/05**

(84) Benannte Vertragsstaaten : **AT BE CH DE ES FR GB GR IT LI LU NL SE**

(56) Entgegenhaltungen :
**US-A- 3 950 363**
**US-A- 4 028 350**
**TETRAHEDRON LETTERS, Nr. 24, 1975, Seiten 1957-1960, Pergamon Press, Oxford, GB; G.L. BUNDY: "The synthesis of prostaglandinendoperoxide analogs"**
**CHEMICAL PHARMACEUTICAL BULLETIN, Band 28, Nr. 6, 1980, Seiten 1814-1819, Tokyo, JP; K. SAKAI et al.: "Synthetic studies onprostanoids. XX. Synthesis of stable (+)-prostaglandin H1 analogs"**

(56) Entgegenhaltungen :
**EUROPEAN JOURNAL OF BIOCHEMISTRY, Band 169, Nr. 3, 1987, Seiten 563-569, FEBS, Berlin, DE; M. HECKER et al.: "Thromboxanesynthase catalyses hydroxylations of prostaglandin H2 analogs in the presence of iodosylbenzene"**

(73) Patentinhaber : **SCHERING AKTIENGESELLSCHAFT**
**Müllerstrasse 170/178**
**W-1000 Berlin 65 (DE)**

(72) Erfinder : **Klar, Ulrich**
**Helmkrautstrasse 21**
**W-1000 Berlin 27 (DE)**
Erfinder : **Vorbrüggen, Helmut**
**Wilkestrasse 7**
**W-1000 Berlin 27 (DE)**
Erfinder : **Rehwinkel, Hartmut**
**Glasower Strasse 41**
**W-1000 Berlin 44 (DE)**
Erfinder : **Thierauch, Karl-Heinz**
**Hochwildpfad 45**
**W-1000 Berlin 37 (DE)**
Erfinder : **Stürzebecher, Claus-Steffen**
**Sponholzstrasse 34**
**W-1000 Berlin 41 (DE)**

EP 0 358 290 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

European Journal of Biochemistry 169, 563-569 betrifft die Hydroxylierung von den Prostaglandin-$H_2$-Analoga 15(S)-Hydroxy-11α,9α-epoxymethano- und 15(S) Hydroxy-9α,11α-epoxymethano-5(Z), 13(E)-prostadiensäure durch Thromboxan Synthase.

U.S. Patentschriften 3950363 und 4028350 betreffen 9,11-Epoxymethano- und 11,9-Epoxymethano-Prostaglandinderivate und ihre Anwendung in der Behandlung von Entzündungen.

Die Erfindung betrifft 2-Oxa-bicyclo[2.2.1]heptanderivate der Formel I

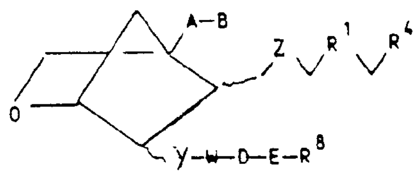

(I),

sowie deren Enantiomere,

worin

A $-(CH_2)_n-$, (E)- oder (Z)-CH=CH- oder -C≡C-,

n 0-7,

B $C_1$-$C_{10}$-Alkyl, $-OR^2$, Halogen, -C≡N, $-N_3$, $-COOR^3$, mit der Einschränkung, daß wenn B $OR^2$, Halogen oder $N_3$ ist und A $-(CH_2)_n-$ ist, n>O sein muß

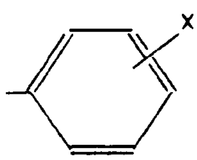
,
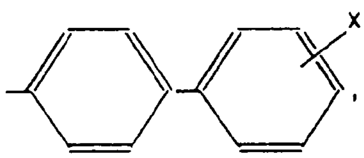
,
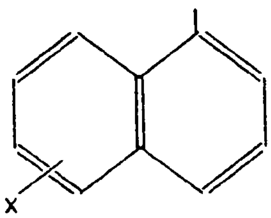
,

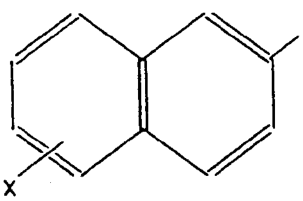
,
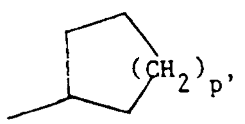
,

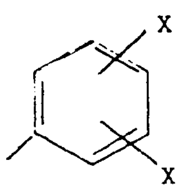
,
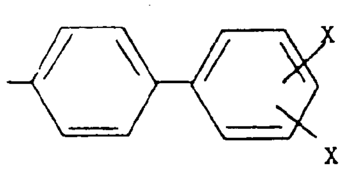
,
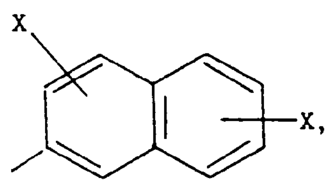
,

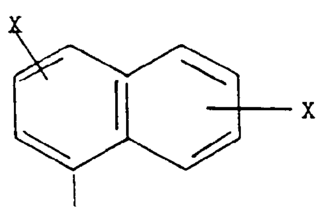

oder einen 5- oder 6-gliedrigen heterocyclischen Rest mit wenigstens einem N, O-oder S-Atom,

$R^1$ Sauerstoff, eine Direktbindung oder eine $-CH_2$-Gruppe,

$R^2$ Wasserstoff, $C_1$-$C_{10}$-Alkyl, durch X substituiertes $C_6$-$C_{12}$-Aryl oder durch X substituiertes $C_7$-$C_{16}$-Aral-

kyl,

$R^3$ Wasserstoff, $C_1$-$C_{10}$-Alkyl, $C_5$-$C_6$-Cycloalkyl, $C_6$-$C_{12}$-Aryl oder $C_7$-$C_{16}$-Aralkyl,

X Wasserstoff, $C_1$-$C_5$-Alkyl,

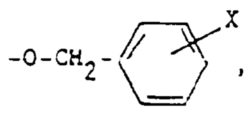,

$-OR^2$, Halogen, -C≡N, $-N_3$ $-NO_2$, $-COOR^3$ Trifluormethyl, oder Phenyl für den Fall $R_8$ ein Gruppe

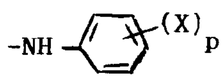

bedeutet, $R^4$, $-COOR^5$, wobei $R^5$ Wasserstoff oder gegenbenenfalls durch Halogen, Phenyl, $C_1$-$C_4$-Alkoxy oder Di-($C_1$-$C_4$)-alkylamino substituiertes $C_1$-$C_{10}$-Alkyl, $C_5$-$C_6$-Cycloalkyl, $C_7$-$C_{16}$-Aralkyl, durch X substituiertes Phenacyl oder $C_6$-$C_{12}$-Aryl oder einen 5- oder 6-gliedrigen heterocyclischen Rest mit wenigstens einem N-, O- oder S-Atom bedeuten kann, oder $-CONHR^6$ mit $R^6$ in der Bedeutung Wasserstoff, $C_1$-$C_{10}$-Alkanoyl oder $C_1$-$C_{10}$-Alkansulfonyl oder $CONR^2R^3$ oder der Rest

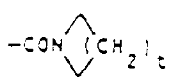

oder den

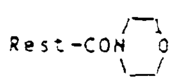

sein kann,

t 2 oder 3,

z,y unabhängig voneinander $-(CH_2)_p-$, (E)CH=CH,$-CH=CR^7-$, -C≡C,

p 0 bis 5,

W eine Direktbindung, die Gruppe

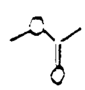,

die Gruppe

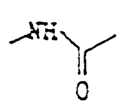,

die Gruppe

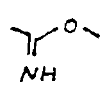,

eine freie oder funktionell abgewandelte Hydroxymethylgengruppe oder eine freie oder funktionell abgewandelte

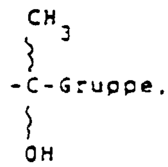

wobei die OH-Gruppe jeweils α- oder β-ständig sein kann,

O eine Direktbindung, die Gruppe

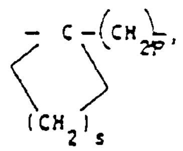

die Gruppe

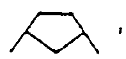

die Gruppe

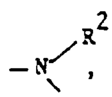

die Gruppe $-NH-SO_2^-$,
eine geradkettige
gesättigte Alkylengruppe mit 1-5 C-Atomen, eine verzweigte gesättigte oder eine geradkettige oder verzweigte ungesättigte Alkylengruppe mit 2-5 C-Atomen, die gegebenenfalls durch Fluoratome substituiert sein können,

s 2 bis 4

E eine Direktbindung, -C≡C- oder $-CH=CR^7-$,

wobei $R^7$ Wasserstoff, $C_1$-$C_5$-Alkyl, Halogen, oder Trifluormethyl,

$R^8$ Wasserstoff, $C_1$-$C_{10}$-Alkyl, $C_3$-$C_{10}$-Cycloalkyl, gegebenenfalls durch X substituiertes $C_7$-$C_{16}$-Aralkyl, gegebenenfalls durch X substituiertes $C_6$-$C_{12}$-Aryl oder einen 5- oder 6-gliedrigen heterocyclischen Rest mit wenigstens einem N-, O- oder S-Atom oder, falls E eine Direktbindung darstellt ,

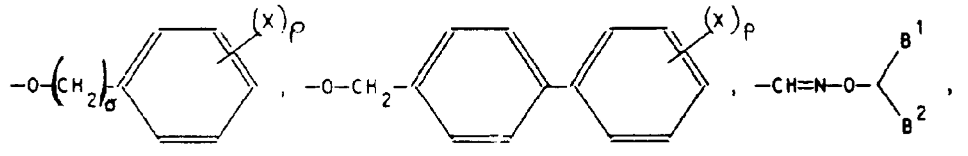

$-O-Si(B^3)_3$,

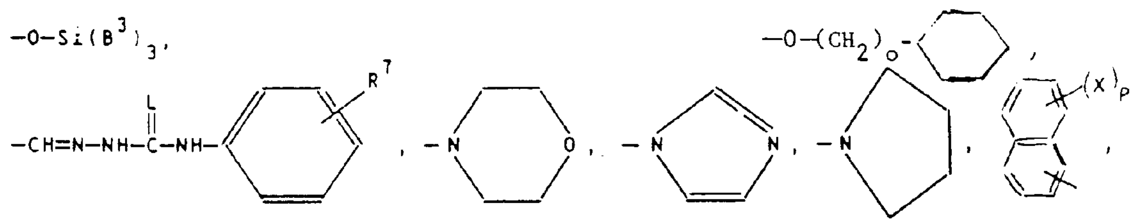

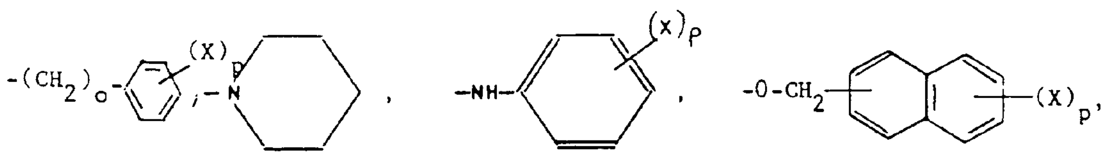

$-O-(C_1-C_8)$-Alkyl, $- O - CH_2 - CH_2 - O-$

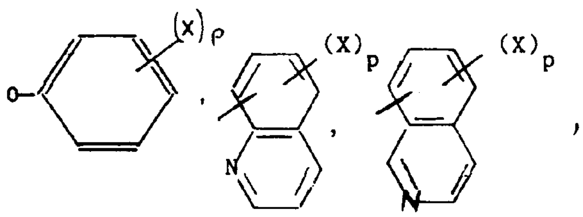

$-O-(CH_2)_S-L-(CH_2)_o-X$, wobei in den Resten $-(X)_p$, wenn p=2 oder 3 ist, X gleich oder verschieden sein kann,

o 0 bis 5,

$B^1$ und $B^2$ gleich oder verschieden sind und mit Ausnahme von Halogen $OR^2$,-C≡N, $N_3$ und $COOR^3$ B bedeuten, und $B^3$ gleich oder verschieden sind und $C_1$-$C_4$-Alkyl, Phenyl oder $C_7$-$C_{16}$-Aralkyl bedeuten,

L Sauerstoff oder Schwefel und, falls $R^5$ Wasserstoff bedeutet, deren Salze mit physiologisch verträglichen Basen bedeuten, sowie die α- β- oder γ-Cyclodextrinclathrate, sowie die mit Liposomen verkapselten Verbindungen der Formel I.

Die Definition "5- oder 6-gliedriger heterocyclischer Rest" betrifft Heterocylen, die wenigstens 1 Heteroatom, vorzugsweise Stickstoff, Sauerstoff oder Schwefel enthalten und mono- oder bicyclisch sind. Beispielsweise seien genannt 2-Furyl, 3-Furyl, 2-Thienyl, 3-Thienyl, 2-Pyridyl, 3-Pyridyl, 4-Pyridyl, Chinolyl, Isochinolyl.

Als Alkylgrupper $R^2$, $R^3$, $R^5$, $R^8$ und B sind gerad- oder verzweigtkettige Alkylgruppen mit 1 - 10 C-Atomen zu betrachten, wie beispielsweise Methyl, Ethyl, Propyl, Isopropyl, Butyl, Isobutyl, tert.-Butyl, Pentyl, Isopentyl, Neopentyl, Heptyl, Hexyl, Decyl.

Die Alkylgruppen $R^2$, $R^3$, $R^5$, $R^8$ und B konnen substituiert sein durch Halogenatome, Hydroxygruppen, $C_1$-$C_4$-Alkoxygruppen, $C_6$-$C_{12}$-Arylgruppen, die durch Halogen substituiert sein können, $D_1$-$C_1$-$C_4$-Alkylamine und Tri-$C_1$-$C_4$-Alkylammonium. Bevorzugt sind solche Alkylgruppen, die einfach substituiert sind.

Als Substituenten seien beispielsweise genannt Fluor-, Chlor- oder Bromatome, Phenyl, Dimethylamino, Diethylamino, Methoxy, Ethoxy.

Als bevorzugte Alkylgruppen $R^2$, $R^3$, $R^5$, $R^8$ und B sind solche mit 1 - 4 C-Atomen, wie z. B. Methyl, Ethyl, Propyl, Isobutyl, Butyl, zu nennen.

Die Alkylgruppe $R^7$ mit 1 - 5 C-Atomen stellt ebenfalls gerad- oder verzweigtkettige Alkygruppen, wie sie bereits für $R_2$ gennant wurden, dar.

Bevorzugte Alkyle ($R^7$) sind Methyl und Ethyl.

Als Arylgruppen $R^2$, $R^3$, $R^5$ und $R^8$ mit 6 - 12 C-Atomen kommen beispielsweise in Betracht: Phenyl, Diphenyl, 1-Naphthyl und 2-Naphthyl, die substituiert sein können durch 1 - 3 Halogenatome, eine Phenylgruppe, 1 - 3 Alkylgruppen mit jeweils 1 - 4 C-Atomen, eine Chlormethyl-, Fluormethyl-, Trifluormethyl-, Carboxyl-, $C_1$-$C_4$-Alkoxy-oder Hydroxygruppe. Bevorzugt ist die Substitution in 3- und 4-Stellung am Phenylring zum Beispiel durch Fluor, Chlor, $C_1$--$C_4$-Alkoxy oder Trifluormethyl oder in 4-Stellung durch Hydroxy.

Die Cycloalkylgruppen $R^3$ und $R^5$ können im Ring 5 und 6 Kohlenstoffatome enthalten. Die Ringe können durch Alkylgruppen mit 1 -4 Kohlenstoffatomen substituiert sein. Beispielsweise seien genannt Cyclopentyl-,

Cyclohexyl, Methylcyclohexyl.

Die Cycloalkylgruppe $R^8$ kann im Ring 3 - 10, vorzugsweise 3 - 6 Kohlenstoffatome enthalten. Die Ringe können durch Alkylgruppen mit 1 - 4 Kohlenstoffatomen substituiert sein. Beispielsweise seien genannt Cyclopropyl, Cyclobutyl, Cyclopentyl, Cyclohexyl, Methylcyclopentyl und Methylcyclohexyl.

Aralkyl mit 7 - 16 C-Atomen für $R^2$, $R^3$, $R^4$ und $B^3$ bedeutet einen der vorstehend genannten niederen Alkylreste mit 1 - 4 C-Atomen mit einem Phenylsubstituenten wie z. B. Benzyl, Phenethyl, der substituiert sein kann durch 1 - 3 Halogenatome, eine Fluormethyl oder Trifluormethylgruppe.

Die unter den Definitionen genannten $C_1$-$C_4$- bzw. $C_1$-$C_5$- bzw. $C_1$-$C_8$-Alkylgruppen sollen geradkettige oder verzweigte Alkylgruppen sein, wie sie für die vorstehenden Alkylgruppen bereits genannt wurden.

Als Alkylengruppe D kommen geradkettige oder verzweigtkettige, gesättigte und ungesättigte Alkylenreste, vorzugsweise gesättigte, mit bis zu 5 C-Atomen infrage, die gegebenenfalls durch Fluoratome substituiert sein können. Beispielsweise seien genannt: Methylen, Fluormethylen, Ethylen, 1,2-Propylen, Ethylethylen, Trimethylen, Tetramethylen, Pentamethylen, 1-Methyltetramethylen, 1-Methyltrimethylen.

Die Hydroxygruppen in W können funktionell abgewandelt sein, beispielsweise durch Veretherung oder Veresterung, wobei die freien oder abgewandelten Hydroxygruppen $\alpha$- oder ß-ständig sein können, wobei freie Hydroxygruppen bevorzugt sind.

Als Ether- und Acylreste kommen die dem Fachmann bekannten Reste in Betracht. Bevorzugt sind leicht abspaltbare Etherreste, wie beispielsweise der Tetrahydropyranyl-, Tetrahydrofuranyl-, Tribenzyl-silylrest. Als Acylreste kommen z.B. Acetyl, Propionyl, Butyryl, Benzoyl in Frage.

Halogen in den Definitionen für B, X und $R^5$ bedeutet Fluor, Chlor und Brom.

Die Reste $C_1$-$C_{10}$-Alkanoyl oder $C_1$-$C_{10}$-Alkansulfonyl für $R^6$ entsprechen den bereits genannten Alkyl-Gruppen gleicher Länge mit dem Unterschied, daß sie an eine Carboxylgruppe gebunden sind. Bevorzugt sind $C_1$-$C_4$-Alkanoyl bzw. -Alkansulfonyl.

Zur Salzbindung mit den freien Säuren ($R^5$ = H) sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin u.s.w.

Bevorzugt werden die Verbindungen der Formel I, in denen

A die Gruppen $-(CH_2)_n-$ mit n = 0 - 2 und -C≡C-,

B Wasserstoff (für n=1-2), Alkyl mit 1 - 4 C-Atomen, Phenyl, p-Halogenphenyl (insbesondere p-Fluorphenyl), Diphenyl,

Z,y unabhängig voneinander die Gruppen $-(CH_2)_p-$ mit p = 0 bis 2 und (E)-CH=CH-, $-CH=CR^7-$, -C≡C-,

W eine Direktbindung oder die Gruppe -CH(OH)-, -O-CO- und -NH-CO-

D eine Direktbindung oder eine geradkettige oder durch $C_1$-$C_2$-Alkylgruppen substituierte Alkylengruppe mit 1 - 3 C-Atomen in der Kette, die Gruppen $-N(R^2)-$ und $-NH-SO_2-$,

E eine Direktbindung, die Gruppe -C≡C-

$R^8$ Wasserstoff, $C_1$-$C_4$-Alkyl, $C_5$-$C_6$-Cycloalkyl, $C_1$-$C_7$-Alkoxy, eine gegebenenfalls p'-fach durch Halogen (insbesondere Fluor), CN, OH,Trifluormethyl oder Phenyl substituierte Benzyloxygruppe, eine gegebenenfalls p'-fach durch Halogen (bevorzugt Fluor), CN, OH oder Phenyl substituierte Anilinogruppe, eine gegebenenfalls p-fach durch Halogen (bevorzugt Fluor), CN, OH oder Phenyl substituierte 5- oder 6-gliedrige heterocyclische Gruppe

mit 1 oder 2 Heteroatomen (N oder O), die mono- oder bicyclisch sein kann, wie sie bereits für $R^8$ im einzelnen genannt wurden, z.B.

, 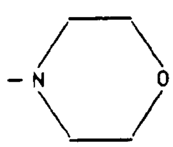 , 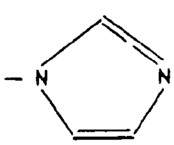 , 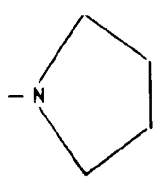 , 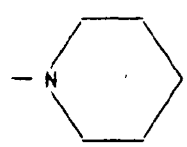 ,

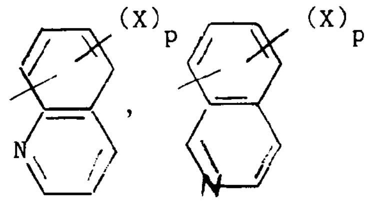 ,

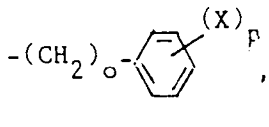 , 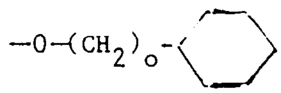 , 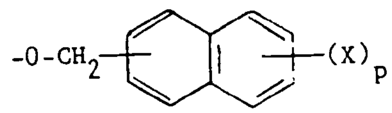

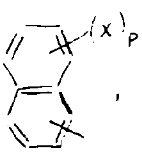 , 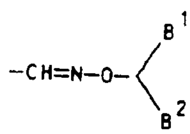 , 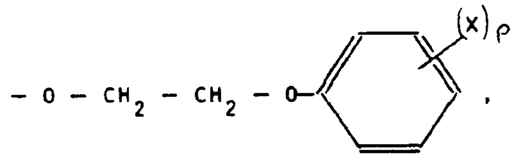 ,

wobei in den Resten $-(X)_p$, wenn p=2 oder 3 ist, X gleich oder verschieden sein kann,
o 0 bis 5,
p 0 bis 5,
p' 1 bis 5
X Halogen (insbesondere Fluor), CN, OH, Trifluormethyl oder Phenyl,
$B^1$ und $B^2$ verschieden sind und Wasserstoff,

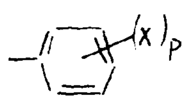 , 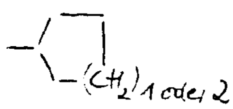

bedeuten,
$R^1$ $-CH_2-$ und
$R^4$ die Reste $-COOR^5$ mit $R^5$ als Wasserstoff, $C_1$-$C_4$-Alkyl oder gegebenenfalls durch Halogen (Fluor) substituiertes $C_7$-$C_8$-Aralkyl oder

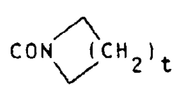

mit t = 2 oder 3 bedeuten.

Bevorzugt ist insbesondere die Kombination aller durch die Beispiele offenbarten Definitionen der Reste A, n, B, $R^1$, $R^2$, $R^3$, $R^4$, X, p, $R^5$, $R^6$, t, Z, y, $R^7$, W, D, s, E, o, $B^1$, $B^2$, $B^3$, L.

Die anmeldungsgemäßen Verbindungen der Formel I lassen sich wie nachstehend näher beschrieben herstellen:

A.

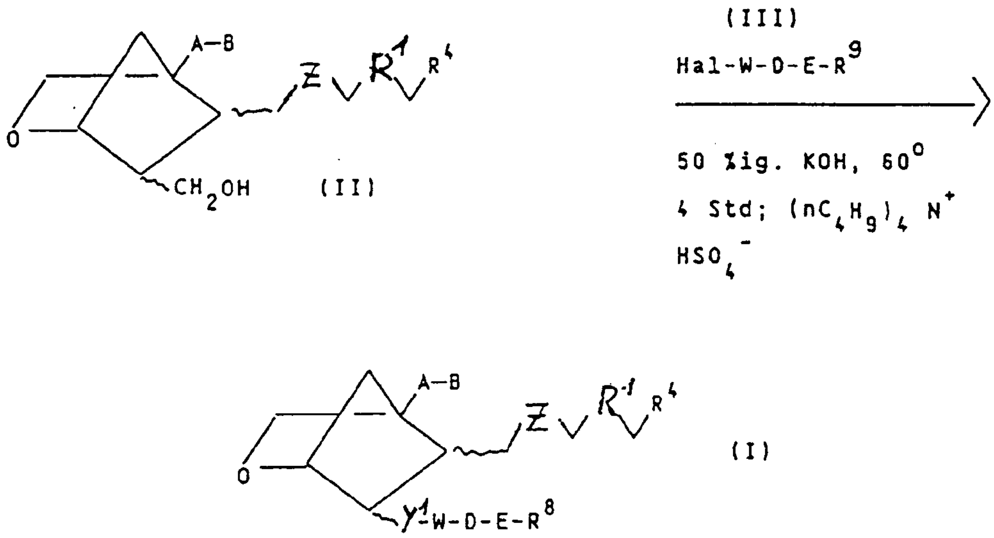


(II)

(I)

mit A, B, Z, $R^1$ in den oben angegebenen Bedeutungen,

Hal als Brom oder Chlor,

W,D,E als Direktbindung, wenn

$R^8$ eine gegebenenfalls durch Halogen, Trifluormethyl, Nitro, Cyano oder Phenyl substituierte Benzyl-, Phenyl-, Benzyl-oxy-, 2-Phenyloxyethoxy- oder Naphthylmethyloxy-gruppe,

$y^1$ die $-(CH_2)_p$-Gruppe mit p=1 bedeutet

$R^9$ eine gegebenenfalls durch Halogen, Trifluormethyl, Nitro, Cyano oder Phenyl substituierte Benzyl-, Phenyl-, 2-Phenyloxyethyl- oder Naphthylmethyl-gruppe

und $R^4$ in der Bedeutung eines $-COOR^5$-Esters.

B.

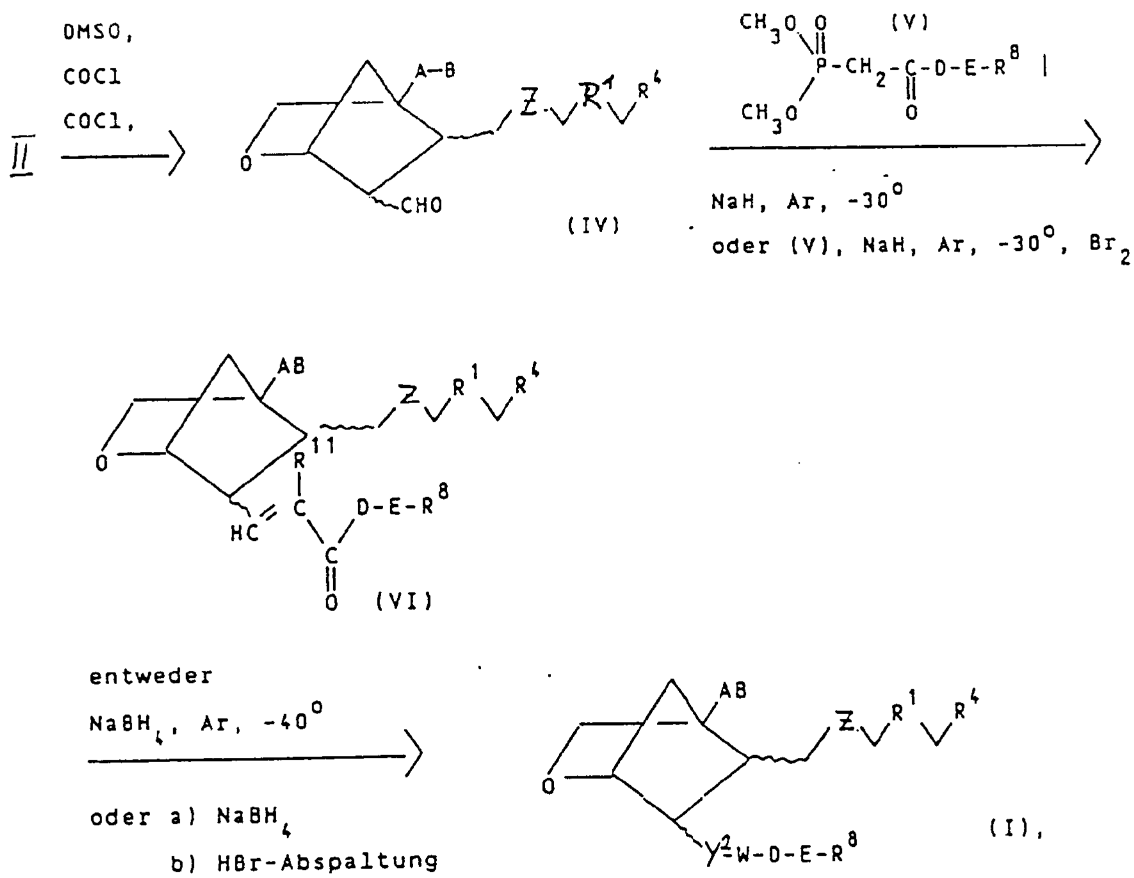


(IV)

(V)

(VI)

(I),

mit A, B, Z, $R^1$ in den obenangegebenen Bedeutungen,

D als gegebenenfalls durch Alkyl substituiertes Alkylen,

E als Direktbindung, als Gruppe -C≡C- oder als gegebenenfalls substituierte Phenoxygruppe

$R^8$ als Wasserstoff, Alkyl

$R^4$ in der Bedeutung eines $-COOR^5$-Esters,

$R^{11}$ als Wasserstoff oder Brom

$y^2$ (E)-CH=CH- oder -C≡C-

W- CH(OH)-, oder

D und E als Direktbindung, wenn $y^1$ die Gruppe -CH=CH-, $R^8$ die Reste $C_1$-$C_{10}$- Alkyl oder $C_7$-$C_{16}$-Aralkyl und W eine Hydroxymethylengruppe bedeutet oder W den Rest

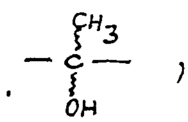

oder

D eine geradkettige oder verzweigte gesättigte Alkylengruppe mit 2-5 C-Atomen,

E die Gruppe -C≡C- bedeuten, wenn

$y^1$ die Gruppen -CH=CH- oder -CH=CBr-

$R^8$ $C_1$-$C_{10}$-Alkyl darstellen.

C.

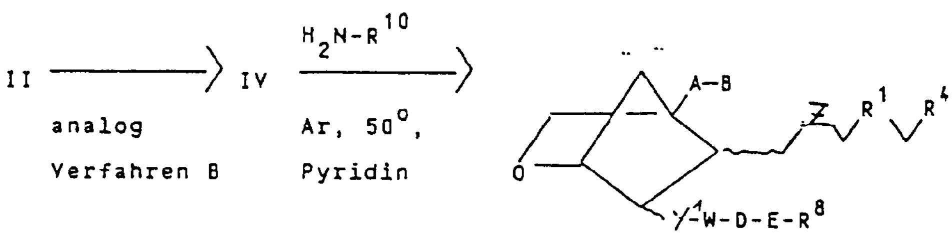


(I),

mit A,B,Z,$R^1$ in den oben angegebenen Bedeutungen,

$y^1$ als $-(CH_2)_p-$ mit p = 0,

W,D,E als Direktbindung

$R^8$ als $-CH=N-O-CH(C_6H_5)_2$ oder

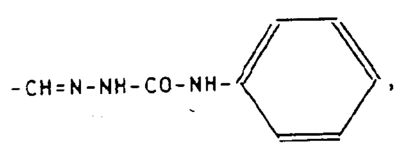

$R^4$ als $-COOR^5$-Ester und

$R^{10}$ als $-O-CH(B^1)B^2$ oder

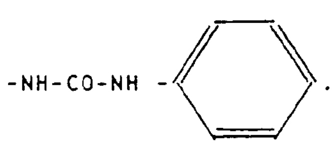

$B^1$ und $B^2$ als erschiedene Reste in der Bedeutung Wasserstoff,

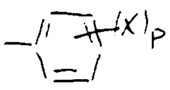, 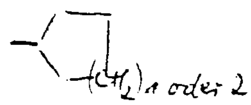

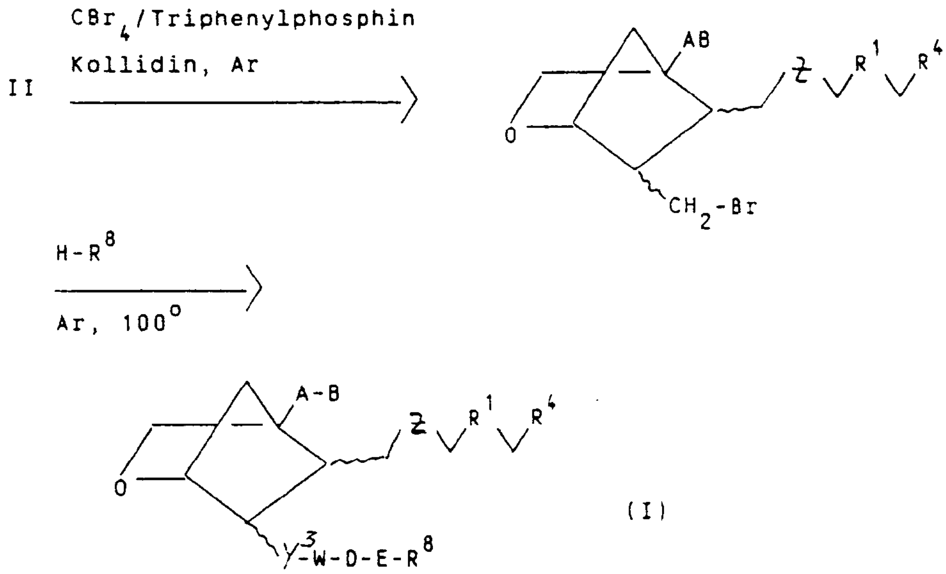


(VII)

(I)

D. mit A,B,Z,$R^1$,X in den oben angegebenen Bedeutungen,

$y^3$ als $-(CH_2)_p-$ mit p = 1
W,D,E als Direktbindung,
$R^8$ als

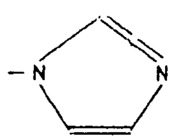, 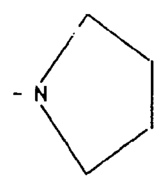, 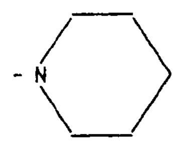, 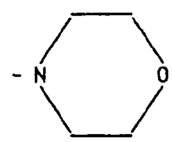,

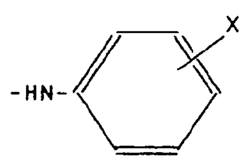, 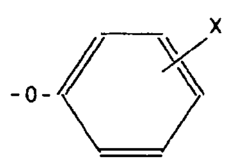

und $R^4$ als $-COOR^5$-Ester.

E.

II → analog Verfahren D → VII → $H - R^8$, Ar, 100°

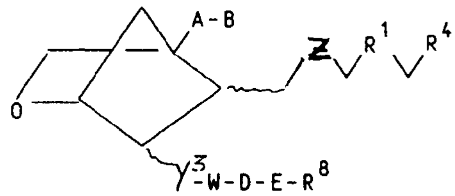

(I),

mit A,B,$R^1$,Z,$y^3$,W,D,E,$R^8$ in den unter Verfahren D angegebenen Bedeutungen und $R^4$ als

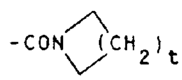

und t in der Bedeutung 2 oder 3.

F.

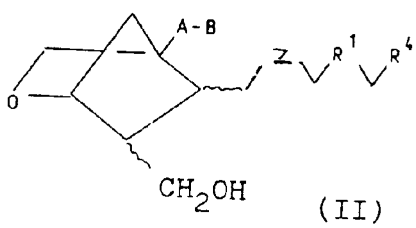

(II)

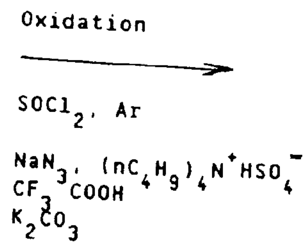


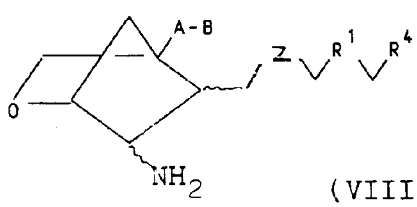

(VIII)

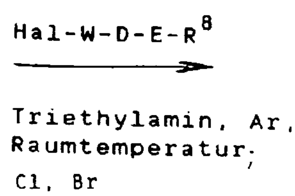


Hal als Cl, Br

W eine Direktbindung oder -CO-

D eine Direktbindung oder $-SO_2-$

E eine Direktbindung

$R^8$ wie Formel I angegeben

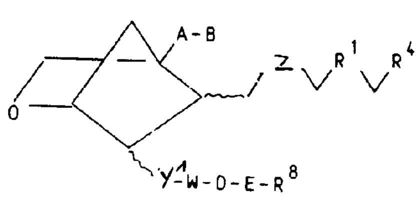

(I)

mit A, B, Z, $R^1$, $R^4$ in den oben angegebenen Bedeutungen,

$y^1$ in der Bedeutung $-(CH_2)_p$-mit p = O

W eine Direktbindung oder die Gruppe -NH-CO-

D eine Direktbindung oder die Gruppe $-NH-SO_2-$

E eine Direktbindung und

$R^8$ Alkyl mit 1-10 C-Atomen, Aralkyl mit 7-16 C-Atomen, Cycloalkyl und Aryl, wie sie zu Beginn der Beschreibung genannt wurden oder gegebenenfalls durch 1-5 X-Reste substituierte, aromatische, carbocyclische oder heterocyclische, mono- oder bicyclische Reste wie sie bereits oben genannt wurden.

Die Verbindungen der Formel I lassen sich nach Anspruch 3 entsprechend den oben beschriebenen Verfahrensalternativen herstellen.

Die Ausgangsverbindungen der Formel II werden entsprechend den in den Beispielen 1 a - 1 l oder in den daraufffolgenden Beispielen (z.B. 274a-274m,276a-276u und 327a angegebenen Vorschriften hergestellt.

Die als Zwischenprodukt für die Herstellung der Ausgangsverbindungen der Formel II dienenden Lactole IX sowie die dazugehörigen Enantiomeren,

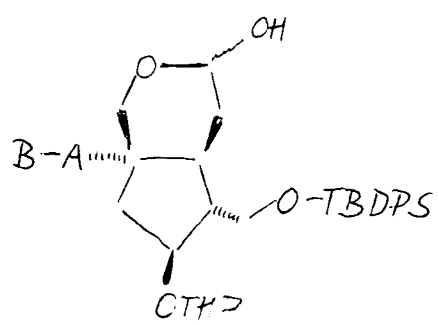

(IX),

worin A und B die obengenannten Bedeutungen haben und THP für Tetrahydropyranyl und TBDPS für tert.-Butyl-diphenylsilyl stehen, können entsprechend dem im Beispiel 274 m angegebenen Hinweis in Analogie zu den Beispielen 17a bis 17g und 1f bis 1l aus enantiomerem Corey-Lacton hergestellt werden. Die Lactole IX werden dann, wie das beispielsweise an den Beispielen 274 m (und folgende Beispiele 274l -274) und 276f (und folgende Beispiele 276e - 276) gezeigt wurde, zu Verbindungen der Formel II umgesetzt.

Die Verseifung der Ester in $-COOR^5$ erfolgt beispielsweise mit Alkali- oder Erdalkali-carbonaten oder -hydroxyden in einem Alkohol oder der waßrigen Lösung eines Alkohols. Als Alkohole kommen aliphatische Alkohole in Betracht, wie zum Beispiel Methanol, Ethanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei -10° C bis 70° C, vorzugsweise bei 25° C.

Die Reaktionsbedingungen der nachfolgenden Verfahrensstufen sind:

1) II → I (Verfahren A)

In Gegenwart von wäßrigen Alkali- oder Erdalkalihydroxidlösungen und unter Verwendung von Phasentransfer-Katalysatoren (wie z. B. Tetrabutylammoniumhydrogenphosphat oder -sulfat) werden Verbindungen der Formel II bei 20 -100° C in 1 - 8 Std. mit dem Reaktanten III als organische Phase oder einer Lösung von III in einem inerten, nicht mit Wasser mischbaren organischen Lösungsmittel umgesetzt.

2) II → IV (Verfahren B)

Die Oxydation von Verbindungen der Formel II erfolgt nach bekannten Verfahren, wie z. B. nach dem von Swern, Collins sowie unter Verwendung von Pyridiniumdichromat bzw. -chlorochromat in Lösungsmitteln wie Methylenchlorid, Diethylether, Tetrahydrofuran, Benzol oder Toluol bei -80° bis -50° (Swern) bzw. bis + 30 ° (bei den übrigen Oxydationen) innerhalb von 10 Minuten bis 24 Stunden, bevorzugt 30 Minuten bis 2 Stunden.

IV → VI (Verfahren B), VI → I (Verfahren B)

Die Umsetzung der Verbindungen IV mit den Phosphonaten V sowie die sich anschließende Reduktion bzw. HBr-Eliminierung erfolgten analog den in der DE-OS 28 45 770 genannten Bedingungen.

3) IV → I (Verfahren C)

Die Reaktion von Verbindungen der Formel IV mit Verbindungen der Formel $H_2N-R^{10}$ (bzw. des Hydrochlorids) wird in alkoholischer Lösung (bevorzugt ethanolischer) in Gegenwart katalytischer (equimolarer) Mengen einer organischen Base (z.B. Pyridin, DABCO, DBN, DBU, Triethylamin, DMAP usw.) bei 20 - 100° C (bevorzugt 40 -60°C) in 2 - 24 Stunden (bevorzugt 5 - 10 Stunden) durchgeführt.

4) II → VII (Verfahren D)

Als Bromierungsmittel für die Reaktion II → VII kommen Thionylbromid, $CBr_4$ bzw. $C_2Br_6$/Triphenylphosphin in Gegenwart einer organischen Base (wie z.B. Pyridin, Kollidin, Anilin usw.) in Betracht. Die Reaktion wird bei 0 - 80° C (vorzugsweise 20 - 50° C) in 30 Minuten bis 2 Stunden durchgeführt. Als Lösungsmittel werden inerte organische Lösungsmittel wie z.B. Methylenchlorid, Diethylether, Chloroform etc. verwendet.

5) VII → I (Verfahren D und E)

Die Umsetzung der Bromide der Formel VII mit $H-R^8$ erfolgt entweder mit uberschüssigem $H-R^8$ oder unter Zusatz eines inerten Lösungsmittels wie Toluol bei 60° - 120° C (bevorzugt 80 - 100° C) in 2 - 28 Stunden (bevorzugt 2 - 12 Stunden). Wenn $H-R^8$ ein Phenol darstellt, muß unter Verwendung eines zusätzlichen Deprotinierungsmittels (wie z.B. NaH, BuLi, Natriummethanolat) gegebenenfalls unter Zusatz eines Kupfersalzes gearbeitet werden.

6) II → VIII (Verfahren F)

Die Oxidation der Verbindungen der Formel II wird bevorzugt mit Jones-Reagenz oder Pyridiniumchlorochromat unter Beachtung der hierfür erforderlichen Reaktionsbedingungen durchgeführt. Anschließend wird unter üblichen Bedingungen mit $SOCl_2$ zum Chlorid umgesetzt, unter Phasentransferkatalyse mit wässriger $NaN_3$-Lösung zur Reaktion gebracht und wie in den zugehörigen Beispielen beschrieben zu Verbindungen VIII umgesetzt.

7) VIII → I (Verfahren F)

Die Umsetzung der Verbindungen VIII mit den Verbindungen der Formel Hal-WDE-$R^8$ erfolgt wie in den dafür genannten Beispielen angegeben.

Die Salze der erfindungsgemäßen Säuren der Formel I werden durch Umsetzen einer Lösung der Säure in einem inerten Lösungsmittel, wie z.B. Methanol, oder in wässrigen Lösungen oder Wasser-haltigen Lösungen und Zugabe des Salz-Bildners [z.B. Tris-(hydroxymethyl)-aminomethan] und anschließendes Abdampfen des Lösungsmittels erhalten. Cyclodextrinclatherate werden analog der Vorschrift in WO 87/05294 erhalten. Liposome werden nach dem in "Pharmazie in unserer Zeit 11, 98 (1982)" beschriebenen Herstellungsverfahren hergestellt.

Alle stereoisomeren Formen gehören ebenfalls zum Gegenstand der Erfindung.

Biologische Wirkung und Anwendungsbereich der neuen TXA2-Antagonisten:

Die Verbindungen dieser Erfindung eignen sich zur Therapie von Erkrankungen des cardiovaskulären Systems, des Magens, des Pankreas, der Leber und der Niere. Sie wirken blutdrucksenkend und bronchodilatorisch. Sie sind geeignet zur Hemmung der Thrombozytenaktivierung. Folglich stellen die neuen TXA2-Antagonisten der Formel I wertvolle pharmazeutische Wirkstoffe dar. Darüber hinaus zeichnen sich die Verbindungen bei ähnlichem Wirkungsspektrum verglichen mit entsprechenden TXA2-Antagonisten durch eine höhere Spezifizät und Selektivität (bei fehlendem Partialagonismus), eine wesentlich längere Wirksamkeit und eine größere Stabilität aus.

Die neuen TXA2-Antagonisten besitzen die für diese Verbindungsklasse typischen Eigenschaften, wie z.B. Senkung des peripheren arteriellen, des koronaren und des pulmonalen Gefäßwiderstandes, Senkung des pulmonalen Blutdrucks, Senkung des systemischen Blutdrucks ohne zugleich Schlagvolumen und koronare Durchblutung zu senken, Förderung der Nierendurchblutung und der Durchblutung anderer peripherer Organe. Erhöhung der cerebralen Durchblutung, Inhibierung der Thrombozytenaktivierung und Auflösung von Thromben, Inhibierung der Bronchokonstriktion, Inhibierung der Magensäuresekretion, Zytoprotektion des Herzens, der Magen- und Darmschleimhaut, der Leber, Zytoprotektion im Pankreas und in der Niere sowie antiallergische Eigenschaften. Daher sind die neuen TXA2-Antagonisten prinzipiell geeignet zur Behandlung des Schlaganfalles, der Prophylaxe und Therapie koronarer Herzerkrankungen, zum Beispiel der Koronarthrombose, zur

Behandlung des Herzinfarktes, peripherer Arterienerkrankungen, zur Prophylaxe und Therapie bei anderen thromboembolischen Erkrankungen und bei Arteriosklerose, bei ischämischen Attacken des ZNS-Systems und anderer Durchblutungsstörungen des Gehirns, zur Behandlung der Hypertonie und zur Behandlung von Krankheiten, die mit einer Erhöhung des pulmonalen Gefäßwiderstandes einhergehen wie z.B. der pulmonalen Hypertonie und zur Therapie des Schocks und des Asthmas. Sie können ferner eingesetzt werden zur Inhibierung von Geburtswehen und zur Behandlung von Schwangerschaftstoxikosen.

Die neuen TXA2-Antagonisten können außerdem Anwendung finden zur Verbesserung der Organfunktion nach Transplantation, zum Beispiel bei der Nierentransplantation, zur Verhinderung von Abstoßungsreaktionen, an Stelle von Heparin oder als Adjuvans bei der Dialyse oder Hämofiltration und bei der Konservierung von Blutplasmakonserven, zum Beispiel von Blutplättchenkonserven.
Die neuen Thromboxanantagonisten besitzen eine antimetastatische Wirkung und antiproliferative Eigenschaften.

Die neuen TXA2-Antagonisten können in Kombination, zum Beispiel mit Carbacyclinen, Prostacyclin und seinen Analoga, 7-Oxo-prostacyclinen, Prostaglandinen und deren Derivate und 6-Keto-$PGE_1$-Derivaten, mit $TXA_2$-Synthetase-Inhibitoren, mit Phosphodiesterase-Inhibitoren, mit Antagonisten und Rezeptorantagonisten verschiedener Thrombozytenstimulatoren (z.B. ADP, Thrombin, Collagen, PAF, Adrenalin, Serotonin, Fibrinogen) mit Calciumantagonisten, mit Fibrinolytika und Thrombolytika, z. B. t-PA, mit Heparin und anderen Antikoagulanzien, mit Zyklooxygenasehemmern, z.B. Acetylsalizylsäure, mit Hemmstoffen der Lipoxygenasen sowie Antagonisten von Lipoxygenaseprodukten, mit Vasodilatatoren wie z.B. Nitroverbindungen, mit Antihypertensiva wie z.B. β-Blockern oder mit Diuretika Verwendung finden.

Die Dosis der Verbindungen ist 0,1 - 500 mg/pro Tag, auch in mehreren Teildosierungen, wenn sie am menschlichen Patienten verabreicht werden. Die Einheitsdosis für den pharmazeutisch akzeptablen Träger beträgt 0,1 - 100 mg. Für die parenterale Verabreichung werden sterile, injizierbare wäßrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z.B. zur Herstellung von Blutdrucksenkern dienen.

Der Einheitsdosisbereich für die Ampulle ist 0,1 - 100 mg, für die Tablette 0,1 - 100 mg.

Beispiel 1

(4S,5R(5Z),6S,1R)-7-[4-Methyl-6-benzyloxymethyl-2-oxabicyclo[2.2.1]heptan-5-yl]-5-heptensäuremethyl (bzw. Benzyl) ester:

16 mg (56,7 μmol) des nach Beispiel 1a dargestellten Alkohols versetzte man mit 160 μl einer 50%igen wässrigen Kaliumhydroxydlösung, 100 μl Benzylchlorid und 1,5 mg Tetrabutylammoniumhydrogensulfat. Das heterogene Gemisch erwärmte man auf 60 °C, ließ 4 Stunden reagieren, extrahierte das erkaltete Gemisch mehrfach mit insgesamt 4 ml Diethylether und reinigte durch Chromatographie an einer analytischen Dünnschichtplatte. Auf eine Trennung des Methylesters von ebenfalls gebildeten Benzylester wurde verzichtet. Nach Elution mit Diethylether isolierte man 18 mg der Titelverbindungen als farbloses Öl.

IR (Film): 3080, 3060, 3020, 3000, 2960, 2940, 2870, 1735, 1455, 1125, 1020, 965, 895, 740 und 700 $cm^{-1}$.

Beispiel 1a

(4S,5R(5Z),6S,1R)-7-[4-Methyl-6-hydroxymethyl-2-oxabicyclo[2.2.1]heptan-5-yl]-5-heptensäuremethylester:

400 mg (768 μmol) des in Beispiel 1b dargestellten Bicyclus löste man in 4 ml wasserfreiem Tetrahydrofuran, versetzte mit 4,0 ml einer 1 molaren Lösung von Tetrabutylammoniumfluorid in Tetrahydrofuran und rührte die gelbe homogene Lösung 1 Stunde bei 23 °C unter einer Atmosphäre aus trockenem Argon. Man goß auf 20 ml einer eiskalten 10%igen Ammoniumchloridlösung, extrahierte mehrfach mit insgesamt 25 ml Diethylether, wusch mit Wasser und isolierte nach Trocknung über Magnesiumsulfat, Filtration und Einengen im Wasserstrahlvakuum 432 mg Rohöl, das durch Chromatographie an ca. 70 ml Kieselgel mittels eines Gradientensystems aus Aceton in n-Hexan gereinigt wurde. Isoliert wurden 206 mg (730 μmol, 95 %) der Titelverbindung als farbloses Öl.

IR (Film): 3200-3600, 2950, 2930, 2870, 1735, 1450, 1435, 1155, 1010, 960 und 890 $cm^{-1}$.

Beispiel 1b

(4S,5R(5Z),6S,1R)-7-[4-Methyl-6-tert-butyldiphenylsilyloxymethyl-2-oxabicyclo-[2.2.1]heptan-5-yl]-5-heptensäuremethylester:

Die farblose Lösung von 598 mg (1,11 mmol) des in Beispiel 1c dargestellten Diols in 5 ml wasserfreiem Toluol versetzte man nacheinander mit 780 mg Triphenylphosphin, 0,7 ml Diazodicarbonsäurediethylester und rührte 7 Stunden bei 23 °C unter einer Atmosphäre aus trockenem Argon. Man versetzte mit 15 ml Diethylether, wusch mehrfach mit Wasser, trocknete über Magnesiumsulfat und isolierte nach Filtration, Einengen im Wasserstrahlvakuum und Chromatographie an Kieselgel mit einem Gradientensystem aus Diethylether in n-Hexan 403 mg (774 μmol, 70 %) der Titelverbindung als farbloses Öl.

IR (Film): 3070, 3050, 2950, 2930, 2860, 1735, 1590, 1425, 1110, 1015, 895, 820, 740 und 705 $cm^{-1}$.

Beispiel 1c

(1S,2R(5Z),3S,4R)-7-[1-Hydroxymethyl-1-methyl-3-tert-butyldiphenylsilyloxymethyl-4-hydroxy-cyclpentan-2-yl]-5-heptensäuremethylester:

Die farblose Lösung von 921 mg (1,48 mmol) der in Beispiel 1d dargestellten Verbindung in 25 ml wasserfreiem Ethylalkohol versetzte man mit 185 mg Pyridiump-toluolsulfonat und rührte die homogene Lösung 3 Stunden bei 55 °C unter einer Atmosphäre aus trockenem Argon. Man versetzte mit 50 ml Diethylether, filtrierte von ausgefallenem Feststoff, engte das Filtrat in Wasserstrahlvakuum bis zur Trockne ein und reinigte durch Chromatographie an Kieselgel unter Verwendung eines Gradientensystems aus Aceton in n-Hexan. Isoliert wurden 616 mg (1,14 mmol, 77 %) der Titelverbindung als farbloses Öl.

IR (Film): 3100-3600, 3070, 3050, 2940, 2855, 1735, 1590, 1425, 1110, 820, 740 und 705 $cm^{-1}$.

Beispiel 1d

(1S,2R(5Z),3S,4R)-7-[1-Hydroxymethyl-1-methyl-3-tert-butyldiphenylsilyloxymethyl-4-(tetrahydropyran-2-yloxy)-cyclopentan-2-yl]-5-heptensäuremethylester:

Die farblose Lösung von 11,7 ml Hexamethyldisilazan in 40 ml wasserfreiem Tetrahydrofuran kühlte man unter einer Atmosphäre aus trockenem Argon auf 0 °C bis 3 °C, versetzte mit 42 ml einer 1,33 M Lösung von n-Butyllithium in n-Hexan und rührte noch 20 Minuten nach. 40 ml dieser Lösung wurden unter Feuchtigkeitsausschluß zu einer gut gerührten Suspension von 5,52 g Carboxybutyltriphenylphosphoniumbromid in 55 ml wasserfreiem Tetrahydrofuran gegeben und 30 Minuten bei Raumtemperatur gerührt. Sodann tropfte man die Lösung von 744 mg (1,42 mmol) der in Beispiel 1e dargestellten polareren Laktole in 15 ml wasserfreiem Tetrahydrofuran zu und rührte noch 2 Stunden bei 35 °C. Man quenchte durch Eingießen in 200 ml einer eiskalten 10%igen Ammoniumchloridlösung, säuerte mit 10%iger Zitronensäure an und extrahierte mehrfach mit insgesamt 300 ml Diethylether. Die vereinigten organischen Extrakte wusch man mit Wasser und gesättigter Natriumchloridlösung, trocknete über Magnesiumsulfat und isolierte nach Filtration und Abzug des Lösungsmittels im Wasserstrahlvakuum 3,5 g eines farblosen Öls. Man versetzte mit 5 ml Diethylether, filtrierte von ausgefallenem Triphenylphoshinoxid und versetzte das Filtrat mit einer etherischen Lösung von Diazomethan bei 0 °C. Man engte erneut ein und reinigte das Rohprodukt (3,32 g) durch Chromatographie an ca. 300 ml feinem Kieselgel unter Druck mittels eines Gradientensystems aus n-Hexan/Diethylether. Isoliert wurden 848 mg (1,36 mmol, 96 %) der Titelverbindung als farbloses Öl.

IR (Film): 3200-3600, 3070, 3050, 2940, 2855, 1735, 1590, 1425, 1360, 1200, 1110, 870, 820, 740 und 705 $cm^{-1}$.

Beispiel 1e

(1S,4RS,6R,7S,8R)-1-Methyl-4-hydroxy-7-tert-butyl-diphenylsilyloxymethyl-8-(tetrahydropyran-2-yloxy)-3-oxabicyclo[4.3.0]nonan (A) und (1R,3RS,6R,7S,8R)-1-Methyl-3-hydroxy-7-tert-butyl-diphenylsilyloxymethyl-8-(tetrahydropyran-2-yloxy)-4-oxabicyco[4.3.0]nonan (B):

Die farblose Lösung eines ca. 1:1-Gemisches aus 1,71 g (3,26 mmol) der in Beispiel 1f dargestellten regioisomeren Laktone in 45 ml wasserfreiem Methylbenzol kühlte man unter einer Atmosphäre aus trockenem Argon auf - 70 °C, versetzte mit 6 ml einer 1 M Lösung von Diisobutylaluminiumhydrid in Methylbenzol, ließ 45 Minuten reagieren und zersetzte überschüssiges Reduktionsmittel durch Zugabe von 3,3 ml Isopropanol. Man ließ auf 0 °C erwärmen, versetzte mit 3,0 ml Wasser, rührte so lange, bis ein feinkörniger Niederschlag entstanden war, filtrierte, wusch den Niederschlag mit 50 ml Dichlormethan nach, entfernte das Lösungsmittel im Vakuum und isolierte 1,68 g (3,21 mmol, 98 %) eines Gemisches der beiden Titelverbindungen A und B.

Durch wiederholte Chromatographie an 400 ml feinem Kieselgel unter Druck mittels eines Gradientensystems aus Diethylether/n-Hexan trennte man die regioisomeren Laktolgemische auf:

unpolare Verbindung B: 930 mg (1,77 mmol, 54 %), farbloses Öl,

polare Verbindung A: 755 mg (1,44 mmol, 44 %), farbloses Öl.

IR (Film): 3200-3600, 3070, 3045, 2940, 2860, 1590, 1425, 1110, 1020, 865, 820, 740 und 705 $cm^{-1}$.

Beispiel 1f

(1S,6R,7S,8R)-1-Methyl-7-tert-butyl-diphenylsilyloxymethyl-8-(tetrahydropyran-2-yloxy)-3-oxabicyclo[4.3.0]nonan-4-on und (1R,6R,7S,8R)-1-Methyl-7-tert-butyl diphenylsilyloxymethyl-8-(tetrahydropyran-2-yloxy)-4-oxabicyclo[4.3.0]nonan-3-on:

Die Lösung von 1,8 g (3,55 mmol) des in Beispiel 1g dargestellten bicyclischen Ketons in 30 ml wasserfreiem Dichlormethan versetzte man mit 870 mg fein pulverisiertem Natriumhydrogencarbonat, 1,43 g m-Chlorperbenzoesäure und ließ 6 Stunden bei Raumtemperatur unter einer Atomosphäre aus trockenem Argon rühren. Man versetzte mit 100 ml einer 10%igen Natriumthiosulfatlösung, trennte nach 15 Minuten die organische Phase ab, wusch mit 50 ml einer 1 n Natronlauge, sodann mehrfach mit Wasser bis zur Neutralreaktion der Waschlösung und trocknete über Magnesiumsulfat. Isoliert wurden 1,9 g eines farblosen Öles, das man unter Druck an ca. 400 ml feinem Kieselgel mit einem 8:2-Gemisch aus n-Hexan/Ethylacetat chromatographisch reinigte. Man erhielt 1,72 g (3,28 mmol, 93 %) eines 1:1-Gemisches der beiden Titelverbindungen als farbloses Öl.

IR (Film): 3070, 3045, 2940, 2855, 1750, 1590, 1470, 1425, 1215, 1110, 1075, 1060, 1035, 975, 910, 870, 820, 740 und 705 $cm^{-1}$.

Beispiel 1g

(1R,5R,6S,7R)-1-Methyl-6-tert-butyl-diphenylsilyloxymethyl-7-(tetrahydropyran-2-yloxy)-bicyclo[3.3.0] octan-3-on:

Die Lösung von 3,04 g (6,2 mmol) des in Beispiel 1h dargestellten bicyclischen ungesättigten Ketons in 70 ml wasserfreiem Tetrahydrofuran versetzte man mit 103 mg Kupfer-II-acetat, kühlte unter einer Atmosphäre aus trockenem Argon auf - 20 °C und tropfte zügig 8,2 ml einer 3 M Methylmagnesiumbromidlösung in Tetrahydrofuran zu. Man ließ 20 Minuten bei - 20 °C bis - 15 °C reagieren, goß in 300 ml einer eiskalten 10%igen Ammoniumchloridlösung und extrahierte mehrfach mit insgesamt 400 ml Diethylether. Die vereinigten organischen Extrakte wusch man mit Wasser und gesättigter Natriumchloridlösung bis zur Neutralreaktion, trocknete über Magnesiumsulfat und isolierte nach Filtration und Abzug des Lösungsmittels im Wasserstrahlvakuum 3,34 g eines gelben Öles, das an ca. 400 ml feinem Kieselgel unter Druck mittels eines Gradientensystems aus n-Hexan/Ethylacetat chromatographisch gereinigt wurde. Man erhielt 1,81 g (3,6 mmol, 58 %) der Titelverbindung als farbloses Öl.

IR (Film): 3070, 3045, 2970, 2930, 1740, 1590, 1425, 1110, 1075, 1020, 970, 870, 820, 790, 740 und 700 $cm^{-1}$.

Beispiel 1h

(5R,6S,7R)-7-(Tetrahydropyran-2-yloxy)-6-tert-butyl-diphenyl-silyloxymethyl-bicyclo[3.3.0]oct-1-en-3-on:

Die Lösung aus 8,79 g (17,2 mmol) der in Beispiel 1i dargestellten Ketosäure in 200 ml wasserfreiem Ethylacetat versetzte man mit 7,14 g Triphenyl(phenyliminovinyliden)phosphoran und refluxierte 3 h unter einer Atmosphäre aus trockenem Argon. Nach dem Erkalten engte man im Wasserstrahlvakuum auf ein Restvolumen von ca. 30 ml ein, versetzte mit 200 ml wasserfreiem Toluol, 5 ml wasserfreiem Ethanol und refluxierte erneut für 20 Stunden unter Argon. Nach dem Erkalten zog man das Lösungsmittel im Wasserstralvakuum ab und chromatographierte das braune Öl an ca. 600 ml freinem Kieselgel unter Druck mit einem Gradientensystem aus n-Hexan und Ethylacetate. Isoliert wurden 4,81 g (9,8 mmol, 57 %) der Titelverbindungen als blaßgelbes Öl.

IR (Film): 3070, 3045, 2940, 2850, 1775, 1705, 1630, 1470, 1425, 1110, 1075, 1030, 970, 915, 870, 820, 740 und $705^{-1}$.

Beispiel 1i

(2R,3S,4R)-2-Carboxymethyl-3-tert-butyl-diphenylsilyloxymethyl-4-(tetrahydropyran-2-yloxy)-cyclopentan-1-on:

Die Lösung von 8,83 g (17,8 mmol) des in Beispiel 1j dargestellten Lactons in 80 ml Diethylether versetzte man mit 80 ml einer 1 N Natronlauge und rührte das Zweiphasensystem kräftig bei Raumtemperatur. Nach 24 h trennte man die wässrige Phase ab, kühlte auf 0 bis + 3 °C und stellte durch Zugabe einer eiskalten gesättigten Citronensäurelösung auf pH 4-5 ein. Die ausgefallene Hydroxysäure wurde mehrfach mit insgesamt 200 ml - 5 °C kaltem Trichlormethan extrahiert, die vereinigten organischen Extrakte rasch über Magnsiumsulfat filtriert, zweimal mit je 20 ml - 5 °C kaltem Trichlormethan nachgewaschen und mit 150 ml - 20 °C kaltem Aceton versetzt. Man kühlte auf - 30 °C und versetzte mit 9,2 ml einer standardisierten Chromschwefelsäurelösung. Nach vierstündigem Rühren bei - 30 °C bis - 20 °C zersetzte man überschüssiges Oxidationsmittel durch Zugabe von 10 ml Isopropanol, ließ auf Raumtemperatur erwärmen und verdünnte durch Zugabe von 200 ml Wasser. Die organische Phase wurde abgetrennt, die wässrige mehrfach mit insgesamt 350 ml Trichlormethan extrahiert und die vereinigten Extrakte mit Wasser und gesättigter Natriumchloridlösung gewaschen bis die Waschlösung neutral reagierte. Nach Trocknen über Magnsiumsulfat, Filtration und Abzug des Lösungsmittels im Vakuum isolierte man 8,79 g (17,2 mmol, 96 %) der Titelverbindung als blaßgrünes Öl, das ohne Reinigung weiter umgesetzt wurde.

IR (Film): 2500-3600, 3070, 3040, 2940, 2860, 1745, 1710, 1590, 1428, 1110, 1075, 1035, 1020, 970, 870, 820, 758, 740 und 705 $cm^{-1}$.

Beispiel 1j

(1S,5R,6S,7R)-7-(Tetrahydropyran-2-yloxy)-6-tert-butyl-diphenylsilyloxymethyl-2-oxabicyclo[3.3.0]octan-3-on:

Die Lösung von 7,74 g (18,9 mmol) der nach Beispiel 1k dargestellten Verbindung in 110 ml wasserfreiem Dichlormethan versetzte man mit 95 mg p-Toluolsulfonsäure und 3,1 ml Dihydropyran. Man ließ 1 h bei Raumtemperatur unter einer Atmosphäre aus trockenem Argon rühren, quenchte die violette klare Lösung durch Zugabe von 20 ml einer 10%igen Natriumhydrogencarbonatlösung, trennte die organische Phase ab, wusch mehrfach mit Wasser nach und trocknete über Magnesiumsulfat. Nach Filtration und Abzug des Lösungsmittels im Wasserstrahlvakuum isolierte man 12,4 g eines gelben Öles, das an ca. 400 ml feinem Kieselgel unter Verwendung eines Gradientensystems aus n-Hexan/Ethylacetat und unter Druck chromatographisch gereinigt wurde. Man isolierte 8,86 g (17,9 mmol, 95 %) der Titelverbindung als farbloses Öl.

IR (Film): 3065, 3045, 2940, 2850, 1770, 1590, 1425, 1175, 1110, 1075, 1035, 1000, 970, 870, 820, 740 und 705 $cm^{-1}$.

Beispiel 1k

(1S,5R,6S,7R)-7-Hydroxy-6-tert-butyl-diphenylsilyoxymethyl-2-oxabicyclo[3.3.0]octan-3-on:

Die farblose Lösung von 93 g (181 mmol) der nach Beispiel 1l dargestellten Verbindung in 800 ml wasserfreiem Methylalkohol versetzte man mit 32,5 g fein pulverisiertem Kaliumcarbonat und rührte 5,5 Stunden bei Raumtemperatur unter einer Atmosphäre aus trockenem Argon. Nach Filtration und Einengen des Filtrates auf ca. 200 ml im Wasserstrahlvakuum setzte man 600 ml Wasser zu, stellte mit 1 n Salzsäure auf pH 3 bis 4 ein und extrahierte mehrfach mit insgesamt 1 l Dichlormethan. Die vereinigten organischen Extrakte wusch man mit Wasser und gesättigter Natriumchloridlösung bis zur Neutralreaktion, trocknete über Magnesiumsulfat und reinigte das nach Lösungsmittelabzug im Wasserstrahlvakuum erhaltende Rohprodukt durch Chromatographie unter Druck an ca. 1,5 l feinem Kieselgel mittels eines Gradientensystems aus n-Hexan/Ethylacetat. Isoliert wurden 59 g (144 mmol, 80 %) der Titelverbindung als kristalliner Feststoff.

IR (KBr): 3440, 3070, 3040, 2955, 2930, 2855, 1740, 1590, 1470, 1425, 1390, 1370, 1200, 1110, 1045, 1030, 995, 900, 865, 820, 795, 740, 705 und 615 $cm^{-1}$.

$$\alpha_D^{23} = -6{,}2\ ° \ (c=1,\ CHCl_3)$$

Smp.: 90 - 91 °C

Beispiel 1l

(1S,5R,6S,7R)-7-Benzoyloxy-6-tert-butyl-diphenylsilyloxymethyl-2-oxabicyclo[3.3.0]octan-3-on:

Die farblose Lösung von 50 g (181 mmol) Corey-Lakton in 850 ml wasserfreiem Dimethylformamid versetzte man mit 25 g Imidazol und tropfte anschließend innerhalb 1 Stunde 47,5 ml tert-Butyl-diphenylchlorsilan zu, spülte mit wasserfreiem Dimethylformamid nach und ließ 2,5 Stunden bei Raumtemperatur unter einer Atmosphäre aus trockenem Argon reagieren. Das nach Eingießen in 1 l Eiswasser gummiartig ausfallende Rohprodukt wurde mehrfach mit insgesamt 1 l Diethylether extrahiert, die vereinigten organischen Extrakte mit

Wasser und gesättigter Natriumchloridlösung bis zur Neutralreaktion der Waschlösung gewaschen und über Magnesiumsulfat getrocknet. Nach Filtration und Abzug des Lösungsmittels im Wasserstrahlvakuum isolierte man nach intensiver Trocknung 93 g (181 mmol, 100 %) der Titelverbindung als farbloses, bei - 20 °C erstarrendes Öl, das ohne Reinigung weiter umgesetzt wurde.

IR (Film): 3070, 3045, 2955, 2930, 2855, 1770, 1715, 1600, 1585, 1425, 1270, 1175, 1110, 820, 740 und 705 $cm^{-1}$.

$$\alpha_D^{23} = -52\,° \; (c=1{,}0, \; CHCl_3)$$

Beispiel 2

(4S,5R(5Z),6S,1R)-7-[4-Methyl-6-benzyloxymethyl-2-oxabicyclo[2.2.1]heptan-5-yl]-5-heptensäure:

18 mg des nach Beispiel 1 dargestellten Estergemisches löste man in 0,5 ml Methanol, versetzte mit 0,5 ml einer 5%igen wässrigen Lithiumhydroxydlösung und rührte 4 bis 6 Stunden bei Raumtemperatur. Man säuerte mit einer 1 n Salzsäure an, extrahierte mehrfach mit insgesamt 30 ml Chloroform, filtrierte über Magnesiumsulfat und reinigte durch Chromatographie an einer analytischen Dünnschichtplatte. Als Laufmittel diente ein Gemisch aus n-Hexan/Ethylacetat, als Elutionsmittel ein Gemisch aus Chloroform/i-Propanol. Isoliert wurden 15 mg (41,8 µmol, 74 %, bezogen auf Startmaterial in Beispiel 1) der Titelverbindung als farbloses Öl.

IR (Film): 3600-2400, 3080, 3060, 3030, 3000, 2950, 2930, 2870, 1730, 1710, 1455, 1125, 1100, 1020, 965, 885, 875, 740 und 700 $cm^{-1}$.

Beispiel 3

(4S,5R(5Z),6S,1R)-7-[4-Phenyl-6-benzyloxymethyl-2-oxabicyclo[2.2.1]heptan-5-yl]-5-heptensäuremethyl (bzw. Benzyl) ester:

16 mg (46,5 µmol) des nach Beispiel 3a dargestellten Alkohols setzte man in Analogie zu Beispiel 1 um und isolierte nach Aufarbeitung und Reinigung 19 mg der Titelverbindungen als farbloses Öl.

IR (Film): 3090, 3060, 3030, 3000, 2930, 2870, 1735, 1605, 1500, 1455, 1075, 970, 895, 740 und 700 $cm^{-1}$.

Beispiel 3a

(4S,5R(5Z),6S,1R)-7-[4-Phenyl-6-hydroxymethyl-2-oxabicyclo[2.2.1]heptan-5-yl]-5-heptensäuremethylester:

460 mg (789 µmol) des nach Beispiel 3b dargestellten Bicyclus setzte man in Analogie zu Beispiel 1a um und isolierte nach Aufarbeitung und Reinigung 254 mg (737 µmol, 93 %) der Titelverbindung als farbloses Öl.

IR (Film): 3600-3400, 3080, 3060, 3030, 2940, 2870, 1735, 1600, 1495, 1445, 1435, 1200, 1150, 1070, 1035, 1000, 895, 760 und 700 $cm^{-1}$.

Beispiel 3b

(4S,5R(5Z),6S,1R)-7-[4-Phenyl-6-tert-butyldiphenylsilyloxymethyl-2-oxabicyclo[2.2.1]heptan-5-yl]-5-heptensäuremethylester:

570 mg (949 µmol) des nach Beispiel 3c dargestellten Diols cyclisierte man in Analogie zu Beispiel 1b und isolierte nach Aufarbeitung und Reinigung 452 mg (776 µmol, 82 %) der Titelverbindung als farbloses Öl.

IR (Film): 3070, 3050, 3020, 2950, 2930, 2855, 1735, 1600, 1590, 1425, 1110, 895, 820, 760, 740 und 700 $cm^{-1}$.

Beispiel 3c

(1S,2R(5Z),3S,4R)-7-[1-Hydroxymethyl-1-phenyl-3-tert-butyldiphenylsilyloxymethyl-4-hydroxy-cyclopentan-2-yl]-5-heptensäuremethylester:

436 mg (662 µmol) der in Beispiel 3d dargestellten Verbindung setzte man in Analogie zu Beispiel 1c um und isolierte nach Aufarbeitung und Reinigung 359 mg (598 µmol, 90 %) der Titelverbindung als farbloses Öl.

IR (Film): 3100-3600, 3070, 3050, 3000, 2950, 2930, 2855, 1735, 1600, 1590, 1425, 1110, 1055, 820, 740 und 700 $cm^{-1}$.

Beispiel 3d

(1S,2R(5Z),3S,4R)-7-[1-Hydroxymethyl-1-phenyl-3-tert-butyldiphenylsilyloxymethyl-4-(methoxyethyl-1-oxy)-cyclopentan-2-yl]-5-heptensäuremethylester:

716 mg (1,28 mmol) der in Beispiel 3e dargestellten unpolareren Laktole wurden in Analogie zu Beispiel 1d umgesetzt. Nach Veresterung und Reinigung isolierte man 705 mg (1,07 mmol, 84 %) der Titelverbindung als farbloses Öl.

IR (Film): 3200-3600, 3070, 3050, 2950, 2930, 2860, 1740, 1600, 1590, 1430, 1110, 820, 740, 700 und 505 $cm^{-1}$.

Beispiel 3e

(1S,4RS,6R,7S,8R)-1-Phenyl-4-hydroxy-7-tert-butyl-diphenylsilyloxymethyl-8-(methoxyethyl-1-oxy)-3-oxabicyclo[4.3.0]nonan (A) und (1S,3RS,6R,7S,8R)-1-Phenyl-3-hydroxy-7-tert.-butyl-diphenylsilyloxymethyl-8-(methoxyethyl-1-oxy)-4-oxabicyclo[4.3.0]nonan (B):

In Analogie zu Beispiel 1e setzte man 1,43 g (2,56 mmol) eines ca. 1:1-Gemisches der in Beispiel 3f dargestellten regioisomeren Laktone um. Nach Aufarbeitung isolierte man 1,42 g (2.53 mmol, 99 %) eines Gemisches der beiden Titelverbindungen A und B.

IR (Film): 3200-3600, 3070, 3050, 3025, 2930, 2860, 1605, 1590, 1495, 1470, 1425, 1390, 1110, 1040, 990, 900, 825, 735 und 700 $cm^{-1}$.

Chromatographische Trennung ergab 716 mg (1,28 mmol, 50 %) der Verbindung A als unpolarere Komponente sowie 692 mg (1,23 mmol, 48 %) der polareren Verbindung B.

Beispiel 3f

(1S,6R,7S,8R)-1-Phenyl-7-tert-butyl-diphenylsilyloxymethyl-8-(methoxyethyl-1-oxy)-3-oxabicyclo[4.3.0]nonan-4-on und (1S,6R,7S,8R)-1-Phenyl-7-tert-butyldiphenylsilyloxymethyl-8- (methoxyethyl-1-oxy)-4-oxabicyclo[4.3.0]nonan-3-on:

1.59 (2.93 mmol) des nach Beispiel 3g dargestellten bicyclischen Ketons oxidierte man in Analogie zu Beispiel 1f und isolierte nach Aufarbeitung und Reinigung 1,43 g (2,56 mmo, 87 %) eines ca. 1:1-Gemisches der beiden Titelverbindungen als kristallinen Feststoff.

Smp.: 113 - 114 °C (n-Hexan/Diisopropylether)

Beispiel 3g

(1S,5R,6S,7R)-1-Phenyl-6-tert-butyl-diphenylsilyloxymethyl-7-(methoxyethyl-1-oxy)-bicyclo[3.3.0]octan-3-on:

1,19 g (2,56 mmol) (5R,6S,7R)-7-(Methoxyethyl-1-oxy)-6-tert-butyldiphenylsilyloxymethyl-bicyclo[3.3.0]oct-1-en-3-on, das man analog den Beispielen 1h, 1i und 1j unter Verwendung von Methylvinylether aus (1S,5R,6S,7R)-7-Hydroxy-6-tert-butyldiphenylsilyloxymethyl-2-oxabicyclo[3.3.0]octan-3-on hergestellt hatte, setzte man in Analogie zu Beispiel 1g mit Phenylmagnesiumjodid unter Kupferkatalyse um. Nach Aufarbeitung und Reinigung isolierte man 995 mg (1,83 mmol, 72 %) der Titelverbindung als farbloses Öl.

IR (Film): 3070, 3050, 2940, 2855, 1740, 1600, 1590, 1470, 1430, 1390, 1110, 995, 900, 825, 740 und 705 $cm^{-1}$.

Beispiel 4

(4S,5R(5Z),6S,1R)-7-[4-Phenyl-6-benzyloxymethyl-2-oxabicyclo[2.2.1]heptan-5-yl]-5-heptensäure:

19 mg des nach Beispiel 3 dargestellten Estergemisches verseifte man in Analogie zu Beispiel 2 und isolierte nach Aufarbeitung und Reinigung 16 mg (38 µmol, 82 %, bezogen auf Startmaterial in Beispiel 3) der Titelverbindung als farbloses Öl.

IR (Film): 3600-2500, 3090, 3060, 3030, 3000, 2930, 2870, 1730, 1710, 1605, 1500, 1455, 1210, 1120, 1075, 970, 900, 760, 740 und 700 $cm^{-1}$.

Beispiel 5

(4R,5S(5Z),6R,1S)-7-[4-Phenyl-6-benzyloxymethyl-2-oxabicyclo[2.2.1]heptan-5-yl]-5-heptensäuremethyl (bzw. Benzyl) ester:

19 mg (55 µmol) des nach Beispiel 5a dargestellten Alkohols setzte man in Analogie zu Beispiel 1 um und isolierte nach Aufarbeitung und Reinigung 22 mg der Titelverbindungen als farbloses Öl.

IR (Film): 3090, 3060, 3030, 2940, 2870, 1735, 1605, 1500, 1455, 1075, 970, 895, 740 und 700 $cm^{-1}$.

Beispiel 5a

(4R,5S(5Z),6R,1S)-7-[4-Phenyl-6-hydroxymethyl-2-oxabicyclo[2.2.1]heptan-5-yl]-5-heptensäuremethylester:

2,81 g (10,2 mmol) enantiomeres Corey-Lakton setzte man in Analogie zu den Beispielen 1a bis 1l um und isolierte 228 mg (662 µmol, 6,5 %) der Titelverbindung als farbloses Öl.

IR (Film): 3600-3200, 3040, 3030, 3000, 2940, 2870, 1735, 1600, 1495, 1440, 1435, 1220, 1200, 1035, 1000, 895, 760 und 700 $cm^{-1}$.

Beispiel 6

(4R,5S(5Z),6R,1S)-7-[4-Phenyl-6-benzyloxymethyl-2-oxabicyclo[2.2.1]heptan-5-yl]-5-heptensäure:

22 mg des nach Beispiel 5 dargestellten Estergemisches verseifte man in Analogie zu Beispiel 2 und isolierte nach Aufarbeitung und Reinigung 20 mg (48 µmol, 86 %, bezogen auf Startmaterial in Beispiel 5) der Titelverbindung als farbloses Öl.

IR (Film): 3600-2300, 3080, 3060, 3030, 3000, 2930, 2870, 1730, 1705, 1600, 1500, 1450, 1240, 1210, 1115, 1095, 1070, 1040, 895, 760, 735 und 700 $cm^{-1}$.

Beispiel 7

(4S,5R(5Z),6S,1R)-7-[4-Benzyl-6-benzoyloxymethyl-2-oxabicyclo[2.2.1]heptan-5-yl]-5-heptensäuremethyl (bzw. Benzyl) ester:

20 mg (55,8 µmol) des nach Beispiel 7a dargestellten Alkohols veretherte man in Analogie zu Beispiel 1 und isolierte nach Aufarbeitung und Reinigung 21 mg der Titelverbindungen als farbloses Öl.

IR (Film): 3090, 3060, 3030, 3000, 2930, 2870, 1735, 1605, 1500, 1455, 1120, 1100, 1075, 1005, 895, 760, 740 und 700 $cm^{-1}$.

Beispiel 7a

(4S,5R(5Z),6S,1R)-7-[4-Benzyl-6-hydroxymethyl-2-oxabicyclo[2.2.1]heptan-5-yl]-5-heptensäuremethylester:

364 mg (611 µmol) des nach Beispiel 7b dargestellten Bicyclus setzte man in Analogie zu Beispiel 1a um und isolierte nach Aufarbeitung und Reinigung 210 mg (586 µmol, 96 %) der Titelverbindung als farbloses Öl.

IR (Film): 3600-3200, 3060, 3020, 2940, 2920, 2870, 1735, 1600, 1450, 1435, 1245, 1200, 1150, 1030, 1000, 890, 765 und 705 $cm^{-1}$.

Beispiel 7b

(4S,5R(5Z),6S,1R)-7-[4-Benzyl-6-tert-butyldiphenylsilyloxymethyl-2-oxabicyclo[2.2.1]heptan-5-yl]-5-heptensäuremethylester:

554 mg (901 µmol) des in Beispiel 7c dargestellten Diols cyclisierte man in Analogie zu Beispiel 1b und isolierte nach Aufarbeitung und Reinigung 364 mg (610 µmol, 68 %) der Titelverbindung als farbloses Öl.

IR (Film): 3070, 3030, 2950, 2930, 2860, 1735, 1600, 1590, 1425, 1110, 1070, 1005, 895, 820, 740 und 700 $cm^{-1}$.

Beispiel 7c

(1S,2R(5Z),3S,4R)-7-[1-Hydroxymethyl-1-benzyl-3-tert-butyldiphenylsilyloxymethyl-4-hydroxy-cyclopentan-2-yl]-5-heptensäuremethylester:

766 mg (1,10 mmol) der in Beispiel 7d dargestellten Verbindung setzte man in Analogie zu Beispiel 1 c um und isolierte nach Aufarbeitung und Reinigung 554 mg (901 µmol, 82 %) der Titelverbindung als farbloses Öl.

IR (Film): 3200-3600, 3070, 3050, 3020, 3000, 2950, 2925, 2855, 1735, 1600, 1590, 1425, 1110, 820, 740

und 700 $cm^{-1}$.

Beispiel 7d

(1S,2R(5Z),3S,4R)-7-[1-Hydroxymethyl-1-benzyl-3-tert-butyldiphenylsilyloxymethyl-4-(tertrahydropyran-2-yloxy)-cyclopentan-2-yl]-5-heptensäuremethylester:

742 mg (1,23 mmol) der in Beispiel 7e dargestellten unpolareren Laktole setzte man in Analogie zu Beispiel 1d um Isoliert wurden nach Veresterung und Reinigung 766 mg (1,10 mmol, 89 %) der Titelverbindung als farbloses Öl.

IR (Film): 3200-3600, 3070, 3050, 3020, 3000, 2940, 2855, 1735, 1600, 1590, 1425, 1265, 1200, 1100, 1030, 870, 820, 740 und 705 $cm^{-1}$.

Beispiel 7e

(1S,4RS,6R,7S,8R)-1-Benzyl-4-hydroxy-7-tert-butyl-diphenylsilyloxymethyl-8-(tetrahydropyran-2-yloxy)-3-oxabicyclo[4.3.0]nonan (A) und (1S,3RS,6R,7S,8R)-1-Benzyl-3-hydroxy-7-tert-butyl-diphenylsilyloxymethyl-8-(tertrahydropyran-2-yloxy)-4-oxabicylo[4.3.0]nonan (B):

1,86 g (3,11 mmol) eines ca. 1:1-Gemisches der in Beispiel 7f dargestellten regioisomeren Laktone reduzierte man in Analogie zu Beispiel 1e und isolierte nach Aufarbeitung 1,85 g (3,08 mmol, 99 %) eines Gemisches der Titelverbindungen A und B.

IR (Film): 3200-3600, 3070, 3045, 3020, 2940, 2855, 1600, 1590, 1425, 1110, 1040, 870, 820, 740 und 705 $cm^{-1}$.

Chromatographische Trennung lieferte 764 mg (1,27 mmol, 41 %) Verbindung A als unpolarere Komponente neben 897 mg (1,49 mmol, 48 %) der Verbindung B.

Beispiel 7f

(1S,6R,7S,8R)-1-Benzyl-7-tert-butyl-diphenylsilyloxymethyl-8-(tetrahydropyran-2-yloxy)-3-oxabicyclo[4.3.0]nonan-4-on und (1R,6R,7S,8R)-1-Benzyl-7-tert-butyl-diphenylsilyloxymethyl-8-(tertrahydropyran-2-yloxy)-4-oxabicyclo[4.3.0]nonan-3-on:

3,86 g (6,62 mmol) des nach Beispiel 7g dargestellten bicyclischen Ketons wurden in Analogie zu Beispiel 1f oxidiert. Nach Aufarbeitung und Reinigung isolierte man 3,04 g (5,08 mmol, 77 %) eines ca. 1:1-Gemisches der beiden Titelverbindungen als farbloses Öl.

IR (Film): 3070, 3045, 3010, 2940, 2855, 1745, 1600, 1590, 1425, 1110, 1035, 970, 870, 820, 740 und 705 $cm^{-1}$.

Beispiel 7g

(1R,5R,6S,7R)-1-Benzyl-6-tert-butyl-diphenylsilyoxymethyl-7-(tetrahydropyran-2-yloxy)-bicyclo[3.3.0]octan-3-on:

2,01 g (4,18 mmol) des nach Beispiel 1h dargestellten bicyclischen ungesättigten Ketons wurden in Analogie zu Beispiel 1g mit Benzylmagnesiumchlorid unter Kupferkatalyse umgesetzt. Nach Aufarbeitung und Reinigung isolierte man 1,90 g (3,23 mmol, 83 %) der Titelverbindung als farbloses Öl.

IR (Film): 3070, 3050, 3025, 2930, 2855, 1740, 1600, 1590, 1425, 1265, 1110, 1075, 1020, 870, 820, 740 und 705 $cm^{-1}$.

Beispiel 8

(4S,5R(5Z),6S,1R)-7-[4-Benzyl-6-benzoyloxymethyl-2-oxabicyclo[2.2.1]heptan-5-yl]-5-heptensäure:

21 mg des nach Beispiel 7 dargestellten Estergemisches verseifte man in Analogie zu Beispiel 2 und isolierte nach Aufarbeitung und Reinigung 10,7 mg (24,6 µmol, 44 %, bezogen auf Startmaterial in Beispiel 7) der Titelverbindung als farbloses Öl.

IR (Film): 3600-2400, 3090, 3060, 3030, 3000, 2930, 2860, 1730, 1710, 1600, 1495, 1455, 1115, 1100, 1075, 1010, 895, 765, 740 und 705 $cm^{-1}$.

Beispiel 9

(4S,5R(5Z),6S,1R)-7-[4-(p-Phenyl-benzyl)-6-benzoyloxymethyl-2-oxabicyclo[2.2.1]heptan-5-yl]-5-heptensäuremethyl (bzw. Benzyl) ester:

29 mg (66,7 μmol) des nach Beispiel 9a dargestellten Alkohols veretherte man in Analogie zu Beispiel 1 und isolierte nach Aufarbeitung und Reinigung 24 mg der Titelverbindungen als farbloses Öl.

IR (Film): 3060, 3030, 3000, 2930, 2870, 1735, 1600, 1490, 1455, 1115, 1075, 1005, 895, 850, 770, 740 und 700 $cm^{-1}$.

Beispiel 9a

(4S,5R(5Z),6S,1R)-7-[4-(p-Phenyl-benzyl)-6-hydroxymethyl-2-oxabicyclo[2.2.1]heptan-5-yl]-5-heptensäuremethylester:

210 mg (312 μmol) des nach Beispiel 9b dargestellten Bicyclus setzte man in Analogie zu Beispiel 1a um und isolierte nach Aufarbeitung und Reinigung 126 mg (290 μmol, 93 %) der Titelverbindung als farbloses Öl.

IR (KBr): 3200-3600, 3020, 2920, 2880, 2850, 1715, 1490, 1440, 1200, 1005, 890, 770, 740 und 695 $cm^{-1}$.

Smp.: 61,5 - 63 °C

Beispiel 9b

(4S,5R(5Z),6S,1R)-7-[4-(p-Phenyl-benzyl)-6-tert-butyldiphenylsilyloxymethyl-2-oxabicyclo[2.2.1]heptan-5-yl]-5-heptensäuremethylester:

296 mg (428 μmol) des in Beispiel 9c dargestellten Diols cyclisierte man in Analogie zu Beispiel 1b und isolierte nach Aufarbeitung und Reinigung 220 mg (327 μmol, 76 %) der Titelverbindung als farbloses Öl.

IR (Film): 3070, 3050, 3020, 3000, 2950, 2920, 2855, 1735, 1600, 1590, 1485, 1425, 1110, 1070, 1005, 900, 845, 820, 765, 740 und 700 $cm^{-1}$.

Beispiel 9c

(1S,2R(5Z),3S,4R)-7-[1-Hydroxymethyl-1-(p-phenyl-benzyl)-3-tert-butyldiphenylsilyloxymethyl-4-hydroxy-cyclopentan-2-yl]-5-heptensäuremethylester:

469 mg (605 μmol) der in Beispiel 9d dargestellten Verbindung setzte man in Analogie zu Beispiel 1c um und isolierte nach Aufarbeitung und Reinigung 308 mg (446 μmol, 74 %) der Titelverbindung als farbloses Öl.

IR (Film): 3200-3600, 3070, 3050, 3030, 3000, 2950, 2930, 2860, 1735, 1600, 1590, 1490, 1430, 1115, 825, 770, 740, 705 und 505 $cm^{-1}$.

Beispiel 9d

(1S,2R(5Z),3S,4R)-7-[1-Hydroxymethyl-1-(p-phenyl-benzyl)-3-tert-butyldiphenylsilyloxymethyl-4-(tetrahydropyran-2-yloxy)-cyclopentan-2-yl]-5-heptensäuremethylester (A) und (1R(5Z),2R,3S,4R)-7-[1-(p-Phenyl-benzyl)-2-hydroxymethyl-3-tert-butyldiphenylsilyloxymethyl-4-(tetrahydropyran-2-yloxy)-cyclopentan-1-yl]-5-heptensäuremethylester (B):

0,77 g (1,14 mmol) eines ca. 1:1-Gemisches der in Beispiel 9a dargestellten regioisomeren Laktole setzte man in Analogie zu Beispiel 1d um. Nach Veresterung, Reinigung und chromatographischer Trennung isolierte man 355 mg (458 μmol, 40 %) der Titelverbindung 8 als unpolarere Komponente sowie 509 mg (657 μmol, 58 %) der Titelverbindung A als polarere Komponente, jeweils als farbloses Öl.

IR (Film) von A: 3200-3600, 3070, 3050, 3020, 3000, 2950, 2855, 1735, 1600, 1590, 1430, 1110, 1025, 860, 820, 765, 740 und 700 $cm^{-1}$.

Beispiel 9e

(1S,4RS,6R,7S,8R)-1-(p-Phenyl-benzyl)-4-hydroxy-7-tert-butyl-diphenylsilyloxymethyl-8-(tetrahydropyran-2-yloxy)-3-oxabicyclo[4.3.0]nonan (A) und [1S,3RS,6R, 7S,8R)-1-(p-Phenyl-benzyl)-3-hydroxy-7-tert-butyl-diphenylsilyloxymethyl-8 (tetrahydropyran-2-yloxy)-4-oxabicyclo[4.3.0]nonan (B):

1,62 g (2,40 mmol) eines ca. 1:1-Gemisches der in Beispiel 9f dargestellten regioisomeren Laktone wurden in Analogie zu Beispiel 1e reduziert. Nach Aufarbeitung erhielt man 1,37 g (2,03 mmol, 84 %) eines Gemisches der Titelverbindungen, das ohne Trennung weiter ungesetzt wurde.

IR (Film): 3200-3600, 3070, 3050, 3020, 2940, 2855, 1600, 1590, 1485, 1425, 1265, 1200, 1110, 1075, 1020, 870, 820, 765, 740 und 700 $cm^{-1}$.

Beispiel 9f

(1S,6R,7S,8R)-1-(p-Phenyl-benzyl)-7-tert-butyl-diphenylsilyloxymethyl-8-(tetrahydropyran-2-yloxy)-3-oxabicyclo[4.3.0]nonan-4-on und (1R,6R,7S,8R)-1-(pPhenyl-benzyl)-7-tert-butyl-diphenylsilyloxymethyl-8-(tetrahydropyran-2-yloxy)4-oxabicyclo[4.3.0]nonan-3-on:

2,20 g (3,34 mmol) des in Beispiel 9g dargestellten bicyclischen Ketons wurden in Analogie zu Beispiel 1f oxidiert. Nach Aufarbeitung und Reinigung isolierte man 1,69 g (2,51 mmol, 75 %) eines ca. 1:1-Gemisches der beiden Titelverbindungen als farbloses Öl.

IR (Film): 3070, 3050, 3020, 2940, 2850, 1745, 1600, 1590, 1485, 1425, 1260, 1110, 1075, 1030, 870, 820, 735 und 700 $cm^{-1}$.

Beispiel 9g

(1R,5R,6S,7R)-1-(p-Phenyl-benzyl)-6-tert-butyldiphenylsilyloxymethyl-7-(tetrahydropyran-2-yloxy)-bicyclo [3.3.0]octan-3-on:

1,91 g (3,88 mmol) des nach Beispiel 1h dargestellten bicyclischen ungesättigten Ketons wurden in Analogie zu Beispiel 1g mit p-Phenylbenzylmagnesiumchlorid unter Kupferkatalyse umgesetzt. Nach Aufarbeitung und Reinigung isolierte man 2,29 g (3,48 mmol, 90 %) der Titelverbindung als farbloses Öl.

IR (Film): 3070, 3050, 3000, 2940, 2855, 1730, 1600, 1590, 1485, 1425, 1110, 1075, 1020, 865, 820, 740 und 700 $cm^{-1}$.

Beispiel 10

(4S,5R(5Z),6S,1R)-7-[4-(p-Phenyl-benzyl)-6-benzoyloxymethyl-2-oxabicyclo[2.2.1]-heptan-5-yl]-5-heptensäure:

24 mg des nach Beispiel 9 dargestellten Estergemisches verseifte man in Analogie zu Beispiel 2 und isolierte nach Aufarbeitung und Reinigung 16,2 mg (31,7 μmol), 48 %, bezogen auf Startmaterial in Beispiel 9) der Titelverbindung als farbloses Öl.

IR (KBr): 3600-2400, 3060, 3030, 3000, 2920, 2860, 1730, 1705, 1600, 1485, 1450, 1110, 1075, 1005, 895, 845, 770, 740 und 695 $cm^{-1}$.

Beispiel 11

(4S,5R(5Z),6S,1R)-7-[4-(1-Pentinyl)-6-benzyloxymethyl-2-oxabicyclo[2.2.1]heptan-5-yl]-5-heptensäuremethyl (bzw. Benzyl) ester:

12,3 mg (36,8 μmol) des nach Beispiel 11a dargestellten Alkohols veretherte man in Analogie zu Beispiel 1 und isolierte nach Aufarbeitung und Reinigung 16,4 mg der Titelverbindungen als farbloses Öl.

IR (Film): 3060, 3015, 2960, 2930, 2870, 1735, 1450, 1155, 1010, 895, 735 und 700 $cm^{-1}$.

Beispiel 11a

(4S,5R(5Z),6S,1R)-7-[4-(1-Pentinyl)-6-hydroxymethyl-2-oxabicyclo[2.2.1]heptan-5-yl]-5-heptensäuremethylester:

22 mg (38 μmol) des nach Beispiel 11b dargestellten Bicyclus setzte man in Analogie zu Beispiel 1a um und isolierte nach Aufarbeitung und Reinigung 12 mg (37 μmol, 96 %) der Titelverbindung als farbloses Öl.

IR (Film): 3200-3600, 2955, 2930, 2870, 1735, 1450, 1435, 1220, 1190, 1155, 1030, 1000, 950 und 890 $cm^{-1}$.

Beispiel 11b

(4S,5R(5Z),6S,1R)-7-[4-(1-Pentinyl)-6-tert-butyldiphenylsilyloxymethyl-2-oxabicyclo[2.2.1]heptan-5-yl]-5-heptensäuremethylester:

46 mg (78 μmol) des in Beispiel 11c wurden dargestellten Diols wurden in Analogie zu Beispiel 1b cyclisiert. Nach Aufarbeitung und Reinigung isolierte man 22 mg (38 μmol, 49 %) der Titelverbindung als farbloses Öl.

IR (Film): 3070, 3050, 2960, 2930, 1735, 1590, 1425, 1245, 1110, 1005, 890, 820, 740 und 705 $cm^{-1}$.

Beispiel 11c

(1S,2R(5Z),3S,4R)-7-[1-Hydroxymethyl-1-(1-pentinyl)-3-tert-butyldiphenylsilyloxymethyl-4-hydroxy-cyclopentan-2-yl]-5-heptensäuremethylester:

75 mg (111 μmol) der in Beispiel 11d dargestellten Verbindung setzte man in Analogie zu Beispiel 1c um und isolierte nach Aufarbeitung und Reinigung 46 mg (78 μmol, 70 %) der Titelverbindung als farbloses Öl.

IR (Film): 3100-3600, 3070, 3050, 3000, 2950, 2930, 2855, 1735, 1590, 1425, 1110, 820, 740 und 705 $cm^{-1}$.

Beispiel 11d

(1S,2R(5Z),3S,4R)-7-[1-Hydroxymethyl-1-(1-pentyl)-3-tert-butyl-diphenylsilyloxymethyl-4-(tetrahydropyran-2-yloxy)-cyclopentan-2-yl]-5-heptensäuremethylester:

78 mg (135 μmol) der in Beispiel 11e dargestellten polareren Laktole setzte man in Analogie zu Beispiel 1d um und isolierte nach Veresterung und Reinigung 75 mg (111 μmol, 72 %) der Titelverbindung als farbloses Öl.

IR (Film): 3200-3600, 3070, 3050, 2950, 2930, 2860, 1735, 1590, 1430, 1200, 1110, 1030, 870, 820, 740 und 705 $cm^{-1}$.

Beispiel 11e

(1S,4RS,6R,7S,8R)-1-(1-Pentinyl)-4-hydroxy-7-tert-butyl-diphenylsilyloxymethyl-8-(tetrahydropyran-2-yloxy)-3-oxa-bicyclo[4.3.0]nonan (A) und (1S,3RS,6R,7S, 8R)-1-(1-Pentinyl)-3-hydroxy-7-tert-butyl-diphenylsilyloxymethyl-8-(tetrahydropyran-2-yloxy)-4-oxabicyclo[4.3.0]nonan (B):

330 mg (574 μmol) eines ca. 1:1-Gemisches der in Beispiel 11f dargestellten regioisomeren Laktone reduzierte man in Analogie zu Beispiel 1e. Isoliert wurden 325 mg (563 μmol. 98 %) eines Gemisches der beiden Titelverbindungen A und B.

IR (Film): 3200-3600, 3070, 3050, 2940, 2860, 1590, 1425, 1265, 1200, 1110, 1075, 1020, 870, 820, 740 und 705 $cm^{-1}$.

Nach chromatographischer Trennung erhielt man 125 mg (218 μmol, 38 %) der Verbindung B neben 80 mg (139 μmol, 24 %) der polareren Komponente A.

Beispiel 11f

(1S,6R,7S,8R)-1-(1-Pentinyl)-7-tert-butyl-diphenylsilyloxymethyl-8-(tetrahydropyran-2-yloxy)-3-oxabicyclo[4.3.0]nonan-4-on und (1S,6R,7S,8R)-1-(1-Pentinyl)-7-tert-butyl-diphenylsilyloxymethyl-8-(tetrahydropyran-2-yloxy)-4-oxabicyclo[4.3.0]nonan-3-on:

770 mg (1,38 mmol) des in Beispiel 11g dargestellten bicyclischen Ketons oxidierte man in Analogie zu Beispiel 1f und isolierte nach Aufarbeitung und Reinigung 570 mg (992 μmol, 72 %) eines ca. 1:1 Gemisches der beiden Titelverbindungen als farbloses Öl.

IR (Film): 3070, 3050, 2940, 2860, 1750, 1590, 1425, 1260, 1110, 1075, 1035, 970, 870, 820, 740 und 705 $cm^{-1}$.

Beispiel 11g

(1S,5R,6S,7R)-1-(1-Pentinyl)-6-tert-butyl-diphenylsilyloxymethyl-7-(tetrahydropyran-2-yloxy)-bicyclo[3.3.0]octan-3-on:

Die unter einer Atmosphäre aus trockenem Argon auf - 40 °C gekühlte Lösung von 587 mg 1-Pentin in 10 ml wasserfreiem Diethylether versetzte man mit 7,2 ml einer 1,18 M Lösung von n-Butyllithium in n-Hexan, ließ 30 Minuten bei - 10 °C rühren, kühlte erneut auf - 40 °C, versetzte mit 8,5 ml einer 1 M Lösung von Diethylaluminiumchlorid in Toluol und rührte weitere 40 Minuten bei 0 °C bis 3 °C. Die gebildete Emulsion gab man unter Feuchtigkeitsausschluß zu einem Gemisch aus 104 mg Nickelacetylacetonat, das man in 20 ml wasserfreiem Diethylether aufgeschlämmt und mit 400 μl einer 1 M Lösung aus Diisobutylaluminiumhydrid in Toluol versetzt hatte, kühlte auf - 10 °C und tropfte zügig die Lösung von 1,85 g (3,77 mmol) des nach Beispiel 1h dargestellten bicyclischen ungesättigten Ketons in 25 ml wasserfreiem Diethylehter zu. Man ließ noch 1,5 h bei - 15 °C bis - 5 °C reagieren, quenchte durch Eingießen in ein gut gerührtes Gemisch aus ca. 200 ml fein gestoßenem Eis und 50 ml einer 1 n HC1, extrahierte mehrfach mit insgesamt 250 ml Diethylether, wusch die vereinigten organischen Extrakte mit Wasser und gesättigter Natriumchloridlösung bis zur Neutralreaktion,

trocknete über Magnesiumsulfat und entfernte das Lösungsmittel im Wasserstralvakuum. Das braune Rohöl (2,41 g) chromatographierte man unter Druck an ca. 400 ml Kieselgel unter Verwendung eines n-Hexan/Ethylacetat-Gradientensystems. Isoliert wurden 890 mg (1,59 mmol, 42 %) der Titelverbindung als farbloses Öl.

IR (Film): 3070, 3045, 2970, 2930, 1740, 1590, 1428, 1155, 1110, 1075, 1020, 870, 820, 740 und 705 $cm^{-1}$.

Beispiel 12

(4S,5R(5Z),6S,1R)-7-[4-(1-Pentinyl)-6-benzyloxymethyl-2-oxabicyclo[2.2.1]heptan-5-yl]-5-heptensäure:

16,4 mg des nach Beispiel 11 dargestellten Estergemisches verseifte man in Analogie zu Beispiel 2 und isolierte nach Aufarbeitung und Reinigung 10,9 mg (26,5 μmol, 72 %, bezogen auf Startmaterial in Beispiel 11) der Titelverbindung als farbloses Öl.

IR (Film): 3600-2400, 3090, 3060, 3030, 3000, 2930, 2870, 1730, 1710, 1605, 1455, 1115, 1090, 1075, 1010, 895, 735 und 700 $cm^{-1}$.

Beispiel 13

(4S,5R(5Z),6S,1R)-7-[4-Phenylacetylen-6-benzoyloxymethyl-2-oxabicyclo[2.2.1]heptan-5-yl]-5-heptensäuremethyl (bzw. Benzyl) ester:

11.7 mg (31,8 μmol) des nach Beispiel 13a dargestellten Alkohols setzte man in Analogie zu Beispiel 1 um und isolierte nach Aufarbeitung und Reinigung 14 mg der Titelverbindungen als farbloses Öl.

IR (Film): 3080, 3060, 3030, 3000, 2930, 2870, 2230, 1735, 1605, 1490, 1455, 1110, 1075, 1005, 895, 755, 740 und 695 $cm^{-1}$.

Beispiel 13a

(4S,5R(5Z),6S,1R)-7-[4-Phenylacetylen-6-hydroxy-2-oxabicyclo[2.2.1]heptan-5-yl]-5-heptensäuremethylester:

91 mg (150 μmol) des nach Beispiel 13b dargestellten Bicyclus setzte man in Analogie zu Beispiel 1a um und isolierte nach Aufarbeitung und Reinigung 55 mg (148 μmol, 99 %) der Titelverbindung als farbloses Öl.

IR ($CHCl_3$): 3200-3700, 3080, 3060, 3030, 3000, 2950, 2880, 2230, 1725, 1600, 1490, 1440, 1365, 1300, 1220, 1175, 1025, 995, 890 und 690 $cm^{-1}$.

Beispiel 13b

(4S,5R(5Z),6S,1R)-7-[4-Phenylacetylen-6-tert-butyldiphenylsiloxymethyl-2-oxabicyclo[2.2.1]heptan-5-yl]-5-heptensäuremethylester:

120 mg (192 μmol) des in Beispiel 13c dargestellten Diols cyclisierte man in Analogie zu Beispiel 1b um und isolierte nach Aufarbeitung und Reinigung 91 mg (150 μmol, 62 %) der Titelverbindung als farbloses Öl.

IR (Film): 3070, 3050, 3000, 2950, 2930, 2860, 2215, 1735, 1600, 1590, 1425, 1110, 1005, 895, 820, 755, 740 und 705 $cm^{-1}$.

Beispiel 13c

(1S,2R(5Z),3S,4R)-7-[1-Hydroxymethyl-1-phenylacetylen-3-tert-butyldiphenylsilyloxymethyl-4-hydroxy-cyclopentan-2-yl]-5-heptensäuremethylester:

205 mg (289 μmol) der in Beispiel 13d dargestellten Verbindung setzte man in Analogie zu Beispiel 1c um und isolierte nach Aufarbeitung und Reinigung 120 mg (192 μmol, 66 %) der Titelverbindung als farbloses Öl.

IR (Film): 3200-3600, 3070, 3050, 3010, 2950, 2930, 2860, 2220, 1735, 1600, 1590, 1425, 1110, 820, 755, 740 und 705 $cm^{-1}$.

Beispiel 13d

(1S,2R(5Z),3S,4R)-7-[1-Hydroxymethyl-1-phenylacetylen-3-tert-butyldiphenylsilyloxymethyl-4-(tetrahydropyran-2-yloxy)-cyclopentan-2-yl]-5-heptensäuremethylester (A) und (1R(5Z),2R,3S,4R)-7-[1-Phenylacetylen-2-hydroxymethyl-3-tert-butyl-diphenylsilyloxymethyl-4-(tetrahydropyran-2-yloxy)-cyclopentan-1-yl]-5-heptensäuremethylester (B):

512 mg (838 µmol) eines ca. 1:1-Gemisches der in Beispiel 13e dargestellten regioisomeren Laktole setzte man in Analogie zu Beispiel 1d um und isolierte nach Methylesterbildung, Reinigung und chromatographischer Trennung 220 mg (310 µmol, 37 %) der Titelverbindung B als unpolarere Komponente sowie 205 mg (289 µmol, 35 %) der Titelverbindung A als polarere Komponente, jeweils als farbloses Öl.

IR (Film) von A: 3200-3600, 3070, 3050, 3010, 2940, 2850, 2220, 1735, 1600, 1590, 1430, 1240, 1110, 1035, 1020, 870, 820, 760, 740 und 705 $cm^{-1}$.

Beispiel 13e

(1S,4RS,6R,7S,8R)-1-Phenylacetylen-4-hydroxy-7-tert-butyl-diphenylsilyloxymethyl-8-(tetrahydropyran-2-yloxy)-3-oxa-bicyclo[4.3.0]nonan und (1S,3RS,6R,7S, 8R)-1-Phenylacetylen-3-hydroxy-7-tert-butyl-diphenylsilyloxymethyl-8-(tetrahydropyran-2-yloxy)-4-oxabicyclo[4.3.0]nonan:

1,01 g (1,66 mmol) eines ca 1:1-Gemisches der in Beispiel 13f dargestellten regioisomeren Laktone reduzierte man in Analogie zu Beispiel 1e. Isoliert wurden nach Aufarbeitung und Reinigung 512 mg (838µmol, 51 %) eines Gemisches der beiden Titelverbindungen als farbloses Öl.

IR ($CHCl_3$): 3400-3600, 3070, 3050, 3000, 2940, 2860, 2220, 1600, 1590, 1425, 1255, 1110, 1075, 1020, 910, 865, 820 und 700 $cm^{-1}$.

Beispiel 13f

(1S,6R,7S,8R)-1-Phenylacetylen-7-tert-butyl-diphenylsilyloxymethyl-8-(tetrahydropyran-2-yloxy>)-3-oxa-bicyclo[4.3.0]nonan-4-on und (1S,6R,7S,8R)-1-Phenylacetylen-7-tert-butyldiphenylsilyloxymethyl-8-(tetrahydropyran-2-yloxy)-4-oxabicyclo[4.3.0]nonan-3-on:

1,7 g (2.87 mmol) des nach Beispiel 13g dargestellten bicyclischen Ketons wurden in Analogie zu Beispiel 1f oxidiert. Nach Aufarbeitung und Reinigung isolierte man 1,01 g (1,66 mmol, 58 %) eines ca. 1:1-Gemisches der beiden Titelverbindungen als schwach gelb gefärbter Schaum.

IR (Film): 3070, 3050, 2940, 2860, 1750, 1600, 1590, 1575, 1430, 1260, 1110, 1035, 970, 915, 870, 820, 740 und 705 $cm^{-1}$.

Beispiel 13g

(1S,5R,6S,7R)-1-Phenylacetylen-6-tert-butyl-diphenylsilyloxymethyl-7-(tetrahydropyran-2-yloxy)-bicyclo [3.3.0]octan-3-on:

2,0 g (4,08 mmol) des nach Beispiel 1h dargestellten bicyclischen ungesättigten Ketons wurden in Analogie zu Beispiel 11g unter Verwendung von Phenylacetylen umgesetzt. Nach Aufarbeitung und Reinigung isolierte man 1,78 g (3,00 mmol, 74 %) der Titelverbindung als gelbes Öl.

IR ($CHCl_3$): 3070, 3050, 3000, 2940, 2860, 2220, 1735, 1600, 1590, 1425, 1260, 1110, 1075, 1020, 915, 870, 820, 740 und 700 $cm^{-1}$.

Beispiel 14

(4S,5R(5Z),6S,1R)-7-[4-Phenylacetylen-6-benzyloxymethyl-2-oxabicyclo[2.2.1]heptan-5-yl]-5-heptensäure:

14 mg des nach Beispiel 13 dargestellten Estergemisches verseifte man in Analogie zu Beispiel 2 und isolierte nach Aufarbeitung und Reinigung 10,1 mg (22,7 µmol, 71 %, bezogen auf Startmaterial in Beispiel 13) der Titelverbindung als farbloses Öl.

IR (Film): 3600-2400, 3080, 3060, 3030, 3000, 2930, 2880, 2230, 1730, 1705, 1600, 1490, 1455, 1110, 1070, 1005, 895, 755, 740 und 690 $cm^{-1}$.

Beispiel 15

(4S,5R(5Z),6S,1R)-7-[4-(2-Phenylethyl)-6-benzoyloxymethyl-2-oxabicyclo[2.2.1]heptan-5-yl]-5-heptensäuremethyl (bzw. Benzyl) ester:

19,5 mg (52,3 µmol) des nach Beispiel 15a dargestellten Alkohols veretherte man in Analogie zu Beispiel 1 und isolierte nach Aufarbeitung und Reinigung 23 mg der Titelverbindungen als farbloses Öl.

IR (Film): 3080, 3060, 3030, 3000, 2930, 2870, 1735, 1600, 1495, 1455, 1240, 1205, 1115, 1095, 1020, 895, 740 und 700 $cm^{-1}$.

Beispiel 15a

(4S,5R(5Z),6S,1R)-7-[4-(2-Phenylethyl)-6-hydroxymethyl-2-oxabicyclo[2.2.1]heptan-5-yl]-5-heptensäuremethylester:

944 mg (1,55 mmol) des nach Beispiel 15b dargestellten Bicyclus setzte man in Analogie zu Beispiel 1a um und isolierte nach Aufarbeitung und Reinigung 528 mg (1,42 mmol, 92 %) der Titelverbindung als farbloses Öl.

IR (Film): 3200-3600, 3060, 3020, 3000, 2940, 2860, 1735, 1605, 1450, 1435, 1240, 1155, 1025, 895, 750 und 700 $cm^{-1}$.

Beispiel 15b

(4S,5R(5Z),6S,1R)-7-[4-(2-Phenylethyl)-6-tert-butyldiphenylsilyloxymethyl-2-oxabicyclo[2.2.1]heptan-5-yl]-5-heptensäuremethylester:

1,11 g (1,77 mmol) des in Beispiel 15c dargestellten Diols setzte man in Analogie zu Beispiel 1b um und isolierte nach Aufarbeitung und Reinigung 945 mg (1,55 mmol, 88 %) der Titelverbindung als farbloses Öl.

IR (Film): 3070, 3050, 3020, 3000, 2940, 2850, 1735, 1600, 1590, 1425, 1110, 895, 820, 740 und 700 $cm^{-1}$.

Beispiel 15c

(1S,2R(5Z),3S,4R)-7-[1-Hydroxymethyl-1-(2-phenylethyl)-3-tert-butyldiphenylsilyloxymethyl-4-hydroxycyclopentan-2-yl]-5-heptensäuremethylester:

1,62 g (2,27 mmol) der in Beispiel 15d dargestellten Verbindung setzte man in Analogie zu Beispiel 1c um und isolierte nach Aufarbeitung und Reinigung 1,11 g (1,77 mmol. 78 %) der Titelverbindung als farbloses Öl.

IR (Film): 3200-3600, 3070, 3050, 3020, 3000, 2940, 2850, 1735, 1600, 1590, 1430, 1240, 1110, 1045, 820, 740 und 700 $cm^{-1}$.

Beispiel 15d

(1S,2R(5Z),3S,4R)-7-[1-Hydroxymethyl-1-(2-phenylethyl)-3-tert-butyl-diphenylsilyloxymethyl-4-(tetrahydropyran-2-yloxy)-cyclopentan-2-yl]-5-heptensäuremethylester (A) und (1R(5Z),2R,3S,4R)-7-[1-(2-Phenylethyl)-2-hydroxymethyl-3-tert-butyl-diphenylsilyloxymethyl-4-(tetrahydropyran-2-yloxy)-cyclopentan-1-yl]-5-heptensäuremethylester (B):

2,62 g (4,26 mmol) des in Beispiel 15e isolierten Gemisches der regioisomeren Laktole setzte man in Analogie zu Beispiel 1d um und isolierte nach Versterung, Reinigung und chromatographischer Trennung 1,29 g (1,81 mmol, 42 %) der Titelverbindung B als unpolarere Komponente neben 1,62 g (2,27 mmol, 53 %) der Titelverbindung A als polarere Komponente.

IR (Film) von A: 3200-3600, 3070, 3050, 3020, 2940, 2855, 1735, 1600, 1590, 1430, 1240, 1200, 1110, 1030, 870, 820, 740 und 705 $cm^{-1}$.

Beispiel 15e

(1S,4RS,6R,7S,8R)-1-(2-Phenylethyl)-4-hydroxy-7-tert-butyl-diphenylsilyloxymethyl-8-(tetrahydropyran-2-yloxy)-3-oxabicyclo[4.3.0]nonan und (1R,3RS,6R,7S, 8R)-1-(2-Phenylethyl)-3-hydroxy-7-tert-butyl-diphenylsilyloxymethyl-8-(tetrahydropyran-2-yloxy)-4-oxabicyclo[4.3.0]nonan:

2,70 g (4,40 mmol) des in Beispiel 15f dargestellten Gemisches der beiden regioisomeren Laktone reduzierte man in Analogie zu Beispiel 1e und isolierte nach Aufarbeitung 2,62 g (4,26 mmol, 97 %) eines Gemisches der Titelverbindungen.

IR (Film): 3200-3600, 3070, 3050, 3020, 2930, 2850, 1600, 1590, 1425, 1155, 1260, 1200, 1110, 1075, 1020, 910, 865, 820, 740 und 700 $cm^{-1}$.

Beispiel 15f

(1S,6R,7S,8R)-1-(2-Phenylethyl)-7-tert-butyl-diphenylsilyloxymethyl-8-(tetrahydropyran-2-yloxy)-3-oxabicyclo[4.3.0]nonan-4-on und (1R,6R,7S,8R)-1-(2-Phenylethyl)-7-tert-butyl-diphenylsilyloxymethyl-8-(tetrahydropyran-2-yloxy)-4-oxabicyclo[4.3.0]nonan-3-on:

3,14 g (5,26 mmol) des in Beispiel 15g dargestellten Ketons oxidierte man in Analogie zu Beispiel 1f

und isolierte nach Aufarbeitung und Reinigung 2,70 g (4,40 mmol, 84 %) eines ca. 1:1-Gemisches der beiden Titelverbindungen als farbloses Öl.

IR (Film): 3070, 3050, 3015, 2940, 2850, 1745, 1600, 1590, 1425, 1110, 1040, 970, 870, 820, 740 und 705 $cm^{-1}$.

Beispiel 15g

(1R,5R,6S,7R)-1-(2-Phenylethyl)-6-tert-butyl-diphenylsiloxymethyl-7-(tetrahydropyran-2-yloxy)-bicyclo [3.3.0]octan-3-on:

4,18 g (8,52 mmol) des in Beispiel 1h dargestellten bicyclischen ungesättigten Ketons setzte man in Analogie zu Beispiel 1g mit Phenethylmagnesiumbromid unter Kupferkatalyse um. Nach Aufarbeitung und Reinigung isolierte man 3,15 g (5,27 mmol, 62 %) der Titelverbindung als farbloses Öl.

IR (Film): 3070, 3050, 3020, 2930, 2850, 1740, 1600, 1590, 1425, 1110, 1075, 1020, 870, 820, 740 und 700 $cm^{-1}$.

Beispiel 16

(4S,5R(5Z),6S,1R)-7-[4-(2-Phenylethyl)-6-benzyloxymethyl-2-oxabicyclo[2.2.1]-heptan-5-yl]-5-heptensäure:

23 mg des nach Beispiel 15 dargestellten Estergemisches verseifte man in Analogie zu Beispiel 2 und isolierte nach Aufarbeitung und Reinigung 21 mg (47 µmol, 90 %, bezogen auf Edukt aus Beispiel 15) der Titelverbindung als farbloses Öl.

IR (Film): 3600-2400, 3080, 3060, 3020, 3000, 2930, 2860, 1730, 1705, 1600, 1495, 1455, 1240, 1205, 1115, 1090, 1025, 895, 740 und 700 $cm^{-1}$.

Beispiel 17

[4S,5R(5Z),6S,1R)-7-[4(p-Fluorphenyl)-6-benzyloxymethyl-2-oxabicyclo[2.2.1]heptan-5-yl]-5-heptensäuremethyl (bzw. benzylester):

17,5 mg (48,3 µmol des nach Beispiel 17a dargestellten Alkohols setzte man in Analogie zu Beispiel 1 um und isolierte nach Aufarbeitung und Reinigung 24 mg der Titelverbindung als farbloses Öl.

IR (Film): 3060, 3020, 3000, 2930, 2870, 1735, 1600, 1510, 1455, 1110, 1075, 895, 820, 740 und 705 $cm^{-1}$.

Beispiel 17a

(4S,5R(5Z),6S,1R)-7-[4-(p-Fluorphenyl)-6-hydroxy-2-oxabicyclo[2.2.1]heptan-5-yl]-5-heptensäuremethylester:

798 mg (1,33 mmol) des nach Beispiel 17b dargestellten Bicyclus setzte man in Analogie zu Beispiel 1a um und isolierte nach Aufarbeitung und Reinigung 448 mg (1,24 mmol, 93 %) der Titelverbindung als farbloses Öl.

IR (Film): 3200-3600, 3080, 3060, 3020, 3000, 2950, 2870, 1735, 1600, 1510, 1435, 1220, 1020, 995, 895, 820 und 700 $cm^{-1}$.

Beispiel 17b

(4S,5R(5Z),6S,1R)-7-[4(p-Fluorphenyl)-6-tert.-butyl-diphenylsilyloxymethyl-2-oxabicyclo[2.2.1]heptan-5-yl]-5-heptensäuremethylester:

1,15 g (1,86 mmol) des nach Beispiel 17c dargestellten Diols cyclisierte man in Analogie zu Beispiel 1b um und isolierte nach Aufarbeitung und Reinigung 800 mg (1,33 mmol, 72 %) der Titelverbindung als farbloses Öl.

IR ($CHCl_3$): 3070, 3050, 3000, 2950, 2930, 2860, 1735, 1605, 1590, 1510, 1425, 1230, 1110, 895, 830, 820, 740 und 705 $cm^{-1}$.

Beispiel 17c

(1S,2R(5Z),3S,4R)-7-(1-Hydroxymethyl-1-(p-fluorphenyl)-3-tert-butyldiphenylsilyoxymethyl-4-hydroxy-cyclopentan-2-yl]-5-heptensäuremethylester (A) und (1R,(5Z),2R,3S,4R)-7-[1-(p-Fluorphenyl)-2-hydroxymethyl-3-tert-butyl-diphenylsilyloxymethyl-4-hydroxy-cyclopentan-1-yl]-5-heptensäuremethylester (B):

2,58 g (3,67 mmol) eines Gemisches der in Beispiel 17d dargestellten regioisomeren Verbindungen setzte man in Analogie zu Beispiel 1c um und isolierte nach Aufarbeitung, Reinigung und Trennung 1,15 g (1,86 mmol, 51 %) der Titelverbindung A, sowie 0,85 g (1,37 mmol, 37 %) der Titelverbindung B, jeweils als farbloses Öl.

IR ($CHCl_3$) von A: 3700-3200, 3070, 3040, 3010, 2950, 2930, 2850, 1730, 1600, 1590, 1510, 1425, 1230, 1110, 1050, 820, 740 und 705 $cm^{-1}$.

Beispiel 17d

(1S,2R(5Z),3S,4R)-7-[1-Hydroxymethyl-1-(p-fluorphenyl)-3-tert-butyldiphenylsilyoxymethyl-4-(tetrahydropyran-2-yloxy)-cyclopentan-2-yl]-5-heptensäuremethylester und (1R, (5Z),2R,3S,4R)-7-[1-(p-Fluorphenyl)-2-hydroxymethyl-3-tert-butyl-diphenylsilyloxymethyl-4-(tetrahydropyran-2-yloxy)-cyclopentan-1-yl]-5-heptensäuremethylester:

2,55 g (4,22 mmol) eines ca. 1:1-Gemisches der in Beispiel 17e dargestellten regioisomeren Laktole setzte man in Analogie zu Beispiel 1d um und isolierte nach Methylesterbildung und Reinigung 2,58 g (3,67 mmol, 87 %) eines Gemisches der beiden Titelverbindungen als farbloses Öl.

IR (Film): 3200-3600, 3070, 3050, 3020, 2950, 2860, 1735, 1600, 1590, 1510, 1430, 1235, 1110, 1035, 870, 820, 740 und 705 $cm^{-1}$.

Beispiel 17e

(1S,4RS,6R,7S,8R)-1-(p-Fluorphenyl)-4-hydroxy-7-tert-butyldiphenylsilyloxymethyl-8-(tetrahydropyran-2-yloxy)-3-oxabicyclo[4.3.0]nonan und (1S,3RS,6R,7S,8R)-1-(p-Fluorphenyl)-3-hydroxy-7-tert-butyl-diphenylsilyloxymethyl-8-(tetrahydropyran-2-yloxy)-4-oxabicyclo[4.3.0]nonan:

In Analogie zu Beispiel 1e reduzierte man 2,57 g (4,26 mmol) eines ca. 1:1-Gemisches der in Beispiel 17f dargestellten regioisomeren Laktone und isolierte nach Aufarbeitung und Reinigung 2,55 g (4,22 mmol, 99 %) eines Gemisches der beiden Titelverbindungen.

IR (Film): 3200-3600, 3070, 3040, 3000, 2950, 2870, 1600, 1590, 1510, 1425, 1260, 1110, 1075, 1040, 910, 865, 820, 740 und 700 $cm^{-1}$.

Beispiel 17f

(1S,6R,7S,8R)-1-(p-Fluorphenyl)-7-tert-butyl-diphenylsilyloxymethyl-8-(tetrahydropyran-2-yloxy)-3-oxabicyclo[4.3.0]nonan-4-on und (1S,6R,7S,8R)-1-(p-Fluorphenyl)-7-tert-butyl-diphenylsilyloxymethyl-8-(tetrahydropyran-2-yloxy)-4-oxabicyclo[4.3.0]nonan-3-on:

2,80 g (4,77 mmol) des nach Beispiel 17g dargestellten bicyclischen Ketons oxidierte man in Analogie zu Beispiel 1f und isolierte nach Aufarbeitung und Reinigung 2,58 g (4,28 mmol, 90 %) eines ca. 1:1-Gemisches der beiden Titelverbindungen.

IR ($CHCl_3$): 3070, 3050, 3000, 2950, 2860, 1745, 1605, 1590, 1510, 1230, 1110, 1030, 820, 740 und 700 $cm^{-1}$.

Beispiel 17g

(1S,5R,6S,7R)-1-(p-Fluorphenyl)-7-tert-butyl-diphenylsilyloxymethyl-7-(tetrahydropyran-2-yloxy)-bicyclo [3.3.0]octan-3-on:

4,06 g (8,27 mmol) des nach Beispiel 1h dargestellten bicyclischen ungesättigten Ketons wurden in Analogie zu Beispiel 1g mit p-Fluorphenylmagnesiumchlorid unter Kupferkatalyse umgesetzt. Nach Aufarbeitung und Reinigung isolierte man 2,80 g (4,77 mmol, 58 %) der Titelverbindungen als farbloses Öl.

IR (Film): 3090, 3040, 2940, 2850, 1740, 1600, 1590, 1510, 1425, 1235, 1110, 1075, 1020, 820, 740 und 700 $cm^{-1}$.

Beispiel 18

(4S,5R(5Z),6S,1R)-7-[4-(p-Fluorphenyl)-6-benzyloxymethyl-2-oxabicyclo[2.2.1]-heptan-5-yl]-5-heptensäure:

24 mg des in Beispiels 17 dargestellten Estergemisches verseifte man in Analogie zu Beispiel 2 und isolierte nach Aufarbeitung und Reinigung 20 mg (45,6 μmol, 94 % bezogen auf Startmaterial in Beispiel 17) der Titelverbindungen als farbloses Öl.

IR (Film): 3600-2400, 3090, 3060, 3030, 3000, 2930, 2870, 1725, 1710, 1605, 1510, 1450, 1230, 1160,

1040, 1025, 890, 830, 735, 695 und 640 $cm^{-1}$.

Beispiel 19

(4S,5R(5Z),6S,1R)-7-[4-Phenyl-6-tert-butyl-diphenylsilyloxymethyl-2-oxabicyclo[2.2.1]heptan-5-yl]-5-heptensäure:

Die farblose Lösung von 22,7 mg (39 µmol) der in Beispiel 3b dargestellten Verbindung in 1 ml Methanol versetzte man mit 0,5 ml einer 5%igen wässrigen Lithiumhydroxid-Lösung und rührte das anfangs heterogene Gemisch 16 Stunden bei 23 °C. Man neutralisierte durch Zugabe entsprechender Mengen einer 10%igen wässrigen Zitronensäure und reinigte durch Chromatographie an einer analytischen Dünnschichtplatte unter Verwendung eines Gemisches aus Dichlormethan und Ethanol. Nach Elution der geeigneten Zone mit einem Gemisch aus Ethylacetat und Ethanol isolierte man 19,5 mg (34,3 µmol, 88 %) der Titelverbindung als farbloses Öl.

IR (Film): 3500-2400, 3070, 3050, 3030, 3010, 2950, 2930, 2860, 1730, 1710, 1600, 1590, 1470, 1430, 1110, 1075, 890, 825, 760, 740, 700, 615, 505 und 490 $cm^{-1}$.

Beispiel 20

(4S,5R(5Z),6S,1R)-7-[4-Phenyl-6-hydroxymethyl-2-oxabicyclo[2.2.1]heptan-5-yl]-5-heptensäure:

15 mg (43,5 µmol) der in Beispiel 3a dargestellten Verbindung verseifte man in Analogie zu Beispiel 19 und isolierte nach Aufarbeitung und Reinigung 10,3 mg (31,2 µmol, 72 %) der Titelverbindung als farbloses Öl.

IR (Film): 3100-2400, 3080, 3060, 3030, 2930, 2870, 1710, 1600, 1500, 1445, 1230, 1200, 1040, 1000, 970, 890, 760 und 700 $cm^{-1}$.

Beispiel 21

(4S,5R(5Z),6S,1R)-7-[4-Phenyl-6-hexyloxymethyl-2-oxabicyclo[2.2.1]heptan-5-yl]-5-heptensäuremethylester:

33,8 mg (98,1 µmol) der in Beispiel 3a dargestellten Verbindung löste man in 3,5 ml wasserfreiem Tetrahydrofuran, versetzte mit 10 mg einer 50%igen Natriumhydriddispension in Weissöl und gab nach beendeter Wasserstoffentwicklung 140 µl 1-Bromhexan zu. Man erwärmte auf 40 - 50 °C und rührte noch 18 Stunden unter einer Atmosphäre aus trockenem Argon. Man versetzte mit Wasser, säuerte mit einer 10%igen Zitronensäure an und extrahierte mehrfach mit Diethylether. Die vereinigten organischen Extrakte wurden mit Wasser gewaschen, über Magnesiumsulfat getrocknet, filtriert und im Wasserstrahlvakuum eingeengt. Den Rückstand reinigte man durch Chromatographie an einer analytischen Dünnschichtplatte unter Verwendung eines Chloroform/Ethanol-Gemisches. Nach Elution geeigneter Zonen isolierte man 16,4 mg (38,3 µmol, 39 %) der Titelverbindung neben Ausgangsmaterial und Esterspaltungsprodukt.

IR (Film): 3070, 3050, 3030, 3000, 2940, 2870, 1735, 1600, 1450, 1040, 895, 760 und 700 $cm^{-1}$.

Beispiel 22

(4S,5R(5Z),6S,1R)-7-[4-Phenyl-6-hexyloxymethyl-2-oxabicyclo[2.2.1]heptan-5-yl]-5-heptensäure:

16,4 mg (38,3 µmol) der in Beispiel 21 dargestellten Verbindung verseifte man in Analogie zu Beispiel 19 und isolierte nach Aufarbeitung und Reinigung 10,0 mg (24,1 µmol, 63 %) der Titelverbindung als farbloses Öl.

IR (Film): 3600-2400, 3070, 3050, 3030, 3010, 2960, 2940, 2870, 1730, 1710, 1600, 1450, 1040, 900, 760 und 700 $cm^{-1}$.

Beispiel 23

(4S,5R(5Z),6R(1E,3R),1R)-7-[4-Phenyl-6-(3-hydroxy-4,4-dimethyl-1-octenyl)-2-oxabicyclo[2.2.1]heptan-5-yl]-5-heptensäuremethylester (A) und (4S,5R(5Z),6R(1E,3S),1R)-7-[4-Phenyl-6-(3-hydroxy-4,4-dimethyl-1-octenyl)-2-oxabicyclo-[2.2.1]heptan-5-yl]-5-heptensäuremethylester (B):

108 mg (231 µmol) des in Beispiels 23a dargestellten ungesättigten Ketons löste man in 2,8 ml wasserfreiem Methanol, kühlte unter einer Atmosphäre aus trockenem Argon auf - 40 °C und versetzte mit 53 mg Natriumborhydrid. Nach 0,5 Stunden quenchte man durch Zugabe von 70 µl Eisessig, ließ auf 23 °C erwärmen und extrahierte mehrfach mit insgesamt 8 ml Diethylether. Man trocknete über Magnesiumsulfat und chromatographierte den nach Abzug des Lösungsmittels im Wasserstrahlvakuum erhaltenen Rückstand an 4 analytischen Kieselgelplatten. Als Laufmittel diente ein Gemisch aus Chloroform/Ethanol, als Elutionsmittel ein Gemisch aus Ethylacetat/Ethanol. Isoliert wurden 49 mg (105 µmol, 45 %) einer unpolareren Komponente, der man die Struktur A zuordnetete neben 31 mg (66 µmol, 29 %) einer polareren Komponente, der die Struktur B zugeordnet wurde.

IR (Film) von A: 3200-3600, 3060, 2950, 2930, 2870, 1735, 1600, 1450, 1195, 1025, 995, 970, 895, 750 und 700 $cm^{-1}$.

IR (Film) von B: 3200-3600, 3060, 2950, 2930, 2870, 1735, 1600, 1445, 1245, 1195, 995, 970, 895, 760 und 700 $cm^{-1}$.

Beispiel 23a

(4S,5R(5Z),6R(1E),1R)-7-[4-Phenyl-6-(3-oxo-4,4-dimethyl-1-octenyl)-2-oxabicyclo[2.2.1]heptan-5-yl]-5-heptensäuremethylester:

Zu der Suspension von 8,9 mg Natriumhydrid in 2,8 ml wasserfreiem Dimethoxyethan tropfte man die Lösung von 96 mg Dimethyl-(2-oxo-3,3-dimethyl-heptyl)-phosphonat in 0,9 ml wasserfreiem Dimethoxyethan, kühlte nach beendeter Gasentwicklung unter einer Atmosphäre aus trockenem Argon auf - 30 °C und tropfte die Lösung von 104 mg (303 µmol) des in Beispiel 23b dargestellten Aldehyds in 1,3 ml wasserfreiem Dimethoxyethan zu. Man ließ innerhalb 6 Stunden auf 22 °C erwärmen, versetzte mit 170 µl Eisessig, verdünnte mit 5 ml Diethylether, wusch mit Wasser und gesättigter Natriumchloridlösung neutral und trocknete über Magnesiumsulfat. Nach Abzug des Lösungsmittels im Wasserstrahlvakuum reinigte man den Rückstand durch Chromatographie an 6 analytischen Kieselgelplatten. Als Laufmittel diente ein Gemisch aus n-Hexan-Ethylacetat, als Elutionsmittel Ethylacetat. Isoliert wurden 108 mg (231 µmol, 76 %) der Titelverbindung als farbloses Öl.

IR (Film): 3060, 2955, 2930, 2870, 1735, 1685, 1620, 1450, 1195, 1045, 995, 900, 760 und 700 $cm^{-1}$.

Beispiel 23b

(4S,5R(5Z),6S,1R)-7-[4-Phenyl-6-formyl-2-oxabicyclo[2.2.1]heptan-5-yl]-5-heptensäuremethylester:

103 mg (299 µmol) des nach Beispiel 3a dargestellten Alkohols oxidierte man in Analogie zu Beispiel 38b und isolierte nach Aufarbeitung und Reinigung 101 mg (295 µmol, 99 %) der Titelverbindung.

IR (Film): 3060, 2950, 2870, 2720, 1735, 1715, 1600, 1500, 1445, 1435, 1245, 1165, 1015, 1000, 900, 765 und 700 $cm^{-1}$.

Beispiel 24

(4S,5R(5Z),6R(1E,3S),1R)-7-[4-Phenyl-6-(3-hydroxy-4,4-dimethyl-1-octenyl)-2-oxabicyclo[2.2.1]heptan-5-yl]-5-heptensäure:

48 mg (105 µmol) des nach Beispiel 23 dargestellten unpolareren Alkohols verseifte man in Analogie zu Beispiel 2 und isolierte nach Aufarbeitung und Reinigung 44 mg (97 µmol, 92 %) der Titelverbindung als farbloses Öl.

IR (Film): 3600-2400, 3080, 3060, 3030, 3000, 2950, 2930, 2870, 1710, 1600, 1450, 1240, 1200, 995, 970, 890, 755 und 700 $cm^{-1}$.

Beispiel 25

(4S,5R(5Z),6R(1E,3R),1R)-7-[4-Phenyl-6-(3-hydroxy-4,4-dimethyl-1-octenyl)-2-oxabicyclo[2.2.1]heptan-5-yl]-5-heptensäure:

31 mg (66 µmol) des nach Beispiel 23 dargestellten polareren Alkohols verseifte man in Analogie zu Beispiel 2 und isolierte nach Aufarbeitung und Reinigung 30 mg (60,8 µmol, 92 %) der Titelverbindung als farbloses Öl.

IR (Film): 3600-2400, 3090, 3060, 3030, 3000, 2960, 2930, 2870, 1710, 1605, 1450, 1240, 1195, 995, 970, 895, 760 und 700 $cm^{-1}$.

Beispiel 26

(4S,5R(5Z),6S,1R)-7-[4-Phenyl-6-[(E/Z)-diphenylmethoxyiminomethyl]-2-oxabicyclo[2.2.1]heptan-5-yl]-5-heptensäuremethylester:

19 mg (40 μmol) des nach Beispiel 23b dargestellten Aldehyds löste man in 200 μl wasserfreiem Ethanol, versetzte mit 12,2 mg Diphenylmethoxyhydroxyamin, einem Tropfen Pyridin und erwärmte unter einer Atmosphäre aus trockenem Argon 7 Stunden auf 50 °C. Man ließ erkalten und reinigte durch Chromatographie an einer analytischen Kieselgelplatte. Als Laufmittel diente ein Gemisch aus Chloroform/ Ethanol, als Elutionsmittel Ethylacetate. Isoliert wurden 20mg (38 μmol, 95 %) der Titelverbindung als farbloses Öl.

IR (Film): 3080, 3060, 3025, 2940, 2870, 1735, 1600, 1495, 1450, 1020, 1000, 935, 895, 760, 740 und 700 $cm^{-1}$.

Beispiel 27

(4S,5R(5Z),6S,1R)-7-[4-(2-Phenylethyl)-6-[(E/Z)-diphenylmethoxyiminomethyl]-2-oxabicyclo[2.2.1]heptan-5-yl]-5-heptensäuremethylester:

18 mg (49 μmol) des nach Beispiel 27a dargestellten Aldehyds setzte man in Analogie zu Beispiel 26 um und isolierte nach Aufarbeitung und Reinigung 26 mg (47 μmol, 97 %) der Titelverbindung als farbloses Öl.

IR (Film): 3080, 3060, 3030, 2940, 2865, 1735, 1600, 1495, 1450, 1020, 935, 895, 745 und 700 $cm^{-1}$.

Beispiel 27a

(4S,5R(5Z),6R,1R)-7-[4-(2-Phenylethyl)-6-formyl-2-oxabicyclo[2.2.1]heptan-5-yl]-5-heptensäuremethylester:

201 mg (540 μmol) des nach Beispiel 15a dargestellten Alkohols oxidierte man in Analogie zu Beispiel 38b und isolierte nach Aufarbeitung und Reinigung 200 mg (540 μmol, 100 %) der Titelverbindung als blassgelbes Öl.

IR (Film): 3070, 3050, 3020, 2950, 2860, 2720, 1735, 1715, 1605, 1450, 1435, 1245, 1160, 1025, 900, 750 und 705 $cm^{-1}$.

Beispiel 28

(4S,5R(5Z),6S,1R)-7-[4-Phenyl-6-[(E/Z)-diphenylmethoxyiminomethyl]-2-oxabicyclo[2.2.1]heptan-5-yl]-5-heptensäure:

20 mg (38 μmol) der nach Beispiel 26 dargestellten Verbindung verseifte man in Analogie zu Beispiel 2 und isolierte nach Aufarbeitung und Reinigung 15 mg (29 μmol, 77 %) der Titelverbindung als farbloses Öl.

IR (Film): 3600-2500, 3090, 3060, 3030, 3010, 2940, 2880, 1730, 1710, 1605, 1495, 1450, 1000, 935, 900, 760, 745 und 700 $cm^{-1}$.

Beispiel 29

(4S,5R(5Z),6S,1R)-7-[4-(2-Phenylethyl)-6-[(E/Z)-diphenylmethoxyiminomethyl]-2-oxabicyclo[2.2.1]heptan-5-yl]-5-heptensäure:

26 mg (47 μmol) der nach Beispiel 27 dargestellten Verbindung verseifte man in Analogie zu Beispiel 2 und isolierte nach Aufarbeitung und Reinigung 20 mg (37 μmol, 79 %) der Titelverbindung als farbloses Öl.

IR (Film): 3600-2400, 3080, 3060, 3030, 3000, 2930, 2870, 1730, 1705, 1605, 1495, 1455, 1020, 935, 895, 745 und 700 $cm^{-1}$.

Beispiel 30

(4S,5R(5Z),6R(1E,3R),1R)-7-[4-Phenyl-6-(3-hydroxy-4-[phenoxy-1-butenyl)-2-oxabicyclo[2.2.1]heptan-5-yl]-5-heptensäuremethylester (A) und (4S,5R(5Z),6R (1E,3S),1R)-7-[4-Phenyl-6-(3-hydroxy-4-phenoxy-1-butenyl)-2-oxabicyclo[2.2.1]-heptan-5-yl]-5-heptensäuremethylester (B):

54 mg (114 μmol) des in Beispiel 30a dargestellten ungesättigten Ketons reduzierte man in Analogie zu Beispiel 23 und isolierte nach Aufarbeitung, Reinigung und Epimerentrennung 26,6 mg (56 μmol, 49 %) eines unpolaren Alkohols, dem man die Struktur A zuordnete sowie 25 mg (52 μmol, 46 %) eines polareren Alkohols, dem man die Struktur zuordnete.

IR (Film) des Epimerengemisches:: 3600-3200, 3060, 3030, 3010, 2930, 2880, 1735, 1600, 1590, 1500, 1455, 1250, 1140, 1000, 970, 895, 760 und 700 $cm^{-1}$.

Beispiel 30a

(4S,5R(5Z),6R(1E),1R)-7-[4-Phenyl-6-(3-oxo-4-phenoxy-1-butenyl)-2-oxabicyclo-[2.2.1]heptan-5-yl]-5-heptensäuremethylester:

84 mg (245 μmol) des nach Beispiel 23b dargestellten Aldehyds löste man in 4 ml wasserfreiem Dimethoxyethan, kühlte unter einer Atmosphäre aus trockenem Argon auf 0 bis 5 °C, versetzte mit 147 mg Dimethyl-(2-oxo-3-phenoxy-propyl)-phosphonatlithiumsalz und ließ 8 Stunden bei 23 °C rühren. Man goß in kalte wässrige Zitronensäure, extrahierte mehrfach mit Diethylether, wusch mit Wasser und gesättigter Natriumchloridlösung neutral und trocknete über Magnesiumsulfat. Den nach Lösungsmittelabzug im Wasserstrahlvakuum verbliebenen Rückstand reinigte man durch Chromatographie an 5 analytischen Kieselgelplatten. Als Laufmittel diente ein Gemisch aus Chloroform/Ethanol, als Elutionsmittel Ethylacetat. Man isolierte 54 mg (114 μmol, 47 %) der Titelverbindung als farbloses Öl.

IR (Film): 3060, 3030, 2940, 2870, 1735, 1690, 1620, 1600, 1495, 1435, 1290, 1240, 1220, 1045, 1000, 900, 755 und 700 $cm^{-1}$.

Beispiel 31

(4S,5R(5Z),6R(1E,3R),1R)-7-[4-(p-Fluorphenyl)-6-(3-hydroxy-4-phenoxy-1-butenyl)-2-oxabicyclo[2.2.1]heptan-5-yl]-5-heptensäuremethylester (A) und (4S,5R (5Z),6R (1E,3S),1R)-7-[4-(p-Fluorphenyl)-6-(3-hydroxy-4-phenoxy-1-butenyl)-2-oxabicyclo[2.2.1]heptan-5-yl]-5-heptensäuremethylester (B):

59 mg (120 μmol) des in Beispiel 31a dargestellten ungesättigten Ketons reduzierte man in Analogie zu Beispiel 23 und isolierte nach Aufarbeitung und Reinigung 54 mg (109 μmol, 91 %) eines Gemisches der beiden Titelverbindungen als farbloses Öl.

IR (Film): 3600-3200, 3060, 3040, 3010, 2940, 2920, 2870, 1730, 1600, 1585, 1510, 1490, 1240, 1160, 1035, 895, 835, 755 und 690 $cm^{-1}$.

Die chromatographische Trennung lieferte 24 mg (49 μmol, 41 %) des unpolareren Alkohols, dem man die Struktur A zuordnete, sowie 29 mg (59 μmol, 49 %) des polareren Alkohols, dem man die Struktur B zuordnete.

Beispiel 31a

(4S,5R(5Z),6R(1E),1R)-7-[4-(p-Fluorphenyl)-6-(3-oxo-4-phenoxy-1-butenyl)-2-oxa-bicyclo[2.2.1]heptan-5-yl]-5-heptensäuremethylester:

54 mg (150 μmol) des in Beispiel 31b dargestellten Aldehyds setzte man in Analogie zu Beispiel 30a unter Verwendung von Dimethyl-(2-oxo-3-phenoxy-propyl)-phosphonat-lithiumsalz um und isolierte nach Aufarbeitung und Reinigung 57 mg (116 μmol, 77 %) der Titelverbindung als farbloses Öl.

IR (Film): 3000, 2940, 2870, 1730, 1690, 1620, 1600, 1430, 1225, 1160, 895, 835, 755 und 690 $cm^{-1}$.

Beispiel 31b

(4S,5R(5Z),6R,1R)-7-[4-(p-Fluorphenyl)-6-formyl-2-oxabicyclo[2.2.1]heptan-5-yl]-5-heptensäuremethylester:

155 mg (428 μmol) des in Beispiel 17a dargestellten Alkohols oxidierte man in Analogie zu Beispiel 38b und isolierte nach Aufarbeitung 155 mg (428 μmol, 100 %) der Titelverbindung als farbloses Öl.

IR (Film): 3060, 2950, 2870, 2730, 1735, 1600, 1505, 1450, 1435, 1245, 1160, 1010, 1005, 900, 820, 760 und 700 $cm^{-1}$.

Beispiel 32

(4S,5R(5Z),6R(1E,3S),1R)-7-[4-Phenyl-6-(3-hydroxy-4-phenoxy-1-butenyl)-2-oxabicyclo[2.2.1]heptan-5-yl]-5-heptensäure:

25 mg (52 μmol) des in Beispiel 30 dargestellten polareren Esters verseifte man in Analogie zu Beispiel 2 und isolierte nach Aufarbeitung und Reinigung 19 mg (41 μmol, 79 %) der Titelverbindung als farbloses Öl.

IR (Film): 3600-2400, 3060, 3030, 3010, 2930, 2880, 1710, 1600, 1590, 1500, 1455, 1245, 1140, 995, 970, 895, 760 und 700 $cm^{-1}$.

Beispiel 33

(4S,5R(5Z),6R(1E,3R),1R)-7-[4-(p-Fluorphenyl)-6-(3-hydroxy-4-phenoxy-1-butenyl)-2-oxabicyclo[2.2.1]

heptan-5-yl]-5-heptensäure:

24 mg (49 µmol) des in Beispiel 31 dargestellten unpolareren Esters verseifte man in Analogie zu Beispiel 2 und isolierte nach Aufarbeitung und Reinigung 19 mg (40 µmol, 81 %) der Titelverbindung als farbloses Öl.

IR (Film): 3600-2400, 3060, 3040, 3010, 2930, 2880, 1710, 1600, 1585, 1515, 1495, 1300, 1240, 1160, 1040, 895, 835, 755, 690 und 585 $cm^{-1}$.

Beispiel 34

(4S,5R(5Z),6S(1E,3R),1R)-7-[4-Phenyl-6-(3-hydroxy-4-phenoxy-1-butyl)-2-oxabicyclo[2.2.1]heptan-5-yl]-5-heptensäure:

26 mg (55 µmol) des in Beispiel 30 dargestellten unpolareren Esters verseifte man in Analogie zu Beispiel 2 und isolierte nach Aufarbeitung und Reinigung 17 mg (37 µmol, 67 %) der Titelverbindung als farbloses Öl.

IR (Film): 3600-2400, 3060, 3030, 3010, 2930, 2880, 1730, 1710, 1600, 1590, 1500, 1455, 1245, 1040, 890, 755, 700 und 690 $cm^{-1}$.

Beispiel 35

(4S,5R(5Z),6R(1E,3S),1R)-7-[4-(p-Fluorphenyl)-6-(3-hydroxy-4-phenoxy-1-butenyl)-2-oxabicyclo[2.2.1] heptan-5-yl]-5-heptensäure:

29 mg (59 µmol) des in Beispiel 31 dargestellten polareren Esters verseifte man in Analogie zu Beispiel 2 und isolierte nach Aufarbeitung und Reinigung 24 mg (50 µmol, 85 %) der Titelverbindung als farbloses Öl.

IR (Film): 3600-2500, 3060, 3040, 3010, 2930, 2880, 1710, 1600, 1585, 1515, 1495, 1455, 1300, 1240, 1160, 1040, 895, 835, 755, 690 und 590 $cm^{-1}$.

Beispiel 36

(4S,5R(5Z),6S,1R)-7-[4-Methyl-6-(phenyl-p-benzyloxymethyl)-2-oxabicyclo[2.2.1]heptan-5-yl]-5-heptensäuremethyl (bzw. 4-phenylbenzyl) ester:

16,7 mg (59 µmol) des in Beispiel 1a gewonnenen Alkohols setzte man in Analogie zu Beispiel 1 unter Verwendung von 4-Phenylbenzylchlorid um. Nach Aufarbeitung und Reinigung isolierte man 26 mg der Titelverbindungen als farbloses Öl.

IR (Film): 3070, 3030, 3010, 2960, 2930, 2880, 1735, 1600, 1485, 1455, 1230, 1110, 1020, 895, 830, 760 und 700 $cm^{-1}$.

Beispiel 37

(4S,5R(5Z),6S,1R)-7-[4-Methyl-6-(phenyl-p-benzyloxymethyl)-2-oxabicyclo[2.2.1]heptan-5-yl]-5-heptensäure:

26 mg des in Beispiel 36 dargestellten Estergemisches verseifte man in Analogie zu Beispiel 2 und isolierte nach Aufarbeitung und Reinigung 18 mg (41 µmol, 69 %) der Titelverbindung als farbloses Öl.

IR (Film): 3600-2400, 3070, 3030, 3000, 2950, 2920, 2870, 1730, 1705, 1600, 1485, 1455, 1235, 1110, 1085, 1020, 1010, 895, 825, 760 und 695 $cm^{-1}$.

Beispiel 38

(4S,5R(5Z),6R(1E,3R),1R)-7-[4-Methyl-6-(3-hydroxy-1-octenyl)-2-oxabicyclo[2.2.1]heptan-5-yl]-5-heptensäuremethylester (A) und (4S,5R(5Z),6R(1E,3S),1R)-7-[4-Methyl-6-(3-hydroxy-1-octenyl)-2-oxabicyclo [2.2.1]heptan-5-yl]-5-heptensäuremethylester (B):

37 mg (98 µmol) des in Beispiel 38a dargestellten ungesättigten Ketons reduzierte man in Analogie zu Beispiel 23 und isolierte nach Aufarbeitung und Reinigung 37 mg (97 µmol, 100 %) eines Gemisches der beiden Titelverbindungen als farbloses Öl.

IR (Film): 3600-3200, 2950, 2930, 2860, 1735, 1450, 1435, 1020, 965 und 890 $cm^{-1}$.

Die chromatographische Trennung lieferte 19 mg (50 µmol, 51 %) des unpolareren Alkohols, dem man die Struktur A zuordnete, sowie 16,5 mg (44 µmol, 44 %) des polareren Alkohols, dem man die Strukur B zuordnete.

Beispiel 38a

(4S,5R(5Z),6R(1E),1R)-7-[4-Methyl-6-(3-oxo-1-octenyl)-2-oxabicyclo[2.2.1]heptan-5-yl]-5-heptensäuremethylester:

50 mg (178 µmol) des in Beispiel 38b dargestellten Aldehyds setzte man in Analogie zu Beispiel 23a unter Verwendung von Dimethyl-(2-oxo-heptyl)-phosphonat um und isolierte nach Aufarbeitung und Reinigung 37,6 mg (100 µmol, 56 %) der Titelverbindung als farbloses Öl.

IR (Film): 2950, 2930, 2870, 1735, 1690, 1670, 1620, 1450, 1435, 1240, 1195, 1160, 1020, 980 und 895 $cm^{-1}$.

Beispiel 38b

(4S,5R(5Z),6R,1R)-7-[4-Methyl-6-formyl-2-oxabicyclo[2.2.1]heptan-5-yl]-5-heptensäuremethylester:

Zu der unter einer Atmosphäre aus trockenem Argon auf - 60 °C gekühlten Lösung von 63 µl frisch destilliertem Oxalylchlorid in 1,6 ml wasserfreiem Dichlormethan gab man die Lösung von 110 µl Dimethylsulfoxid in 310 µl Dichlormethan und nach ca. 2 Minuten die Lösung von 168 mg (595 µmol) des in Beispiel 1a dargestellten Alkohols in 630 µl Dichlormethan. Man ließ noch 30 - 40 Minuten reagieren, versetzte mit 440 µl Triethylamin, ließ auf Raumtemperatur erwärmen, verdünnte mit 10 ml Diethylether, wusch mehrfach mit Wasser und gesättigter Natriumchloridlösung und trocknete über Magnesiumsulfat. Nach Filtration und Einengen im Wasserstrahlvakuum isolierte man 155 mg (553 µmol, 93 %) der Titelverbindung als blassgelbes Öl.

IR (Film): 2950, 2930, 2870, 2720, 1735, 1715, 1450, 1435, 1245, 1215, 1195, 1160, 1010, 990, 970 und 895 $cm^{-1}$.

Beispiel 39

(4R,5S(5Z),6S(1E,3S),1S)-7-[4-Phenyl-6-(3-hydroxy-1-octenyl)-2-oxabicyclo[2.2.1]heptan-5-yl]-5-heptensäuremethylester (A) und (4R,5S(5Z),6S(1E,3R),1S)-7-[4-Phenyl-6-(3-hydroxy-1-octenyl)-2-oxabicyclo [2.2.1]heptan-5-yl]-5-heptensäuremethylester (B):

44 mg (100 µmol) des in Beispiel 39a dargestellten ungesättigten Ketons reduzierte man in Analogie zu Beispiel 23 und isolierte nach Aufarbeitung und Reinigung 40 mg (91 µmol, 91 %) eines Gemisches der beiden Titelverbindungen als farbloses Öl.

IR (Film): 3600-3200, 3060, 2950, 2930, 2860, 1730, 1600, 1450, 1435, 1265, 995, 970, 895, 735 und 700 $cm^{-1}$.

Die chromatographische Trennung lieferte 20 mg (45 µmol, 45 %) des unpolareren Alkohols, dem man die Struktur A zuordnete, sowie 17 mg (39 µmol, 39 %) des polareren Alkohols, dem man die Strukur B zuordnete.

Beispiel 39a

(4R,5S(5Z),6S(1E),1S)-7-[4-Phenyl-6-(3-oxo-1-octenyl)-2-oxabicyclo[2.2.1]heptan-5-yl]-5-heptensäuremethylester:

43 mg (126 µmol) des in Beispiel 39b dargestellten Aldehyds setzte man in Analogie zu Beispiel 23a unter Verwendung von Dimethyl-(2-oxo-heptyl)-phosphonat um und isolierte nach Aufarbeitung und Reinigung 44 mg (100 µmol, 80 %) der Titelverbindung als farbloses Öl.

IR (Film): 3060, 3000, 2950, 2930, 2870, 1735, 1690, 1670, 1625, 1600, 1500, 1450, 1435, 1245, 1195, 1170, 1045, 995, 975, 900, 760 und 700 $cm^{-1}$.

Beispiel 39b

(4R,5S(5Z),6S,1S)-7-[4-Phenyl-6-formyl-2-oxabicyclo[2.2.1]heptan-5-yl]-5-heptensäuremethylester:

93 mg (270 µmol) des nach Beispiel 5a dargestellten Alkohols oxidierte man in Analogie zu Beispiel 38b und isolierte nach Aufarbeitung und Reinigung 86 mg (251 µmol, 93 %) der Titelverbindung.

IR (Film): 3060, 3030, 2940, 2870, 2720, 1735, 1715, 1600, 1500, 1450, 1435, 1245, 1165, 1015, 1000, 895, 765 und 700 $cm^{-1}$.

Beispiel 40

(4S,5R(5Z),6R(1E,3R),1R)-7-[4-Benzyl-6-(3-hydroxy-1-octenyl)-2-oxabicyclo[2.2.1]heptan-5-yl]-5-heptensäuremethylester (A) und (4S,5R(5Z),6R(1E,3S),1R)-7-[4-Benzyl-6-(3-hydroxy-1-octenyl)-2-oxabicyclo[2.2.1]heptan-5-yl]-5-heptensäuremethylester (B):

55 mg (121 µmol) des in Beispiel 40a dargestellten ungesättigten Ketons reduzierte man in Analogie zu Beispiel 23 und isolierte nach Aufarbeitung und Reinigung 51 mg (112 µmol, 93 %) eines Gemisches der beiden Titelverbindungen als farbloses Öl.

IR (Film): 3600-3300, 3060, 3030, 2950, 2920, 2860, 1735, 1600, 1450, 1435, 1245, 1150, 1015, 995, 965, 895, 765 und 705 $cm^{-1}$.

Die chromatographische Trennung lieferte 27,7 mg (61 µmol, 50 %) des unpolareren Alkohols, dem man die Struktur A zuordnete, sowie 21,2 mg (47 µmol, 39 %) des polareren Alkohols, dem man die Strukur B zuordnete.

Beispiel 40a

(4S,5R(5Z),6R(1E),1R)-7-[4-Benzyl-6-(3-oxo-1-octenyl)-2-oxabicyclo[2.2.1]heptan-5-yl]-2-heptensäuremethylester:

49 mg (137 µmol) des in Beispiel 40b dargestellten Aldehyds setzte man in Analogie zu Beispiel 23a unter Verwendung von Dimethyl-(2-oxo-heptyl)-phosphonat um und isolierte nach Aufarbeitung und Reinigung 55 mg (121 µmol, 88 %) der Titelverbindung als farbloses Öl.

IR (Film): 3060, 3030, 2950, 2930, 2860, 1735, 1690, 1670, 1620, 1450, 1245, 1160, 995, 900, 765 und 705 $cm^{-1}$.

Beispiel 40b

(4S,5R(5Z),6R,1R)-7-[4-Benzyl-6-formyl-2-oxabicyclo[2.2.1]heptan-5-yl]-5-heptensäuremethylester:

100 mg (279 µmol) des nach Beispiel 7a dargestellten Alkohols oxidierte man in Analogie zu Beispiel 38b und isolierte nach Aufarbeitung und Reinigung 98 mg (275 µmol, 99 %) der Titelverbindung als blassgelbes Öl.

IR (Film): 3060, 2950, 2720, 1735, 1715, 1600, 1450, 1435, 1245, 1160, 1010, 995, 895, 760 und 700 $cm^{-1}$.

Beispiel 41

(4S,5R(5Z),6R(1E,3R),1R)-7-[4-(p-Phenyl-benzyl)-6-(3-hydroxy-1-octenyl)-2-oxabicyclo[2.2.1]heptan-5-yl]-5-heptensäuremethylester (A) und (4S,5R(5Z),6R(1E,3S),1R)-7-[4-(p-Phenyl-benzyl)-6-(3-hydroxy-1-octenyl)-2-oxabicyclo[2.2.1]heptan-5-yl]-5-heptensäuremethylester (B):

44 mg (83 µmol) des in Beispiel 41a dargestellten ungesättigten Ketons reduzierte man in Analogie zu Beispiel 23 und isolierte nach Aufarbeitung und Reinigung 43 mg (81 µmol, 98 %) eines Gemisches der beiden Titelverbindungen als farbloses Öl.

IR (Film): 3600-3200, 3060, 3030, 2930, 2870, 1735, 1600, 1455, 1200, 1110, 1010, 895, 850, 740 und 700 $cm^{-1}$.

Die chromatographische Trennung lieferte 23 mg (43 µmol, 52 %) des unpolareren Alkohols, dem man die Struktur A zuordnete, sowie 19 mg (36 µmol, 43 %) des polareren Alkohols, dem man die Strukur B zuordnete.

Beispiel 41a

(4S,5R(5Z),6R(1E),1R)-7-[4-(p-Phenyl-benzyl)-6-(3-oxo-1-octenyl)-2-oxabicyclo-[2.2.1]heptan-5-yl]-5-heptensäuremethylester:

43 mg (99 µmol) des in Beispiel 41b dargestellten Aldehyds setzte man in Analogie zu Beispiel 23a unter Verwendung von Dimethyl-(2-oxo-heptyl)-phosphonat um und isolierte nach Aufarbeitung und Reinigung 44 mg (83 µmol, 84 %) der Titelverbindung als kristallinen Feststoff.

Smp.: 75 - 76 °C

Beispiel 41b

(4S,5R(5Z),6R,1S)-7-[4-(p-Phenyl-benzyl)-6-formyl-2-oxabicyclo[2.2.1]heptan-5-yl]-5-heptensäuremethylester:

103 mg (237 µmol) des nach Beispiel 9a dargestellten Alkohols oxidierte man in Analogie zu Beispiel 38b und isolierte nach Aufarbeitung und Reinigung 87 mg (201 µmol, 85 %) der Titelverbindung als wachsartigen Feststoff.

IR (Film): 3070, 3050, 3020, 2920, 2860, 2720, 1735, 1715, 1600, 1490, 1440, 1005, 895, 770, 740 und

700 $cm^{-1}$.

Beispiel 42

(4S,5R(5Z),6R(1E,3R),1R)-7-[4-(2-Phenylethyl)-6-(3-hydroxy-1-octenyl)-2-oxabicyclo[2.2.1]heptan-5-yl]-5-heptensäuremethylester (A) und (4S,5R(5Z),6R(1E,3S),1R)-7-[4-(2-Phenylethyl)-6-(3-hydroxy-1-octenyl)-2-oxabicyclo[2.2.1]heptan-5-yl]-5-heptensäuremethylester (B):

33,7 mg (72 µmol) des in Beispiel 42a dargestellten ungesättigten Ketons reduzierte man in Analogie zu Beispiel 23 und isolierte nach Aufarbeitung und Reinigung 30 mg (64 µmol, 89 %) eines Gemisches der beiden Titelverbindungen als farbloses Öl.

IR (Film): 3600-3200, 3080, 3060, 3020, 3000, 2930, 2860, 1735, 1600, 1450, 1435, 1245, 1200, 1155, 1025, 1015, 970, 895, 755 und 700 $cm^{-1}$.

Die chromatographische Trennung lieferte 14 mg (30 µmol, 41 %) des unpolareren Alkohols, dem man die Struktur A zuordnete, sowie 13 mg (28 µmol, 39 %) des polareren Alkohols, dem man die Strukur B zuordnete.

Beispiel 42a

(4S,5R(5Z),6R(1E),1R)-7-[4-(2-Phenylethyl)-6-(3-oxo-1-octenyl)-2-oxabicyclo[2.2.1]heptan-5-yl]-5-heptensäuremethylester:

52 mg (140 µmol) des in Beispiel 27a dargestellten Aldehyds setzte man in Analogie zu Beispiel 23a unter Verwendung von Dimethyl-(2-oxo-heptyl)-phosphonat um und isolierte nach Aufarbeitung und Reinigung 33,7 mg (72 µmol, 52 %) der Titelverbindung als farbloses Öl.

IR (Film): 3080, 3060, 3020, 3000, 2950, 2920, 2860, 1735, 1690, 1670, 1620, 1450, 1435, 1245, 1170, 980, 900, 755 und 700 $cm^{-1}$.

Beispiel 43

(4S,5R(5Z),6R(1E,3R)1R)-7-[4-(p-Fluorphenyl)-6-(3-hydroxy-1-octenyl)-2-oxabicyclo[2.2.1]heptan-5-yl]-5-heptensäuremethylester (A) und (4S,5R(5Z),6R(1E,3S),1R)-7-[4-(p-Fluorphenyl)-6-(3-hydroxy-1-octenyl)-2-oxabicyclo[2.2.1]heptan-5-yl]-5-heptensäuremethylester (B):

52 mg (114 µmol) des in Beispiel 43a dargestellten ungesättigten Ketons redzuzierte man in Analogie zu Beispiel 23 und isolierte nach Aufarbeitung und Reinigung 49 mg (107 µmol, 94 %) eines Gemisches der beiden Titelverbindungen als farbloses Öl.

IR (Film): 3600-3200, 3050, 3000, 2950, 2930, 2860, 1735, 1500, 1510, 1230, 1160, 995, 970, 895, 830 und 735 $cm^{-1}$.

Die chromatographische Trennung lieferte 26 mg (57 µmol, 50 %) des unpolareren Alkohols, dem man die Struktur A zuordnete, sowie 20 mg (44 µmol, 38 %) des polareren Alkohols, dem man die Struktur B zuordnete.

Beispiel 43a

(4S,5R(5Z),6R(1E),1R)-7-[4-(p-Fluorphenyl)-6-(3-oxo-1-octenyl)-2-oxabicyclo[2.2.1]heptan-5-yl]-5-heptensäuremethylester:

46 mg (128 µmol) des in Beispiel 31b dargestellten Aldehyds setzte man in Analogie zu Beispiel 23a unter Verwendung von Dimethyl-(2-oxo-heptyl)-phosphonat um und isolierte nach Aufarbeitung und Reinigung 52 mg (114 µmol, 89 %) der Titelverbindung als farbloses Öl.

IR (Film): 3000, 2950, 2930, 2860, 1735, 1690, 1670, 1620, 1510, 1435, 1230, 1160, 995, 975, 900 und 835 $cm^{-1}$.

Beispiel 44

(4S,5R(5Z),6R(1E,3R),1R)-7-[4-Methyl-6-(3-hydroxy-1-octenyl)-2-oxabicyclo[2.2.1]heptan-5-yl]-5-heptensäure:

17,5 mg (46 µmol) des in Beispiel 38 dargestellten unpolareren Esters verseifte man in Analogie zu Beispiel 2 und isolierte nach Aufarbeitung und Reinigung 15,7 mg (43 µmol, 94 %) der Titelverbindung als farbloses Öl.

IR (Film): 3600-2400, 2970, 2930, 2870, 1710, 1460, 1240, 1025, 970 und 890 $cm^{-1}$.

Beispiel 45

(4R,5S(5Z),6S(1E,3S),1S)-7-[4-Phenyl-6-(3-hydroxy-1-octenyl)-2-oxabicyclo[2.2.1]heptan-5-yl]-5-heptensäure:

20 mg (45 µmol) des in Beispiel39 dargestellten unpolareren Esters verseifte man in Analogie zu Beispiel 2 und isolierte nach Aufarbeitung und Reinigung 19 mg (45 µmol, 98 %) der Titelverbindung als farbloses Öl.

IR (Film): 3600-2300, 3080, 3060, 3030, 3000, 2930, 2870, 1710, 1605, 1500, 1450, 1240, 1195, 1045, 995, 970, 890, 760 und 700 $cm^{-1}$.

Beispiel 46

(4S,5R(5Z),6R(1E,3R),1R)-7-[4-Benzyl-6-(3-hydroxy-1-octenyl)-2-oxabicyclo[2.2.1]heptan-5-yl]-5-heptensäure:

27,7 mg (61 µmol) des in Beispiel 40 dargestellten unpolareren Esters verseifte man in Analogie zu Beispiel 2 und isolierte nach Aufarbeitung und Reinigung 26 mg (59 µmol, 97 %) der Titelverbindung als farbloses Öl.

IR (Film): 3600-2400, 3090, 3070, 3030, 2940, 2870, 1710, 1605, 1455, 1245, 995, 970, 895, 765 und 705 $cm^{-1}$.

Beispiel 47

(4S,5R(5Z),6R(1E,3R),1R)-7-[4-(p-Phenyl-benzyl)-6-(3-hydroxy-2-octenyl)-2-oxabicyclo[2.2.1]heptan-5-yl]-5-heptensäure:

23 mg (43 µmol) des in Beispiel 41 dargestellten unpolareren Esters verseifte man in Analogie zu Beispiel 2 und isolierte nach Aufarbeitung und Reinigung 19,7 mg (38 µmol, 89 %) der Titelverbindung als kristallinen Feststoff.

IR (KBr): 3600-2200, 3010, 2930, 2870, 2850, 1690, 1600, 1490, 1265, 1200, 890, 765, 735 und 690 $cm^{-1}$.

Beispiel 48

(4S,5R(5Z),6R(1E,3R),1R)-7-[4-(2-Phenylethyl)-6-(3-hydroxy-1-octenyl)-2-oxabicyclo[2.2.1]heptan-5-yl]-5-heptensäure:

14 mg (30 µmol) des in Beispiel 42 dargestellten unpolareren Esters verseifte man in Analogie zu Beispiel 2 und isolierte nach Aufarbeitung und Reinigung 13 mg (29 µmol, 95 %) der Titelverbindung als farbloses Öl.

IR (Film): 3600-2400, 3080, 3060, 3020, 3000, 2930, 2860, 1725, 1710, 1605, 1455, 1240, 1030, 1115, 970, 890, 755 und 700 $cm^{-1}$.

Beispiel 49

(4S,5R(5Z),6R(1E,3R),1R)-7-[4-(p-Fluorphenyl)-6-(3-hydroxy-1-octenyl)-2-oxabicyclo[2.2.1]heptan-5-yl]-5-heptensäure:

26 mg (57 µmol) des in Beispiel 43 dargestellten unpolareren Esters verseifte man in Analogie zu Beispiel 2 und isolierte nach Aufarbeitung und Reinigung 21 mg (47 µmol, 83 %) der Titelverbindung als farbloses Öl.

IR (Film): 3600-2400, 3060, 3040, 3000, 2950, 2930, 2860, 1710, 1610, 1515, 1235, 1160, 995, 970, 895, 835 und 585 $cm^{-1}$.

Beispiel 50

(4S,5R(5Z),6R(1E,3S),1R)-7-[4-Methyl-6-(3-hydroxy-1-octenyl)-2-oxabicyclo[2.2.1]heptan-5-yl]-5-heptensäure:

14,7 mg (39 µmol) des in Beispiel 38 dargestellten polareren Esters verseifte man in Analogie zu Beispiel 2 und isolierte nach Aufarbeitung und Reinigung 11,8 mg (32 µmol, 83 %) der Titelverbindung als farbloses Öl.

IR (Film): 3600-2400, 2960, 2930, 2870, 1730, 1710, 1460, 1240, 1025, 970 und 890 $cm^{-1}$.

Beispiel 51

(4R,5S(5Z),6S(1E,3R),1S)-7-[4-Phenyl-6-(3-hydroxy-1-octenyl)-2-oxabicyclo[2.2.1]heptan-5-yl]-5-heptensäure:

17 mg (39 µmol) des in Beispiel 39 dargestellen unpolareren Esters verseifte man in Analogie zu Beispiel 2 und isolierte nach Aufarbeitung und Reinigung 16 mg (38 µmol, 96 %) der Titelverbindung als farbloses Öl.

IR (Film): 3600-2400, 3080, 3060, 3030, 3000, 2950, 2930, 2860, 1710, 1605, 1500, 1450, 1295, 1240, 1195, 1045, 995, 970, 890, 760 und 700 $cm^{-1}$.

Beispiel 52

(4S,5R(5Z),6R(1E,3S),1R)-7-[4-Benzyl-6-(3-hydroxy-1-octenyl)-2-oxabicyclo[2.2.1]heptan-5-yl]-5-heptensäure:

21,2 mg (47 µmol) des in Beispiel 40 dargestellten polareren Esters verseifte man in Analogie zu Beispiel 2 und isolierte nach Aufarbeitung und Reinigung 19 mg (43 µmol, 92 %) der Titelverbindung als farbloses Öl.

IR (Film): 3600-2400, 3090, 3070, 3030, 2930, 2870, 1730, 1710, 1605, 1455, 1245, 995, 970, 890, 765 und 705 $cm^{-1}$.

Beispiel 53

(4S,5R(5Z),6R(1E,3S),1R)-7-[4-(p-Phenyl-benzyl)-6-(3-hydroxy-1-octenyl)-2-oxabicyclo[2.2.1]heptan-5-yl]-5-heptensäure:

19 mg (36 µmol) des in Beispiel 41 dargestellten polareren Esters verseifte man in Analogie zu Beispiel 2 und isolierte nach Aufarbeitung und Reinigung 14,6 mg (28 µmol, 78 %) der Titelverbindung als kristallinen Feststoff.

IR (KBr): 3600-2200, 3010, 2960, 2930, 2880, 2850, 1695, 1600, 1490, 1460, 1250, 1135, 1055, 1005, 970, 890, 770, 735 und 695 $cm^{-1}$.

Beispiel 54

(4S,5R(5Z),6R(1E,3S),1R)-7-[4-(2-Phenylethyl)-6-(3-hydroxy-1-octenyl)-2-oxabiciclo[2.2.1]heptan-5-yl]-5-heptensäure:

13 mg (28 µmol) des in Beispiel 42 dargestellten polareren Esters verseifte man in Analogie zu Beispiel 2 und isolierte nach Aufarbeitung und Reinigung 11 mg (24 µmol, 86%) der Titelverbindung als farbloses Öl.

IR (Film): 3600-2400, 3080, 3060, 3030, 3000, 2930, 2860, 1725, 1710, 1605, 1455, 1260, 1240, 1030, 970, 890, 755 und 700 $cm^{-1}$.

Beispiel 55

(4S,5R(5Z),6R(1E,3S),1R)-7-[4-(p-Fluorphenyl)-6-(3-hydroxy-1-octenyl)-2-oxabicyclo[2.2.1]heptan-5-yl]-5-heptensäure:

20 mg (44 µmol) des in Beispiel 43 dargestellten polaren Esters verseifte man in Analogie zu Beispiel 2 und isolierte nach Aufarbeitung und Reinigung 18 mg (40 µmol, 92 %) der Titelverbindung als farbloses Öl.

IR (Film): 3600-2500, 3040, 3000, 2950, 2930, 2860, 1710, 1610, 1515, 1235, 1160, 995, 970, 895, 835 und 585 $cm^{-1}$.

Beispiel 56

(4S,5R(5Z),6R(1E,3R,4RS),1R)-7-[4-Methyl-6-(3-hydroxy-4-methyl-6-in-1-octenyl)-2-oxabicyclo[2.2.1]heptan-5-yl]-5-heptensäuremethylester (A) und (4S,5R(5Z),6R (1E,3S,4RS),1R)-7-[4-Methyl-6-(3-hydroxy-4-methyl-6-in-1-octenyl)-2-oxabicyclo[2.2.1]heptan-5-yl]-5-heptensäuremethylester (B):

53 mg (137 µmol) des in Beispiel 56a dargestellten ungesättigten Ketons reduzierte man in Analogie zu Beispiel 23 und isolierte nach Aufarbeitung und Reinigung 52 mg (134 µmol, 98 %) eines Gemisches der beiden Titelverbindung als farbloses Öl.

IR (Film): 3600-3200, 2950, 2920, 2870, 1735, 1450, 1435, 1245, 1160, 1020, 970 und 890 $cm^{-1}$.

Die chromatographische Trennung lieferte 28 mg (72 µmol, 53 %) des unpolareren Alkohols, dem man die Struktur A zuordnete, sowie 22 mg (57 µmol, 41 %) des polareren Alkohols, dem man die Strukur B zuordnete.

Beispiel 56a

(4S,5R(5Z),6R(1E,4RS),1R)-7-[4-Methyl-6-(3-oxo-4-methyl-6-in-1-octenyl)-2-oxabicyclo[2.2.1]heptan-5-yl]-5-heptensäuremethylester:

50 mg (178 μmol) des in Beispiel 38b dargestellten Aldehyds setzte man in Analogie zu Beispiel 23a unter Verwendung von racemischen Dimethyl-(2-oxo-3-methylhept-5-inyl)-phosphonat um und isolierte nach Aufarbeitung und Reinigung 53 mg (137 μmol, 77 %) der Titelverbindung als farbloses Öl.

IR (Film): 2950, 2930, 2870, 1735, 1690, 1665, 1620, 1450, 1435, 1370, 1215, 1195, 1160, 1025, 975 und 895 $cm^{-1}$.

Beispiel 57

(4S,5R(5Z),6R(1E,3R,4RS),1R)-7-[4-(p-Phenyl-benzyl)-6-(3-hydroxy-4-methyl-6-in-1-octenyl)-2-oxabicyclo[2.2.1]heptan-5-yl]-5-heptensäuremethylester (A) und (4S,5R(5Z),6R(1E,3S,4RS),1R)-7-[4-(p-Phenyl-benzyl)-6-(3-hydroxy-4-methyl-6-in-1-octenyl)-2-oxabicyclo[2.2.1]heptan-5-yl]-5-heptensäuremethylester (B):

44 mg (82 μmol) des in Beispiel 57a dargestellten ungesättigten Ketons reduzierte man in Analogie zu Beispiel 23 und isolierte nach Aufarbeitung und Reinigung 43 mg (80 μmol, 97 %) eines Gemisches der beiden Titelverbindungen.

IR (KBr): 3600-3200, 3010, 2960, 2930, 2870, 1735, 1600, 1490, 1245, 1120, 970, 895, 770, 735 und 695 $cm^{-1}$.

Die chromatographische Trennung lieferte 24,6 mg (45 μmol, 55 %) des unpolareren Alkohols, dem man die Struktur A zuordnete, sowie 17 mg (31 μmol, 38 %) des polareren Alkohols, dem man die Strukur B zuordnete.

Beispiel 57a

(4S,5R(5Z),6R(1E,4RS),1R)-7-[4-(p-Phenyl-benzyl)-6-(3-oxo-4-methyl-6-in-1-octenyl)-2-oxabicyclo[2.2.1]heptan-5-yl]-5-heptensäuremethylester:

43 mg (99 μmol) des in Beispiel 41b dargestellten Aldehyds setzte man in Analogie zu Beispiel 23a unter Verwendung von racemischem Dimethyl-(2-oxo-3-methylhept-5-inyl)-phosphonat um und isolierte nach Aufarbeitung und Reinigung 45 mg (84 μmol, 84 %) der Titelverbindung als farbloses Öl.

Smp.: 82 - 83 °C

Beispiel 58

(4S,5R(5Z),6R(1E,3R,4RS),1R)-7-[4-Methyl-6-(3-hydroxy-4-methyl-6-in-1-octenyl)-2-oxabicyclo[2.2.1]heptan-5-yl]-5-heptensäure:

28 mg (72 μmol) des in Beispiel 56 dargestellten unpolareren Esters verseifte man in Analogie zu Beispiel 2 und isolierte nach Aufarbeitung und Reinigung 23,6 mg (63 μmol, 88 %) der Titelverbindung als farbloses Öl.

IR (Film): 3600-2400, 2960, 2930, 2870, 1730, 1710, 1455, 1240, 1020, 970 und 890 $cm^{-1}$.

Beispiel 59

(4S,5R(5Z),6R(1E,3R,4RS),1R)-7-[4-(p-Phenyl-benzyl)-6-(3-hydroxy-4-methyl-6-in-1-octenyl)-2-oxabicyclo[2.2.1]heptan-5-yl]-5-heptensäure:

24,6 mg (45 μmol) des in Beispiel 57 dargestellten unpolareren Esters verseifte man in Analogie zu Beispiel 2 und isolierte nach Aufarbeitung und Reinigung 20 mg (38 μmol, 84 %) der Titelverbindung als kristallinen Feststoff.

IR (KBr): 3600-2200, 3080, 3060, 3030, 2970, 2920, 1730, 1710, 1605, 1490, 1450, 1410, 1245, 1010, 995, 970, 890, 850, 770, 740 und 700 $cm^{-1}$.

Beispiel 60

(4S,5R(5Z),6R(1E,3S,4RS),1R)-7-[4-Methyl-6-(3-hydroxy-4-methyl-6-in-1-octenyl)-2-oxabicyclo[2.2.1]heptan-5-yl]-5-heptensäure:

22 mg (57 μmol) des in Beispiel 56 dargestellten polareren Esters verseifte man in Analogie zu Beispiel

2 und isolierte nach Aufarbeitung und Reinigung 20 mg (53 μmol, 94 %) der Titelverbindung als farbloses Öl.
IR (Film): 3600-2400, 2960, 2930, 2870, 1730, 1710, 1455, 1240, 1020, 970 und 885 $cm^{-1}$.

Beispiel 61

(4S,5R(5Z),6R(1E,3S,4RS),1R)-7-[4-(p-Phenyl-benzyl)-6-(3-hydroxy-4-methyl-6-in-1-octenyl)-2-oxabicyclo[2.2.1]heptan-5-yl]-5-heptensäure:
17 mg (31 μmol) des in Beispiel 57 dargestellten polareren Esters verseifte man in Analogie zu Beispiel 2 und isolierte nach Aufarbeitung und Reinigung 12,4 mg (24 μmol, 76 %) der Titelverbindung als kristallinen Feststoff.
IR (KBr): 3600-2200, 3080, 3060, 3030, 2960, 2920, 1725, 1705, 1600, 1490, 1450, 1410, 1295, 1010, 995, 920, 890, 845, 765, 740 und 700 $cm^{-1}$.

Beispiel 62

(4R,5S(5Z),6R,1S)-7-[4-Phenyl-6-(N-morpholinomethyl)-2-oxabicyclo[2.2.1]heptan-5-yl]-5-heptensäuremethylester:
22 mg (54 μmol) des in Beispiel 62a dargestellten Bromids setzte man in Analogie zu Beispiel 63 um und isolierte nach Aufarbeitung und Reinigung 15 mg (36 μmol, 67 %) der Titelverbindung als farbloses Öl.
IR (Film): 3080, 3060, 3030, 3000, 2950, 2870, 2810, 1735, 1670, 1600, 1440, 1225, 995, 895, 865 und 695 $cm^{-1}$.

Beispiel 62a

(4R,5S(5Z),6S,1S)-7-[4-Phenyl-6-brommethyl-2-oxabicyclo[2.2.1]heptan-5-yl]-5-heptensäuremethylester:
100 mg (290 μmol) des in Beispiel 5a dargestellten Alkohols bromierte man in Analogie zu Beispiel 63a und isolierte nach Aufarbeitung und Reinigung 116 mg (285 μmol, 98 %) der Titelverbindung als farbloses Öl.
IR (Film): 3080, 3060, 3030, 3000, 2950, 2930, 2870, 1735, 1600, 1450, 1435, 1225, 1165, 995, 895, 765, 740, 705 und 640 $cm^{-1}$.

Beispiel 63

(4S,5R(5Z),6S,1R)-7-[4-Benzyl-6-(N-morpholinomethyl)-2-oxabicyclo[2.2.1]heptan-5-yl]-5-heptensäuremethylester:
17 mg (40 μmol) des nach Beispiel 63a dargestellten Bromids versetzte man mit 205 μl Morpholin und erwärmte unter einer Atmosphäre aus trockenem Argon 3 Stunden auf 100 °C. Nach dem Erkalten verdünnte man mit 10 ml Diethylether, wusch mehrmals mit Wasser, trocknete die organische Phase über Magnesiumsulfat, filtrierte und zog das Lösungsmittel in Wasserstrahlvakuum ab. Isoliert wurden 14,2 mg (33 μmol, 83 %) der Titelverbindung als farbloses Öl.
IR (Film): 3060, 3030, 3000, 2950, 2930, 2870, 2815, 1730, 1670, 1605, 1440, 1225, 1000, 895, 865 und 695 $cm^{-1}$.

Beispiel 63a

(4S,5R(5Z),6R,1R)-7-[4-Benzyl-6-brommethyl-2-oxabicyclo[2.2.1]heptan-5-yl]-5-heptensäuremethylester:
Die Lösung von 66 mg (184 μmol) des nach Beispiel 7a dargestellten Alkohols löste man in 1,4 ml wasserfreiem Acetonitril, versetzte mit 305 mg Tetrabrommethan, 120 μl Kollidin, 241 mg Triphenylphosphin und rührte 9 Stunden bei 23 °C unter einer Atmosphäre aus trockenem Argon. Man versetzte mit 8 ml Diethylether, wusch mit eiskaltem Wasser, trocknete die organische Phase über Magnesiumsulfat, filtrierte und engte im Wasserstrahlvakuum zur Trockne ein. Den Rückstand chromatographierte man an ca. 35 ml Kieselgel. Als Laufmittel diente ein Gradientensystem aus n-Hexan/Ethylacetat. Isoliert wurden 77 mg (183 μmol, 99 %) der Titelverbindung als farbloses Öl.
IR (Film): 3080, 3060, 3020, 3000, 2950, 2930, 2860, 1735, 1605, 1450, 1435, 1220, 1165, 1015, 995, 895, 765, 735, 705 und 635 $cm^{-1}$.

Beispiel 64

(4S,5R(5Z),6S,1R)-7-[4-Phenylacetylen-6-(N-morpholinomethyl)-2-oxabicyclo[2.2.1]heptan-5-yl]-5-hep-

tensäuremethylester:

16 mg (37 μmol) des in Beispiel 64a dargestellten Bromids setzte man in Analogie zu Beispiel 63 um und isolierte nach Aufarbeitung und Reinigung 14,5 mg (33 μmol, 90 %) der Titelverbindung als farbloses Öl.

IR (Film): 3060, 3030, 3000, 2950, 2860, 2810, 2230, 1730, 1670, 1600, 1440, 1220, 1110, 1000, 890, 865 und 690 $cm^{-1}$.

Beispiel 64a

(4S,5R(5Z),6R,1R)-7-[4-Phenylacetylen-6-brommethyl-2-oxabicyclo[2.2.1]heptan-5-yl]-5-heptensäuremethylester:

25 mg (68 μmol) des in Beispiel 13a dargestellten Alkohols setzte man in Analogie zu Beispiel 63a um und isolierte nach Aufarbeitung und Reinigung 16,3 mg (38 μmol, 56 %) der Titelverbindung als farbloses Öl.

IR ($CHCl_3$): 3080, 3060, 3020, 3000, 2950, 2880, 2230, 1725, 1600, 1490, 1435, 1300, 1220, 1165, 995, 895, 690 und 645 $cm^{-1}$.

Beispiel 65

(4S,5R(5Z),6S,1R)-7-[4-(2-Phenylethyl)-6-(N-morpholinomethyl)-2-oxabicyclo[2.2.1]heptan-5-yl]-5-heptensäuremethylester:

22 mg (51 μmol) des in Beispiel 65a dargestellten Bromids setzte man in Analogie zu Beispiel 63 um und isolierte nach Aufarbeitung und Reinigung 20,6 mg (47 μmol, 91 %) der Titelverbindung als farbloses Öl.

IR (Film): 3080, 3060, 3030, 3000, 2950, 2870, 2810, 1735, 1665, 1600, 1440, 1220, 995, 895, 865 und 700 $cm^{-1}$.

Beispiel 65a

(4S,5R(5Z),6R,1R)-7-[4-(2-Phenylethyl)-6-brommethyl-2-oxabicyclo[2.2.1]heptan-5-yl]-5-heptensäuremethylester:

98 mg (263 μmol) des in Beispiel 15a dargestellten Alkohols bromierte man in Analogie zu Beispiel 63a und isolierte nach Aufarbeitung und Reinigung 101 mg (232 μmol, 88 %) der Titelverbindung als farbloses Öl.

IR (Film): 3080, 3060, 3030, 3000, 2950, 2930, 2860, 1735, 1605, 1450, 1435, 1245, 1220, 1165, 1025, 980, 900, 750, 700 und 635 $cm^{-1}$.

Beispiel 66

(4R,5S(5Z),6R,1S)-7-[4-Phenyl-6-(N-morpholinomethyl)-2-oxabicyclo[2.2.1]heptan-5-yl]-5-heptensäure:

15 mg (36 μmol) des in Beispiel 62 dargestellten Esters verseifte man in Analogie zu Beispiel 2 und isolierte nach Aufarbeitung und Reinigung 14 mg (35 μmol, 97 %) der Titelverbindung als farblosen Feststoff.

IR (KBr): 3600-3200, 3080, 3060, 3030, 3000, 2950, 2920, 2870, 2700-2300, 2100-1850, 1710, 1600, 1500, 1460, 1445, 1200, 1115, 1025, 1005, 900, 870, 760 und 700 $cm^{-1}$.

Beispiel 67

(4S,5R(5Z),6S,1R)-7-[4-Benzyl-6-(N-morpholinomethyl)-2-oxabicyclo[2.2.1]heptan-5-yl]-5-heptensäure:

14,2 mg (33 μmol) des in Beispiel 63 dargestellten Esters verseifte man in Analogie zu Beispiel 2 und isolierte nach Aufarbeitung und Reinigung 10 mg (24 μmol, 73 %) der Titelverbindung als farbloses Öl.

IR (Film): 3600-2300, 3080, 3060, 3030, 3000, 2930, 2870, 2810, 1720, 1605, 1495, 1455, 1265, 1245, 1120, 1005, 895, 870, 765 und 705 $cm^{-1}$.

Beispiel 68

(4S,5R(5Z),6S,1R)-7-[4-Phenylacetylen-6-(N-morpholinomethyl)-2-oxabicyclo[2.2.1]heptan-5-yl]-5-heptensäure:

14,5 mg (33 μmol) des in Beispiel 64 dargestellten Esters verseifte man in Analogie zu Beispiel 2 und isolierte nach Aufarbeitung und Reinigung 11,5 mg (27 μmol, 82 %) der Titelverbindung als farbloses Öl.

IR (Film): 3600-2400, 3080, 3060, 2930, 2880, 2860, 2810, 2240, 1720, 1600, 1490, 1460, 1445, 1140, 1120, 1005, 895, 870 und 695 $cm^{-1}$.

Beispiel 69

(4S,5R(5Z),6S,1R)-7-[4-(2-Phenylethyl)-6-(N-morpholinomethyl)-2-oxabicyclo[2.2.1]heptan-5-yl]-5-heptensäure:

20,6 mg (47 μmol) der in Beispiel 65 dargestellten Verbindung verseifte man in Analogie zu Beispiel 2 und isolierte nach Aufarbeitung und Reinigung 16,5 mg (39 μmol, 82 %) der Titelverbindung als kristallinen Stoff.

IR (KBr): 3600-2300, 3090, 3060, 3010, 2930, 2860, 1710, 1605, 1500, 1455, 1230, 1120, 1010, 890, 870 und 705 $cm^{-1}$.

Beispiel 70

(4R,5S(5Z),6R,1S)-7-[4-Phenyl-6-(1-imidazoylmethyl)-2-oxabicyclo[2.2.1]heptan-5-yl]-5-heptensäuremethylester:

26 mg (64 μmol) des in Beispiel 62a dargestellten Bromids setzte man in Analogie zu Beispiel 63 um und isolierte nach Aufarbeitung und Reinigung 15 mg (38 μmol, 59 %) der Titelverbindung als farbloses Öl.

IR (Film): 3110, 3060, 3020, 3000, 2950, 2880, 1735, 1600, 1510, 1450, 1300, 1235, 1110, 1080, 1005, 900, 750, 700 und 665 $cm^{-1}$.

Beispiel 71

(4S,5R(5Z),6S,1R)-7-[4-Benzyl-6-(1-imidazoylmethyl)-2-oxabicyclo[2.2.1]heptan-5-yl]-5-heptensäuremethylester:

15 mg (36 μmol) des in Beispiel 63a dargestellten Bromids setzte man in Analogie zu Beispiel 63 unter Verwendung von Imidazol um und isolierte nach Aufarbeitung und Reinigung 9,2 mg (23 μmol, 64 %) der Titelverbindung als farbloses Öl.

IR (Film): 3115, 3060, 3030, 3000, 2940, 2870, 1735, 1605, 1510, 1455, 1235, 1110, 1085, 1000, 900, 760, 705 und 660 $cm^{-1}$.

Beispiel 72

(4S,5R(5Z),6S,1R)-7-[4-(2-Phenylethyl)-6-(1-imidazoylmethyl)-2-oxabicyclo[2.2.1]heptan-5-yl]-5-heptensäuremethylester:

19 mg (44 μmol) des in Beispiel 65a dargestellten Bromids setzte man in Analogie zu Beispiel 63 unter Verwendung von Imidazol um und isolierte nach Aufarbeitung und Reinigung 18 mg (43 μmol, 97 %) der Titelverbindung als farbloses Öl.

IR (Film): 3110, 3080, 3060, 3030, 2970, 2930, 2870, 1735, 1605, 1455, 1320, 1295, 1235, 1110, 1075, 1010, 895, 750, 700 und 660 $cm^{-1}$.

Beispiel 73

(4R,5S(5Z),6R,1S)-7-[4-Phenyl-6-(1-imidazoylmethyl)-2-oxabicyclo[2.2.1]heptan-5-yl]-5-heptensäure:

15 mg (38 μmol) des in Beispiel 70 dargestellten Esters verseifte man in Analogie zu Beispiel 2 und isolierte nach Aufarbeitung und Reinigung 11 mg (29 μmol, 76 %) der Titelverbindung als farblosen Feststoff.

IR (KBr): 3600-3200, 3110, 3060, 3020, 3000, 2940, 2880, 2700-2200, 2100-1800, 1700,1600,1500, 1445, 1315, 1290, 1235, 1110, 1085, 1025, 1005, 900, 760, 750, 700 und 665 $cm^{-1}$.

Beispiel 74

(4S,5R(5Z),6S,1R)-7-[4-Benzyl-6-(1-imidazoylmethyl)-2-oxabicyclo[2.2.1]heptan-5-yl]-5-heptensäure:

9,2 mg (23 μmol) des in Beispiel 71 dargestellten Esters verseifte man in Analogie zu Beispiel 2 und isolierte nach Aufarbeitung und Reinigung 6,5 mg (16 μmol, 70 %) der Titelverbindung als farbloses Öl.

IR (Film): 3600-2300, 3120, 3060, 3030, 3000, 2930, 2860, 1710, 1605, 1510, 1455, 1235, 1110, 1085, 1005, 900, 760, 705 und 660 $cm^{-1}$.

Beispiel 75

(4S,5R(5Z),6S,1R)-7-[4-(2-Phenylethyl)-6-(1-imidazoylmethyl)-2-oxabicyclo[2.2.1]heptan-5-yl]-5-hepten-

säure:

18 mg (43 μmol) der in Beispiel 72 dargestellten Verbindung verseifte man in Analogie zu Beispiel 2 und isolierte nach Aufarbeitung und Reinigung 13,3 mg (33 μmol, 76 %) der Titelverbindung als farbloses Öl.

IR (KBr): 3600-3000, 3100, 3080, 3060, 3020, 2970, 2920, 2870, 2700-2200, 2100-1800, 1700, 1600, 1455, 1320, 1290, 1255, 1235, 1110, 1080, 1030, 1010, 890, 750, 700, 660 und 630 $cm^{-1}$.

Beispiel 76

(4S,5R(5Z),6R(1E,3S,4S),1R)-7-[4-Benzyl-6-(3-hydroxy-4-methyl-6-in-1-nonenyl)-2-oxabicyclo[2.2.1]heptan-5-yl]-5-heptensäuremethylester (A) und (4S,5R(5Z),6R(1E,3R,4S),1R)-7-[4-Benzyl-6-(3-hydroxy-4-methyl-6-in-1-nonenyl)-2-oxabicyclo[2.2.1]heptan-5-yl]-5-heptensäuremethylester (B):

53 mg (111 μmol) des in Beispiel 76a dargestellten ungesättigten Ketons reduzierte man in Analogie zu Beispiel 23 und isolierte nach Aufarbeitung und Reinigung 51 mg (107 μmol, 96 %) eines Gemisches der beiden Titelverbindung als farbloses Öl.

IR (Film): 3600-3200, 3080, 3060, 3030, 2970, 2920, 2870, 1735, 1600, 1450, 1435, 1245, 1155, 1015, 995, 970, 895, 765 und 705 $cm^{-1}$.

Die chromatographische Trennung lieferte 33 mg (69 μmol, 62 %) des unpolareren Alkohols, dem man die Struktur A zuordnete, sowie 15,5 mg (32 μmol, 29 %) des polareren Alkohols, dem man die Strukur B zuordnete.

Beispiel 76a

(4S,5R(5Z),6R(1E,4S),1R)-7-[4-Benzyl-6-(3-oxo-4-methyl-6-in-1-nonenyl)-2-oxabicyclo[2.2.1]heptan-5-yl]-5-heptensäuremethylester:

49 mg (137 μmol) des in Beispiel 40b dargestellten Aldehyds setzte man in Analogie zu Beispiel 23a unter Verwendung von Dimethyl-(2-oxo-3S-methyl-oct-5-inyl)-phosphonat um und isolierte nach Aufarbeitung und Reinigung 53 mg (111 μmol, 81 %) der Titelverbindung als farbloses Öl.

IR (Film): 3080, 3060, 3030, 2970, 2930, 2870, 1735, 1690, 1665, 1620, 1450, 1435, 1245, 1165, 995, 895, 765 und 705 $cm^{-1}$.

Beispiel 77

(4S,5R(5Z),6R(1E,3R,4S),1R)-7-[4-Benzyl-6-(3-hydroxy-4-methyl-6-in-1-nonenyl)-2-oxabicyclo[2.2.1]heptan-5-yl]-5-heptensäure:

33 mg (69 μmol) des in Beispiel 76 dargestellten unpolareren Esters verseifte man in Analogie zu Beispiel 2 und isolierte nach Aufarbeitung und Reinigung 32 mg (68 μmol, 99 %) der Titelverbindung als farbloses Öl.

IR (Film): 3600-2400, 3090, 3060, 3030, 2980, 2930, 2880, 1710, 1605, 1495, 1455, 1245, 995, 970, 890, 765 und 705 $cm^{-1}$.

Beispiel 78

(4S,5R(5Z),6R(1E,3S,4S),1R)-7-[4-Benzyl-6-(3-hydroxy-4-methyl-6-in-1-nonenyl)-2-oxabicyclo[2.2.1]heptan-5-yl]-5-heptensäure:

15,5 mg (32 μmol) des in Beispiel 76 dargestellten polareren Esters verseifte man in Analogie zu Beispiel 2 und isolierte nach Aufarbeitung und Reinigung 14 mg (30 μmol, 94 %) der Titelverbindung als farbloses Öl.

IR (Film): 3600-2400, 3090, 3070, 3030, 2980, 2940, 2880, 1730, 1710, 1670, 1605, 1495, 1455, 1250, 1100, 1015, 995, 970, 890, 765 und 705 $cm^{-1}$.

Beispiel 79

(4S,5R(5Z),6S,1R)-7-[4-(2-Phenylethyl)-6-((2-phenylamino-carbonyl)-hydroazino)-methyl)-2-oxabicyclo[2.2.1]heptan-5-yl]-5-heptensäuremethylester:

17,4 mg (47 μmol) des in Beispiel 27a dargestellten Aldehyds setzte man in Analogie zu Beispiel 26 unter Verwendung von 4-Phenylsemicarbazid um und isolierte nach Aufarbeitung und Reinigung 21,3 mg (42 μmol, 90 %) der Titelverbindung als farbloses Öl.

IR (Film): 3370, 3200, 3140-3040, 3030, 2940, 2860, 1730, 1690, 1595, 1530, 1445, 1230, 1030, 980, 895, 755, 735 und 695 $cm^{-1}$.

Beispiel 80

(4S,5R(5Z),6S,1R)-7-[4-(2-Phenylethyl)-6-((2-(phenylamino-carbonyl)-hydrazino)-methyl)-2-oxabicyclo [2.2.1]heptan-5-yl]-5-heptensäure:

21,3 mg (47 μmol) des in Beispiel 79 dargestellten Esters verseifte man in Analogie zu Beispiel 2 und isolierte nach Aufarbeitung und Reinigung 18 mg (37 μmol, 78 %) der Titelverbindung als farbloses Öl.

IR (Film): 3500-2300, 3380, 3200, 3090, 3060, 3030, 3010, 2940, 2870, 1700, 1660, 1595, 1540, 1500, 1450, 1235, 900, 755 und 695 $cm^{-1}$.

Beispiel 81

(4R,5S(5Z),6R,1S)-7-[4-Phenyl-6-(1-pyrrolidinomethyl)-2-oxabicyclo[2.2.1]heptan-5-yl]-5-heptensäurepyrrolidinamid:

22 mg (54 μmol) des in Beispiel 62a dargestellten Bromids setzte man in Analogie zu Beispiel 63 unter Verwendung von Pyrrolidin um und isolierte nach Aufarbeitung und Reinigung 19 mg (44 μmol, 81 %) der Titelverbindung als farbloses Öl.

IR (Film): 3060, 3030, 2950, 2880, 2600, 1630, 1450, 1260, 1005, 895, 765, 705, 665, 620 und 450 $cm^{-1}$.

Beispiel 82

(4S,5R(5Z),6S,1R)-7-[4-(2-Phenylethyl)-6-(N-pyrrolidinomethyl)-2-oxabicyclo[2.2.1]heptan-5-yl]-5-heptensäurepyrrolidinamid:

18 mg (41 μmol) des in Beispiel 65a dargestellten Bromids setzte man in Analogie zu Beispiel 63 unter Verwendung von Pyrrolidin um und isolierte nach Aufarbeitung und Reinigung 15 mg (32 μmol, 78 %) der Titelverbindung als farbloses Öl.

IR (Film): 3080, 3060, 3030, 2930, 2870, 2780, 1640, 1605, 1435, 895, 755 und 700 $cm^{-1}$.

Beispiel 83

(4R,5S(5Z),6S(1E,3S),1S)-7-[4-Phenyl-6-(3-hydroxy-4-(p-fluorphenoxy)-1-butenyl)-2-oxabicyclo[2.2.1] heptan-5-yl]-5-heptensäuremethylester (A) und (4R,5S(5Z),6S(1E,3R),1S)-7-[4-Phenyl-6-(3-hydroxy-4-(p-fluorphenoxy)-1-butenyl)-2-oxabicyclo[2.2.1]heptan-5-yl]-5-heptensäuremethylester (B):

21 mg (43 μmol des in Beispiel 83a dargestellten ungesättigten Ketons reduzierte man in Analogie zu Beispiel 23 und isolierte nach Aufarbeitung und Reinigung 19 mg (38 μmol, 89 %) eines Gemisches der beiden Titelverbindungen als farbloses Öl.

IR (Film): 3600-3200, 3080, 3060, 3030, 3010, 2930, 2870, 1735, 1600, 1505, 1250, 1210, 1040, 1030, 895, 830, 760 und 700 $cm^{-1}$.

Die chromatographische Trennung lieferte 7,5 mg (15 μmol, 36 %) des unpolareren Alkohols, dem man die Struktur A zuordnete, sowie 9 mg (18 μmol, 43 %) des polareren Alkohols, dem man die Struktur B zuordnete.

Beispiel 83 a

(4R,5S(5Z),6S(1E),1S)-7-[4-Phenyl-6-(3-oxo-4-(p-fluorphenoxy)-1-butenyl)-2-oxabicyclo[2.2.1]heptan-5-yl]-5-heptensäuremethylester:

43 mg (125 μmol) des in Beispiel 39b dargestellten Aldehyds setzte man in Analogie zu Beispiel 84a um und isolierte nach Aufarbeitung und Reinigung 21 mg (43 μmol, 34 %) der Titelverbindung als farbloses Öl.

IR (Film): 3080, 3060, 3000, 2940, 2870, 1730, 1690, 1620, 1500, 1440, 1290, 1245, 1200, 1040, 1000, 895, 830, 765, 730 und 700 $cm^{-1}$.

Beispiel 84

(4S,5R(5Z),6R(1E,3R),1R)-7-[4-(2-Phenylethyl)-6-(3-hydroxy-4-(p-flurophenoxy)-1-butenyl)-2-oxabicyclo [2.2.1]heptan-5-yl]-5-heptensäuremethylester (A) und (4S,5R(5Z),6R(1E,3S),1R)-7-[4-(2-Phenylethyl)-6-(3-hydroxy-4-(p-fluorphenoxy)-1-butenyl)-2-oxabicyclo[2.2.1]heptan-5-yl]-5-heptensäuremethylester (B):

40 mg (77 μmol) des in Beispiel 84a dargestellten ungesättigten Ketons reduzierte man in Analogie zu Beispiel 23 und isolierte nach Aufarbeitung und Reinigung 34 mg (65 μmol, 85 %) eines Gemisches der beiden

Titelverbindung als farbloses Öl.

IR (Film): 3600-3200, 3080, 3060, 3020, 3000, 2930, 2860, 1735, 1600, 1505, 1450, 1435, 1250, 1210, 1030, 975, 895, 830, 760 und 700 $cm^{-1}$.

Die chromatographische Trennung lieferte 13 mg (25 µmol, 32 %) des unpolareren Alkohols, dem man die Struktur A zuordnete, sowie 18 mg (34 µmol, 45 %) des polareren Alkohols, dem man die Strukur B zuordnete.

Beispiel 84a

(4S,5R(5Z),6R(1E),1R)-7-[4-(2-Phenylethyl)-6-(3-oxo-4-(p-fluorphenoxy)-1-butenyl)-2-oxabicyclo[2.2.1] heptan-5-yl]-5-heptensäuremethylester:

Zu der Lösung von 7,6 mg wasserfreiem Lithiumchlorid in 500 µl wasserfreiem Acetonitril tropfte man unter einer Atmosphäre aus trockenem Argon die Lösung von 47 mg Dimethyl-(2-oxo-3-(p-fluorphenoxy) propyl)-phosphonat in 500 µl wasserfreiem Acetonitril, versetzte mit 20 µl DBU und der Lösung von 55 mg (148 µmol) des in Beispiel 27a dargestellten Aldehyds in 250 µl Acetonitril und rührte 4 bis 5 Stunden bei 23 °C. Man verdünnte mit 10 ml Diethylether, wusch mit 10%iger Ammoniumchloridlösung, Wasser und gesättigter Natriumchloridlösung neutral, trocknete über Magnesiumsulfat und chromatographierte den nach Filtration und Einengen im Wasserstrahlvakuum erhaltenen Rückstand an zwei analytischen Kieselgelplatten. Als Laufmittel diente ein Gemisch aus Chloroform/Diethylether, als Elutionsmittel Ethylacetat. Isoliert wurden 40 mg (77 µmol, 52 %) der Titelverbindung als farbloses Öl.

IR (Film): 3080, 3060, 3030, 3000, 2940, 2870, 1735, 1690, 1620, 1505, 1450, 1435, 1245, 1210, 1155, 1035, 980, 895, 830, 760, 730 und 700 $cm^{-1}$.

Beispiel 85

(4S,5R(5Z),6R(1E,3R),1R)-7-[4-(p-Fluorphenyl)-6-(3-hydroxy-4-(p-fluorphenoxy)-1-butenyl)-2-oxabicyclo [2.2.1]heptan-5-yl]-5-heptensäuremethylester (A) und (4S,5R(5Z),6R(1E,3S),1R)-7-[4-(p-Fluorphenyl)-6-(3-hydroxy-4-(p-fluorphenoxy)-1-butenyl)-2-oxabicyclo[2.2.1]heptan-5-yl]-5-heptensäuremethylester (B):

39 mg (76 µmol) des in Beispiel 85a dargestellten ungesättigten Ketons reduzierte man in Analogie zu Beispiel 23 und isolierte nach Aufarbeitung und Reinigung 37 mg (72 µmol, 95 %) eines Gemisches der beiden Titelverbindungen als farbloses Öl.

IR (Film): 3600-3200, 3050, 3000, 2970, 2940, 2870, 1730, 1600, 1505, 1245, 1220, 1160, 1035, 895, 830, 760 und 735 $cm^{-1}$.

Die chromatographische Trennung lieferte 15 mg (29 µmol, 38 %) des unpolareren Alkohols, dem man die Struktur A zuordnete, sowie 19 mg (37 µmol, 49 %) des polareren Alkohols, dem man die Strukur B zuordnete.

Beispiel 85a

(4S,5R(5Z),6R(1E),1R)-7-[4-(p-Fluorphenyl)-6-(3-oxo-4-(p-fluorphenoxy)-1-buteny 1)-2-oxabicyclo[2.2.1]heptan-5-yl]-5-heptensäuremethylester:

54 mg (150 µmol) des in Beispiel 31b dargestellten Aldehyds setzte man in Analogie zu Beispiel 84a unter Verwendung von Dimethyl-(2-oxo-3-(p-fluorphenoxy)-propyl)-phosphonat um und isolierte nach Aufarbeitung und Reinigung 39 mg (76 µmol, 51 %) der Titelverbindung als farbloses Öl.

IR(Film): 3010, 2940, 2870, 1730, 1715, 1690, 1620, 1505, 1435, 1220, 1200, 1160, 900 und 830 $cm^{-1}$.

Beispiel 86

(4R,5S(5Z),6S(1E,3R),1S)-7-[4-Phenyl-6-(3-hydroxy-4-(p-fluorphenoxy)-1-butenyl) -2-oxabicyclo[2.2.1]heptan-5-yl]-5-heptensäure:

9 mg (18 µmol) des in Beispiel 83 dargestellten polaren Esters verseifte man in Analogie zu Beispiel 2 und isolierte nach Aufarbeitung und Reinigung 7 mg (15 µmol, 81 %) der Titelverbindung als farbloses Öl.

IR (Film): 3600-2400, 3080, 3060, 3030, 3010, 2930, 2870, 1710, 1600, 1505, 1250, 1210, 1035, 890, 830, 760 und 700 $cm^{-1}$.

Beispiel 87

(4S,5R(5Z),6R(1E,3R),1R)-7-[4-(2-Phenylethyl)-6-(3-hydroxy-4-(p-fluorphenoxy)-1-butenyl)-2-oxabicyclo [2.2.1]heptan-5-yl]-5-heptensäure:

13 mg (25 µmol) der in Beispiel 84 dargestellten unpolareren Verbindung verseifte man in Analogie zu

Beispiel 2 und isolierte nach Aufarbeitung und Reinigung 12 mg (24 μmol, 95 %) der Titelverbindung als farbloses Öl.

IR (Film): 3600-2200, 3030, 3010, 2930, 2870, 1725, 1710, 1605, 1505, 1250, 1210, 1030, 975, 890, 830, 760 und 700 $cm^{-1}$.

Beispiel 88

(4S,5R(5Z),6R(1E,3S),1R)-7-[4-(p-Fluorphenyl)-6-(3-hydroxy-4-(p-fluorphenoxy)-1-butenyl)-2-oxabicyclo [2.2.1]heptan-5-yl]-5-heptensäuremethylester:

14 mg (27 μmol) des in Beispiel 85 dargestellten unpolareren Esters verseifte man in Analogie zu Beispiel 2 und isolierte nach Aufarbeitung und Reinigung 12 mg (24 μmol, 88 %) der Titelverbindung als farbloses Öl.

IR (Film): 3600-2500, 3070, 3050, 3000, 2930, 2880, 1710, 1610, 1600, 1505, 1295, 1220, 1160, 1035, 895, 830, 760, 585 und 510 $cm^{-1}$.

Beispiel 89

(4R,5S(5Z),6S(1E,3S),1S)-7-[4-Phenyl-6-(3-hydroxy-4-(p-fluorphenoxy)-1-butenyl)-2-oxabicyclo[2.2.1] heptan-5-yl]-5-heptensäure:

7,5 mg (15 μmol) des in Beispiel 83 dargestellten unpolareren Esters verseifte man in Analogie zu Beispiel 2 und isolierte nach Aufarbeitung und Reinigung 7 mg (15 μmol, 96 %) der Titelverbindung als farbloses Öl.

IR (Film): 3600-2400, 3080, 3060, 3030, 3010, 2930, 2880, 1710, 1600, 1505, 1250, 1210, 1040, 1030, 890, 830, 760 und 700 $cm^{-1}$.

Beispiel 90

(4S,5R(5Z),6R(1E,3S),1R)-7-[4-(2-Phenylethyl)-6-(3-hydroxy-4-(p-fluorphenoxy)-1-butenyl)-2-oxabicyclo [2.2.1]heptan-5-yl]-5-heptensäure:

18 mg (34 μmol) der in Beispiel 84 dargestellten polareren Verbindung verseifte man in Analogie zu Beispiel 2 und isolierte nach Aufarbeitung und Reinigung 16 mg (31 μmol, 91 %) der Titelverbindung als farbloses Öl.

IR (Film): 3600-2400, 3080, 3060, 3030, 3000, 2930, 2870, 1725, 1710, 1505, 1455, 1250, 1210, 1030, 975, 890, 830, 760 und 700 $cm^{-1}$.

Beispiel 91

(4S,5R(5Z),6R(1E,3R),1R)-7-[4-(p-Fluorphenyl)-6-(3-hydroxy-4-(p-fluorphenoxy)-1-butenyl)-2-oxabicyclo [2.2.1]heptan-5-yl]-5-heptensäure:

19 mg (37 μmol) des im Beispiel 85 dargestellten polareren Esters verseifte man in Analogie zu Beispiel 2 und isolierte nach Aufarbeitung und Reinigung 18 mg (36 μmol, 97 %) der Titelverbindung als farbloses Öl.

IR (Film): 3600-2500, 3080, 3030, 3010, 2980, 2940, 2880, 1710, 1610, 1605, 1510, 1460, 1300, 1225, 1165, 1040, 970, 895, 830, 760, 590 und 515 $cm^{-1}$.

Beispiel 92

(4R,5S(5Z),6R,1S)-7-[4-Phenyl-6-(1-piperidinomethyl)-2-oxabicyclo[2.2.1]heptan-5-yl]-5-heptensäuremethylester:

21 mg (52 μmol) des in Beispiel 62a dargestellten Bromids setzte man in Analogie zu Beispiel 63 unter Verwendung von Piperidin um und isolierte nach Aufarbeitung und Reinigung 20 mg (49 μmol, 94 %) der Titelverbindung als farbloses Öl.

IR (Film): 3080, 3060, 3030, 3000, 2930, 2860, 1735, 1605, 1500, 1450, 1400, 1040, 895, 760 und 700 $cm^{-1}$.

Beispiel 93

(4R,5S(5Z),6R,1S)-7-[4-Phenyl-6-(1-piperidinomethyl)-2-oxabicyclo[2.2.1]heptan-5-yl]-5-heptensäure:

20 mg (52 μmol) des in Beispiel 92 dargestellten Esters verseifte man in Analogie zu Beispiel 2 und isolierte nach Aufarbeitung und Reinigung 15 mg (38 µmol, 78 %) der Titelverbindung als farbloses Öl.

IR (Film): 3600-2300, 3440, 3080, 3060, 3000, 2930, 2870, 1715, 1630, 1605, 1500, 1450, 1400, 1035, 895, 760 und 700 $cm^{-1}$.

Beispiel 94

(4S,5R(5Z),6S,1R)-7-[4-(2-Phenylethyl)-6-(m-nitrobenzyloxymethyl)-2-oxabicyclo[2.2.1]heptan-5-yl]-5-heptensäuremethyl-(bzw. m-nitrobenzyl)ester:

18,7 mg (50 µmol) des in Beispiel 15a dargestellten Alkohols veretherte man in Analogie zu Beispiel 1 mit m-Nitrobenzylchlorid und isolierte nach Aufarbeitung und Reinigung 20 mg der Titelverbindungen als farbloses Öl.

IR (Film): 3080, 3060, 3020, 3000, 2930, 2860, 1735, 1600, 1530, 1350, 1100, 895, 810, 730 und 700 $cm^{-1}$.

Beispiel 95

(4S,5R(5Z),6S,1R)-7-[4-(2-Phenylethyl)-6-(m-nitrobenzyloxymethyl)-2-oxabicyclo[2.2.1]heptan-5-yl]-5-heptensäure:

20 mg des in Beispiel 94 dargestellten Estergemisches verseifte man in Analogie zu Beispiel 2 und isolierte nach Aufarbeitung und Reinigung 13 mg (26 µmol, 53 % bezogen auf Edukt aus Beispiel 94) der Titelverbindung als farbloses Öl.

IR (Film): 3600-2400, 3080, 3060, 3020, 3000, 2930, 2860, 1730, 1705, 1600, 1530, 1350, 1120, 1095, 890, 805, 730 und 700 $cm^{-1}$.

Beispiel 96

(4S,5R(5Z),6S,1R)-7-[4-(2-Phenylethyl)-6-(o-fluorbenzyloxymethyl)-2-oxabicyclo[2.2.1]heptan-5-yl]-5-heptensäuremethyl(bzw. o-fluorbenzyl)-ester:

19 mg (51 µmol) des in Beispiel 15a dargestellten Alkohols veretherte man in Analogie zu Beispiel 1 mit o-Fluorbenzylchlorid und isolierte nach Aufarbeitung und Reinigung 27 mg der Titelverbindungen als farbloses Öl.

IR (Film): 3080, 3060, 3030, 3000, 2930, 2870, 1735, 1620, 1605, 1585, 1490, 1455, 1230, 1120, 1085, 895, 760 und 700 $cm^{-1}$.

Beispiel 97

(4S,5R,(5Z),6S,1R)-7-[4-(2-Phenylethyl)-6-(o-fluorbenzyloxymethyl)-2-oxabicyclo[2.2.1]heptan-5-yl]-5-heptensäure:

27 mg des in Beispiel 96 dargestellten Estergemisches verseifte man in Analogie zu Beispiel 2 und isolierte nach Aufarbeitung und Reinigung 18 mg (39 µmol, 76 % bezogen auf Edukt aus Beispiel 96) der Titelverbindung als farbloses Öl.

IR (Film): 3600-2500, 3080, 3060, 3020, 3000, 2930, 2860, 1730, 1710, 1620, 1600, 1585, 1490, 1455, 1230, 1115, 1085, 890, 760 und 700 $cm^{-1}$.

Beispiel 98

(4S,5R(5Z),6S,1R)-7-[4-(2-Phenylethyl)-6-(m-fluorbenzyloxymethyl)-2-oxabicyclo[2.2.1]heptan-5-yl]-5-heptensäuremethyl(bzw. m-fluorbenzyl)ester:

18 mg (48 µmol) des in Beispiel 15a dargestellten Alkohols veretherte man in Analogie zu Beispiel 1 mit m-Fluorbenzylchlorid und isolierte nach Aufarbeitung und Reinigung 24 mg der Titelverbindungen als farbloses Öl.

IR (Film): 3080, 3060, 3020, 3000, 2930, 2860, 1735, 1615, 1590, 1485, 1450, 1255, 1140, 895, 785, 750, 700 und 685 $cm^{-1}$.

Beispiel 99

(4S,5R(5Z),6S,1R)-7-[4-(2-Phenylethyl)-6-(m-fluorbenzyloxymethyl)-2-oxabicyclo[2.2.1]heptan-5-yl]-5-heptensäure:

24 mg des in Beispiel 98 dargestellten Estergemisches verseifte man in Analogie zu Beispiel 2 und isolierte nach Aufarbeitung und Reinigung 17 mg (36 µmol, 75 % bezogen auf Edukt aus Beispiel 98) der Titelverbindung als farbloses Öl.

IR (Film): 3600-2400, 3080, 3060, 3020, 3000, 2930, 2860, 1725, 1710, 1615, 1590, 1485, 1450, 1250, 890, 785, 745, 700 und 685 $cm^{-1}$.

Beispiel 100

(4S,5R(5Z),6S,1R)-7-[4-(2-Phenylethyl)-6-(p-fluorbenzyloxymethyl)-2-oxabicyclo[2.2.1]heptan-5-yl]-5-heptensäuremethyl (bzw. p-fluorbenzyl)ester:

19 mg (50 µmol) des in Beispiel 15a dargestellten Alkohols veretherte man in Analogie zu Beispiel 1 mit p-Fluorbenzylchlorid und isolierte nach Aufarbeitung und Reinigung 21 mg der Titelverbindungen als farbloses Öl.

IR (Film): 3080,. 3060, 3020, 3000, 2930, 2860, 1735, 1605, 1510, 1220, 1150, 895, 825, 755 und 700 $cm^{-1}$.

Beispiel 101

(4S,5R(5Z),6S,1R)-7-[4-(p-Fluorphenyl)-6-(p-fluorbenzyloxymethyl)-2-oxabicyclo[2.2.1]heptan-5-yl]-5-heptensäuremethyl(bzw. p-fluorbenzyl)ester:

18 mg (50 µmol) des in Beispiel 17 a dargestellten Alkohols setzte man in Analogie zu Beispiel 1 um und isolierte nach Aufarbeitung und Reinigung 26 mg der Titelverbindungen als farbloses Öl.

IR (Film): 3080, 3060, 3030, 3000, 2940, 2860, 1735, 1605, 1510, 1225, 1145, 895, 820, 760 und 705 $cm^{-1}$.

Beispiel 102

(4S,5R(5Z),6S,1R)-7-[4-Phenyl-6-(p-fluorbenzyloxymethyl)-2-oxabicyclo[2.2.1]heptan-5-yl]-5-heptensäuremethyl(bzw. p-fluorbenzyl)ester:

11,2 mg (33 µmol) des in Beispiel 3a dargestellten Alkohols setzte man in Analogie zu Beispiel 1 um und isolierte nach Aufarbeitung und Reinigung 10 mg der Titelverbindungen als farbloses Öl.

IR (Film): 3080, 3060, 3020, 3000, 2950, 2860, 1735, 1605, 1510, 1220, 1145, 900, 825, 760 und 700 $cm^{-1}$.

Beispiel 103

(4S,5R(5Z),6S,1R)-7-[4-(2-Phenylethyl)-6-(p-fluorbenzyloxymethyl)-2-oxabicyclo[2.2.1]heptan-5-yl]-5-heptensäure:

21 mg des in Beispiel 100 dargestellten Estergemisches verseifte man in Analogie zu Beispiel 2 und solierte nach Aufarbeitung und Reinigung 16 mg (34 µmol, 68 % bezogen auf Edukt aus Beispiel 100) der Titelverbindung als farbloses Öl.

IR (Film): 3600-2400, 3080, 3060, 3020, 3000, 2930, 2860, 1730, 1710, 1605, 1510, 1455, 1220, 1090, 890, 825, 755 und 700 $cm^{-1}$.

Beispiel 104

(4S,5R(5Z),6S,1R)-7-[4-(p-Fluorphenyl)-6-(p-fluorbenzyloxymethyl)-2-oxabicyclo[2.2.1]heptan-5-yl]-5-heptensäure:

26 mg des in Beispiel 101 dargestellten Estergemisches verseifte man in Analogie zu Beispiel 2 und isolierte nach Aufarbeitung und Reinigung 21 mg (46 µmol, 92 % bezogen auf Edukt aus Beispiel 101) der Titelverbindung als farbloses Öl.

IR (Film): 3600-2500, 3070, 3040, 3000, 2930, 2870, 1725, 1710, 1600, 1510, 1220, 1160, 1090, 890 und 830 $cm^{-1}$.

Beispiel 105

(4S,5R(5Z),6S,1R)-7-[4-Phenyl-6-(p-fluorbenzyloxymethyl)-2-oxabicyclo[2.2.1]heptan-5-yl]-5-heptensäure:

10 mg des in Beispiel 102 dargestellten Estergemisches verseifte man in Analogie zu Beispiel 2 und isolierte nach Aufarbeitung und Reinigung 9 mg (20 µmol, 59 % bezogen auf Edukt aus Beispiel 102) der Titelverbindung als farbloses Öl.

IR (Film): 3600-2400, 3060, 3030, 3000, 2930, 2870, 1730, 1705, 1600, 1510, 1220, 1115, 1090, 895, 825, 760 und 700 $cm^{-1}$.

Beispiel 106

(4S,5R(5Z),6S,1R)-7-[4-(2-Phenylethyl)-6-(m-trifluormethylbenzyloxymethyl)-2-oxabicyclo[2.2.1]heptan-5-yl]-5-heptensäuremethyl(bzw. m-trifluormethylbenzyl)ester:

17 mg (46 μmol) des in Beispiel 15a dargestellten Alkohols veretherte man in Analogie zu Beispiel 1 mit m-Trifluormethylbenzylchlorid und isolierte nach Aufarbeitung und Reinigung 23 mg der Titelverbindungen als farbloses Öl.

IR (Film): 3080, 3060, 3030, 3000, 2930, 2860, 1735, 1605, 1495, 1330, 1200, 1165, 1130, 1070, 890, 750, 700 und 665 $cm^{-1}$.

Beispiel 107

(4S,5R(5Z),6S,1R)-7-[4-(2-Phenylethyl)-6-(m-trifluormethylbenzyloxymethyl)-2-oxabicyclo[2.2.1]heptan-5-yl]-5-heptensäure:

23 mg des in Beispiel 106 dargestellten Estergemisches verseifte man in Analogie zu Beispiel 2 und isolierte nach Aufarbeitung und Reinigung 17 mg (33 μmol, 72 % bezogen auf Edukt aus Beispiel 106) der Titelverbindung als farbloses Öl.

IR (Film): 3600-2500, 3080, 3060, 3030, 3000, 2930, 2860, 1730, 1710, 1605, 1455, 1330, 1195, 1165, 1125, 1075, 890, 800, 755, 700 und 660 $cm^{-1}$.

Beispiel 108

(4S,5R(5Z),6S,1R)-7-[4-(2-Phenylethyl)-6-(p-trifluormethylbenzyloxymethyl)-2-oxabicyclo[2.2.1]heptan-5-yl]-5-heptensäuremethyl(bzw. p-trifluormethylbenzyl)ester:

15 mg (40 μmol) des in Beispiel 15a dargestellten Alkohols veretherte man in Analogie zu Beispiel 1 mit p-Trifluormethylbenzylchlorid und isolierte nach Aufarbeitung und Reinigung 28 mg der Titelverbindungen als farbloses Öl.

IR (Film): 3080, 3060, 3030, 3000, 2930, 2860, 1735, 1620, 1605, 1330, 1160, 1130, 1065, 1015, 895, 820, 760 und 700 $cm^{-1}$.

Beispiel 109

(4S,5R(5Z),6S,1R)-7-[4-(2-Phenylethyl)-6-(p-trifluormethylbenzyloxymethyl)-2-oxabicyclo[2.2.1]heptan-5-yl]-5-heptensäure:

28 mg des in Beispiel 108 dargestellten Estergemisches verseifte man in Analogie zu Beispiel 2 und isolierte nach Aufarbeitung und Reinigung 19 mg (37 μmol, 91 % bezogen auf Edukt aus Beispiel 108) der Titelverbindung als farbloses Öl.

IR (Film): 3600-2400, 3090, 3060, 3030, 3010, 2940, 2860, 1730, 1710, 1620, 1605, 1330, 1165, 1125, 1065, 1020, 890, 825, 755 und 705 $cm^{-1}$.

Beispiel 110

(4S,5R(5Z),6S,1R)-7-[4-(2-Phenylethyl)-6-(p-chlorbenzyloxymethyl)-2-oxabicyclo[2.2.1]heptan-5-yl]-5-heptensäuremethyl(bzw. p-chlorbenzyl)ester:

18 mg (48 μmol) des in Beispiel 15a dargestellten Alkohols veretherte man in Analogie zu Beispiel 1 mit p-Chlorbenzylchlorid und isolierte nach Aufarbeitung und Reinigung 30 mg der Titelverbindungen als farbloses Öl.

IR (Film): 3080, 3060, 3030, 3000, 2930, 2860, 1735, 1600, 1495, 1450, 1235, 890, 810, 760 und 700 $cm^{-1}$.

Beispiel 111

(4S,5R(5Z),6S,1R)-7-[4-(2-Phenylethyl)-6-(p-chlorbenzyloxymethyl)-2-oxabicyclo[2.2.1]heptan-5-yl]-5-heptensäure:

30 mg des in Beispiel 110 dargestellten Estergemisches verseifte man in Analogie zu Beispiel 2 und isolierte nach Aufarbeitung und Reinigung 18 mg (37 μmol, 77 % bezogen auf Edukt aus Beispiel 110) der Titelverbindung als farbloses Öl.

IR (Film): 3600-2400, 3080, 3060, 3030, 3000, 2930, 2860, 1730, 1710, 1600, 1490, 1455, 1240, 1090, 1030, 890, 810, 755 und 700 $cm^{-1}$.

Beispiel 112

(4S,5R(5Z),6S,1R)-7-[4-(2 Phenylethyl)-6-(phenoxyethyloxymethyl)-2-oxabicyclo[2.2.1]heptan-5-yl]-5-heptensäuremethyl(bzw. Phenoxyethyl)ester:

17 mg (46 μmol) des in Beispiel 15a dargestellten Alkohols veretherte man in Analogie zu Beispiel 1 mit 1-Chlor-2-phenoxyethan und isolierte nach Aufarbeitung und Reinigung 18 mg der Titelverbindungen als farbloses Öl.

IR (Film): 3060, 3030, 3000, 2930, 2870, 1735, 1600, 1590, 1500, 1455, 1250, 1125, 890, 760, 700 und 690 $cm^{-1}$.

Beispiel 113

(4S,5R(5Z),6S,1R)-7-[4-(2-Phenylethyl)-6-(phenoxyethyloxymethyl)-2-oxabicyclo[2.2.1]heptan-5-yl]-5-heptensäure:

18 mg des in Beispiel 112 dargestellten Estergemisches verseifte man in Analo gie zu Beispiel 2 und isolierte nach Aufarbeitung und Reinigung 11 mg (23 μmol, 50 % bezogen auf Edukt aus Beispiel 112) der Titelverbindung als farbloses Öl.

IR (Film): 3600-2500, 3080, 3060, 3030, 3000, 2930, 2870, 1730, 1710, 1600, 1585, 1495, 1455, 1245, 1130, 890, 755, 700 and 690 $cm^{-1}$.

Beispiel 114

(4S,5R(5Z),6S,1R)-7-[4-(2-Phenylethyl)-6-N-anilinomethyl-2-oxabicyclo[2.2.1]heptan-5-yl]-5-heptensäuremethylester:

19 mg (44 μmol) des in Beispiel 65a dargestellten Bromids setzte man in Analogie zu Beispiel 63 unter Verwendung von Anilin um und isolierte nach Aufarbeitung und Reinigung 17 mg (38 μmol, 87 %) der Titelverbindung als farbloses Öl.

IR (Film): 3440-3300, 3080, 3060, 3020, 2930, 2860, 1730, 1600, 1505, 1320, 1250, 895, 750 und 695 $cm^{-1}$.

Beispiel 115

(4S,5R(5Z),6S,1R)-7-[4-(2-Phenylethyl)-6-N-anilinomethyl-2-oxabicyclo[2.2.1]heptan-5-yl]-5-heptensäure:

17 mg (38 μmol) des in Beispiel 114 dargestellten Esters verseifte man in Analogie zu Beispiel 2 und isolierte nach Aufarbeitung und Reinigung 15 mg (35 μmol, 91 %) der Titelverbindung als farbloses Öl.

IR (Film): 3400, 3600-2300, 3080, 3060, 3030, 3000, 2930, 2860, 1725, 1710, 1605, 1510, 1495, 1325, 1255, 980, 890, 750, 700 und 695 $cm^{-1}$.

Beispiel 116

(4S,5R(5Z),6S,1R)-7-[4-(2-Phenylethyl)-6-(p-fluor-N-anilinomethyl)-2-oxabicyclo[2.2.1]heptan-5-yl]-5-heptensäuremethylester:

19 mg (44 μmol) des in Beispiel 65a dargestellten Bromids setzte man in Analogie zu Beispiel 63 unter Verwendung von p-Fluoranilin um und isolierte nach Aufarbeitung und Reinigung 13 mg (28 μmol, 64 %) der Titelverbindung als farbloses Öl.

IR (Film): 3450-3300, 3080, 3060, 3030, 3000, 2930, 2870, 1735, 1610, 1605, 1515, 1220, 895, 820 und 700 $cm^{-1}$.

Beispiel 117

(4S,5R(5Z),6S,1R)-7-[4-(2-Phenylethyl)-6-(p-fluor-N-anilinomethyl)-2-oxabicyclo[2.2.1]heptan-5-yl]-5-heptensäure:

13 mg (28 μmol) des in Beispiel 116 dargestellten Esters verseifte man in Analogie zu Beispiel 2 und isolierte nach Aufarbeitung und Reinigung 11 mg (24 μmol, 87 %) der Titelverbindung als farbloses Öl.

IR (Film): 3390, 3600-2300, 3080, 3060, 3020, 3000. 2930, 2870, 1710, 1610, 1605, 1510, 1220, 890, 820 und 700 $cm^{-1}$.

Beispiel 118

(4S,5R(5Z),6S,1R)-7-[4-(p-Fluorphenyl)-6-phenoxymethyl-2-oxabicyclo[2.2.1]heptan-5-yl]-5-heptensäuremethylester:

Zu der klaren Lösung von 90 mg Natriumphenolat in einem Gemisch aus 300 µl wasserfreiem Methylbenzol und 300 µl Tetrahydrofuran gab man die Lösung von 36 mg (84,6 µmol) des in Beispiel 118a dargestellten Bromids in 300 µl Tetrahydrofuran und erwärmte 28 Stunden auf 80-90° C. Man versetzte mit Wasser, extrahierte mehrfach mit Diethylether, wusch mit Wasser und gesättigter Natriumchloridlösung neutral, trocknete über Magnesiumsulfat und reinigte den nach Filtration und Lösungsmittelabzug erhaltenen Rückstand durch Chromatographie an analytischen Kieselgelplatten. Als Laufmittel diente ein Gemisch aus Chloroform/Diethylether, als Elutionsmittel ein Gemisch aus Ethylacetat/Ethanol. Isoliert wurde neben Ausgangsmaterial und verseiftem Ester 18 mg (40,3 µmol, 48 %) der Titelverbindung als blassgelbes Öl.

IR (Film): 3080, 3060, 3030, 3000, 2950, 2930, 2870, 1735, 1600, 1510, 1445, 1230, 1110, 900, 820, 760 und 700 $cm^{-1}$.

Beispiel 118a

(4S,5R(5Z),6R,1R)-7-[4-(p-Fluorphenyl)-6-brommethyl-2-oxabicyclo[2.2.1]heptan-5-yl]-5-heptensäuremethylester:

28 mg (77 µmol) des in Beispiel 17a dargestellten Alkohols bromierte man in Analogie zu Beispiel 63a und isolierte nach Aufarbeitung und Reinigung 32 mg (75 µmol, 97 %) der Titelverbindung als farbloses Öl.

IR (Film): 3080, 3060, 3030, 3000, 2950, 2860, 1735, 1600, 1505, 1450, 1435, 1220, 1165, 1010, 895, 825, 760, 700 und 635 $cm^{-1}$.

Beispiel 119

(4S,5R(5Z),6S,1R)-7-[4-(p-Fluorphenyl)-6-phenoxymethyl-2-oxabicyclo[2.2.1]heptan-5-yl]-5-heptensäure:

18 mg (40,3 µmol) des in Beispiel 118 dargestellten Esters verseifte man in Analogie zu Beispiel 2 und isolierte nach Aufarbeitung und Reinigung 14 mg (32,3 µmol, 82 %) der Titelverbindung als farbloses Öl.

IR (Film): 3600-2400, 3080, 3040, 2950, 2930, 2870, 1710, 1605, 1600, 1515, 1230, 1160, 895, 835, 720 und 580 $cm^{-1}$.

**Beispiel 120**

(1S,4R,5S(5Z),6R)-7-[4-(4-Fluorphenyl)-6-benzyloxymethyl-2-oxabicyclo[2.2.1]hept-5-yl]-5-heptensäuremethyl (bzw. benzyl)ester:

17,5 mg (48,3 µmol) des in Beispiel 120a dargestellten Alkohols setzte man in Analogie zu Beispiel 1 um und isolierte nach Aufarbeitung und Reinigung 22,6 mg der Titelverbindungen als farbloses Öl.

IR (Film): 3060, 3020, 3000, 2930, 2870, 1735, 1600, 1510, 1455, 1110, 1075, 895, 820, 740 und 705 $cm^{-1}$.

**Beispiel 120a**

(1S,4R,5S(5Z),6R)-7-[4-(4-Fluorphenyl)-6-hydroxymethyl-2-oxabicyclo[2.2.1]hept-5-yl]-5-hepten-säuremethylester:

40,7 g (148 mmol) enantiomeres Corey-Lakton setzte man in Analogie zu den Beispielen 17a bis 17g und 1h bis 1l um und isolierte 1,88 g (5,19 mol, 3,5%) der Titelverbindung als farbloses Öl.

IR (Film): 3600-3200, 3040, 3000, 2960, 2920, 2870, 1730, 1600, 1510, 1225, 1160, 1025, 890 und 830 $cm^{-1}$.

**Beispiel 121**

(1S,4R,5S(5Z)6R)-7-[4-(4-Fluorphenyl)-6-benzyloxymethyl-2-oxabicyclo[2.2.1]hept-5-yl]-5-heptensäure:

22,6 mg des nach Beispiel 120 dargestellten Estergemisches verseifte man in Analogie zu Beispiel 2 und isoliert nach Aufarbeitung und Reinigung 17,4 mg (39,7 µmol, 82%, bezogen auf Startmaterial in Beispiel 120) der Titelverbindung als farbloses Öl.

IR (Film): 3600-2400, 3090, 3060, 3020, 3000, 2940, 2870, 1725, 1710, 1605, 1510, 1450, 1230, 1160, 1090, 1030, 890, 830, 735, 690 und 640 $cm^{-1}$.

**Beispiel 122**

(1S,4R,5S(5Z),6R)-7-[4-(4-Fluorphenyl)-6-hexyloxymethyl-2-oxabicyclo[2.2.1]hept-5-yl]-5-hepten-säuremethylester:

18,1 mg (50 µmol) des in Beispiel 120a dargestellten Alkohols veretherte man unter Verwendung von 1-Bromhexan in Analogie zu Beispiel 1 und isolierte nach Aufarbeitung und Reinigung 25,5 mg (49 µmol, 98%) der Titelverbindung als farbloses Öl.

IR (Film): 3040, 3000, 2960, 2930, 2860, 1730, 1605, 1515, 1230, 1160, 1120, 1090, 1030, 895 und 830 $cm^{-1}$.

**Beispiel 123**

(1S,4R,5S(5Z),6R)-7-[4-(4-Fluorphenyl)-6-hexyloxymethyl-2-oxabicyclo[2.2.1]hept-5-yl]-5-hepten-säure:

25,5 mg des nach Beispiel 122 dargestellten Esters verseifte man in Analogie zu Beispiel 2 und isolierte nach Aufarbeitung und Reinigung 20,0 mg (46,2 µmol, 94%) der Titelverbindung als farbloses Öl.

IR (Film):3600-2400, 3050, 3000, 2950, 2930, 2860, 1730, 1710, 1605, 1515, 1235, 1160, 1120, 1090, 1030, 895 und 830 $cm^{-1}$.

**Beispiel 124**

(1S,4R,5S(5Z)6R)-7-[4-(4-Fluorphenyl)-6-[2-(4-bromphenoxy)-ethoxymethyl]-2-oxabicyclo [2.2.1]hept-5-yl]-5-heptensäuremethylester:

16,3 mg (45 µmol) des in Beispiel 120a dargestellten Alkohols veretherte man unter Verwendung von 1-Chlor-2-(4-bromphenyoxy)-ethan in Analogie zu Beispiel 1 und isolierte nach Aufarbeitung und Reinigung 18,5 mg (34 µmol, 76%) der Titelverbindung als farbloses Öl.

IR (Film): 3040, 3000, 2940, 2870, 1730, 1590, 1580, 1515, 1490, 1450, 1290, 1245, 895, 825 und 645 $cm^{-1}$.

**Beispiel 125**

(1S,4R,5S(5Z),6R)-7-[4-(4-Fluorphenyl)-6-[2-(4-bromphenoxy)-ethoxymethyl]-2-oxabicyclo [2.2.1]hept-5-yl]-5-heptensäure:

18,5 mg (34 µmol) des nach Beispiel 124 dargestellten Esters verseifte man in Analogie zu Beispiel 2 und isolierte nach Aufarbeitung und Reinigung 15,4 mg (29 µmol, 85%) der Titelverbindung als farbloses Öl.

IR (Film): 3600-2500, 3070, 3040, 3000, 2930, 2870, 1725, 1710, 1590, 1580, 1515, 1490, 1455, 1285, 1245, 895, 825 und 645 $cm^{-1}$.

**Beispiel 126**

(1S,4R,5S(5Z),6R)-7-[4-(4-Fluorphenyl)-6-(3-nitrobenzyloxymethyl)-2-oxabicyclo[2.2.1]hep t-5-yl]-5-heptensäuremethyl(bzw.3-nitrobenzyl)ester:

19,4 mg (53 µmol) des in Beispiel 120a dargestellten Alkohols setzte man in Analogie zu Beispiel 1 um und isolierte nach Aufarbeitung und Reinigung 19,9 mg der Titelverbindungen als farbloses Öl.

IR (Film): 3070, 3050, 3000, 2930, 2870, 1735, 1610, 1595, 1530, 1510, 1230, 1160, 1120, 1095, 1030, 895, 830, 815 und 735 $cm^{-1}$.

**Beispiel 127**

(1S,4R,5S(5Z),6R)-7-[4-(4-Fluorphenyl)-6-(3-nitrobenzyloxymethyl)-2-oxabicyclo[2.2.1]hep t-5-yl]-5-heptensäure:

19,9 mg des nach Beispiel 126 dargestellten Estergemisches verseifte man in Analogie zu Beispiel 2 und isolierte nach Aufarbeitung und Reinigung 15 mg (31 µmol, 58%, bezogen auf Startmaterial in Beispiel 126) der Titelverbindung als farbloses Öl.

IR (Film): 3600-2400, 3090, 3070, 3050, 3000, 2930, 2870, 1730, 1705, 1610, 1595, 1530, 1510, 1350, 1230, 1160, 1120, 1095, 1030, 895, 830, 815 und 730 $cm^{-1}$.

**Beispiel 128**

(1S,4R,5S(5Z),6R)-7-[4-(4-Fluorphenyl)-6-(4-chlorbenzyloxymethyl)-2-oxabicyclo[2.2.1]he pt-5-yl]-5-heptensäuremethyl(bzw.4-chlorbenzyl)ester:

18,1 mg (50 μmol) des in Beispiel 120a dargestellten Alkohols setzte man in Analogie zu Beispiel 1 um und isolierte nach Aufarbeitung und Reinigung 22 mg der Titelverbindungen als farbloses Öl.

IR (Film): 3060, 3030, 3000, 2930, 2870, 1735, 1605, 1600, 1515, 1495, 1450, 1235, 1160, 1120, 1090, 1015, 895, 830, 820, 810 und 800 $cm^{-1}$.

**Beispiel 129**

(1S,4R,5S(5Z),6R)-7-[4-(4-Fluorphenyl)-6-(4-chlorbenzyloxymethyl)-2-oxabicyclo[2.2.1]he pt-5-yl]-5-heptensäure:

22 mg des nach Beispiel dargestellten Estergemisches verseifte man in Analogie zu Beispiel 2 und isolierte nach Aufarbeitung und Reinigung 13,3 mg (28 μmol, 56%, bezogen auf Startmaterial in Beispiel 128) der Titelverbindung als farbloses Öl.

IR (Film): 3600-2400, 3050, 3000, 2930, 2870, 1725, 1710, 1605, 1600, 1515, 1490, 1410, 1230, 1160, 1120, 1090, 1015, 895, 830, 820, 810 und 800 $cm^{-1}$.

**Beispiel 130**

(1S,4R,5S(5Z),6R)-7-[4-(4-Fluorphenyl)-6-(4-fluorbenzyloxymethyl)-2-oxabicyclo[2.2.1]hep t-5-yl]-5-heptensäuremethyl(bzw.4-fluorbenzyl)ester:

17,8 mg (49 μmol) des in Beispiel 120a dargestellten Alkohols setzte man in Analogie zu Beispiel 1 um und isolierte nach Aufarbeitung und Reinigung 21 mg der Titelverbindungen als farbloses Öl.

IR (Film): 3080, 3060, 3030, 3000, 2950, 2860, 1735, 1605, 1510, 1225, 1150, 895, 820, 760 und 705 $cm^{-1}$.

**Beispiel 131**

(1S,4R,5S(5Z),6R)-7-[4-(4-Fluorphenyl)-6-(4-fluorbenzyloxymethyl)-2-oxabicyclo[2.2.1]hep t-5-yl]-5-heptensäure:

21 mg des nach Beispiel 130 dargestellten Estergemisches verseifte man in Analogie zu Beispiel 2 und isolierte nach Aufarbeitung und Reinigung 18,1 mg (40 μmol, 81%, bezogen auf Startmaterial in Beispiel 130) der Titelverbindung als farbloses Öl.

IR (Film): 3600-2500, 3070, 3040, 3000, 2930, 2870, 1725, 1710, 1605, 1510, 1220, 1160, 1090, 1030, 1015, 895 und 830 $cm^{-1}$.

**Beispiel 132**

(1S,4R,5S(5Z),6R)-7-[4-(4-Fluorphenyl)-6-(4-trifluormethylbenzyloxymethyl)-2-oxabicyclo[ 2.2.1]hept-5-yl]-5-heptensäuremethyl(bzw.4-trifluormethylbenzyl)ester:

17,7 mg (49 μmol) des in Beispiel dargestellten Alkohols setzte man in Analogie zu Beispiel 1 um und isolierte nach Aufarbeitung und Reinigung 26,5 mg der Titelverbindungen als farbloses Öl.

IR (Film): 3040, 3000, 2940, 2870, 1735, 1620, 1610, 1515, 1325, 1240, 1165, 1125, 1065, 1020, 900, 830 und 825 $cm^{-1}$.

**Biespiel 133**

(1S,4R,5S(5Z),6R)-7-[4-(4-Fluorphenyl)-6-(4-trifluormethylbenzyloxymethyl)-2-oxabicyclo[ 2.2.1]hept-5-yl]-5-heptensäure:

26,5 mg des nach Beispiel 132 dargestellten Estergemisches verseifte man in Analogie zu Beispiel 2 und isolierte nach Aufarbeitung und Reinigung 22,5 mg (44 μmol, 91%, bezogen auf Startmaterial in Beispiel 132) der Titelverbindung als farbloses Öl.

IR (Film): 3600-2500, 3040, 3000, 2940, 2870, 1725, 1710, 1620, 1610, 1515, 1325, 1235, 1165, 1125, 1065, 1020, 895, 830 und 825 $cm^{-1}$.

**Beispiel 134**

(1S,4R,5S(5Z),6R)-7-[4-(4-Fluorphenyl)-6-(4-phenylbenzyloxymethyl)-2-oxabicyclo[2.2.1]h ept-5-yl]-5-heptensäuremethylester:

17 mg (47 µmol) des in Beispiel 120a dargestellten Alkohols setzte man in Analogie zu Beispiel 1 um und isolierte nach Aufarbeitung und Reinigung 21 mg (40 µmol, 85%) der Titelverbindung als farbloses Öl.

IR (Film): 3060, 3030, 3000, 2940, 2870, 1735, 1605, 1515, 1490, 1410, 1225, 1160, 1120, 1090, 1080, 1025, 1010, 895, 830, 760, 735 und 705 $cm^{-1}$.

**Beispiel 135**

(1S,4R,5S(5Z),6R)-7-[4-(4-Fluorphenyl)-6-(4-phenylbenzyloxymethyl)-2-oxabicyclo[2.2.1]h ept-5-yl]-5-heptensäure:

21 mg (40 µmol) des nach Beispiel 134 dargestellten Esters verseifte man in Analogie zu Beispiel 2 und isolierte nach Aufarbeitung und Reinigung 16,5 mg (32 µmol, 80%) der Titelverbindung als farbloses Öl.

IR (Film): 3600-2400, 3060, 3030, 3000, 2940, 2870, 1730, 1710, 1605, 1515, 1490, 1410, 1230, 1160, 1120, 1090, 1080, 1030, 1010, 895, 830, 760, 735 und 700 $cm^{-1}$.

**Beispiel 136**

(1S,4R,5S(5Z),6R)-7-[4-(4-Fluorphenyl)-6-(4-fluoranilinomethyl)-2-oxabicyclo[2.2.1]hept-5-yl]-5-heptensäuremethylester:

28 mg (66 µmol) des in Beispiel 136a dargestellten Bromides setzte man in Analogie zu Beispiel 63 unter Verwendung von 4-Fluoranilin um und isolierte nach Aufarbeitung und Reinigung 20,5 mg (45 µmol, 68%) der Titelverbindung als farbloses Öl.

IR (Film): 3450-3300, 3040, 3000, 2930, 2870, 1735, 1610, 1510, 1320, 1300, 1220, 1160, 895, 820 und 760 $cm^{-1}$.

**Beispiel 136a**

(1S,4R,5S(5Z),6R)-7-[4-(4-Fluorphenyl)-6-brommethyl-2-oxabicyclo[2.2.1]hept-5-yl-5-hept ensäuremethylester:

25,9 mg (71 µmol) des in Beispiel 120a dargestellten Alkohols bromierte man in Analogie zu Beispiel 63a und isolierte nach Aufarbeitung und Reinigung 28 mg (66 µmol, 93%) der Titelverbindung als farbloses Öl.

IR (Film): 3040, 2970, 2940, 2870, 1730, 1605, 1510, 1450, 1430, 1295, 1230, 1160, 1035, 900, 830 und 735 $cm^{-1}$.

**Beispiel 137**

(1S,4R,5S(5Z),6R)-7-[4-(4-Fluorphenyl)-6-(4-fluoranilinomethyl)-2-oxabicyclo[2.2.1]hept-5-yl]-5-heptensäure:

20,5 mg (45 µmol) des nach Beispiel 136 dargestellten Esters verseifte man in Analogie zu Beispiel 2 und isolierte nach Aufarbeitung und Reinigung 18 mg (41 µmol, 91%) der Titelverbindung als farbloses Öl.

IR (Film): 3600-2400, 3390, 3040, 3000, 2930, 2870, 1710, 1610, 1510, 1320, 1300, 1220, 1160, 895, 820 und 760 $cm^{-1}$.

**Beispiel 138**

(1S,4R,5S(5Z),6S)-7-[4-(4-Fluorphenyl)-6-[3(S)-hydroxy-4-phenoxy-1(E)-butenyl]-2-oxabicyclo[2.2.1]hept-5-yl]-5-heptensäuremethylester (A) und (1S,4R,5S(5Z),6S)-7-[4-(4-Fluorphenyl)-6-[3(R)-hydroxy-4-phenoxy-1(E)-butenyl]-2-oxabicyclo[2.2.1]hept-5-yl]-5-heptensäuremethylester (B):

43 mg (87 µmol) des in Beispiel 138a dargestellten ungesättigten Ketons reduzierte man in Analogie zu Beispiel 23 und isolierte nach Aufarbeitung und Reinigung 43 mg (87 µmol, 99%) eines Gemisches der beiden Titelverbindungen als farbloses Öl.

IR (Film): 3700-3100, 3050, 3000, 2970, 2940, 2930, 2870, 1730, 1595, 1585, 1510, 1490, 1450, 1430, 1290, 1230, 1160, 1080, 1035, 995, 970, 895, 830, 755, 735 und 690 $cm^{-1}$.

Die chromatographie Trennung lieferte 19,8 mg (40 µmol, 46%) des unpolareren Alkohols, dem man die Struktur A zuordnete, sowie 19,6 mg (40 µmol, 46%) des polareren Alkohols dem man die Struktur B zuordnete.

**Beispiel 138a**

(1S,4R,5S(5Z),6S)-7-[4-(4-Fluorphenyl)-6-(3-oxo-4-phenoxy-1(E)-butenyl)-2-oxabicyclo[2.2.1]hept-5-yl]-5-heptensäure methylester:

47,4 mg (131 µmol) des in Beispiel 138b dargestellten Aldehyds setzte man in Analogie zu Beispiel 30a unter Verwendung von Dimethyl-(2-oxo-3-phenoxy-propyl)-phosphonat-Lithiumsalz um und isolierte nach Aufarbeitung und Reinigung 43,5 mg (88 µmol, 67%) der Titelverbindung als farbloses Öl.

IR (Film): 3060, 3040, 3000, 2970, 2940, 2870, 1730, 1690, 1620, 1600, 1510, 1490, 1450, 1435, 1290, 1230, 1160, 1040, 995, 900, 835, 755 und 690 $cm^{-1}$.

**Beispiel 138b**

(1S,4R,5S(5Z),6S)-7-[4-(4-Fluorphenyl)-6-formyl-2-oxabicyclo[2.2.1]hept-5-yl]-5-heptensäure:

647 mg (1,79 mmol) des in Beispiel 120a dargestellten Alkohols oxidierte man in Analogie zu Beispiel 38b und isolierte nach Aufarbeitung 637 mg (1,77 mmol, 99%) der Titelverbindung als farbloses Öl, das man ohne Reinigung weiter umsetzte.

**Beispiel 139**

(1S,4R,5S(5Z),6S)-7-[4-(4-Fluorphenyl)-6-[3(S)-hydroxy-4-phenoxy-1(E)-butenyl]-2-oxabicyclo[2.2.1]hept-5-yl]-5-heptensäure:

19,8 mg (40 µmol) des in Beispiel 138 dargestellten unpolaren Esters verseifte man in Analogie zu Beispiel 2 und isolierte nach Aufarbeitung und Reinigung 15,9 mg (33 µmol, 83%) der Titelver-bindung als farbloses Öl.

IR (Film): 3700-2400, 3060, 3040, 3010, 2930, 2880, 1725, 1710, 1600, 1585, 1455, 1300, 1245, 1235, 1160, 1080, 1040, 995, 970, 895, 835, 820, 755, 690 und 590 $cm^{-1}$.

**Beispiel 140**

(1S,4R,5S(5Z),6S)-7-[4-(4-Fluorphenyl)-6-[3(R)-hydroxy-4-phenoxy-1(E)-butenyl]-2-oxabicyclo[2.2.1]hept-5-yl]-5-heptensäure:

19,6 mg (39,6 µmol) des in Beispiel 138 dargestellten polaren Esters verseifte man in Analogie zu Beispiel 2 und isolierte nach Aufarbeitung und Reinigung 13,8 mg (28,7 µmol, 73%) der Titelver-bindung als farbloses Öl.

IR (Film): 3600-2400, 3050, 3040, 3000, 2930, 2880, 1725, 1710, 1600, 1585, 1455, 1300, 1250, 1235, 1160, 1080, 1040, 995, 970, 895, 840, 820, 755, 690 und 590 $cm^{-1}$.

**Beispiel 141**

(1S,4R,5S(5Z),6S)-7-[4-(4-Fluorphenyl)-6-[3(S)-hydroxy-1(E)-octenyl]-2-oxabicyclo[2.2.1]hept-5-yl]-5-heptensäuremethylester (A) und (1S,4R,5S(5Z),6S)-7-[4-(4-Fluorphenyl)-6-[3(R)-hydroxy-1(E)-octenyl[-2-oxabicyclo [2.2.1]hept-5-yl]-5-heptensäuremethylester (B):

43 mg (94 µmol) des in Beispiel 141a dargestellten ungesättigten Ketons reduzierte man in Analogie zu Beispiel 23 und isolierte nach Aufarbeitung und Reinigung 42 mg (92 µmol, 97%) eines Gemisches der beiden Titelverbindungen als farbloses Öl.

IR (Film): 3600-3200, 3040, 3000, 2950, 2920, 2860, 1605, 1510, 1450, 1435, 1230, 1160, 995, 970, 895 und 830 $cm^{-1}$.

Die chromatographische Trennung lieferte 21 mg (46 µmol, 49%) des unpolareren Alkohols, dem man die Struktur A zuordnete, sowie 19 mg (41 µmol, 44%) des polareren Alkohols dem man die Struktur B zuordnete.

**Beispiel 141a**

(1S,4R,5S(5Z),6S)-7-[4-(4-Fluorphenyl)-6-(3-oxo-1(E)-octenyl)-2-oxabicyclo[2.2.1]hept-5-yl]-5-heptensäuremethylester:

47 mg (130 µmol) des in Beispiel 138b dargestellten Aldehyds setzte man in Analogie zu Beispiel 23a unter Verwendung von Dimethyl-(2-oxo-heptyl)-phosphonat um und isolierte nach Aufarbeitung und Reinigung 43 mg (94 µmol, 72%) der Titelverbindung als farbloses Öl.

IR (Film): 3040, 3000, 2950, 2920, 2860, 1730, 1690, 1665, 1620, 1510, 1450, 1430, 1230, 1160, 995, 900

und 835 $cm^{-1}$.

**Beispiel 142**

(1S,4R,5S(5Z),6S)-7-[4-(4-Fluorphenyl)-6-[3(S)-hydroxy-1(E)-octenyl]-2-oxabicyclo[2.2.1]hept-5-yl]-5-heptensäure:

21 mg (46 µmol) des in Beispiel 141 dargestellten unpolaren Esters verseifte man in Analogie zu Beispiel 2 und isolierte nach Aufarbeitung und Reinigung 19 mg (43 µmol, 93%) der Titelver-bindung als farbloses Öl.

IR (Film): 3600-2400, 3040, 3000, 2950, 2930, 2870, 2860, 1710, 1610, 1515, 1455, 1300, 1235, 1160, 995, 970, 895 und 835 $cm^{-1}$.

**Beispiel 143**

(1S,4R,5S(5Z),6S)-7-[4-(4-Fluorphenyl)-6-[3(R)-hydroxy-1(E)-octenyl]-2-oxabicyclo[2.2.1]hept-5-yl]-5-heptensäure:

19 mg (41 µmol) des in Beispiel 141 dargestellten polaren Esters verseifte man in Analogie zu Beispiel 2 und isolierte nach Aufarbeitung und Reinigung 15,6 mg (35 µmol, 86%) der Titelver-bindung als farbloses Öl.

IR (Film): 3600-2400, 3040, 3000, 2940, 2870, 1725, 1710, 1610, 1515, 1455, 1300, 1230, 1160, 995, 970, 895 und 835 $cm^{-1}$.

**Beispiel 144**

(1S,4R,5S(5Z),6S)-7-[4-(4-Fluorphenyl)-6-[(1E,3R,4RS)-3-hydroxy-4,7-dimethyl-octa-1,6-dienyl]-2-oxabicyclo [2.2.1]hept-5-yl]-5-hepten-säuremethylester(A) und (1S,4R,5S(5Z),6S)-7-[4-(4-Fluorphenyl)-6-[(1E,3S,4RS)-3-hydroxy-4,7-dimethyl-octa-1,6-dienyl]-2-oxabicyclo[2.2.1]hept-5-yl]-5-heptensäuremethylester (B):

53 mg (110µmol) des in Beispiel 144a dargestellten ungesättigten Ketons reduzierte man in Analogie zu Beispiel 23 und isolierte nach Aufarbeitung und Reinigung 48 mg (99 µmol, 90%) eines Gemisches der beiden Titelverbindungen als farbloses Öl.

IR (Film): 3600-3200, 3040, 2960, 2920, 2870, 1735, 1605, 1510, 1450, 1435, 1230, 1160, 995, 970, 895, 835 und 735 $cm^{-1}$.

Die chromatographische Trennung lieferte 27 mg (56 µmol, 51%) des unpolareren Alkohols, dem man die Struktur A zuordnete, sowie 18 mg (37 µmol, 34%) des polareren Alkohols dem man die Struktur B zuordnete.

**Beispiel 144a**

(1S,4R,5S(5Z),6S)-7-[4-(4-Fluorphenyl)-6-[(1E,4RS)-3-oxo-4,7-dimethyl-octa-1,6-dienyl]-2-oxa-bicyclo[2.2.1] hept-5-yl]-5-heptensäuremethylester:

48,6 mg (135 µmol) des in Beispiel 138b dargestellten Aldehyds setzte man in Analogie zu Beispiel 23a unter Verwendung von Dimethyl-(2-oxo-3,6-dimethyl-5-heptenyl)-phosphonat um und isolierte nach Aufarbeitung und Reinigung 53 mg (110 µmol, 81%) der Titelverbindung als farbloses Öl.

IR (Film): 3040, 3000, 2960, 2930, 2870, 1735, 1690, 1665, 1620, 1510, 1450, 1435, 1370, 1230, 1160, 1040, 995, 975, 900 und 830 $cm^{-1}$.

**Beispiel 145**

(1S,4R,5S(5Z),6S)-7-[4-(4-Fluorphenyl)-6-[(1E,3S,4RS)-3-hydroxy-4,7-dimethyl-octa-1,6-dienyl]-2-oxabicyclo [2.2.1]hept-5-yl]-5-heptensäure:

27 mg (56 µmol) des in Beispiel 144 dargestellten unpolaren Esters verseifte man in Analogie zu Beispiel 2 und isolierte nach Aufarbeitung und Reinigung 23 mg (49 µmol, 87%) der Titelver-bindung als farbloses Öl.

IR (Film): 3600-2400, 3040, 2970, 2930, 2880, 1710, 1610, 1515, 1455, 1300, 1235, 1160, 995, 970, 895 und 835 $cm^{-1}$.

**Beispiel 146**

(1S,4R,5S(5Z),6S)-7-[4-(4-Fluorphenyl)-6-[(1E,3R,4RS)-3-hydroxy-4,7-dimethyl-octa-1,6-dienyl]-2-oxabicyclo [2.2.1]hept-5-yl]-5-heptensäure:

18 mg (37 µmol) des in Beispiel 144 dargestellten polaren Esters verseifte man in Analogie zu Beispiel

2 und isolierte nach Aufarbeitung und Reinigung 16 mg (34 µmol, 92%) der Titelver-bindung als farbloses Öl.

IR (Film): 3600-2400, 3040, 2970, 2930, 2880, 1725, 1710, 1610, 1515, 1450, 1300, 1235, 1160, 995, 970, 895 und 835 $cm^{-1}$.

**Beispiel 147**

(1S,4R,5S(5Z),6R)-7-[4-(4-Fluorphenyl)-6-(3-phenylureidoiminomethyl)-2-oxabicyclo[2.2.1]hept-5-yl]-5-heptensäuremethylester:

18 mg (50 µmol) des in Beispiel 138b dargestellten Aldehyds setzte man in Analogie zu Beispiel 26 unter Verwendung von 4-Phenylsemicarbazid um und isolierte nach Aufarbeitung und Reinigung 22,3 mg (45 µmol, 90%) der Titelverbindung als farbloses Öl.

IR (Film): 3390, 3200, 3150-3050, 3010, 2940, 2880, 1730, 1690, 1595, 1535, 1515, 1450, 1235, 895, 830, 755 und 690 $cm^{-1}$.

**Beispiel 148**

(1S,4R,5S(5Z),6R)-7-[4-(4-Fluorphenyl)-6-(3-phenylureidoiminomethyl)-2-oxabicyclo[2.2.1]hept-5-yl]-5-heptensäure:

22,3 mg (45 µmol) des in Beispiel 147 dargestellten Esters verseifte man in Analogie zu Beispiel 2 und isolierte nach Aufarbeitung und Reinigung 18 mg (37,5 µmol, 83%) der Titelverbindung als farbloses Öl.

IR (Film): 3600-2400, 3390, 3200, 3080, 3010, 2940, 2880, 1700, 1690, 1595, 1535, 1515, 1450, 1235, 895, 830, 755 und 690 $cm^{-1}$.

**Beispiel 149**

(1S,4R,5S(5Z),6R)-7-[4-(4-Fluorphenyl)-6-diphenylmethoxyiminomethyl-2-oxabicyclo[2.2.1]hept-5-yl]-5-heptensäuremethylester:

17,4 mg (48 µmol) des in Beispiel 138b dargestellten Aldehyds setzte man in Analogie zu Beispiel 26 um und isolierte nach Aufarbeitung und Reinigung 25,7 mg (47 µmol, 98%) der Titelverbindung als farbloses Öl.

IR (Film): 3090, 3060, 3030, 3000, 2930, 2870, 1735, 1605, 1515, 1495, 1450, 1300, 1235, 1160, 1040, 1020, 935, 895, 830, 740 und 700 $cm^{-1}$.

**Beispiel 150**

(1S,4R,5S(5Z),6R)-7-[4-(4-Fluorphenyl)-6-diphenylmethoxyiminomethyl-2-oxabicyclo[2.2.1]hept-5-yl]-5-heptensäure:

25,7 mg (47 µmol) des in Beispiel 149 dargestellten Esters verseifte man in Analogie zu Beispiel 2 und isolierte nach Aufarbeitung und Reinigung 24 mg (45 µmol, 97%) der Titelverbindung als farbloses Öl.

IR (Film): 3600-2400, 3090, 3060, 3030, 3000, 2930, 2870, 1730, 1705, 1605, 1515, 1495, 1450, 1300, 1235, 1160, 1040, 1020, 1000, 935, 900, 830, 740 und 700 $cm^{-1}$.

**Beispiel 151**

(1S,4R,5S(5Z),6R)-7-[4-(4-Fluorphenyl)-6-(1-naphtylmethoxyiminomethyl)-2-oxabicyclo[2.2.1]hept-5-yl]-5-heptensäuremethylester:

17,9 mg (50 µmol) des in Beispiel 138b dargestellten Aldehyds setzte man in Analogie zu Beispiel 26 unter Verwendung von 1-Naphtylmethoxyamin um und isolierte nach Aufarbeitung und Reini-qung 24 mg (47 µmol, 93%) der Titelverbindung als farbloses Öl.

IR (Film): 3060, 3040, 3000, 2940, 2870, 1730, 1605, 1595, 1510, 1450, 1435, 1365, 1230, 1160, 1035, 1010, 1000, 895, 830, 800, 790, 775 und 635 $cm^{-1}$.

**Beispiel 152**

(1S,4R,5S(5Z),6R)-7-[4-(4-Fluorphenyl)-6-(1-naphtylmethoxyiminomethyl)-2-oxabicyclo[2.2.1]hept-5-yl]-5-heptensäure:

24 mg (47 µmol) des nach Beispiel 151 dargestellten Esters verseifte man in Analogie zu Beispiel 2 und isolierte nach Aufarbeitung und Reinigung 20 mg (40 µmol, 85%) der Titelverbindung als wachsartigen Feststoff.

IR (KBr): 3600-2400, 3070, 3050, 3010, 2940, 2880, 1730, 1705, 1610, 1600, 1515, 1230, 1160, 1040, 1015, 1000, 920, 900, 835, 800, 795, 780 und 585 $cm^{-1}$.

**Beispiel 153**

(1S,4R,5S(5Z),6S)-7-[4-(4-Fluorphenyl)-6-[3(S)-hydroxy-4-(4-fluor-phenoxy)-1(E)-butenyl]-2-oxa-bicyclo[2.2.1]hept-5-yl]-5-heptensäuremethylester (A) und (1S,4R,5S(5Z),6S)-7-[4-(4-Fluorphenyl)-6-[3-(R)-hydroxy-4-(4-fluor-phenoxy)-1(E)-butenyl]-2-oxabicyclo[2.2.1]hept-5-yl]-5-hepten-säuremethylester (B):

36 mg (70,5 μmol) des in Beispiel 153a dargestellten ungesättigten Ketons reduzierte man in Analogie zu Beispiel 23 und isolierte nach Aufarbeitung und Reinigung 31 mg (60,5 μmol, 86%) eines Gemisches der beiden Titelverbindungen als Farbloses Öl.

IR (Film): 3600-3200, 3050, 3000, 2970, 2940, 2870, 1735, 1605, 1510, 1245, 1220, 1160, 1035, 895, 830, 760 und 735 $cm^{-1}$.

Die chromatographische Trennung lieferte 11 mg (21,5 μmol, 30%) des unpolareren Alkohols, dem man die Struktur A zuordnete, sowie 16 mg (31 μmol, 44%) des polareren Alkohols dem man die Struktur B zuordnete.

**Beispiel 153a**

(1S,4R,5S(5Z),6S)-7-[4-(4-Fluorphenyl)-6-[3-oxo-4-(4-fluorphenoxy)-1(E)-butenyl]-2-oxabicyclo[2.2.1]hept-5-yl]-5-heptensäuremethylester:

55,4 mg (154 μmol) des in Beispiel 138b dargestellten Aldehyds setzte man in Analogie zu Beispiel 30a unter Verwendung von Dimethyl-(2-oxo-3-(p-Fluorphenoxy)-propyl)-phosphonat um und isolierte nach Aufarbeitung und Reinigung 36 mg (70,5 μmol, 46%) der Titelverbindung als farb-loses Öl.

IR (Film): 3070, 3040, 3000, 2970, 2940, 2870, 1730, 1690, 1620, 1510, 1505, 1435, 1290, 1220, 1200, 1160, 1040, 995, 900 und 830 $cm^{-1}$.

**Beispiel 154**

(1S,4R,5S(5Z),6S)-7-[4-(4-Fluorphenyl)-6-[3(S)-hydroxy-4-(4-fluor-phenoxy)-1(E)-butenyl]-2-oxa-bicyclo[2.2.1]hept-5-yl]-5-heptensäure:

11 mg (21,5 μmol) des in Beispiel 153 dargestellten unpolaren Esters verseifte man in Analogie zu Beispiel 2 und isolierte nach Aufarbeitung und Reinigung 10 mg (20 μmol, 93%) der Titelver-bindung als farbloses Öl.

IR (Film): 3600-2400, 3080, 3030, 3010, 2980, 2940, 2870, 1710, 1610, 1605, 1510, 1460, 1300, 1225, 1165, 1035, 890, 835, 760, 590 und 520 $cm^{-1}$.

**Beispiel 155**

(1S,4R,5S(5Z),6S)-7-[4-(4-Fluorphenyl)-6-[3(R)-hydroxy-4-(4-fluor-phenoxy)-1(E)-butenyl]-2-oxa-bicyclo[2.2.1]hept-5-yl]-5-heptensäure:

16 mg (31 μmol) des in Beispiel 153 dargestellten polaren Esters verseifte man in Analogie zu Beispiel 2 und isolierte nach Aufarbeitung und Reinigung 13 mg (26 μmol, 84%) der Titelver-bindung als farbloses Öl.

IR (Film): 3600-2400, 3080, 3030, 3010, 2980, 2940, 2880, 1710, 1610, 1605, 1510, 1460, 1300, 1225, 1165, 1040, 895, 830, 760, 590 und 520 $cm^{-1}$.

**Beispiel 156**

(1S,4R,5S(5Z),6S)-7-[4-(4-Fluorphenyl)-6-[3(S)-hydroxy-4(RS)-fluor-1(E)-octenyl]-2-oxabicyclo[2.2.1]hept-5-yl]-5-heptensäuremethylester (A) und (1S,4R,5S(5Z),6S)-7-[4-(4-Fluorphenyl)-6-[3(R)-hydroxy-4(RS)-fluor-1(E)-octenyl]-2-oxabicyclo[2.2.1]hept-5-yl]-5-heptensäuremethyl ester (B):

51 mg (107 μmol) des in Beispiel 156a dargstellten ungesättigten Ketons reduzierte man in Analogie zu Beispiel 23 und isolierte nach Aufarbeitung und Reinigung 49 mg (103 μmol, 96%) eines Gemisches der beiden Titelverbindungen als farbloses Öl.

IR (Film): 3600-3200, 3040, 3000, 2950, 2930, 2860, 1735, 1605, 1510, 1450, 1435, 1230, 1160, 1040, 995, 970, 895 und 835 $cm^{-1}$.

Die chromatographische Trennung lieferte 26 mg (54,5 μmol, 51%) des unpolareren Alkohols, dem man

die Struktur A zuordnete, sowie 19 mg (40 µmol, 37%) des polareren Alkohols dem man die Struktur B zuordnete.

**Beispiel 156a**

(1S,4R,5S(5Z),6S)-7-[4-(4-Fluorphenyl)-6-[3-oxo-4(RS)-fluor-1(E)-octenyl]-2-oxabicyclo[2.2.1]hept-5-yl]-5-heptensäuremethylester:

48 mg (133 µmol) des in Beispiel dargestellten Aldehyds setzte man in Analogie zu Beispiel 23a unter Verwendung von Dimethyl-(2-oxo-3-fluor-heptyl)-phosphonat um und isolierte nach Aufarbeitung und Reinigung 51 mg (107 µmol, 81%) der Titelverbindung als farbloses Öl.

IR (Film): 3040, 3020, 3000, 2950, 2920, 2870, 1730, 1690, 1615, 1510, 1450, 1430, 1310, 1290, 1230, 1160, 1045, 995, 975, 900 und 830 $cm^{-1}$.

**Beispiel 157**

(1S,4R,5S(5Z),6S)-7-[4-(4-Fluorphenyl)-6-[3(S)-hydroxy-4(RS)-fluor-1(E)-octenyl]-2-oxabicyclo[2.2.1]hept-5-yl]-5-heptensäure:

26 mg (55 µmol) des in Beispiel 156 dargestellten unpolaren Esters verseifte man in Analogie zu Beispiel 2 und isolierte nach Aufarbeitung und Reinigung 24 mg (52 µmol, 95%) der Titelver-bindung als farbloses Öl.

IR (Film): 3600-2400, 3050, 3010, 2940, 2920, 2880, 1710, 1610, 1515, 1455, 1300, 1235, 1115, 1040, 995, 970, 895 und 835 $cm^{-1}$.

**Beispiel 158**

(1S,4R,5S(5Z),6S)-7-[4-(4-Fluorphenyl)-6-[3(R)-hydroxy-4(RS)-fluor-1(E)-octenyl]-2-oxabicyclo[2.2.1]hept-5-yl]-5-heptensäure:

19 mg (40 µmol) des in Beispiel 156 dargestellten polaren Esters verseifte man in Analogie zu Beispiel 2 und isolierte nach Aufarbeitung und Reinigung 18 mg (39 µmol, 97%) der Titelver-bindung als farbloses Öl.

IR (Film): 3600-2400, 3050, 3010, 2940, 2920, 2880, 1725, 1710, 1610, 1515, 1300, 1235, 1115, 1040, 995, 970, 895 und 835 $cm^{-1}$.

**Beispiel 159**

(1S,4R,5S(5Z),6S)-7-[4-(4-Fluorphenyl)-6-[3(S)-hydroxy-4(RS)-fluor-1(E)-nonenyl]-2-oxabicyclo[2.2.1]hept-5-yl]-5-heptensäuremethylester (A) und (1S,4R,5S(5Z),6S)-7-[4-(4-Fluorphenyl)-6-[3(R)-hydroxy-4(RS)-fluor-1(E)-nonenyl]-2-oxabicyclo[2.2.1]hept-5-yl]-5-heptensäuremethylester (B):

46 mg (94 µmol) des in Beispiel 159a dargestellten ungesättigten Ketons reduzierte man in Analogie zu Beispiel 23 und isolierte nach Aufarbeitung und Reinigung 43 mg (88 µmol, 97%) eines Gemisches der beiden Titelverbindungen als farbloses Öl.

IR (Film): 3600-3200, 3080, 3040, 3000, 2960, 2930, 2870, 1735, 1605, 1510, 1435, 1230, 1160, 1040, 995, 970, 900 und 835 $cm^{-1}$.

Die chromatographische Trennung lieferte 24 mg (50 µmol, 52%) des unpolareren Alkohols, dem man die Struktur A zuordnete, sowie 17 mg (34 µmol, 37%) des polareren Alkohols dem man die Struktur B zuordnete.

**Beispiel 159a**

(1S,4R,5S(5Z),6S)-7-[4-(4-Fluorphenyl)-6-[3-oxo-4(RS)-fluor-1(E)-nonenyl]-2-oxabicyclo[2.2.1]hept-5-yl]-5-heptensäuremethylester:

46 mg (128 µmol) des in Beispiel 138b dargestellten Aldehyds setzte man in Analogie zu Beispiel 23a unter Verwendung von Dimethyl-(2-oxo-3-fluor-octyl)-phosphonat um und isolierte nach Aufarbeitung und Reinigung 46 mg (94 µmol, 74%) der Titelverbindung als farbloses Öl.

IR (Film): 3070, 3040, 3000, 2950, 2920, 2860, 1735, 1690, 1615, 1510, 1450, 1435, 1230, 1160, 1045, 995, 900 und 830 $cm^{-1}$.

**Beispiel 160**

(1S,4R,5S(5Z),6S)-7-[4-(4-Fluorphenyl)-6-[3(S)-hydroxy-4(RS)-fluor-1(E)-nonenyl]-2-oxabicyclo[2.2.1]hept-5-yl]-5-heptensäure:

24 mg (50 µmol) des in Beispiel 159 dargestellten unpolaren Esters verseifte man in Analogie zu Beispiel

2 und isolierte nach Aufarbeitung und Reinigung 21 mg (44 µmol, 88%) der Titelver-bindung als farbloses Öl.

IR (Film): 3600-2400, 3050, 3010, 2960, 2930, 2870, 1725, 1710, 1610, 1515, 1455, 1410, 1300, 1235, 1155, 1000, 975, 895 und 835 $cm^{-1}$.

**Beispiel 161**

(1S,4R,5S(5Z),6S)-7-[4-(4-Fluorphenyl)-6-[3(R)-hydroxy-4(RS)-fluor-1(E)-nonenyl]-2-oxabicyclo[2.2.1]hept-5-yl]-5-heptensäure:

17 mg (34 µmol) des in Beispiel 159 dargestellten polaren Esters verseifte man in Analogie zu Beispiel 2 und isolierte nach Aufarbeitung und Reinigung 16 mg (33 µmol, 98%) der Titelver-bindung als farbloses Öl.

IR (Film): 3600-2400, 3050, 3010, 2950, 2930, 2870, 1710, 1610, 1515, 1455, 1410, 1300, 1235, 1155, 1045, 1000, 975, 895 und 835 $cm^{-1}$.

**Beispiel 162**

(1S,4R,5S(5Z),6S)-7-[4-(4-Fluorphenyl)-6-[3(S)-hydroxy-4,4-trimethylenon-1(E)-nonen-7-inyl]-2-oxabicyclo[2.2.1]hept-5-yl]-5-heptensäuremethylester (A) und (1S,4R,5S(5Z),6S)-7-[4-(4-Fluorphenyl)-6-[3(R)-hydroxy-4,4-trimethylenon-1(E)-nonen-7-inyl[-2-oxabicyclo[2.2.1]hept-5-yl]-5-heptensäuremethylester (B):

30 mg (50 µmol) des in Beispiel 162a dargestellten ungesättigten Ketons reduzierte man in Analogie zu Beispiel 23 und isolierte nach Aufarbeitung und Reinigung 28 mg (55 µmol, 95%) eines Gemisches der beiden Titelverbindungen als farbloses Öl.

IR (Film): 3600-3200, 3080, 3030, 2970, 2930, 2870, 1735, 1605, 1510, 1450, 1435, 1225, 1160, 1040, 995, 970, 895 und 835 $cm^{-1}$.

Die chromatographische Trennung lieferte 18 mg (35 µmol, 60%) des unpolareren Alkohols, dem man die Struktur A zuordnete, sowie 8 mg (16 µmol, 27%) des polareren Alkohols dem man die Struktur B zuordnete.

**Beispiel 162a**

(1S,4R,5S(5Z),6S)-7-[4-(4-Fluorphenyl)-6-[3-oxo-4,4-trimethylenon-1(E)-nonen-7-inyl]-2-oxa-bicyclo[2.2.1]hept-5-yl]-5-heptensäuremethylester:

46 mg (128 µmol) des in Beispiel 138b dargestellten Aldehyds setzte man in Analogie zu Beispiel 23a unter Verwendung von Dimethyl-(2-oxo-3,3-trimethylenon-oct-6-inyl)-phosphonat um und isolierte nach Aufarbeitung und Reinigung 31 mg (61 µmol, 48%) der Titelverbindung als farbloses Öl.

IR (Film): 3040, 2970, 2940, 2860, 1735, 1680, 1620, 1510, 1450, 1453, 1230, 1160, 995, 900 und 830 $cm^{-1}$.

**Beispiel 163**

(1S,4R,5S(5Z),6S)-7-[4-(4-Fluorphenyl)-6-[3(S)-hydroxy-4,4-trimethylenon-1(E)-nonen-7-inyl]-2-oxabicyclo[2.2.1]hept-5-yl]-5-heptensäure:

18 mg (35 µmol) des in Beispiel 162 dargestellten unpolaren Esters verseifte man in Analogie zu Beispiel 2 und isolierte nach Aufarbeitung und Reinigung 15 mg (30 µmol, 87%) der Titelver-bindung als farbloses Öl.

IR (Film): 3700-2400, 3040, 2980, 2940, 2880, 2840, 1710, 1610, 1515, 1445, 1435, 1410, 1300, 1230, 1160, 1040, 1015, 995, 970, 895 und 835 $cm^{-1}$.

**Beispiel 164**

(1S,4R,5S(5Z),6S)-7-[4-(4-Fluorphenyl)-6-[3(R)-hydroxy-4,4-trimethylen-1(E)-nonen-7-inyl]-2-oxabicyclo[2.2.1]hept-5-yl]-5-heptensäure:

8 mg (16 µmol) des in Beispiel 162 dargestellten polaren Esters verseifte man in Analogie zu Beispiel 2 und isolierte nach Aufarbeitung und Reinigung 7 mg (14 µmol, 88%) der Titelverbindung als farbloses Öl.

IR (Film): 3600-2400, 3040, 2980, 2940, 2880, 2840, 1725, 1710, 1610, 1515, 1450, 1435, 1410, 1300, 1235, 1160, 1040, 1015, 995, 970, 890 und 835 $cm^{-1}$.

**Beispiel 165**

(1S,4R,5S(5Z),6S)-7-[4-(4-Fluorphenyl)-6-[3(S)-hydroxy-4,4-trimethylen-1(E)-nonen-6-inyl]-2-oxabicyclo[2.2.1]hept-5-yl]-5-heptensäuremethylester (A) und (1S,4R,5S(5Z),6S)-7-[4-(4-Fluorphenyl)-6-[3(R)-hydroxy-4,4-trimethylen-1(E)-nonen-6-inyl]-2-oxabicyclo[2.2.1]hept-5-yl]-5-heptensäuremethylester (B):

50 mg (99 μmol) des in Beispiel 165a dargestellten ungesättigten Ketons reduzierte man in Analogie zu Beispiel 23 und isolierte nach Aufarbeitung und Reinigung 46 mg (90 μmol, 91 %) eines Gemisches der beiden Titelverbindungen als farbloses Öl.

IR (Film): 3600-3200, 3040, 2970, 2930, 2870, 1735, 1605, 1510, 1450, 1435, 1230, 1160, 1040, 995, 970, 895 und 835 $cm^{-1}$.

Die chromatographische Trennung lieferte 27 mg (53 μmol, 54%) des unpolareren Alkohols, dem man die Struktur A zuordnete, sowie 17 mg (33 μmol, 34%) des polareren Alkohols dem man die Struktur B zuordnete.

**Beispiel 165a**

(1S,4R,5S(5Z),6S)-7-[4-(4-Fluorphenyl)-6-[3-oxo-4,4-trimethylen-1(E)-nonen-6-inyl]-2-oxabicyclo[2.2. 1]hept-5-yl]-5-heptensäuremethylester:

45 mg (125 μmol) des in Beispiel 138b dargestellten Aldehyds setzte man in Analogie zu Beispiel 23a unter Verwendung von Dimethyl-(2-oxo-3,3-trimethylen-5-octinyl)-phosphonat um und isolierte nach Aufarbeitung und Reinigung 50 mg (99 μmol,79 %) der Titelverbindung als farbloses Öl.

IR (Film): 3070, 3040, 2970, 2940, 2870, 1730, 1685, 1620, 1510, 1450, 1430, 1230, 1160, 1045, 995, 900 und 830 $cm^{-1}$.

**Beispiel 166**

(1S,4R,5S(5Z),6S)-7-[4-(4-Fluorphenyl)-6-[3(S)-hydroxy-4,4-trimethylen-1(E)-nonen-6-inyl]-2-oxabicyclo[2.2. 1]hept-5-yl]-5-heptensäure:

26 mg (51 μmol) des in Beispiel 165 dargestellten unpolaren Esters verseifte man in Analogie zu Beispiel 2 und isolierte nach Aufarbeitung und Reinigung 24 mg (49 μmol,95%) der Titelver-bindung als farbloses Öl.

IR (Film): 3600-2400, 3040, 2980, 2940, 2880, 1710, 1610, 1515, 1455, 1430, 1405, 1320, 1300, 1235, 1160, 995, 970, 895 und 835 $cm^{-1}$.

**Beispiel 167**

(1S,4R,5S(5Z),6S)-7-[4-(4-Fluorphenyl)-6-[3(R)-hydroxy-4,4-trimethylen-1(E)-nonen-6-inyl]-2-oxabicyclo[2.2. 1]hept-5-yl]-5-heptensäure:

16 mg (31 μmol) des in Beispiel 165 dargestellten polaren Esters verseifte man in Analogie zu Beispiel 2 und isolierte nach Aufarbeitung und Reinigung 15 mg (30 μmol, 98%) der Titelverbindung als farbloses Öl.

IR (Film): 3600-2400, 3040, 2970, 2940, 2870, 1725, 1710, 1610, 1515, 1455, 1430, 1405, 1320, 1300, 1230, 1160, 1040, 995, 970, 895 und 835 $cm^{-1}$.

**Beispiel 168**

(1S,4R,5S(5Z),6S)-7-[4-(4-Fluorphenyl)-6-[(1E,3R,4RS,6Z)-3-hydroxy-4-methyl-7-chlor-octa-1,6-dienyl]-2-o xabicyclo[2.2.1]hept-5-yl]-5-heptensäuremethylester (A) und (1S,4R,5S(5Z),6S)-7-[4-(4-Fluorphenyl)-6-[(1E, 3S,4RS,6Z)-3-hydroxy-4-methyl-7-chlor-octa- 1,6-dienyl]-2-oxabicyclo[2.2.1]hept-5-yl]-5-heptensäuremethylester (B):

52 mg (103 μmol) des in Beispiel 168a dargestellten ungesättigten Ketons reduzierte man in Analogie zu Beispiel 23 und isolierte nach Aufarbeitung und Reinigung 52 mg (103 μmol, 99%) eines Gemisches der beiden Titelverbindungen als farbloses Öl.

IR (Film): 3600-3200, 3040, 2950, 2920, 2870, 1730, 1605, 1510, 1430, 1230, 1160, 995, 970, 895 und 830 $cm^{-1}$.

Die chromatographische Trennung lieferte 30 mg (59 μmol, 58%) des unpolareren Alkohols, dem man die Struktur A zuordnete, sowie 21 mg (42 μmol, 40%) des polareren Alkohols dem man die Struktur B zuordnete.

**Beispiel 168a**

(1S,4R,5S(5Z),6S)-7-[4-(4-Fluorphenyl)-6-[(1E,4RS,6Z)-3-oxo-4-methyl-7-chlor-octa-1,6-dienyl]-2-oxabicyclo [2.2.1]hept-5-yl]-5-hepten-säuremethylester:

48 mg (133 μmol) des in Beispiel 138b dargestellten Aldehyds setzte man in Analogie zu Beispiel 23a unter Verwendung von Dimethyl-(2-oxo-3-methyl-6-chlor-hept-5(Z)-en)-phosphonat um und isolierte nach Aufarbeitung und Reinigung 52 mg (103 μmol, 78%) der Titelverbindung als farbloses Öl.

IR (Film): 3040, 2970, 2940, 2930, 2870, 1730, 1690, 1665, 1620, 1510, 1450, 1430, 1360, 1225, 1160, 1040, 995, 975, 900 und 830 $cm^{-1}$.

**Beispiel 169**

(1S,4R,5S(5Z),6S)-7-[4-(4-fluorphenyl)-6-[(1E,3R,4RS,6Z)-3-hydroxy-4-methyl-7-chlor-octa-1,6-dienyl]-2-oxa-bicyclo[2.2.1]hept-5-yl]-5-heptensäure:

30 mg (59 µmol) des in Beispiel 168 dargestellten unpolaren Esters verseifte man in Analogie zu Beispiel 2 und isolierte nach Aufarbeitung und Reinigung 25 mg (51 µmol, 86%) der Titelver-bindung als farbloses Öl.

IR (Film): 3600-2400, 3050, 3010, 2970, 2960, 2930, 2880, 1710, 1610, 1515, 1300, 1235, 1160, 1015, 995, 970, 895 und 835 $cm^{-1}$.

**Beispiel 170**

(1S,4R,5S(5Z),6S)-7-[4-(4-Fluorphenyl)-6-[(1E,3S,4RS)-3-hydroxy-4-methyl-7-chlor-octa-1,6-dienyl]-2-oxabicyclo[2.2.1]hept-5-yl]-5-heptensäure:

21 mg (42 µmol) des in Beispiel 168 dargestellten polaren Esters verseifte man in Analogie zu Beispiel 2 und isolierte nach Aufarbeitung und Reinigung 18 mg (37 µmol, 87%) der Titelver-bindung als farbloses Öl.

IR (Film): 3600-2400, 3060, 3020, 2970, 2930, 2880, 1710, 1610, 1515, 1455, 1410, 1300, 1235, 1160, 1015, 995, 970, 895 und 835 $cm^{-1}$.

**Beispiel 171**

(1S,4R,5S(5Z),6S)-7-[4-(4-Fluorphenyl)-6-[(1E,3R,4RS)-3-hydroxy-4,6-dimethyl-heptenyl]-2-oxa-bicyclo[2.2.1] hept-5-yl]-5-heptensäuremethylester (A) und (1S,4R,5S(5Z),6S)-7-[4-(4-Fluorphenyl)-6-[(1E,3S,4RS)-3-hydroxy-4,6-dimethyl-heptenyl]-2-oxabicyclo[2.2.1]hept-5-yl]-5-hepten-säuremethylester (B):

47 mg (100 µmol) des in Beispiel 171a dargestellten ungesättigten Ketons reduzierte man in Analogie zu Beispiel 23 und isolierte nach Aufarbeitung und Reinigung 47 mg (99 µmol,99 %) eines Gemisches der beiden Titelverbindungen als farbloses Öl.

IR (Film): 3600-3250, 3040, 2950, 2920, 2860, 1735, 1605, 1450, 1435, 1365, 1230, 1160, 995, 970, 895 und 835 $cm^{-1}$.

Die chromatographische Trennung lieferte 28 mg (59 µmol, 59%) des unpolareren Alkohols, dem man die Struktur A zuordnete, sowie 18 mg (38 µmol, 38%) des polareren Alkohols dem man die Struktur B zuordnete.

**Biespiel 171a**

(1S,4R,5S(5Z),6S)-7-[4-(4-Fluorphenyl)-6-[(1E,4RS)-3-oxo-4,6-dimethyl-heptenyl]-2-oxabicyclo[2.2.1]hept-5-yl]-5-heptensäuremethylester:

47 mg (130 µmol) des in Beispiel 138b dargestellten Aldehyds setzte man in Analogie zu Beispiel 23a unter Verwendung von Dimethyl-(2-oxo-3,5-dimethyl-hexyl)-phosphonat um und isolierte nach Aufarbeitung und Reinigung 48 mg (102 µmol, 78%) der Titelverbindung als farbloses Öl.

IR (Film): 3060, 3040, 2950, 2920, 2860, 1735, 1690, 1665, 1620, 1510, 1450, 1435, 1365, 1230, 1190, 1160, 1040, 995, 975, 900 und 830 $cm^{-1}$.

**Beispiel 172**

(1S,4R,5S(5Z),6S)-7-[4-(4-Fluorphenyl)-6-[(1E,3R,4RS)-3-hydroxy-4,6-dimethyl-heptenyl]-2-oxa-bicyclo[2.2.1] hept-5-yl]-5-heptensäure:

28 mg (69 µmol) des in Beispiel 171 dargestellten unpolaren Esters verseifte man in Analogie zu Beispiel 2 und isolierte nach Aufarbeitung und Reinigung 24 mg (52 µmol, 76%) der Titelver-bindung als farbloses Öl.

IR (Film): 3600-2400, 3050, 3010, 2960, 2930, 2870, 1725, 1710, 1610, 1515, 1460, 1300, 1235, 1165, 995, 970, 895 und 835 $cm^{-1}$.

**Beispiel 173**

(1S,4R,5S(5Z),6S)-7-[4-(4-Fluorphenyl)-6-[(1E,3S,4RS)-3-hydroxy-4,6-dimethyl-heptenyl]-2-oxa-bicyclo[2.2.1] hept-5-yl]-5-heptensäure:

18 mg (38 µmol) des in Beispiel 171 dargestellten polaren Esters verseifte man in Analogie zu Beispiel

2 und isolierte nach Aufarbeitung und Reinigung 17 mg (37 µmol, 97%) der Titelver-bindung als farbloses Öl.

IR (Film): 3600-2400, 3050, 3010, 2960, 2930, 2870, 1710, 1610, 1515, 1460, 1385, 1300, 1235, 1165, 995, 970, 895 und 835 $cm^{-1}$.

**Beispiel 174**

(1S,4R,5S(5Z),6S)-7-[4-(4-Fluorphenyl)-6-(2-pyridyl-2-vinyl)-2-oxabicyclo[2.2.1]hept-5-yl]-5-heptensäuremethylester:

Die blassgelbe Lösung aus 26,4 mg 2-Picolyl-triphenylphosphoniumchlorid-natriumamid in 250 µl wasserfreiem Tetrahydrofuran versetzte man mit der Lösung von 19 mg (53 µmol) des in Beispiel 138b dargestellten Aldehyds in 200 µl wasserfreiem Tetrahydrofuran und ließ zwei Stunden bei 25°C unter einer Atmosphäre aus trockenem Argon reagieren. Die Reaktionslösung wurde direkt auf zwei analytische Dünnschichtplatten aufgetragen, mit einem Gemisch aus Trichlormethan/Ethanol entwickelt und mit Trichlormethan eluiert. Isoliert wurden 19 mg (44 µmol, 82%) der Titelver-bindung als farbloses Öl.

IR(Film): 3430, 3040, 3000, 2970, 2930, 2870, 1735, 1650, 1595, 1585, 1565, 1515, 1465, 1430, 1295, 1225, 1040, 995, 970, 900, 835, 760, 590 und 530 $cm^{-1}$.

**Beispiel 175**

(1S,4R,5S(5Z),6S)-7-[4-(4-Fluorphenyl)-6-(2-pyridyl-2-vinyl)-2-oxabicyclo[2.2.1]hept-5-yl]-5-heptensäure:

19 mg (44 µmol) des in Beispiel 174 dargestellten Esters verseifte man in Analogie zu Beispiel 2 und isolierte nach Aufarbeitung und Reinigung 16 mg (38 µmol, 86%) der Titelverbindung als farb-loses Öl.

IR (Film): 3600-2300, 3430, 3040, 3000, 2970, 2930, 2870, 2540, 2100-1800, 1725, 1710, 1650, 1595, 1585, 1565, 1515, 1470, 1430, 1295, 1230, 1040, 995, 970, 900, 835, 760, 585 und 530 $cm^{-1}$.

**Beispiel 176**

(1S,4R,5S(5Z),6S)-7-[4-(4-Fluorphenyl)-6-(2-phenylvinyl)-2-oxabicyclo[2.2.1]hept-5-yl]-5-hepten-säuremethylester:

18 mg (50 µmol) des in Beispiel 138b dargestellten Aldehyds setzte man in Analogie zu Beispiel 174 unter Verwendung von Benzyltriphenylphosphoniumbromid-natriumamid um und isolierte nach Aufarbeitung und Reinigung 17 mg (40 µmol, 81%) der Titelverbindung als farbloses Öl.

IR(Film): 3080, 3060, 3020, 3000, 2970, 2940, 2870, 1735, 1595, 1510, 1445, 1435, 1230, 1195, 1160, 1040, 995, 965, 895, 835, 745 und 695 $cm^{-1}$.

**Biespiel 177**

(1S,4R,5S(5Z),6S)-7-[4-(4-Fluorphenyl)-6-(2-phenylvinyl)-2-oxabicyclo[2.2.1]hept-5-yl]-5-hepten-säure:

17 mg (40 µmol) des in Beispiel 176 dargestellten Esters verseifte man in Analogie zu Beispiel 2 und isolierte nach Aufarbeitung und Reinigung 15 mg (36 µmol, 89%) der Titelverbindung als farb-loses Öl.

IR (Film): 3600-2400, 3080, 3060, 3030, 3010, 2980, 2940, 2880, 1730, 1710, 1610, 1600, 1515, 1450, 1410, 1300, 1230, 1160, 1040, 995, 965, 895, 835, 745, 695, 585 und 520 $cm^{-1}$.

**Beispiel 178**

(1S,4R,5S(5Z),6S)-7-[4-(4-Fluorphenyl)-6-[1(E/Z),4(E)-pentadienyl]-2-oxabicyclo[2.2.1]hept-5-yl]-5-heptensäuremethylester:

20 mg (55 µmol) des in Beispiel 138b dargestellten Aldehyds setzte man in Analogie zu Beispiel 174 unter Verwendung von Crotyl-triphenylphosphoniumbromid-natriumamid um und isolierte nach Aufarbeitung und Reinigung 7 mg (18 µmol, 32%) der Titelverbindung als farbloses Öl.

IR(Film): 3040, 3010, 2930, 2870, 1735, 1610, 1515, 1450, 1410, 1300, 1235, 1160, 995, 900 und 835 $cm^{-1}$.

**Beispiel 179**

(1S,4R,5S(5Z),6S)-7-[4-(4-Fluorphenyl)-6-[1(E/Z),4(E)-pentadienyl]-2-oxabicyclo[2.2.1]hept-5-yl]-5-heptensäure:

7 mg (18 µmol) des in Beispiel 178 dargestellten Esters verseifte man in Analogie zu Beispiel 2 und isolierte nach Aufarbeitung und Reinigung 5 mg (13 µmol, 72%) der Titelverbindung als farbloses Öl.

IR (Film): 3600-2400, 3040, 3010, 2930, 2880, 1730, 1710, 1610, 1515, 1450, 1410, 1300, 1235, 1160,

990, 900 und 835 $cm^{-1}$.

**Beispiel 180**

(1S,4R,5S(5Z),6R)-7-[4-(4-Fluorphenyl)-6-(4-trifluormethylbenzyloxyiminomethyl)-2-oxabicyclo[2.2.1]hept-5-yl]-5-heptensäuremethylester:

17,9 mg (49 µmol) des in Beispiel 138b dargestellten Aldehyds setzte man in Analogie zu Beispiel 26 unter Verwendung von p-Trifluormethylphenylmethoxyamin um und isolierte nach Aufarbeitung und Reinigung 22,8 mg (43 µmol, 87%) der Titelverbindung als farbloses Öl.

IR (Film): 3070, 3040, 2940, 2870, 1735, 1620, 1605, 1510, 1435, 1415, 1325, 1230, 1160, 1125, 1065, 1015, 900, 830 und 820 $cm^{-1}$.

**Beispiel 181**

(1S,4R,5S(5Z),6R)-7-[4-(4-Fluorphenyl)-6-(4-trifluormethylbenzyloxyiminomethyl)-2-oxabicyclo[2.2.1]hept-5-yl]-5-heptensäure:

22 mg (43 µmol) des nach Beispiel 180 dargestellten Esters verseifte man in Analogie zu Beispiel 2 und isolierte nach Aufarbeitung und Reinigung 19 mg (37 µmol, 85%) der Titelverbindung als farbloses Öl.

IR (Film): 3600-2400, 3050, 3010, 2930, 2880, 1730, 1710, 1620, 1610, 1515, 1415, 1325, 1235, 1165, 1125, 1065, 1015, 900, 835 und 820 $cm^{-1}$.

**Beispiel 182**

(1S,4R,5S(5Z),6R)-7-[4-(4-Fluorphenyl)-6-(4-fluorbenzyloxyiminomethyl)-2-oxa*bicyclo[2.2.1]hept-5-yl]-5-heptensäuremethylester:

19,3 mg (53,5 µmol) des in Beispiel 138b dargestellten Aldehyds setzte man in Analogie zu Beispiel 26 unter Verwendung von p-Fluorphenylmethoxyamin um und isolierte nach Aufarbeitung und Reinigung 23,6 mg (48,8 µmol, 91%) der Titelverbindung als farbloses Öl.

IR (Film): 3070, 3040, 3000, 2940, 2870, 1735, 1605, 1510, 1435, 1365, 1220, 1160, 1040, 1015, 1000, 900 und 830 $cm^{-1}$.

**Beispiel 183**

(1S,4R,5S(5Z),6R)-7-[4-(4-Fluorphenyl)-6-(4-fluorbenzyloxyiminomethyl)-2-oxabicyclo[2.2.1]hept-5-yl]-5-heptensäure:

23,6 mg (48,8 µmol) des nach Beispiel 182 dargestellten Esters verseifte man in Analogie zu Beispiel 2 und isolierte nach Aufarbeitung und Reinigung 22 mg (46,9 µmol, 96%) der Titelverbindung als farbloses Öl.

IR (Film): 3600-2400, 3070, 3040, 3010, 2930, 2880, 1730, 1710, 1605, 1515, 1225, 1160, 1040, 1015, 1000, 900, 835 und 820 $cm^{-1}$.

**Beispiel 184**

(1S,4R,5S(5Z),6R)-7-[4-(4-Fluorphenyl)-6-(benzyloxyiminomethyl)-2-oxabicyclo[2.2.1]hept-5-yl]-5-heptensäuremethylester:

17,1 mg (47,4 µmol) des in Beispiel 138b dargestellten Aldehyds setzte man in Analogie zu Beispiel 26 unter Verwendung von Phenylmethoxyamin um und isolierte nach Aufarbeitung und Reinigung 17,8 mg (38,2 µmol, 81%) der Titelverbindung als farbloses Öl.

IR (Film): 3090, 3060, 3030, 3000, 2940, 2920, 2870, 1735, 1605, 1510, 1450, 1430, 1365, 1230, 1160, 1040, 1015, 1000, 900, 835, 750, 735 und 700 $cm^{-1}$.

**Beispiel 185**

(1S,4R,5S(5Z),6R)-7-[4-(4-Fluorphenyl)-6-(benzyloxyiminomethyl)-2-oxabicyclo[2.2.1]hept-5-yl]-5-heptensäure:

17,8 mg (38,2 µmol) des nach Beispiel 184 dargestellten Esters verseifte man in Analogie zu Beispiel 2 und isolierte nach Aufarbeitung und Reinigung 17 mg (31,4 µmol, 82%)der Titel verbindung als farbloses Öl.

IR (Film): 3600-2400, 3090, 3060, 3030, 3010, 2930, 2880, 1730, 1710, 1610, 1515, 1455, 1235, 1165, 1045, 1015, 1000, 900, 835, 820, 735 und 700 $cm^{-1}$.

**Beispiel 186**

(1S,4R,5S(5Z),6R(3S,4S))-7-[4-(4-Fluorphenyl)-6-(3-hydroxy-4-methyl-nona-1,6-diinyl)-2-oxabicyclo[2.2.1]hept-5-yl]-5-heptensäuremethylester:

3 mg (6,4 μmol) der nach Beispiel 187 dargestellten Säure löste man in 1 ml Dichlormethan, kühlte auf 0-5 °C und versetzte mit einer etherischen Lösung von Diazomethan. Nach Lösungsmittelabzug isolierte man 3 mg (6,4 μmol, 100%) der Titelverbindung als farbloses Öl.

IR (Film): 3600-3200, 3050, 3010, 2970, 2940, 2880, 2230, 1735, 1610, 1595, 1515, 1445, 1435, 1290, 1235, 1165, 1040, 1020, 990, 895, 835 und 820 $cm^{-1}$.

**Beispiel 187**

(1S,4R,5S(5Z),6R(3S,4S))-7-[4-(4-Fluorphenyl)-6-(3-hydroxy-4-methyl-nona-1,6-diinyl)-2-oxabicyclo[2.2.1]hept-5-yl]-5-heptensäure:

26 mg (46,3 μmol) des nach Beispiel 190 dargestellten unpolaren Alkohols A löste man in einem Gemisch aus 460 μl wasserfreiem Dimethylsulfoxid und 190 μl wasserfreiem Tetrahydrofuran, versetzte mit 15,6 mg Kalium-t.-butanolat und rührte 2,5 Stunden bei 23°C unter einer Atmosphäre aus trockenem Argon. Man versetzte mit Eiswasser, säuerte durch Zugabe einer gesättigten Zitronensäurelösung an, extrahierte mit Diethylether, trocknete über Magnesiumsulfat und reinigte den nach Filtration und Lösungsmittelabzug erhaltenen Rückstand durch Chromatographie an 2 analytischen Dünnschichtplatten. Als Laufmittel diente ein Gemisch aus Trichlormethan und Ethanol, als Elutionsmittel ein Gemisch aus Ethylacetat und Ethanol. Isoliert wurden 11,5 mg (24,6 μmol, 53%) der Titelverbindung als farbloses Öl.

IR (Film): 3700-2400, 3050, 3010, 2980, 2940, 2880, 2230, 1710, 1610, 1595, 1515, 1445, 1435, 1410, 1290, 1235, 1165, 1040, 1020, 990, 900, 835 und 820 $cm^{-1}$.

**Beispiel 188**

(1S,4R,5S(5Z),6R(3R,4S))-7-[4-(4-Fluorphenyl)-6-(3-hydroxy-4-methyl-nona-1,6-diinyl)-2-oxabicyclo[2.2.1]hept-5-yl]-5-heptensäuremethylester:

2,7 mg (5,8 μmol) der nach Beispiel 189 dargestellten Säure veresterte man in Analogie zu Beispiel 186 und isolierte nach Lösungsmittelabzug 2,7 mg (5,8 μmol, 100%) der Titelverbindung als farbloses Öl.

IR (Film): 3600-3200, 3050, 3010, 2980, 2940, 2880, 2230, 1735, 1610, 1515, 1445, 1435, 1410, 1295, 1230, 1165, 1040, 1020, 990, 900, 835 und 820 $cm^{-1}$.

**Beispiel 189**

(1S,4R,5S(5Z),6R(3R,4S))-7-[4-(4-Fluorphenyl)-6-(3-hydroxy-4-methyl-nona-1,6-diinyl)-2-oxabicyclo[2.2.1]hept-5-yl]-5-heptensäure:

38 mg (67,7 μmol) des nach Beispiel 190 dargestellten polaren Alkohols B setzte man in Analogie zu Beispiel 187 um und isolierte nach Aufarbeitung und Reinigung 17,3 mg (37,1 μmol, 55%) der Titelverbindung als farbloses Öl.

IR (Film): 3700-2400, 3050, 3010, 2980, 2940, 2880, 2230, 1710, 1610, 1515, 1445, 1435, 1410, 1295, 1235, 1165, 1040, 1020, 990, 900, 835 und 820 $cm^{-1}$.

**Beispiel 190**

(1S,4R,5S(5Z),6R(1E/Z,3S,4S))-7-[4-(4-Fluorphenyl)-6-(2-brom-3-hydroxy-4-methyl-non-1-en-6-inyl)-2-oxabicyclo[2.2.1]hept-5-yl]-5-heptensäuremethylester (A) und (1S,4R,5S(5Z),6R(1E/Z,-3R,4S))-7-[4-(4-Fluorphenyl)-6-(2-brom-3-hydroxy-4-methyl-non-1-en-6-inyl)-2-oxabicyclo[2.2.1]hept-5-yl]-5-heptensäuremethylester (B):

80 mg (143 μmol) des in Beispiel 190a dargestellten ungesättigten Ketons reduzierte man in Analogie zu Beispiel 23 und isolierte nach Aufarbeitung und Reinigung 66 mg (118 μmol, 82%) eines Gemisches der beiden Titelverbindungen als farbloses Öl.

IR (Film): 3600-3200, 3040, 2970, 2930, 2870, 1735, 1605, 1510, 1450, 1430, 1230, 1160, 1040, 990, 895, 830 und 735 $cm^{-1}$.

Die chromatographische Trennung lieferte 26 mg (46,3 μmol, 32%) des unpolareren Alkohols, dem man die Struktur A zuordnete, sowie 38 mg (67,7 μmol, 47%) des polareren Alkohols dem man die Struktur B zuordnete.

**Beispiel 190a**

(1S,4R,5S(5Z),6R(1E/Z,4S))-7-[4-(4-Fluorphenyl)-6-(2-brom-3-oxo-4-methyl-non-1-en-6-inyl)-2-oxabicyclo[2.2.1]hept-5-yl]-5-heptensäuremethylester:

Zur Suspension bestehend aus 12,4 mg einer 50%igen Natriumhydriddispersion in Weissöl und 1,1 ml wasserfreiem Tetrahydrofuran tropfte man bei 0°C unter einer Atmosphäre aus trockenem Argon die Lösung von 62 mg Dimethyl-(2-oxo-3S-methyl-oct-5-in)-phosphonat in 650 µl wasserfreiem Tetrahydrofuran zu, versetzte nach 30 Minuten mit 47,5 mg fein pulverisiertem N-Brom-succinimid und tropfte nach 1 Stunde die Lösung von 77,7 mg (215 µmol) des nach Beispiel 138b dargestellten Aldehyds in 750 µl wasserfreiem Tetrahydrofuran zu. Man ließ auf 23°C erwärmen, rührte noch 5 Stunden, verdünnte mit Diethylether, wusch mit einer 10%igen Natriumhydrogencarbonatlösung und Wasser bis zur Neutralreaktion, trocknete über Magnesiumsulfat und reinigte den nach Filtration und Lösungsmittelabzug erhaltenen Rückstand durch Chromatographie an 3 analytischen Dünnschichtplatten. Als Laufmittel diente ein Gemisch aus Chloroform und Diethylether, als Elutionsmittel Ethylacetat. Isoliert wurden 80 mg (143 µmol, 66%) der Titelverbindung als farbloses Öl.

IR (Film): 3040, 3010, 2970, 2930, 2870, 1730, 1680, 1600, 1510, 1450, 1430, 1230, 1160, 1040, 985, 900 und 830 $cm^{-1}$.

**Beispiel 191**

(1S,4R,5S(5Z),6R(1E/Z,3R,4S))-7-[4-(4-Fluorphenyl)-6-(2-brom-3-hydroxy-4-methyl-non-1-en-6-inyl)-2-oxabicyclo[2.2.1]hept-5-yl]-5-heptensäure:

7,3 mg (13 µmol) des nach Beipsiel 190 dargestellten polaren Esters B verseifte man in Analogie zu Beispiel 2 und isolierte nach Aufarbeitung und Reinigung 5,1 mg (9,3 µmol, 72%) der Titelverbindung als farbloses Öl.

IR (Film): 3600-2400, 3040, 3010, 2960, 2930, 2870, 1735, 1605, 1510, 1450, 1410, 1305, 1230, 1160, 1040, 990, 895, 830 und 730 $cm^{-1}$.

**Beispiel 192**

(1S,4R,5S(5Z),6R(1E/Z,3S,4S))-7-[4-(4-Fluorphenyl)-6-(2-brom-3-hydroxy-4-methyl-non-1-en-6-inyl)-2-oxabicyclo[2.2.1]hept-5-yl]-5-heptensäure:

8,1 mg (14,4 µmol) des nach Beispiel 190 dargestellten unpolaren Esters A verseifte man in Analogie zu Beispiel 2 und isolierte nach Aufarbeitung und Reinigung 7,5 mg (13,7 µmol, 95%) der Titelverbindung als farbloses Öl.

IR (Film): 3600-2300, 3050, 3010, 2970, 2930, 2880, 1735, 1605, 1510, 1455, 1410, 1305, 1235, 1160, 1040, 990, 895, 830 und 730 $cm^{-1}$.

**Beispiel 193**

(1S,4R,5S(5Z),6S(1E,3S,4S))-7-[4-(4-Fluorphenyl)-6-(3-hydroxy-4-methyl-non-1-en-6-inyl)-2-oxabicyclo[2.2.1]hept-5-yl]-5-heptensäure-methylester (A) und (1S,4R,5S(5Z),6S(1E,3R,4S))-7-[4-(4-Fluor-phenyl)-6-(3-hydroxy-4-methyl-non-1-en-6-inyl)-2-oxabicyclo[2.2.1]hept-5-yl]-5-heptensäuremethylester (B):

40 mg (83,2 µmol) des in Beispiel 193a dargestellten ungesättigten Ketons reduzierte man in Analogie zu Beispiel 23 und isolierte nach Aufarbeitung und Reinigung 39 mg (80,8 µmol, 97%) eines Gemisches der beiden Titelverbindungen als farbloses Öl.

IR (Film): 3600-3200, 3040, 2970, 2930, 2870, 1730, 1605, 1510, 1450, 1435, 1230, 1160, 995, 970, 895, 830 und 735 $cm^{-1}$.

Die chromatographische Trennung lieferte 16,4 mg (34 µmol, 41%) des unpolareren Alkohols, dem man die Struktur A zuordnete, sowie 20 mg (41 µmol, 50%) des polareren Alkohols dem man die Struktur B zuordnete.

**Beispiel 193a**

(1S,4R,5S(5Z),6S(1E,4S))-7-[4-(4-Fluorphenyl)-6-(3-oxo-4-methyl-non-1-en-6-inyl)-2-oxabicyclo[2.2.1]hept-5-yl]-5-heptensäuremethylester:

49,3 mg (136 µmol) des in Beispiel 138b dargestellten Aldehyds setzte man in Analogie zu Beispiel 23a unter Verwendung von Dimethyl-(2-oxo-3S-methyl-oct-5-in)-phosphonat um und isolierte nach Aufarbeitung und Reinigung 37 mg (77 µmol, 57%) der Titelverbindung als farbloses Öl.

IR (Film): 3060, 3040, 2970, 2930, 2870, 1735, 1690, 1665, 1620, 1510, 1450, 1430, 1230, 1160, 1040, 995, 975, 900 und 835 $cm^{-1}$.

**Beispiel 194**

(1S,4R,5S(5Z),6S(1E,3S,4S))-7-[4-(4-Fluorphenyl)-6-(3-hydroxy-4-methyl-non-1-en-6-inyl)-2-oxabicyclo[2.2.1]hept-5-yl]-5-heptensäure:

16 mg (33,2 μmol) des nach Beispiel 193 dargestellten unpolaren Esters A verseifte man in Analogie zu Beispiel 2 und isolierte nach Aufarbeitung und Reinigung 15 mg (32 μmol, 97%) der Titelverbindung als farbloses Öl.

IR (Film): 3700-2400, 3070, 3050, 3010, 2970, 2930, 2880, 2100, 1710, 1610, 1515, 1455, 1410, 1320, 1300, 1235, 1165, 1040, 1015, 995, 975, 895, 835 und 820 $cm^{-1}$.

**Beispiel 195**

(1S,4R,5S(5Z),6S(1E,3R,4S))-7-[4-(4-Fluorphenyl)-6-(3-hydroxy-4-methyl-non-1-en-6-inyl)-2-oxabicyclo[2.2.1]hept-5-yl]-5-heptensäure:

20 mg (41,4 μmol) des nach Beispiel 193 dargestellten polaren Esters B verseifte man in Analogie zu Beispiel 2 und isolierte nach Aufarbeitung und Reinigung 16 mg (34,1 μmol, 82%) der Titelverbindung als farbloses Öl.

IR (Film): 3700-2300, 3070, 3040, 3000, 2970, 2880, 1710, 1610, 1515, 1455, 1410, 1320, 1300, 1235, 1160, 995, 970, 895 und 835 $cm^{-1}$.

**Beispiel 196**

(1S,4R,5S(5Z),6R)-7-[4-(4-Fluorphenyl)-6-(4-cyanobenzyloxymethyl)-2-oxabicyclo[2.2.1]hept-5-yl]-5-heptensäuremethyl(bzw.4-cyanobenzyl)ester:

19,6 mg (54,1 μmol) des in Beispiel 120a dargestellten Alkohols setzte man in Analogie zu Beispiel 1 mit 4-Brommethylbenzonitril um und isolierte nach Aufarbeitung und Reinigung 23 mg der Titelverbindungen als farbloses Öl.

IR (Film): 3050, 3040, 3010, 2930, 2860, 2220, 1730, 1605, 1510, 1435, 1360, 1225, 1160, 1120, 1090, 1030, 895, 830 und 815 $cm^{-1}$.

**Beispiel 197**

(1S,4R,5S(5Z),6R)-7-[4-(4-Fluorphenyl)-6-(4-cyanobenzyloxymethyl)-2-oxabicyclo[2.2.1]hept-5-yl]-5-heptensäure:

23 mg des nach Beispiel 196 dargestellten Estergemisches verseifte man in Analogie zu Beispiel 2 und isolierte nach Aufarbeitung und Reinigung 18 mg (38,8 μmol, 72%, bezogen auf Startmaterial in Beispiel 196) der Titelverbindung als farbloses Öl.

IR (Film): 3600-2400, 3070, 3050, 3010, 2930, 2870, 2230, 1725, 1710, 1610, 1515, 1410, 1230, 1160, 1125, 995, 930, 920, 895, 835 und 820 $cm^{-1}$.

**Beispiel 198**

(1S,4R,5S(5Z),6R)-7-[4-(4-Fluorphenyl)-6-(3-cyanobenzyloxymethyl)-2-oxabicyclo[2.2.1]hept-5-yl]-5-heptensäuremethyl(bzw.3-cyanobenzyl)ester:

18,4 mg (50,8 μmol) des in Beispiel 120a dargestellten Alkohols setzte man in Analogie zu Beispiel 1 mit 3-Brommethylbenzonitril um und isolierte nach Aufarbeitung und Reinigung 25 mg der Titelverbindungen als farbloses Öl.

IR (Film): 3060, 3040, 3000, 2940, 2860, 2230, 1730, 1605, 1510, 1435, 1230, 1160, 1090, 1030, 895, 830, 795, 730 und 685 $cm^{-1}$.

**Beispiel 199**

(1S,4R,5S(5Z),6R)-7-[4-(4-Fluorphenyl)-6-(3-cyanobenzyloxymethyl)-2-oxabicyclo[2.2.1]hept-5-yl]-5-heptensäure:

25 mg des nach Beispiel 198 dargestellten Estergemisches verseifte man in Analogie zu Beispiel 2 und

isolierte nach Aufarbeitung und Reinigung 16,8 mg (36,2 µmol, 71%, bezogen auf Startmaterial in Beispiel 198) der Titelverbindung als farbloses Öl.

IR (Film): 3600-2400, 3050, 3010, 2930, 2870, 2230, 1725, 1710, 1605, 1515, 1230, 1160, 1120, 1090, 1030, 890, 835, 795 und 690 $cm^{-1}$.

**Beispiel 200**

(1S,4R,5S(5Z),6R)-7-[4-(4-Fluorphenyl)-6-(3-phenylpropoxymethyl)-2-oxabicyclo[2.2.1]hept-5-yl]-5-heptensäuremethylester:

19,4 mg (53,5 µmol) des in Beispiel 120a dargestellten Alkohols setzte man in Analogie zu Beispiel 1 mit 3-Brom-1-phenylpropan um und isolierte nach Aufarbeitung und Reinigung 23,4 mg der Titelverbindung als farbloses Öl.

IR (Film): 3080, 3060, 3020, 2930, 2860, 1730, 1600, 1510, 1450, 1230, 1160, 1115, 1025, 895, 830, 735 und 700 $cm^{-1}$.

**Beispiel 201**

(1S,4R,5S(5Z),6R)-7-[4-(4-Fluorphenyl)-6-(3-phenylpropoxymethyl)-2-oxabicyclo[2.2.1]hep t-5-yl]-5-heptensäure:

23,4 mg des nach Beispiel 200 dargestellten Esters verseifte man in Analogie zu Beispiel 2 und isolierte nach Aufarbeitung und Reinigung 17 mg (36,4 µmol, 68%, bezogen auf Startmaterial in Beispiel 200) der Titelverbindung als farbloses Öl.

IR (Film): 3700-2400, 3090, 3060, 3030, 3000, 2940, 2870, 1725, 1710, 1515, 1455, 1230, 1160, 1120, 1030,895, 835, 745 und 700 $cm^{-1}$.

**Beispiel 202**

(1S,4R,5S(5Z),6R)-7-[4-(4-Fluorphenyl)-6-[2-(4-fluorphenoxy)-ethoxymethyl]-2-oxabicyclo[2.2.1]hept-5-yl]-5-heptensäuremethyl(bzw.2-(4-fluorphenoxy)-ethyl)ester:

21,4 mg (59 µmol) des in Beispiel 120a dargestellten Alkohols setzte man in Analogie zu Beispiel 1 mit 4-Fluorphenoxy-2-ethylbromid um und isolierte nach Aufarbeitung 21 mg der Titelverbindungen als farbloses Öl.

IR (Film): 3070, 3050, 3010, 2940, 2870, 1735, 1605, 1505, 1455, 1255, 1220, 1210, 1130, 895, 830 und 745 $cm^{-1}$.

**Beispiel 203**

(1S,4R,5S(5Z),6R)-7-[4-(4-Fluorphenyl)-6-[2-(4-fluorphenoxy)-ethoxymethyl]-2-oxabicyclo[2.2.1]hept-5-yl]-5-heptensäure:

21 mg des nach Beispiel 202 dargestellten Estergemisches verseifte man in Analogie zu Beispiel 2 und isolierte nach Aufarbeitung und Reinigung 10,6 mg (21,8 µmol, 37%, bezogen auf Startmaterial in Beispiel 202) der Titelverbindung als farbloses Öl.

IR (Film): 3600-2400, 3070, 3050, 3010, 2930, 2870, 1730, 1710, 1605, 1505, 1455, 1250, 1220, 1210, 1130, 895, 830 und 745 $cm^{-1}$.

**Beispiel 204**

(1S,4R,5S(5Z),6R)-7-[4-(4-Fluorphenyl)-6-[2-(methoxycarbonylmethoxy)-ethoxymethyl]-2-oxabicyclo[2.2.1]hept-5-yl]-5-heptensäuremethylester:

2,5 mg (5,5 µmol) der nach Beispiel 205 dargestellten Säure veresterte man in Analogie zu Beispiel 186 und isolierte nach Lösungsmittelabzug 2,5 mg (5,5 µmol, 100%) der Titelverbindung als farbloses Öl.

IR (Film): 3070, 3050, 3010, 2940, 2880, 1735, 1605, 1505, 1455, 1250, 1215, 1125, 895, 830 und 745 $cm^{-1}$.

**Beispiel 205**

(1S,4R,5S(5Z),6R)-7-[4-(4-Fluorphenyl)-6-[2-(carboxymethoxy)-ethoxymethyl]-2-oxabicyclo[2.2.1]hept-5-yl]-5-heptensäure:

18,4 mg (50,8 µmol) des in Beispiel 120a dargestellten Alkohols setzte man in Analogie zu Beispiel 1 mit 6-Chlor-3-oxa-hexansäureethylester um und isolierte nach Aufarbeitung und Reinigung 12,8 mg (28,4 µmol, 56%) der Titelverbindung als farbloses Öl.

IR (Film): 3700-2300, 3050, 3010, 2930, 2880, 1720, 1605, 1515, 1430, 1230, 1120, 1030, 895 und 835 $cm^{-1}$.

**Beispiel 206**

(1S,4R,5S(5Z),6R)-7-[4-(4-Fluorphenyl)-6-(cyclohexylmethoxyiminomethyl)-2-oxabicyclo[2.2.1]hept-5-yl]-5-heptensäuremethylester:

23,8 mg (66 µmol) des in Beispiel 138b dargestellten Aldehyds setzte man in Analogie zu Beispiel 26 unter Verwendung von Cyclohexylmethoxyamin um und isolierte nach Aufarbeitung und Reinigung 21 mg (44,5 µmol, 67%) der Titelverbindung als farbloses Öl.

IR (Film): 3050, 3010, 2920, 2850, 1735, 1605, 1510, 1450, 1230, 1160, 1040, 1000, 900, 830 und 735 $cm^{-1}$.

**Beispiel 207**

(1S,4R,5S(5Z),6R)-7-[4-(4-Fluorphenyl)-6-(cyclohexylmethoxyiminomethyl)-2-oxabicyclo[2.2.1]hept-5-yl]-5-heptensäure:

21 mg (44,5 µmol) des nach Beispiel 206 dargetstellten Esters verseifte man in Analogie zu Beispiel 2 und isolierte nach Aufarbeitung und Reinigung 20 mg (43,7 µmol, 98%) der Titelverbindung als farbloses Öl.

IR (Film): 3600-2400, 3070, 3050, 3010, 2930, 2850, 2635, 1730, 1710, 1610, 1515, 1450, 1235, 1160, 1040, 1025, 995, 895, 835 und 820 $cm^{-1}$.

**Beispiel 208**

(1S,4R,5S(5Z),6R(3S,4S))-7-[4-(4-Fluorphenyl)-6-(3-hydroxy-oct-1-inyl)-2-oxabicyclo[2.2.1]hept-5-yl]-5-heptensäuremethylester:

4,1 mg (9.3 µmol) der nach Beispiel 209 dargestellten Säure veresterte man in Analogie zu Beispiel 186 und isolierte nach Lösungsmittelabzug 4 mg (8,8 µmol,95 %) der Titelverbindung als farbloses Öl.

IR (Film): 3600-3200, 3050, 3020, 2950, 2930, 2860, 2230, 1735, 1610, 1515, 1455, 1410, 1295, 1230, 1165, 1045, 1015,995, 895 und 830 $cm^{-1}$.

**Beispiel 209**

(1S,4R,5S(5Z),6R(3S,4S))-7-[4-(4-Fluorphenyl)-6-(3-hydroxy-oct-1-inyl)-2-oxabicyclo[2.2.1]hept-5-yl]-5-heptensäure:

24 mg (44,6 µmol) des nach Beispiel 212 dargestellten unpolaren Alkohols A setzte man in Analogie zu Beispiel 187 um und isolierte nach Aufarbeitung und Reinigung 13,8 mg (31,2 µmol, 70%) der Titelverbindung als farbloses Öl.

IR (Film): 3600-2500, 3050, 3010, 2950, 2930, 2870, 2860, 2230, 1710, 1610, 1515, 1455, 1410, 1235, 1160, 1045, 1015, 990, 900 und 830 $cm^{-1}$.

**Beispiel 210**

(1S,4R,5S(5Z),6R(3S,4S))-7-[4-(4-Fluorphenyl)-6-(3-hydroxy-oct-1-inyl)-2-oxabicyclo[2.2.1]hept-5-yl]-5-heptensäuremethylester:

2,9 mg (6,6 µmol) der nach Beispiel 211 dargestellten Säure veresterte man in Analogie zu Beispiel 186 und isolierte nach Lösungsmittelabzug 3 mg (6,6 µmol, 100%) der Titelverbindung als farbloses Öl.

IR (Film): 3600-3200, 3050, 3020, 2950, 2930, 2860, 2230, 1735, 1610, 1515, 1450, 1410, 1300, 1230, 1160, 1040, 1010, 995, 895 und 830 $cm^{-1}$.

**Beispiel 211**

(1S,4R,5S(5Z),6R(3R,4S))-7-[4-(4-Fluorphenyl)-6-(3-hydroxy-oct-1-inyl)-2-oxabicyclo[2.2.1]hept-5-yl]-5-heptensäure:

17 mg (31,6 µmol) des nach Beispiel 212 dargestellten polaren Alkohols B setzte man in Analogie zu

Beispiel 187 um und isolierte nach Aufarbeitung und Reinigung 10,2 mg (23 µmol, 73%) der Titelverbindung als farbloses Öl.

IR (Film): 3700-2400, 3070, 3050, 3010, 2950, 2930, 2870, 2860, 2230, 1710, 1610, 1515, 1455, 1410, 1295, 1235, 1160, 1045, 1015, 990, 895 und 830 $cm^{-1}$.

**Beispiel 212**

(1S,4R,5S(5Z),6R(1E/Z,3S,4S))-7-[4-(4-Fluorphenyl)-6-(2-brom-3-hydroxy-oct-1-enyl)-2-oxa-bicyclo[2.2.1]hept-5-yl]-5-heptensäuremethylester (A) und (1S,4R,5S(5Z),6R(1E/Z,3R,4S))-7-[4-(4-Fluorphenyl)-6-(2-brom-3-hydroxy-oct-1-enyl)-2-oxa-bicyclo[2.2.1]hept-5-yl]-5-heptensäure-methylester (B):

80 mg (149 µmol) des in Beispiel 212a dargestellten ungesättigten Ketons reduzierte man in Analogie zu Beispiel 23 und isolierte nach Aufarbeitung und Reinigung 67 mg (125 µmol, 84%) eines Gemisches der beiden Titelverbindungen als farbloses Öl.

IR (Film): 3600-3300, 3070, 3050, 3010, 2960, 2930, 2860, 1735, 1605, 1515, 1455, 1410, 1305, 1235, 1160, 1040, 995, 940, 895, 835 und 725 $cm^{-1}$.

Die chromatographische Trennung lieferte 37 mg (68,8 µmol, 46%) des unpolareren Alkohols, dem man die Struktur A zuordnete, sowie 27 mg (50,2 µmol, 34%) des polareren Alkohols dem man die Struktur B zuordnete.

**Beispiel 212a**

(1S,4R,5S(5Z),6R(1E/Z,4S))-7-[4-(4-Fluorphenyl)-6-(2-brom-3-oxo-oct-1-enyl)-2-oxa-bicyclo[2.2.1]hept-5-yl]-5-heptensäuremethylester:

80,7 mg (224 µmol) des in Beispiel 138b dargestellten Aldehyds setzte man in Analogie zu Beispiel 190a unter Verwendung von Dimethyl-(2-oxo-heptyl)-phosphonat um und isolierte nach Aufarbeitung und Reinigung 80 mg (149 µmol, 67%) der Titelverbindung als farbloses Öl.

IR (Film): 3070, 3050, 3010, 2950, 2930, 2870, 1735, 1685, 1600, 1510, 1450, 1435, 1365, 1305, 1230, 1160, 1045, 990, 900 und 830 $cm^{-1}$.

**Beispiel 213**

(1S,4R,5S(5Z),6R(1E/Z,3S,4S))-7-[4-(4-Fluorphenyl)-6-(2-brom-3-hydroxy-oct-1-enyl)-2-oxa-bicyclo[2.2.1]hept-5-yl]-5-heptensäure:

12,6 mg (23,4 µmol) des nach Beispiel 212 dargestellten unpolaren Esters A verseifte man in Analogie zu Beispiel 2 und isolierte nach Aufarbeitung und Reinigung 12 mg (22,9 µmol, 98%) der Titelverbindung als farbloses Öl.

IR (Film): 3600-2400, 3050, 3010, 2950, 2930, 2870, 2860, 1710, 1605, 1515, 1455, 1410, 1305, 1235, 1160, 1045, 995, 895, 830 und 725 $cm^{-1}$.

**Beispiel 214**

(1R,4S,5R(5Z),6S)-7-[4-(4-Fluorphenyl)-6-(4-cyanobenzyloxymethyl)-2-oxabicyclo[2.2.1]hept-5-yl]-5-heptensäure:

19 mg des nach Beispiel 214a dargestellten Estergemisches verseifte man in Analogie zu Beispiel 2 und isolierte nach Aufarbeitung und Reinigung 14,8 mg (31,9 µmol, 72%, bezogen auf Startmaterial in Beispiel 214a) der Titelverbindung als farbloses Öl.

IR (Film): 3600-2400, 3070, 3050, 3010, 2930, 2870, 2230, 1725, 1710, 1610, 1515, 1410, 1230, 1160, 1125, 995, 930, 920, 895, 835 und 820 $cm^{-1}$.

**Beispiel 214a**

(1R,4S,5R(5Z),6S)-7-[4-(4-Fluorphenyl)-6-(4-cyanobenzyloxymethyl)-2-oxabicyclo[2.2.1]hept-5-yl]-5-heptensäuremethyl(bzw.4-cyanobenzyl)ester:

16,1 mg (44,4 µmol) des in Beispiel 17a dargestellten Alkohols setzte man in Analogie zu Beispiel 1 mit 4-Brommethylbenzonitril um und isolierte nach Aufarbeitung und Reinigung 19 mg der Titelverbindungen als farbloses Öl.

IR (Film): 3050, 3040, 3010, 2930, 2860, 2220, 1730, 1605, 1510, 1435, 1360, 1225, 1160, 1120, 1090, 1030, 895, 830 und 815 $cm^{-1}$.

**Beispiel 215**

(1S,4R,5S(5Z),6R(1E/Z,3R,4S))-7-[4-(4-Fluorphenyl)-6-(2-brom-3-hydroxy-oct-1-enyl)-2-oxa-bicyclo[2.2.1]hept-5-yl]-5-heptensäure:

8,8 mg (16,4 μmol) des nach Beispiel 212 dargestellten polaren Esters B verseifte man in Analogie zu Beispiel 2 und isolierte nach Aufarbeitung und Reinigung 8,3 mg (15,9 μmol, 97%) der Titelverbindung als farbloses Öl.

IR (Film): 3600-2400, 3050, 3010, 2950, 2930, 2870, 2860, 1710, 1610, 1515, 1455, 1410, 1300, 1235, 1165, 1040, 995, 940, 895, 835 und 725 $cm^{-1}$.

**Beispiel 216**

(1S,4R,5S(5Z),6R)-7-[4-(4-Fluorphenyl)-6-[(1E,3S,4RS)-3-hydroxy-4,7-dimethyl-octa-1-in-6-enyl]-2-oxabicyclo [2.2.1]hept-5-yl]-5-heptensäuremethylester:

3,3 mg (7 μmol) der nach Beispiel 217 dargestellten Säure veresterte man in Analogie zu Beispiel 186 und isolierte nach Lösungsmittelabzug 3,2 mg (6,6 μmol, 94%) der Titelverbindung als farbloses Öl.

IR (Film): 3050, 3010, 2960, 2930, 2870, 2230, 1735, 1605, 1515, 1455, 1235, 1160, 1040, 1020, 1000, 900 und 830 $cm^{-1}$.

**Beispiel 217**

(1S,4R,5S(5Z),6R)-7-[4-(4-Fluorphenyl)-6-[(1E,3S,4RS)-3-hydroxy-4,7-dimethyl-octa-1-in-6-enyl]-2-oxabicyclo [2.2.1]hept-5-yl]-5-heptensäure:

27 mg (47,9 μmol) des nach Beispiel 220 dargestellten unpolaren Alkohols A setzte man in Analogie zu Beispiel 187 um und isolierte nach Aufarbeitung und Reinigung 18 mg (38,4 μmol, 80%) der Titelverbindung als farbloses Öl.

IR (Film): 3600-2500, 3070, 3050, 3010, 2960, 2930, 2880, 2230, 1710, 1610, 1515, 1455, 1235, 1165, 1040, 1020, 990, 940, 900 und 830 $cm^{-1}$.

**Beispiel 218**

(1S,4R,5S(5Z),6R)-7-[4-(4-Fluorphenyl)-6-[(1E,3S,4RS)-3-hydroxy-4,7-dimethyl-octa-1-in-6-enyl]-2-oxabicyclo [2.2.1]hept-5-yl]-5-heptensäuremethylester:

3 mg (6,4 μmol) der nach Beispiel 219 dargestellten Säure veresterte man in Analogie zu Beispiel 186 und isolierte nach Lösungsmittelabzug 3 mg (6,2 μmol, 97%) der Titelverbindung als farbloses Öl.

IR (Film): 3600-3200, 3050, 3010, 2960, 2930, 2880, 2230, 1730, 1610, 1515, 1455, 1410, 1375, 1300, 1265, 1235, 1160, 1040, 1020, 995, 940, 895, 830 und 815 $cm^{-1}$.

**Beispiel 219**

(1S,4R,5S(5Z),6R)-7-[4-(4-Fluorphenyl)-6-[(1E,3S,4RS)-3-hydroxy-4,7-dimethyl-octa-1-in-6-enyl]-2-oxabicyclo [2.2.1]hept-5-yl]-5-heptensäure:

18 mg (31,9 μmol) des nach Beispiel 220 dargestellten polaren Alkohols B setzte man in Analogie zu Beispiel 187 um und isolierte nach Aufarbeitung und Reinigung 10,6 mg (22,6 μmol, 71%) der Titelverbindung als farbloses Öl.

IR (Film): 3700-2400, 3050, 3010, 2960, 2930, 2880, 2230, 1710, 1610, 1515, 1455, 1410, 1375, 1295, 1265, 1235, 1160, 1040, 1020, 990, 940, 895, 830 und 815 $cm^{-1}$.

**Beispiel 220**

(1S,4R,5S(5Z),6R)-7-[4-(4-Fluorphenyl)-6-[(1E/Z,3S,4RS)-2-brom-3-hydroxy-4,7-dimethyl-oct-1,6-dienyl]-2-oxa-bicyclo[2.2.1]hept-5-yl]-5-heptensäuremethylester (A) und (1S,4R,5S(5Z),6R)-7-[4-(4-Fluorphenyl)-6-[(1E/Z, 3R,4RS)-2-brom-3-hydroxy-4,7-dimethyl-oct-1,6-dienyl]-2-oxabicyclo[2.2.1]hept-5-yl]-5-heptensäuremethylester (B):

50 mg (89 μmol) des in Beispiel 220a dargestellten ungesättigten Ketons reduzierte man in Analogie zu Beispiel 23 und isolierte nach Aufarbeitung und Reinigung 48 mg (85,2 μmol, 96%) eines Gemisches der beiden Titelverbindungen als farbloses Öl.

IR (Film): 3600-3200, 3050, 3010, 2960, 2920, 2870, 1730, 1605, 1510, 1450, 1435, 1375, 1230, 1160,

1040, 990, 895, 830 und 735 $cm^{-1}$.

Die chromatographische Trennung lieferte 27 mg (47,9 µmol, 54%) des unpolareren Alkohols, dem man die Struktur A zuordnete, sowie 18 mg (31,9 µmol, 36%) des polareren Alkohols dem man die Struktur B zuordnete.

## Beispiel 220a

(1S,4R,5S(5Z),6R)-7-[4-(4-Fluorphenyl)-6-[(1E/Z,4RS)-2-brom-3-oxo-4,7-dimethyl-oct-1,6-dienyl]-2-oxabicyclo [2.2.1]hept-5-yl]-5-heptensäuremethylester:

67,9 mg (188 µmol) des in Beispiel 138b dargestellten Aldehyds setzte man in Analogie zu Beispiel 190a unter Verwendung von Dimethyl-(2-oxo-3(RS),6-dimethyl-hept-5-enyl)-phosphonat um und isolierte nach Aufarbeitung und Reinigung 50 mg (89 µmol, 47%) der Titelverbindung als farbloses Öl.

IR (Film): 3070, 3050, 3010, 2970, 2930, 2870, 1735, 1680, 1600, 1510, 1450, 1435, 1370, 1305, 1230, 1160, 1045, 985, 900 und 835 $cm^{-1}$.

## Beispiel 221

(1S,4R,5S(5Z),6R)-7-[4-(4-Fluorphenyl)-6-[(1E/Z,3R,4RS)-2-brom-3-hydroxy-4,7-dimethyl-oct-1,6-dienyl]-2-oxabicyclo[2.2.1]hept-5-yl]-5-heptensäure:

12 mg (21,3 µmol) des nach Beispiel 220 dargestellten polaren Esters B verseifte man in Analogie zu Beispiel 2 und isolierte nach Aufarbeitung und Reinigung 9,3 mg (16,9 µmol, 79%) der Titelverbindung als farbloses Öl.

IR (Film): 3600-2400, 3050,3010, 2950, 2920, 2870, 1710, 1605, 1510, 1455, 1420, 1375, 1230, 1160, 1045, 995, 895, 830 und 730 $cm^{-1}$.

## Beispiel 222

(1S,4R,5S(5Z),6R)-7-[4-(4-Fluorphenyl)-6-[(1E/Z,3S,4RS)-2-brom-3-hydroxy-4,7-dimethyl-oct-1,6-dienyl]-2-oxabicyclo[2.2.1]hept-5-yl]-5-heptensäure:

13,7 mg (24,3 µmol) des nach Beispiel 220 dargestellten unpolaren Esters A verseifte man in Analogie zu Beispiel 2 und isolierte nach Aufarbeitung und Reinigung 11,8 mg (21,5 µmol, 88%) der Titelverbindung als farbloses Öl.

IR (Film): 3600-2400, 3050, 3010, 2950, 2930, 2870, 1710, 1605, 1515, 1455, 1420, 1305, 1230, 1160, 1040, 995, 895, 830 und 730 $cm^{-1}$.

## Beispiel 223

(1S,4R,5S(5Z),6R)-7-[4-(4-Fluorphenyl)-6-[(3S)-3-hydroxy-3-cyclohexyl-prop-1-inyl]-2-oxabicyclo[2.2.1]hept-5-yl]-5-heptensäuremethylester:

3,5 mg (7,7 µmol) der nach Beispiel 224 dargestellten Säure veresterte man in Analogie zu Beispiel 186 und isolierte nach Lösungsmittelabzug 3,5 mg (7,5 µmol, 97%) der Titelverbindung als farbloses Öl.

IR (Film): 3600-3200, 3050, 3010, 2940, 2860, 2230, 1735, 1605, 1510, 1445, 1265, 1230, 1160, 1015, 995, 895 und 835 $cm^{-1}$.

## Beispiel 224

(1S,4R,5S(5Z),6R)-7-[4-(4-Fluorphenyl)-6-[(3S)-3-hydroxy-3-cyclohexyl-prop-1-inyl]-2-oxabicyclo[2.2.1]hept-5-yl]-5-heptensäure:

29,6 mg (53,9 µmol) des nach Beispiel 227 dargestellten unpolaren Alkohols A setzte man in Analogie zu Beispiel 187 um und isolierte nach Aufarbeitung und Reinigung 13,8 mg (30,4 µmol, 56%) der Titelverbindung als farbloses Öl.

IR (Film): 3600-2400, 3050, 3010, 2930, 2850, 2230, 1710, 1605, 1510, 1450, 1265, 1230, 1160, 1015, 990, 895, 830 und 735 $cm^{-1}$.

## Beispiel 225

(1S,4R,5S(5Z),6R)-7-[4-(4-Fluorphenyl)-6-[(3R)-3-hydroxy-3-cyclohexyl-prop-1-inyl]-2-oxabicyclo[2.2.1]hept-5-yl]-5-heptensäuremethylester:

2,8 mg (6,2 µmol) der nach Beispiel 226 dargestellten Säure veresterte man in Analogie zu Beispiel 186 und isolierte nach Lösungsmittelabzug 2,9 mg (6,2 µmol, 100%) der Titelverbindung als farbloses Öl.

IR (Film): 3600-3200, 3070, 3050, 3010, 2940, 2850, 2230, 1735, 1605, 1515, 1450, 1410, 1295, 1235, 1160, 1045, 1010, 990, 940, 895 und 830 $cm^{-1}$.

**Beispiel 226**

(1S,4R,5S(5Z),6R)-7-[4-(4-Fluorphenyl)-6-[(3R)-3-hydroxy-3-cyclohexyl-prop-1-inyl]-2-oxabicyclo[2.2.1]hept-5-yl]-5-heptensäure:

23,5 mg (42,8 µmol) des nach Beispiel 227 dargestellten polaren Alkohols B setzte man in Analogie zu Beispiel 187 um und isolierte nach Aufarbeitung und Reinigung 11,5 mg (25,3 µmol, 59%) der Titelverbindung als farbloses Öl.

IR (Film): 3700-2400, 3070, 3050, 3010, 2930, 2850, 2230, 1710, 1610, 1515, 1450, 1410, 1295, 1235, 1160, 1045, 1015, 990, 940, 895, 830 und 815 $cm^{-1}$.

**Beispiel 227**

(1S,4R,5S(5Z),6R)-7-[4-(4-Fluorphenyl)-6-[(1E/Z,3S)-2-brom-3-hydroxy-3-cyclohexyl-prop-1-enyl]-2-oxabicyclo [2.2.1]hept-5-yl]-5-heptensäuremethylester (A) und (1S,4R,5S(5Z),6R)-7-[4-(4-Fluorphenyl)-6-[(1E/Z,3R)-2-brom-3-hydroxy-3-cyclohexyl-prop-1-enyl]-2-oxabicyclo[2.2.1]hept-5-yl]-5-heptensäuremethylester (B):

60 mg (110 µmol) des in Beispiel 227a dargestellten ungesättigten Ketons reduzierte man in Analogie zu Beispiel 23 und isolierte nach Aufarbeitung und Reinigung 57,8 mg (105 µmol, 96%) eines Gemisches der beiden Titelverbindungen als farbloses Öl.

IR (Film): 3600-3200, 3050, 3000, 2930, 2850, 1730, 1605, 1510, 1450, 1230, 1160, 990, 895, 830 und 735 $cm^{-1}$.

Die chromatographie Trennung lieferte 29,6 mg (53,9 µmol, 49%) des unpolareren Alkohols, dem man die Struktur A zuordnete, sowie 23,5 mg (42,8 µmol, 39%) des polareren Alkohols dem man die Struktur B zuordnete.

**Beispiel 227a**

(1S,4R,5S(5Z),6R)-7-[4-(4-Fluorphenyl)-6-[(1E/Z)-2-brom-3-oxo-3-cyclohexyl-prop-1-enyl]-2-oxabicyclo[2.2.1] hept-5-yl]-5-heptensäuremethylester:

70,7 mg (196 µmol) des in Beispiel 138b dargestellten Aldehyds setzte man in Analogie zu Beispiel 190a unter Verwendung von Dimethyl-(2-oxo-2-cyclohexyl-ethyl)-phosphonat um und isolierte nach Aufarbeitung und Reinigung 60 mg (110 µmol, 56%) der Titelverbindung als farbloses Öl.

IR (Film): 3050, 3000, 2930, 2850, 1735, 1680, 1600, 1510, 1445, 1435, 1370, 1305, 1235, 1160, 1045, 985, 900 und 830 $cm^{-1}$.

**Beispiel 228**

(1S,4R,5S(5Z),6R)-7-[4-(4-Fluorphenyl)-6-[(1E/Z,3R)-2-brom-3-hydroxy-3-cyclohexyl-prop-1-enyl]-2-oxabicyclo [2.2.1]hept-5-yl]-5-heptensäure:

14,7 mg (26,7 µmol) des nach Beispiel 227 dargestellten polaren Esters B verseifte man in Analogie zu Beispiel 2 und isolierte nach Aufarbeitung und Reinigung 12,6 mg (23,5 µmol, 88%) der Titelverbindung als farbloses Öl.

IR (Film): 3600-2400, 3070, 3040, 3010, 2930, 2850, 1710, 1600, 1510, 1450, 1435, 1230, 1160, 1045, 985, 900, 830 und 735 $cm^{-1}$.

**Beispiel 229**

(1S,4R,5S(5Z),6R)-7-[4-(4-Fluorphenyl)-6-[(1E/Z,3S)-2-brom-3-hydroxy-3-cyclohexyl-prop-1-enyl]-2-oxabicyclo [2.2.1]hept-5-yl]-5-heptensäure:

13,4 mg (24,4 µmol) des nach Beispiel 227 dargestellten unpolaren Esters A verseifte man in Analogie zu Beispiel 2 und isolierte nach Aufarbeitung und Reinigung 11,6 mg (21,7 µmol, 89%) der Titelverbindung als farbloses Öl.

IR (Film): 3600-2400, 3040, 3010, 2930, 2850, 1710, 1605, 1510, 1450, 1435, 1225, 1160, 1040, 995, 895, 830 und 735 $cm^{-1}$.

**Beispiel 230**

(1S,4R,5S(5Z),6S)-7-[4-(4-Fluorphenyl)-6-[(1E,3S)-3-hydroxy-3-cyclohexyl-prop-1-enyl]-2-oxa-bicyclo[2.2.1] hept-5-yl]-5-heptensäuremethylester (A) und (1S,4R,5S(5Z),6S)-7-[4-(4-Fluorphenyl)-6-[(1E,3R)-3-hydroxy-3-cyclohexyl-prop-1-enyl]-2-oxabicyclo[2.2.1]hept-5-yl]-5-heptensäuremethylester (B):

39 mg (83,2 μmol) des in Beispiel 230a dargestellen ungesättigten Ketons reduzierte man in Analogie zu Beispiel 23 und isolierte nach Aufarbeitung und Reinigung 33 mg (70 μmol, 84%) eines Gemisches der beiden Titelverbindungen als farbloses Öl.

IR (Film): 3600-3200, 3040, 3010, 2920, 2850, 1730, 1605, 1510, 1450, 1230, 1160, 995, 970, 895, 835 und 735 $cm^{-1}$.

Die chromatographische Trennung lieferte 19 mg (40,4 μmol, 49%) des unpolareren Alkohols, dem man die Struktur A zuordnete, sowie 14 mg (29,7 μmol, 36%) des polareren Alkohols dem man die Struktur B zuordnete.

**Beispiel 230a**

(1S,4R,5S(5Z),6S)-7-[4-(4-Fluorphenyl)-6-[(1E)-3-oxo-3-cyclohexyl-prop-1-enyl]-2-oxa-bicyclo[2.2.1]hept-5-yl]-5-heptensäuremethylester:

50,3 mg (140 μmol) des in Beispiel 138b dargestellten Aldehyds setzte man in Analogie zu Beispiel 23a unter Verwendung von Dimethyl-(2-oxo-2-cyclohexyl-ethyl)-phosphonat um und isolierte nach Aufarbeitung und Reinigung 39 mg (83,2 μmol, 60%) der Titelverbindung als farbloses Öl.

IR (Film): 3070, 3050, 3010, 2930, 2850, 1735, 1690, 1660, 1620, 1510, 1445, 1370, 1310, 1230, 1160, 995, 900 und 835 $cm^{-1}$.

**Beispiel 231**

(1S,4R,5S(5Z),6S)-7-[4-(4-Fluorphenyl)-6-[(1E,3S)-3-hydroxy-3-cyclohexyl-prop-1-enyl]-2-oxa-bicyclo[2.2.1] hept-5-yl]-5-heptensäure:

19 mg (40,4 μmol) des nach Beispiel 230 dargestellen unpolaren Esters A verseifte man in Analogie zu Beispiel 2 und isolierte nach Aufarbeitung und Reinigung 18 mg (39,4 μmol, 98%) der Titelverbindung als wachsartigen Stoff.

IR (KBr): 3700-2300, 3050, 3010, 2930, 2860, 1710, 1610, 1515, 1450, 1235, 1160, 995, 970, 895 und 835 $cm^{-1}$.

**Beispiel 232**

(1S,4R,5S(5Z),6S)-7-[4-(4-Fluorphenyl)-6-[(1E,3R)-3-hydroxy-3-cyclohexyl-prop-1-enyl]-2-oxa-bicyclo[2.2.1] hept-5yl]-5-heptensäure:

14 mg (29,7 μmol) des nach Beispiel 230 dargestellen polaren Esters B verseifte man in Analogie zu Beispiel 2 und isolierte nach Aufarbeitung und Reinigung 12 mg (26,3 μmol, 88%) der Titelverbindung als farbloses Öl.

IR (Film): 3700-2400, 3050, 3010, 2930, 2850, 1710, 1610, 1515, 1450, 1410, 1300, 1235, 1160,995, 970,895 und 835 $cm^{-1}$.

**Beispiel 233**

(1S,4R,5S(5Z),6R)-7-[4-(4-Fluorphenyl)-6-[3(S)-hydroxy-4,4-trimethylenon-non-1,7-diinyl]-2-oxa-bicyclo[2.2.1] hept-5-yl]-5-heptensäuremethylester:

2,6 mg (5,3 μmol) der nach Beispiel 234 dargestellten Säure veresterte man in Analogie zu Beispiel 186 und isolierte nach Lösungsmittelabzug 2,3 mg (4,5 μmol, 86%) der Titelverbindung als farbloses Öl.

IR (Film): 3600-3200, 3040, 3000, 2980, 2940, 2870, 2230, 1735, 1610, 1515, 1450, 1410, 1300, 1230, 1160, 1040, 1015, 995, 895 und 835 $cm^{-1}$.

**Beispiel 234**

(1S,4R,5S(5Z),6R)-7-[4-(4-Fluorphenyl)-6-[3(S)-hydroxy-4,4-trimethylenon-non-1,7-diinyl]-2-oxa-bicyclo[2.2.1] hept-5-yl]-5-heptensäure:

17 mg (28,9 μmol) des nach Beispiel 237 dargestellen unpolaren Alkohols A setzte man in Analogie zu

Beispiel 187 um und isolierte nach Aufarbeitung und Reinigung 8,7 mg (17,7 µmol, 61%) der Titelverbindung als farbloses Öl.

IR (Film): 3600-2400, 3040, 3020, 2980, 2940, 2880, 2230, 1710, 1610, 1515, 1450, 1410, 1295, 1230, 1160, 1040, 1015, 995, 970, 895 und 835 $cm^{-1}$.

**Beispiel 235**

(1S,4R,5S(5Z),6R)-7-[4-(4-Fluorphenyl)-6-[3(R)-hydroxy-4,4-trimethylen-non-1,7-diinyl]-2-oxa-bicyclo[2.2.1]hept-5-yl]-5-heptensäuremethylester:

2,2 mg (4,5 µmol) der nach Beispiel 236 dargestellten Säure veresterte man in Analogie zu Beispiel 186 und isolierte nach Lösungsmittelabzug 2,2 mg (4,3 µmol, 96%) der Titelverbindung als farbloses Öl.

IR (Film): 3600-3200, 3050, 2980, 2940, 2870, 2230, 1735, 1610, 1515, 1450, 1435, 1410, 1300, 1235, 1160, 1040, 1020, 995, 895 und 835 $cm^{-1}$.

**Beispiel 236**

(1S,4R,5S(5Z),6R)-7-[4-(4-Fluorphenyl)-6-[3(R)-hydroxy-4,4-trimethylen-1,7-diinyl]-2-oxa-bicyclo[2.2.1]hept-5-yl]-5-heptensäure:

9,2 mg (15,7 µmol) des nach Beispiel 237 dargestellten polaren Alkohols B setzte man in Analogie zu Beispiel 187 um und isolierte nach Aufarbeitung und Reinigung 4,8 mg (9,7 µmol, 62%) der Titelverbindung als farbloses Öl.

IR (Film): 3600-2400, 3040, 2980, 2940, 2870, 2230, 1710, 1610, 1515, 1445, 1435, 1410, 1295, 1235, 1160, 1035, 1015, 995, 970, 895 und 835 $cm^{-1}$.

**Beispiel 237**

(1S,4R,5S(5Z),6R)-7-[4-(4-Fluorphenyl)-6-[(1E/Z,3S)-2-brom-3-hydroxy-4,4-trimethylen-non-1-en-7-inyl]-2-oxa-bicyclo[2.2.1]hept-5-yl]-5-heptensäuremethylester (A) und (1S,4R,5S(5Z),6R)-7-[4-(4-Fluorphenyl)-6-[(1E/Z, 3R)-2-brom-3-hydroxy-4,4-trimethylenon-non-1-en-7-inyl]-2-oxabicyclo[2.2.1]hept-5-yl]-5-heptensäuremethylester (B):

32 mg (54,6 µmol) des in Beispiel 237a dargestellten ungesättigten Ketons reduzierte man in Analogie zu Beispiel 23 und isolierte nach Aufarbeitung und Reinigung 28,5 mg (48,5 µmol, 89%) eines Gemisches der beiden Titelverbindungen als farbloses Öl.

IR (Film): 3600-3200, 3050, 2940, 2860, 1730, 1605, 1510, 1435, 1230, 1160, 1040, 1015, 990,895 und 835 $cm^{-1}$.

Die chromatographische Trennung lieferte 17 mg (28,9 µmol, 53%) des unpolareren Alkohols, dem man die Struktur A zuordnete, sowie 9,2 mg (15,7 µmol, 29%) des polareren Alkohols dem man die Struktur B zuordnete.

**Beispiel 237a**

(1S,4R,5S(5Z),6R)-7-[4-(4-Fluorphenyl)-6-[(1E/Z)-2-brom-3-oxo-4,4-trimethylen-non-1-en-7-inyl]-2-oxabicyclo[2.2.1]hept-5-yl]-5-heptensäuremethylester:

65,1 mg (181 µmol) des in Beispiel 138b dargestellten Aldehyds setzte man in Analogie zu Beispiel 190a unter Verwendung von Dimethyl-(2-oxo-3,3-trimethylen-oct-6-inyl)-phosphonat um und isolierte nach Aufarbeitung und Reinigung 32 mg (54,6 µmol, 30%) der Titelverbindung als farbloses Öl.

IR (Film): 3050, 2940, 2870, 1735, 1675, 1605, 1515, 1435, 1230, 1160, 1045, 900 und 835 $cm^{-1}$.

**Beispiel 238**

(1S,4R,5S(5Z),6R)-7-[4-(4-Fluorphenyl)-6-[(1E/Z,3R)-2-brom-3-hydroxy-4,4-trimethylen-non-1-en-7-inyl]-2-oxa-bicyclo[2.2.1]hept-5-yl]-5-heptensäure:

9,9 mg (16,8 µmol) des nach Beispiel 237 dargestellten polaren Esters B verseifte man in Analogie zu Beispiel 2 und isolierte nach Aufarbeitung und Reinigung 8,3 mg (14,5 µmol, 86%) der Titelverbindung als farbloses Öl.

IR (Film): 3600-2400, 3050, 3010, 2950, 2930, 2860, 1710, 1605, 1510, 1440, 1230, 1160, 1040, 1015, 990, 900 und 835 $cm^{-1}$.

**Beispiel 239**

(1S,4R,5S(5Z),6R)-7-[4-(4-Fluorphenyl)-6-[1E/Z,3S)-2-brom-3-hydroxy-4,4-trimethylen-non-1-en-7-inyl]-2-oxabicyclo[2.2.1]hept-5-yl]-5-heptensäure:

10,5 mg (17,9 μmol) des nach Beispiel 237 dargestellten unpolaren Esters A verseifte man in Analogie zu Beispiel 2 und isolierte nach Aufarbeitung und Reinigung 8,8 mg (15,3 μmol, 86%) der Titelverbindung als farbloses Öl.

IR (Film): 3600-2300, 3070, 3050, 3010, 2940, 2860, 1715, 1605, 1515, 1440, 1235, 1160, 1040, 1010, 990, 895 und 835 $cm^{-1}$.

**Beispiel 240**

(1S,4R,5S(5Z),6R)-7-[4-(4-Fluorphenyl)-6-(1-naphtylmethyloxymethyl)-2-oxabicyclo[2.2.1]hept-5-yl]-5-heptensäuremethyl(bzw.1-naphtylmethyl)ester:

16,4 mg (45,2 μmol) des in Beispiel 120a dargestellten Alkohols setzte man in Analogie zu Beispiel 1 mit 1-Chlormethyl-naphtyl um und isolierte nach Aufarbeitung und Reinigung 22,3 mg der Titelverbindungen als farbloses Öl.

IR (Film): 3070, 3050, 3010, 2930, 2870, 1735, 1605, 1595, 1515, 1230, 1160, 1115, 1095, 1075, 1030, 970, 895, 835, 800, 795 und 775 $cm^{-1}$.

**Beispiel 241**

(1S,4R,5S(5Z),6R)-7-[4-(4-Fluorphenyl)-6-(1-naphtylmethyloxymethyl)-2-oxabicyclo[2.2.1]hept-5-yl]-5-heptensäure:

22,3 mg des nach Beispiel 240 dargestellten Estergemisches verseifte man in Analogie zu Beispiel 2 und isolierte nach Aufarbeitung und Reinigung 12 mg (24,6 μmol, 54%, bezogen auf Startmaterial in Beispiel 240) der Titelverbindung als farbloses Öl.

IR (Film): 3600-2500, 3070, 3050, 3010, 2930, 2870, 1730, 1710, 1605, 1595, 1515, 1235, 1160, 1115, 1095, 1075, 1030, 975, 895, 835, 800, 795 und 775 $cm^{-1}$.

**Beispiel 242**

(1S,4R,5S(5Z),6R)-7-[4-(4-Fluorphenyl)-6-(2-naphtylmethyloxymethyl)-2-oxabicyclo[2.2.1]hept-5-yl]-5-heptensäuremethyl(bzw.1-naphtylmethyl)ester:

16,9 mg (46,6 μmol) des in Beispiel 120a dargestellten Alkohols setzte man in Analogie zu Beispiel 1 mit 2-Brommethyl-naphtyl um und isolierte nach Aufarbeitung und Reinigung 23,4 mg der Titelverbindungen als farbloses Öl.

IR (Film): 3040, 3010, 2930, 2870, 1735, 1605, 1510, 1230, 1160, 1125, 1090, 890, 855, 830, 815 und 750 $cm^{-1}$.

**Beispiel 243**

(1S,4R,5S(5Z),6R)-7-[4-(4-Fluorphenyl)-6-(2-naphtylmethyloxymethyl)-2-oxabicyclo[2.2.1]hept-5-yl]-5-heptensäure:

23,4 mg des nach Beispiel 242 dargestellten Estergemisches verseifte man in Analogie zu Beispiel 2 und isolierte nach Aufarbeitung und Reinigung 20 mg (40,9 μmol, 88%, bezogen auf Startmaterial in Beispiel 242) der Titelverbindung als farbloses Öl.

IR (Film): 3600-2400, 3050, 3010, 2930, 2870, 1730, 1705, 1605, 1515, 1230, 1160, 1125, 1080, 895, 855, 830, 820 und 750 $cm^{-1}$.

**Beispiel 244**

(1S,4R,5S(5Z),6R)-7-[4-(4-Fluorphenyl)-6-(2,4-bis-trifluormethylbenzyloxymethyl)-2-oxabicyclo[2.2.1]hept-5-yl]-5-heptensäuremethyl(bzw.2,4-bis-trifluormethylbenzyl)ester:

17,6 mg (48,5 μmol) des in Beispiel 120a dargestellten Alkohols setzte man in Analogie zu Beispiel 1 mit 2,4-Bis-trifluormethyl-benzylbromid um und isolierte nach Aufarbeitung und Reinigung 29,6 mg der Titelverbindungen als farbloses Öl.

IR (Film): 3070, 3050, 3010, 2930, 2870, 1735, 1625, 1605, 1515, 1345, 1300, 1275, 1235, 1175, 1130,

1085, 1055, 910, 900 und 835 $cm^{-1}$.

**Beispiel 245**

(1S,4R,5S(5Z),6R)-7-[4-(4-Fluorphenyl)-6-(2,4-bis-trifluormethylbenzyloxymethyl)-2-oxabicyclo[2.2.1]hept-5-yl]-5-heptensäure:

29,6 mg des nach Beispiel 244 dargestellten Estergemisches verseifte man in Analogie zu Beispiel 2 und isolierte nach Aufarbeitung und Reinigung 22 mg (38,3 µmol, 79%, bezogen auf Startmaterial in Beispiel 244) der Titelverbindung als farbloses Öl.

IR (Film): 3600-2400, 3050, 3000, 2930, 2870, 1730, 1710, 1630, 1610, 1515, 1345, 1300, 1275, 1235, 1175, 1130, 1085, 1055, 910 und 835 $cm^{-1}$.

**Beispiel 246**

(1S,4R,5S(5Z),6R)-7-[4-(4-Fluorphenyl)-6-(3,5-bis-trifluormethylbenzyloxymethyl)-2-oxabicyclo[2.2.1]hept-5-yl]-5-heptensäuremethyl(bzw.3,5-bis-trifluormethylbenzyl)ester:

17,7 mg (48,8 µmol) des in Beispiel 120a dargestellten Alkohols setzte man in Analogie zu Beispiel 1 mit 3,5-Bis-trifluormethyl-benzylbromid um und isolierte nach Aufarbeitung und Reinigung 26,5 mg der Titelverbindungen als farbloses Öl.

IR (Film): 3050, 3000, 2940, 2870, 1735, 1620, 1605, 1510, 1455, 1435, 1380, 1355, 1280, 1230, 1175, 1130, 900, 885, 835, 705 und 680 $cm^{-1}$.

**Beispiel 247**

(1S,4R,5S(5Z),6R)-7-[4-(4-Fluorphenyl)-6-(3,5-bis-trifluormethylbenzyloxymethyl)-2-oxabicyclo[2.2.1]hept-5-yl]-5-heptensäure:

26,5 mg des nach Beispiel 246 dargestellten Estergemisches verseifte man in Analogie zu Beispiel 2 und isolierte nach Aufarbeitung und Reinigung 23 mg (40 µmol, 82%, bezogen auf Startmaterial in Beispiel 246) der Titelverbindung als farbloses Öl.

IR (Film): 3700-2400, 3050, 3010, 2930, 2870, 1730, 1710, 1625, 1610, 1515, 1385, 1355, 1280, 1235, 1175, 1135, 890, 845, 835, 820, 705 und 685 $cm^{-1}$.

**Beispiel 248**

(1S,4R,5S(5Z),6R)-7-[4-(4-Fluorphenyl)-6-(3-methylbenzyloxymethyl)-2-oxabicyclo[2.2.1]hept-5-yl]-5-heptensäuremethyl(bzw.3-methylbenzyl)ester:

16,9 mg (46,6 µmol) des in Beispiel 120a dargestellten Alkohols setzte man in Analogie zu Beispiel 1 mit 3-Methyl-benzylbromid um und isolierte nach Aufarbeitung und Reinigung 23,7 mg der Titelverbindungen als farbloses Öl.

IR (Film): 3050, 3010, 2930, 2870, 1735, 1605, 1595, 1515, 1235, 1160, 1115, 1090, 1025, 975, 895, 835, 785 und 700 $cm^{-1}$.

**Beispiel 249**

(1S,4R,5S(5Z),6R)-7-[4-(4-Fluorphenyl)-6-(3-methylbenzyloxymethyl)-2-oxabicyclo[2.2.1]hept-5-yl]-5-heptensäure:

23,7 mg des nach Beispiel 248 dargestellten Estergemisches verseifte man in Analogie zu Beispiel 2 und isolierte nach Aufarbeitung und Reinigung 17,4 mg (38,4 µmol, 82%, bezogen auf Startmaterial in Beispiel 248) der Titelverbindung als farbloses Öl.

IR (Film): 3700-2400, 3050, 3010, 2930, 2870, 1730, 1710, 1610, 1595, 1515, 1235, 1160, 1115, 1090, 1030, 975, 895, 835, 785 und 695 $cm^{-1}$.

**Beispiel 250**

(1S,4R,5S(5Z),6R)-7-[4-(4-Fluorphenyl)-6-(4-methylbenzyloxymethyl)-2-oxabicyclo[2.2.1]hept-5-yl]-5-heptensäuremethyl(bzw.4-methylbenzyl)ester:

18,5 mg (51 µmol) des in Beispiel 120a dargestellten Alkohols setzte man in Analogie zu Beispiel 1 mit 4-Methyl-benzylbromid um und isolierte nach Aufarbeitung und Reinigung 24,2 mg der Titelverbindungen als

farbloses Öl.

IR (Film): 3050, 3000, 2950, 2930, 2870, 1735, 1610, 1595, 1515, 1455, 1410, 1360, 1295, 1230, 1160, 1120, 1090, 1030, 1020, 970, 895, 830, 815 und 805 $cm^{-1}$.

**Beispiel 251**

(1S,4R,5S(5Z),6R)-7-[4-(4-Fluorphenyl)-6-(4-methylbenzyloxymethyl)-2-oxabicyclo[2.2.1]hept-5-yl]-5-heptensäure:

24,2 mg des nach Beispiel 250 dargestellten Estergemisches verseifte man in Analogie zu Beispiel 2 und isolierte nach Aufarbeitung und Reinigung 19,9 mg (44 µmol, 86%, bezogen auf Startmaterial in Beispiel 250) der Titelverbindung als farbloses Öl.

IR (Film): 3600-2400, 3050, 3010, 2940, 2870, 1730, 1710, 1610, 1515, 1455, 1410, 1360, 1300, 1230, 1160, 1115, 1090, 1030, 1020, 970, 895, 830, 820 und 805 $cm^{-1}$.

**Beispiel 252**

(1S,4R,5S(5Z),6R)-7-[4-(4-Fluorphenyl)-6-(4-(benzyloxy)-benzyloxymethyl)-2-oxabicyclo[2.2.1]hept-5-yl]-5-heptensäuremethyl(bzw.4-(benzyloxy)Benzyl)ester:

21,4 mg (59 µmol) des in Beispiel 120a dargestellten Alkohols setzte man in Analogie zu Beispiel 1 mit 4-Benzyloxy-benzylchlorid um und isolierte nach Aufarbeitung und Reinigung 32 mg der Titelverbindungen als farbloses Öl.

IR (Film): 3060, 3030, 3010, 2930, 2860, 1735, 1610, 1585, 1515, 1450, 1300, 1235, 1165, 1115, 1090, 1080, 1025, 895, 830, 820, 740 und 700 $cm^{-1}$.

**Beispiel 253**

(1S,4R,5S(5Z),6R)-7-[4-(4-Fluorphenyl)-6-(4-(benzyloxy)-benzyloxymethyl)-2-oxabicyclo[2.2.1]hept-5-yl]-5-heptensäure:

32 mg des nach Beispiel 252 dargestellten Estergemisches verseifte man in Analogie zu Beispiel 2 und isolierte nach Aufarbeitung und Reinigung 25,8 mg (47,4 µmol, 80%, bezogen auf Startmaterial in Beispiel 252) der Titelverbindung als farbloses Öl.

IR (Film): 3600-2400, 3060, 3030, 3000, 2930, 2860, 1730, 1705, 1610, 1585, 1510, 1450, 1300, 1235, 1170, 1160, 1115, 1090, 1080, 1025, 895, 830, 820, 740 und 695 $cm^{-1}$.

**Beispiel 254**

(1S,4R,5S(5Z),6S)-7-[4-(2-Phenylethinyl)-6-[3(S)-hydroxy-1(E)-octenyl]-2-oxabicyclo[2.2.1]hept-5-yl]-5-heptensäure:

17 mg (36,6 µmol) des nach Beispiel 256 dargestellten unpolaren Esters A verseifte man in Analogie zu Beispiel 2 und isolierte nach Aufarbeitung und Reinigung 16 mg (35,5 µmol, 97%) der Titelverbindung als farbloses Öl.

IR (Film): 3600-2300, 3080, 3060, 3010, 2950, 2930, 2880, 2860, 2230, 1710, 1600, 1490, 1455, 1445, 1405, 1305, 1240, 1200, 1120, 1015, 995, 970, 895, 755 und 690 $cm^{-1}$.

**Beispiel 255**

(1S,4R,5S(5Z),6S)-7-[4-(2-Phenylethinyl)-6-[3(R)-hydroxy-1(E)-octenyl]-2-oxabicyclo[2.2.1]hept-5-yl]-5-heptensäure:

16 mg (34,4 µmol) des nach Beispiel 256 dargestellten polaren Esters B verseifte man in Analogie zu Beispiel 2 und isolierte nach Aufarbeitung und Reinigung 12,7 mg (28,2 µmol, 82%) der Titelverbindung als farbloses Öl.

IR (Film): 3700-2400, 3080, 3060, 3010, 2960, 2930, 2870, 2860, 2230, 1710, 1600, 1490, 1455, 1445, 1300, 1240, 1200, 1015, 995, 970, 895, 755 und 695 $cm^{-1}$.

**Beispiel 256**

(1S,4R,5S(5Z),6S)-7-[4-(2-Phenylethinyl)-6-[3(S)-hydroxy-1(E)-octenyl]-2-oxabicyclo[2.2.1]hept-5-yl]-5-heptensäuremethylester (A) und (1S,4R,5S(5Z),6S)-7-[4-(2-Phenylethinyl)-6-[3(R)-hydroxy-1(E)-octenyl]-2-oxabicyclo[2.2.1]hept-5-yl]-5-heptensäuremethylester (B):

39 mg (84,3 µmol) des in Beispiel 256a dargestellten ungesättigten Ketons reduzierte man in Analogie zu Beispiel 23 und isolierte nach Aufarbeitung und Reinigung 36,5 mg (78,6 µmol, 93%) eines Gemisches der beiden Titelverbindungen als farbloses Öl.

IR (Film): 3600-3200, 3070, 3050, 3010, 2950, 2930, 2860, 2220, 1735, 1595, 1490, 1440, 1360, 1300, 1240, 1195, 1155, 1070, 1010, 995, 970, 895, 755 und 690 $cm^{-1}$.

Die chromatographische Trennung lieferte 17 mg (36,6 µmol, 43%) des unpolareren Alkohols, dem man die Struktur A zuordnete, sowie 16 mg (34,4 µmol, 41%) des polareren Alkohols dem man die Struktur B zuordnete.

## Beispiel 256a

(1S,4R,5S(5Z),6S)-7-[4-(2-Phenylethinyl)-6-[3-oxo-1(E)-octenyl]-2-oxabicyclo[2.2.1]hept-5-yl]-5-heptensäuremethylester:

52 mg (142 µmol) des in Beispiel 256b dargestellten Aldehyds setzte man in Analogie zu Beispiel 23a unter Verwendung von Dimethyl-(2-oxo-heptyl)-phosphonat um und isolierte nach Aufarbeitung und Reinigung 39 mg (84,3 µmol, 59%) der Titelverbindung als farbloses Öl.

IR (Film): 3070, 3050, 2950, 2930, 2870, 2230, 1735, 1690, 1670, 1620, 1595, 1490, 1450, 1440, 1365, 1310, 1240, 1190, 1170, 1070, 1010, 995, 975, 895, 755 und 690 $cm^{-1}$.

## Beispiel 256b

(1S,4R,5S(5Z),6S)-7-[4-(2-Phenylethinyl)-6-formyl-2-oxabicyclo[2.2.1]hept-5-yl]-5-heptensäuremethylester:

55,7 mg (151 µmol) des in Beispiel 256c dargestellten Alkohols oxidierte man in Analogie zu Beispiel 38b und isolierte nach Aufarbeitung 52 mg (142 µmol, 94%) der Titelverbindung als farbloses Öl.

IR (Film): 3080, 3050, 3010, 2950, 2930, 2870, 2720, 2230, 1735, 1715, 1595, 1490, 1440, 1360, 1310, 1245, 1190, 1170, 1070, 1025, 980, 895, 755 und 690 $cm^{-1}$.

## Beispiel 256c

(1S,4R,5S(5Z),6R)-7-[4-(2-Phenylethinyl)-6-hydroxymethyl-2-oxabicyclo[2.2.1]hept-5-yl]-5-heptensäuremethylester:

5,3 g (19,2 mmol) enantiomeres Corey-Lakton setzte man in Analogie zu den Beispielen 11a bis 11g und 1h bis 11 um und isolierte 208 mg (564 µmol, 2,9%) der Titelverbindung als farbloses Öl.

IR (Film): 3600-3200, 3070, 3050, 2950, 2930, 2870, 2230, 1735, 1490, 1440, 1365, 1300, 1245, 1200, 1155, 1065, 1025, 1000, 890, 755 und 690 $cm^{-1}$.

## Beispiel 257

(1S,4R,5S(5Z),6R)-7-[4-(2-Phenylethinyl)-6-benzyloxymethyl-2-oxabicyclo[2.2.1]hept-5-yl]-5-heptensäuremethyl(bzw.benzyl)ester:

17,8 mg (48,3 µmol) des in Beispiel 256c dargestellten Alkohols setzte man in Analogie zu Beispiel 1 mit Benzylchlorid um und isolierte nach Aufarbeitung und Reinigung 18 mg der Titelverbindungen als farbloses Öl.

IR (Film): 3090, 3060, 3020, 3000, 2930, 2870, 2230, 1730, 1595, 1490, 1450, 1360, 1305, 1210, 1150, 1110, 1070, 1005, 895, 755, 735 und 695 $cm^{-1}$.

## Beispiel 258

(1S,4R,5S(5Z),6R)-7-[4-(2-Phenylethinyl)-6-benzyloxymethyl-2-oxabicyclo[2.2.1]hept-5-yl]-5-heptensäure:

18 mg des nach Beispiel 257 dargestellten Estergemisches verseifte man in Analogie zu Beispiel 2 und isolierte nach Aufarbeitung und Reinigung 16,7 mg (37,6 µmol, 78%, bezogen auf Startmaterial in Beispiel 257) der Titelverbindung als farbloses Öl.

IR (Film): 3600-2400, 3080, 3060, 3030, 3000, 2930, 2870, 2230, 1730, 1705, 1600, 1490, 1450, 1440, 1365, 1305, 1240, 1210, 1110, 1070, 1005, 895, 755, 735 und 695 $cm^{-1}$.

**Beispiel 259**

(1S,4R,5S(5Z),6R)-7-[4-(2-Phenylethinyl)-6-(4-cyanobenzyloxymethyl)-2-oxabicyclo[2.2.1]hept-5-yl]-5-heptensäuremethyl(bzw.4-cyanobenzyl)ester:

16,9 mg (45,9 µmol) des in Beispiel 256c dargestellten Alkohols setzte man in Analogie zu Beispiel 1 mit 4-Brommethylbenzonitril um und isolierte nach Aufarbeitung und Reinigung 20 mg der Titelverbindungen als farbloses Öl.

IR (Film): 3060, 3000, 2940, 2870, 2220, 1730, 1610, 1600, 1440, 1360, 1210, 1105, 1005, 895, 820, 755 und 690 $cm^{-1}$.

**Beispiel 260**

(1S,4R,5S(5Z),6R)-7-[4-(2-Phenylethinyl)-6-(4-cyanobenzyloxymethyl)-2-oxabicyclo[2.2.1]hept-5-yl]-5-heptensäure:

20 mg des nach Beispiel 259 dargestellten Estergemisches verseifte man in Analogie zu Beispiel 2 und isolierte nach Aufarbeitung und Reinigung 18 mg (38 µmol, 84%, bezogen auf Startmaterial in Beispiel 259) der Titelverbindung als farbloses Öl.

IR (Film): 3600-2300, 3080, 3050, 3000, 2930, 2870, 2850, 2230, 1710, 1610, 1600, 1490, 1445, 1310, 1280, 1210, 1110, 1005, 895, 820, 760 und 690 $cm^{-1}$.

**Beispiel 261**

(1S,4R,5S(5Z),6S(1E,3S,4S))-7-[4-(2-Phenylethinyl)-6-(3-hydroxy-4-methyl-non-1-en-6-inyl)-2-oxabicyclo[2.2.1]hept-5-yl]-5-heptensäure:

11,2 mg (22,9 µmol) des nach Beispiel 263 dargestellten unpolaren Esters A verseifte man in Analogie zu Beispiel 2 und isolierte nach Aufarbeitung und Reinigung 10 mg (21,1 µmol, 92%) der Titelverbindung als farbloses Öl.

IR (Film): 3600-2500, 3070, 3050, 3030, 3010, 2970, 2930, 2850, 2230, 1710, 1605, 1490, 1455, 1390, 1240, 1195, 1015, 990, 970, 895, 755 und 690 $cm^{-1}$.

**Beispiel 262**

(1S,4R,5S(5Z),6S(1E,3R,4S))-7-[4-(2-Phenylethinyl)-6-(3-hydroxy-4-methyl-non-1-en-6-inyl)-2-oxabicyclo[2.2.1]hept-5-yl]-5-heptensäure:

18,1 mg (37 µmol) des nach Beispiel 263 dargestellten polaren Esters B verseifte man in Analogie zu Beispiel 2 und isolierte nach Aufarbeitung und Reinigung 14,7 mg (31 µmol, 84%) der Titelverbindung als farbloses Öl.

IR (Film): 3600-2400, 3080, 3050, 3030, 3010, 2970, 2930, 2850, 2230, 1710, 1600, 1490, 1455, 1390, 1240, 1200, 1015, 990, 970, 890, 755 und 690 $cm^{-1}$.

**Beispiel 263**

(1S,4R,5S(5Z),6S(1E,3S,4S))-7-[4-(2-Phenylethinyl)-6-(3-hydroxy-4-methyl-non-1-en-6-inyl)-2-oxabicyclo[2.2.1]hept-5-yl]-5-heptensäuremethylester (A) und (1S,4R,5S(5Z),6S(1E,3R,4S))-7-[4-(2-Phenylethinyl)-6-(3-hydroxy-4-methyl-non-1-en-6-inyl)-2-oxabicyclo[2.2.1]hept-5-yl]-5-heptensäuremethylester (B):

32 mg (65,8 µmol) des in Beispiel 263a dargestellten ungesättigten Ketons reduzierte man in Analogie zu Beispiel 23 und isolierte nach Aufarbeitung und Reinigung 31,1 mg (63,6 µmol, 97%) eines Gemisches der beiden Titelverbindungen als farbloses Öl.

IR (Film): 3600-3200, 3080, 3050, 2970, 2950, 2930, 2870, 2850, 2220, 1730, 1595, 1450, 1435, 1265, 1195, 1155, 1010, 995, 970, 890, 755, 735 und 690 $cm^{-1}$.

Die chromatographische Trennung lieferte 11,2 mg (22,9 µmol, 35%) des unpolareren Alkohols, dem man die Struktur A zuordnete, sowie 18,1 mg (37,0 µmol, 56%) des polareren Alkohols dem man die Struktur B zuordnete.

**Beispiel 263a**

(1S,4R,5S(5Z),6S(1E,4S))-7-[4-(2-Phenylethinyl)-6-(3-oxo-4-methyl-non-1-en-6-inyl-2-oxa bicyclo[2.2.1]hept-5-yl]-5-heptensäuremethylester:

48,6 mg (133 µmol) des in Beispiel 256b dargestellten Aldehyds setzte man in Analogie zu Beispiel 23a unter Verwendung von Dimethyl-(2-oxo-3S-methyl-oct-5-in)-phosphonat um und isolierte nach Aufarbeitung und Reinigung 32 mg (65,8 µmol, 50%) der Titelverbindung als farbloses Öl.

IR (Film): 3080, 3050, 2970, 2930, 2870, 2230, 1735, 1685, 1665 1615, 1450, 1435, 1240, 1160, 1040, 1015, 985, 895, 755, 735 und 695 $cm^{-1}$.

**Beispiel 264**

(1S,4R,5S(5Z),6R(3S,4S))-7-[4-(2-Phenylethinyl)-6-(3-hydroxy-4-methyl-nona-1,6-diinyl)-2-oxabicyclo[2.2.1]hept-5-yl]-5-heptensäuremethylester:

2,5 mg (5,3 µmol) der nach Beispiel 265 dargestellten Säure veresterte man in Analogie zu Beispiel 186 und isolierte nach Lösungsmittelabzug 2,5 mg (5,1 µmol, 97%) der Titelverbindung als farbloses Öl.

IR (Film): 3600-3200, 3080, 3060, 3030, 3010, 2970, 2930, 2880, 2230, 1735, 1605, 1490, 1450, 1410, 1320, 1305, 1290, 1270, 1240, 1160, 1020, 990, 895, 760 und 690 $cm^{-1}$.

**Beispiel 265**

(1S,4R,5S(5Z),6R(3S,4S))-7-[4-(2-Phenylethinyl)-6-(3-hydroxy-4-methyl-nona-1,6-diinyl)-2-oxabicyclo[2.2.1]hept-5-yl]-5-heptensäure:

23,9 mg (42,1 µmol) des nach Beispiel 268 dargestellten unpolaren Alkohols A setzte man in Analogie zu Beispiel 187 um und isolierte nach Aufarbeitung und Reinigung 11,7 mg (24,8 µmol, 59%) der Titelverbindung als farbloses Öl.

IR (Film): 3600-2400, 3060, 3030, 3010, 2970, 2940, 2880, 2230, 1705, 1600, 1490, 1455, 1445, 1410, 1320, 1305, 1280, 1240, 1155, 1020, 990, 895, 760 und 690 $cm^{-1}$.

**Beispiel 266**

(1S,4R,5S(5Z),6R(3R,4S))-7-[4-(2-Phenylethinyl)-6-(3-hydroxy-4-methyl-nona-1,6-diinyl)-2-oxabicyclo[2.2.1]hept-5-yl]-5-heptensäuremethylester:

2,9 mg (6,1 µmol) der nach Beispiel 267 dargestellten Säure veresterte man in Analogie zu Beispiel 186 und isolierte nach Lösungsmittelabzug 3 mg (6,1 µmol, 100%) der Titelverbindung als farbloses Öl.

IR (Film): 3600-3200, 3070, 3050, 3030, 3010, 2970, 2930, 2880, 2230, 1735, 1600, 1490, 1450, 1410, 1320, 1305, 1290, 1270, 1240, 1160, 1020, 990, 895, 760 und 690 $cm^{-1}$.

**Beispiel 267**

(1S,4R,5S(5Z),6R(3R,4S))-7-[4-(2-Phenylethinyl)-6-(3-hydroxy-4-methyl-nona-1,6-diinyl)-2-oxabicyclo[2.2.1]hept-5-yl]-5-heptensäure:

25,7 mg (45 µmol) des nach Beispiel 268 dargestellten polaren Alkohols B setzte man in Analogie zu Beispiel 187 um und isolierte nach Aufarbeitung und Reinigung 15,5 mg (32,8 µmol, 73%) der Titelverbindung als farbloses Öl.

IR (Film): 3700-2400, 3080, 3060, 3030, 3010, 2970, 2930, 2880, 2230, 1710, 1600, 1490, 1455, 1445, 1410, 1320, 1305, 1290, 1270, 1240, 1155, 1020, 990, 895, 760 und 690 $cm^{-1}$.

**Beispiel 268**

(1S,4R,5S(5Z),6R(1E/Z,3S,4S))-7-[4-(2-Phenylethinyl)-6-(2-brom-3-hydroxy-4-methyl-non-1-en-6-inyl)-2-oxabicyclo[2.2.1]hept-5-yl]-5-heptensäuremethylester (A) und (1S,4R,5S(5Z),6R(1E/Z,3R,4S))-7-[4-(2-Phenylethinyl)-6-(2-brom-3-hydroxy-4-methyl-non-1-en-6-inyl)-2-oxabicyclo[2.2.1]hept-5-yl]-5-heptensäuremethylester (B):

58 mg (102 µmol) des in Beispiel 268a dargestellten ungesättigten Ketons reduzierte man in Analogie zu Beispiel 23 und isolierte nach Aufarbeitung und Reinigung 52,8 mg (93 µmol, 91%) eines Gemisches der beiden Titelverbindungen als farbloses Öl.

IR (Film): 3600-3200, 3080, 3050, 2970, 2950, 2930, 2880, 2230, 1735, 1595, 1490, 1450, 1435, 1315, 1240, 1200, 1155, 1025, 1010, 990, 895, 755, 735 und 690 $cm^{-1}$.

Die chromatographische Trennung lieferte 23,9 mg (42,1 µmol, 41%) des unpolareren Alkohols, dem man die Struktur A zuordnete, sowie 25,7 mg (45 µmol, 45%) des polareren Alkohols dem man die Struktur B zuordnete.

**Beispiel 268a**

(1S,4R,5S(5Z),6R(1E/Z,4S))-7-[4-(2-Phenylethinyl)-6-(2-brom-3-oxo-4-methyl-non-1-en-6-inyl)-2-oxabicyclo [2.2.1]hept-5-yl]-5-heptensäuremethylester:

39,5 mg (108 μmol) des in Beispiel 256b dargestellten Aldehyds setzte man in Analogie zu Beispiel 190a unter Verwendung von Dimethyl-(2-oxo-3S-methyl-oct-5-in)-phosphonat um und isolierte nach Aufarbeitung und Reinigung 58 mg (102 μmol, 94%) der Titelverbindung als farbloses Öl.

IR (Film): 3080, 3050, 2970, 2950, 2930, 2880, 2230, 1730, 1685, 1600, 1485, 1450, 1435, 1310, 1265, 1240, 1160, 1070, 1015, 985, 895, 755, 735 und 690 $cm^{-1}$.

**Beispiel 269**

(1S,4R,5S(5Z),6R(1E/Z,3S,4S))-7-[4-(2-Phenylethinyl)-6-(2-brom-3-hydroxy-4-methyl-non-1-en-6-inyl)-2-oxabicyclo[2.2.1]hept-5-yl]-5-heptensäure:

10,2 mg (18 μmol) des nach Beispiel 268 dargestellten unpolaren Esters A verseifte man in Analogie zu Beispiel 2 und isolierte nach Aufarbeitung und Reinigung 8,5 mg (15,4 μmol, 85%) der Titelverbindung als farbloses Öl.

IR (Film): 3700-2400, 3070, 3050, 2970, 2950, 2930, 2880, 2230, 1705, 1595, 1490, 1450, 1435, 1315, 1240, 1200, 1160, 1025, 1010, 990, 895, 755, 735 und 695 $cm^{-1}$.

**Beispiel 270**

(1S,4R,5S(5Z),6R(1E/Z,3R,4S))-7-[4-(2-Phenylethinyl)-6-(2-brom-3-hydroxy-4-methyl-non-1-en-6-inyl)-2-oxabicyclo[2.2.1]hept-5-yl]-5-heptensäure:

12,3 mg (21,7 μmol) des nach Beispiel 268 dargestellten polaren Esters B verseifte man in Analogie zu Beispiel 2 und isolierte nach Aufarbeitung und Reinigung 10,6 mg (19,1 μmol, 88%) der Titelverbindung als farbloses Öl.

IR (Film): 3600-2400, 3080, 3050, 2970, 2950, 2930, 2880, 2230, 1710, 1595, 1495, 1450, 1435, 1315, 1240, 1200, 1160, 1025, 1010, 990, 895, 755, 735 und 690 $cm^{-1}$.

**Beispiel 271**

(1S,4R,5S(5Z),6R)-7-[4-(2-Phenylethinyl)-6-(4-(benzyloxy)-benzyloxymethyl)-2-oxabicyclo[2.2.1]hept-5-yl]-5-heptensäuremethyl[bzw.4-(benzyloxy)benzyl)ester:

28 mg (76 μmol) des in Beispiel 256c dargestellten Alkohols setzte man in Analogie zu Beispiel 1 mit 4-Benzyloxybenzylchlorid um und isolierte nach Aufarbeitung und Reinigung 36 mg der Titelverbindungen als farbloses Öl.

IR (Film): 3070, 3030, 3010, 2930, 2880, 2230, 1735, 1610, 1580, 1510, 1445, 1295, 1240, 1170, 1110, 1080, 1025, 1005, 895, 825, 760, 740 und 695 $cm^{-1}$.

**Beispiel 272**

(1S,4R,5S(5Z),6R)-7-[4-(2-Phenylethinyl)-6-(4-(benzyloxy)-benzyloxymethyl)-2-oxabicyclo[2.2.1]hept-5-yl]-5-heptensäure:

36 mg des nach Beispiel 271 dargestellten Estergemisches verseifte man in Analogie zu Beispiel 2 und isolierte nach Aufarbeitung und Reinigung 28,4 mg (51,6 μmol, 68%, bezogen auf Startmaterial in Beispiel 271) der Titelverbindung als farbloses Öl.

IR (Film): 3600-2400, 3060, 3030, 3010, 2930, 2880, 2230, 1730, 1710, 1610, 1585, 1510, 1300, 1240, 1170, 1105, 1080, 1025, 1005, 895, 825, 760, 740 und 695 $cm^{-1}$.

**Beispiel 273**

(1S,4R,5S.6R)-7-[4-(4-Fluorphenyl)-6-(4-fluorbenzyloxymethyl)-2-oxabicyclo[2.2.1]hept-5-yl]-3-oxa-5(Z)-heptensäure:

9,2 mg des nach Beispiel 274 dargestellten Estergemisches verseifte man in Analogie zu Beispiel 2 und isolierte nach Aufarbeitung und Reinigung 5,8 mg (12,6 μmol, 31%, bezogen auf Startmaterial in Beispiel 274) der Titelverbindung als farbloses Öl.

IR (Film): 3700-2400, 3070, 3040, 2930, 2870, 1730, 1605, 1510, 1225, 1115, 895 und 830 $cm^{-1}$.

**Beispiel 274**

(1S,4R,5S.6R)-7-[4-(4-Fluorphenyl)-6-(4-fluorbenzyloxymethyl)-2-oxabicyclo[2.2.1]hept-5-yl]-3-oxa-5(Z)-heptensäure-t-butyl(bzw.4-fluorbenzyl)ester:

16,5 mg (40 μmol) des in Beispiel 274a dargestellten Alkohols setzte man in Analogie zu Beispiel 1 um und isolierte nach Aufarbeitung und Reinigung 9,2 mg der Titelverbindungen als farbloses Öl.

IR (Film): 3050, 2980, 2930, 2870, 1745, 1605, 1510, 1370, 1265, 1225, 1160, 1120, 1030, 830 und 735 $cm^{-1}$.

**Beispiel 274a**

(1S,4R,5S,6R)-7-[4-(4-Fluorphenyl)-6-hydroxymethyl-2-oxabicyclo[2.2.1]hept-5-yl]-3-oxa-5(Z)-heptensäure-tert.-butylester:

686 mg (1,06 mmol) der in Beispiel 274b dargestellten Verbindung setzte man in Analogie zu Beispiel 1a um und isolierte nach Aufarbeitung und Reinigung 385 mg (947 μmol, 89%) der Titelverbindung als farbloses Öl.

IR (Film): 3600-3200, 3040, 2970, 2920, 2870, 1740, 1605, 1510, 1365, 1230, 1160, 1130, 1025, 970, 895 und 735 $cm^{-1}$.

**Beispiel 274b**

(1S,4R,5S,6R)-7-[4-(4-Fluorphenyl)-6-tert.-butyldiphenylsilyoxymethyl-2-oxabicyclo[2.2.1]hept-5-yl]-3-oxa-5 (Z)-heptensäure-t-butylester:

580 mg (1,09 mmol) des in Beisoiel 274c dargestellten Alkohols setzte man in Analogie zu Beispiel 1 mit Bromessigsäure-tert.-butylester um und isolierte nach Aufarbeitung und Reinigung 686 mg (1,06 mmol, 97%) der Titelverbindung als farbloses Öl.

IR (Film): 3050, 3040, 3010, 2970, 2930, 2850, 1745, 1605, 1590, 1510, 1470, 1425, 1390, 1365, 1295, 1225, 1160, 1130, 1110, 940, 895, 830, 820, 740 und 700 $cm^{-1}$.

**Beispiel 274c**

(1S,4R,5S,6R)-4-[4-(4-Fluorphenyl)-6-tert.-butyldiphenylsilyloxymethyl-2-oxabicyclo[2.2.1]hept-5-yl]-2(Z)-butenol:

620 mg (1,11 mmol) des in Beispiel 274d dargestelten Esters A reduzierte man in Analogie zu Beispiel 274j und isolierte nach Aufarbeitung 581 mg (1,09 mmol, 99%) der Titelverbindung als farbloses Öl.

IR (Film): 3600-3200, 3070, 3040, 3010, 2950, 2930, 2860, 1605, 1590, 1510, 1470, 1425, 1265, 1230, 1160, 1110, 1025, 1015, 895, 830, 820, 735 und 700 $cm^{-1}$.

**Beispiel 274d**

(1S,4R,5S,6R)-4-[4-(4-Fluorphenyl)-6-tert.-butyldiphenylsilyloxymethyl-2-oxabicyclo[2.2.1]hept-5-yl]-2(Z)-butensäuremethylester (A) und (1S,4R,5S,6R)-4-[4-(4-Fluorphenyl)-6-tert.-butyldiphenylsilyloxymethyl-2-oxabicyclo[2.2.1]hept-5-yl]-2(E)-butensäuremethylester (B):

Die Lösung von 1,25 ml bis-(2.2.2-Trifluorethyl)-methoxycarbonylmethyl-phosphonat in 30 ml wasserfreiem Tetrahydrofuran versetzte man mit 1,96 g 18-Krone-6, kühlte unter einer Atmosphäre aus trockenem Argon auf -70°C und tropfte 5,9 ml einer 1M Lösung von Natriumhexamethyl-disilazid in Tetrahydrofuran zu. Man ließ 15 Minuten reagieren und tropfte anschließend die Lösung von 748 mg (1,48 mmol) des in Beispiel 274e dargestellten Aldehyds in 15 ml wasserfreiem Tetra-hydrofuran zu und ließ 2,5 Stunden bei -70°C bis -60°C rühren. Anschließend goß man auf eine ge-sättigte Ammoniumchloridlösung, extrahierte mehrfach mit Diethylether, wusch mit Wasser und gesättigter Natriumchloridlösung, trocknete über Magnesiumsulfat und reinigte den nach Filtration und Lösungsmittelabzug erhaltenen Rückstand durch Chromatographie an ca. 100 ml Kieselgel unter Verwendung eines Gradientensystems aus n-Hexan und Ethylacetat. Isoliert wurden 622 mg (1,11 mmol, 75%) der Titelverbindung A sowie 54 mg (96,6 μmol, 7%) der Titelverbindung B.

IR (Film) von A: 3070, 3040, 2950, 2930, 2860, 1720, 1645, 1605, 1590, 1510, 1425, 1230, 1205, 1170, 1110, 1085, 1025, 895, 820, 740 und 700 $cm^{-1}$.

IR (Film) von B: 3070, 3040, 2950, 2930, 2860, 1720, 1655, 1645, 1605, 1590, 1510, 1425, 1265, 1230, 1160, 1110, 1085, 1025, 1015, 895, 830, 820, 740 und 700 $cm^{-1}$.

**Beispiel 274e**

(1S,4R,5S,6R)-2-[4-(4-Fluorphenyl)-6-tert.-butyldiphenylsilyloxymethyl-2-oxabicyclo[2.2.1]hept-5-yl]-1-oxo-ethan:

748 mg (1,48 mmol) des in Beispiel 274f dargestellten Alkohols oxidierte man in Analogie zu Beispiel 38b und isolierte nach aufarbeitung 748 mg (1,48 mmol, 100%) der Titelverbindung als blassgelbes Öl, das ohne Reinigung weiter umgesetzt wurde.

**Beispiel 274f**

(1S,4R,5S,6R)-2-[4-(4-Fluorphenyl)-6-tert.-butyldiphenylsilyloxymethyl-2-oxabicyclo[2.2.1]hept-5-yl]-1-hydroxy-ethan:

1,03 g (1,68 mmol) des in Beispiel 274g dargestellten Benzoates verseifte man in Analogie zu Beispiel 2 und isolierte nach Aufarbeitung und Reinigung 748 mg (1,48 mmol, 88%) der Titelverbindung als farbloses Öl.

IR (Film): 3600-3200, 3070, 3050, 2920, 2850, 1605, 1590, 1510, 1465, 1425, 1260, 1230, 1160, 1110, 1075, 1065, 1015, 975, 935, 895, 830, 820, 740 und 700 $cm^{-1}$.

**Beispiel 274g**

(1S,4R,5S,6R)-2-[4-(4-Fluorphenyl)-6-tert.-butyldiphenylsilyloxymethyl-2-oxabicyclo[2.2.1]hept-5-yl]-1-benzoyloxy-ethan:

1,11 g (1,76 mmol) des in Beispiel 274h dargestellten Diols setzte man in Analogie zu Beispiel 1b um und isolierte nach Aufarbeitung und Reinigung 1,03 g (1,68 mmol, 95%) der Titelverbindung als farbloses Öl.

IR (Film): 3070, 3050, 2950, 2930, 2850, 1775, 1715, 1600, 1585, 1510, 1465, 1450, 1425, 1270, 1230, 1110, 1070, 1020, 830, 820, 740 und 705 $cm^{-1}$.

**Beispiel 274h**

(1R,2S,3R,4S)-2-[1-Hydroxymethyl-1-(4-fluorphenyl)-3-tert.-butyldiphenylsilyloxymethyl-4-hydroxy-cyclopentan-2-yl]-1-benzoyloxyethan:

1,79 g (2,17 mmol) des in Beispiel 274i dargestellten Benzoates setzte man in Analogie zu Beispiel 1c um und isolierte nach Aufarbeitung und Reinigung 1,11 g (1,76 mmol, 81%) der Titelverbindung als farbloses Öl.

IR (Film): 3700-3100, 3070, 3040, 2950, 2930, 2860, 1715, 1600, 1585, 1510, 1470, 1450, 1425, 1270, 1235, 1110, 1070, 1045, 825, 740 und 705 $cm^{-1}$.

**Beispiel 274i**

(1R,2S,3R,4S)-2-[1-tert.-Butyldimethylsilyloxymethyl-1-(4-fluorphenyl)-3-tert.-butyldiphenylsilyloxymethyl-4-(tetrahydropyran-2-yloxy)-cyclopentan-2-yl]-1-benzoyloxyethan:

1,70 g (2,36 mmol) des in Beispiel 274j dargestellten Alkohols löste man in 17 ml wasserfreiem Pyridin, versetzte mit 850 µl Benzoylchlorid und ließ 4 Stunden bei 23°C unter einer Atmosphäre aus trockenem Argon reagieren. Man verdünnte mit Diethylether, wusch mehrfach mit Eiswasser, trochnete über Magnesiumsulfat und reinigte den nach Filtration und Lösungsmittelabzug erhaltenen Rückstand durch Chromatographie an ca. 75 ml Kieselgel. Als Laufmittel diente ein Gradientensystem aus n-Hexan und Diethylether. Isoliert wurden 1,79 g (2,17 mmol, 92%) der Titelverbindung als farbloses Öl.

IR (Film): 3070, 3050, 2950, 2860, 1715, 1600, 1585, 1510, 1475, 1450, 1425, 1270, 1250, 1110, 1025, 875, 840, 740 und 705 $cm^{-1}$.

**Beispiel 274j**

(1R,2S,3R,4S)-2-[1-tert.-Butyldimethylsilyloxymethyl-1-(4-fluorphenyl)-3-tert.-butyldiphenylsilyloxymethyl-4-(tetrahydropyran-2-yloxy)-cyclopentan-2-yl]-1-hydroxyethan:

1,77 g (2,36 mmol) der in Beispiel 274k dargestellten Verbindung löste man in 30 ml wasserfreien Toluol, kühlte unter einer Atmosphäre aus trockenem Argon auf 0-5°C, versetzte mit 8,3 ml einer 1M Lösung von Diisobutylaluminiumhydrid in Toluol und ließ 30 Minuten reagieren. Die Auf-arbeitung erfolgte in Analogie zu Beispiel 1e. Man isolierte 1,70 g (2,36 mmol, 100%) der Titel-verbindung als farbloses Öl.

IR (Film): 3600-3400, 3070, 3050, 2940, 2860, 1605, 1590, 1470, 1425, 1265, 1250, 1235, 1165, 1130,

1110, 1075, 1025, 875, 840, 740 und 705 $cm^{-1}$.

**Beispiel 274k**

(1R,2S,3R,4S)-2-[1-tert.-Butyldimethylsilyloxymethyl-1-(4-fluorphenyl)-3-tert.-butyldipheny lsilyloxymethyl-4-(tetrahydropyran-2-yloxy)-cyclopentan-2-yl]-ethansäuremethylester:

1,55 g (2,44 mmol) des in Beispiel 274l dargestellten Hydroxyesters setzte man in Analogie zu Beispiel 11 unter Verwendung von tert.-Butyldimethylchlorsilan um und isolierte nach Aufarbeitung und chromatographischer Reinigung 1,77 g (2,36 mmol, 97%) der Titelverbindung als farbloses Öl.

IR (Film): 3070, 3040, 2940, 2850, 1735, 1600, 1585, 1510, 1465, 1425, 1250, 1235, 1160, 1110, 1075, 1030, 1020, 875, 840, 740 und 700 $cm^{-1}$.

**Beispiel 274l**

(1R,2S,3R,4S)-2-[1-hydroxymethyl-1-(4-fluorphenyl)-3-tert.-butyldiphenylsilyloxymethyl-4-(tetra-hydropyran-2-yloxy)-cyclopentan-2-yl]-ethansäuremethylester:

1,48 g (2,46 mmol) des nach Beispiel 274m dargestellten Laktons löste man in 15 ml Tetrahydrofuran, versetzte mit 15 ml einer 1n Kaliumhydroxidlösung und ließ 16 Stunden bei 23°C kräftig rühren. Das organische Lösungsmittel wurde weitestgehend abdestilliert, die wässrige Phase mit eiskalter gesättigter Zitronensäure auf pH 4 bis 5 angesäuert, rasch mehrfach mit eiskaltem Dichlormethan extrahiert und die vereinigten Extrakte zu einer vorgekühlten etherischen Lösung von Diazomethan getropft. Anschließend trocknete man über Magnesiumsulfat und isolierte nach Filtration und Lösungsmittelabzug 1,55 g (2,44 mmol, 99%) der Titelverbindung als blass gelbes Öl, das rasch ohne Reinigung weiter umgesetzt wurde.

**Beispiel 274m**

(1R,6S,7R,8S)-1-(4-Fluorphenyl)-7-tert.-butyldiphenylsilyloxymethyl-8-(tetrahydropyran-2-yloxy)-3-oxabicyclo [4.3.0]nonan-4-on:

1,007g (1,66 mmol) (1R,4RS,6S,7R,8S)-1-(4-Fluorphenyl)-4-hydroxy-7-tert.-butyldiphenylsilyloxy-methyl-8-(tetrahydropyran-2-yloxy)-3-oxabicyclo[4.3.0]nonan, das man in Analogie zu den Bei-spielen 1f bis 1l aus enantiomerem Corey-Lakton darstellte (vgl. Beispiel 120a) löste man in 20 ml wasserfreiem Aceton, kühlte unter einer Atmosphäre aus trockenem Argon auf -30°C, versetzte mit 1 ml einer standardisierten Jones-Lösung und rührte 2 Stunden bei -30°C. Die Aufarbeitung erfolgte in Analogie zu Beispiel 1i. Isoliert wurden 994 mg (1,65 mmol, 99%) der Titelverbindung als blass gelbes Öl.

IR (Film): 3070, 3040, 2940, 2850, 1750, 1605, 1580, 1510, 1465, 1425, 1260, 1235, 1110, 1080, 1030, 970, 820, 740 und 700 $cm^{-1}$.

**Beispiel 275**

(1S,4R,5S.6R)-7-[4-(4-Fluorphenyl)-6-(4-fluorbenzyloxymethyl)-2-oxabicyclo[2.2.1]hept-5-yl]-3-oxa-5(E)-heptensäure:

17,4 mg des nach Beispiel 276 dargestellten Estergemisches verseifte man in Analogie zu Beispiel 2 und isolierte nach Aufarbeitung und Reinigung 12,2 mg (26,6 µmol, 54%, bezogen auf Startmaterial in Beispiel 276) der Titelverbindung als farbloses Öl.

IR (Film): 3600-2400, 3070, 3040, 2920, 2860, 1730, 1605, 1515, 1225, 1160, 1115, 970, 900 und 830 $cm^{-1}$.

**Beispiel 276**

(1S,4R,5S.6R)-7-[4-(4-Fluorphenyl)-6-(4-fluorbenzyloxymethyl)-2-oxabicyclo[2.2.1]hept-5-yl]-3-oxa-5(E)-heptensäure-t-butyl(bzw.4-fluorbenzyl)ester:

20,2 mg (49 µmol) des in Beispiel 276a dargestellten Alkohols setzte man in Analogie zu Beispiel 1 um und isolierte nach Aufarbeitung und Reinigung 17,4 mg der Titelverbindungen als farbloses Öl.

IR (Film): 3070, 3040, 2980, 2930, 2860, 1745, 1605, 1510, 1365, 1225, 1160, 1125, 1025, 970, 895 und 830 $cm^{-1}$.

**Beispiel 276a**

(1S,4R,5S,6R)-7-[4-(4-Fluorphenyl)-6-hydroxymethyl-2-oxabicyclo[2.2.1]hept-5-yl]-3-oxa-5(E)-heptensäure-t-butylester:

388 mg (602 µmol) der in Beispiel 276b dargestellten Verbindung setzte man in Analogie zu Beispiel 1a um und isolierte nach Aufarbeitung und Reinigung 218 mg (536 µmol, 89%) der Titelverbindung als farbloses Öl.

IR (Film): 3600-3200, 3070, 3040, 2970, 2920, 2870, 1740, 1605, 1510, 1365, 1300, 1230, 1160, 1130, 1025, 970, 895 und 835 $cm^{-1}$.

**Beispiel 276b**

(1S,4R,5S,6R)-7-[4-(4-Fluorphenyl)-6-tert.-butyldiphenylsilyloxymethyl-2-oxabicyclo[2.2.1]hept-5-yl]-3-oxa-5(E)-heptensäure-t-butylester:

364 mg (686 µmol) der in Beispiel 276c dargestellten Verbindung A veretherte man in Analogie zu Beispiel 1 mit Bromessigsäure-tert.-butylester und isolierte nach Aufarbeitung und Reinigung 388 mg (602 µmol, 88%) der Titelverbindung als farbloses Öl.

IR (Film): 3090, 3070, 3040, 3010, 2950, 2930, 2860, 1745, 1605, 1590, 1510, 1470, 1425, 1390, 1365, 1295, 1225, 1160, 1130, 1110, 895, 830, 820, 740 und 700 $cm^{-1}$.

**Beispiel 276c**

(1S,4R,5S,6R)-4-[4-(4-Fluorphenyl)-6-tert.-butyldiphenylsilyloxymethyl-2-oxabicyclo[2.2.1]hept-5-yl]-2(E)-butenol (A) und (1S,4R,5S,6R)-4-[4-(4-Fluorphenyl)-6-tert.-butyldiphenylsilyloxymethyl-2-oxabicyclo[2.2.1]hept-5-yl]-2(Z)-butenol (B):

506 mg (883 µmol) der in Beispiel 276d dargestellten Verbindungen reduzierte man in Analogie zu Beispiel 274j und isolierte nach Aufarbeitung und chromatographischer Trennung an ca. 100 ml Kieselgel unter Verwendung eines Gradientensystems aus n-Hexan und Ethylacetat 374 mg (705 µmol, 80%) der Titelverbindung A sowie 25 mg (47 µmol, 5%) der Titelverbindung B jeweils als farbloses Öl.

IR (Film) von A: 3600-3200, 3070, 3050, 3010, 2950, 2920, 2850, 1605, 1590, 1510, 1470, 1425, 1265, 1230, 1160, 1110, 1025, 1000, 970, 895, 825, 735 und 705 $cm^{-1}$.

IR (Film) von B: 3600-3200, 3070, 3050, 3010, 2950, 2920, 2850, 1605, 1590, 1510, 1470, 1425, 1265, 1230, 1160, 1110, 1025, 1015, 1000, 890, 830, 820, 735 und 705 $cm^{-1}$.

**Beispiel 276d**

(1S,4R,5S,6R)-4-[4-(4-Fluorphenyl)-6-tert.-butyldiphenylsilyloxymethyl-2-oxabicyclo[2.2.1]hept-5-yl]-2(E)-butensäureethylester und (1S,4R,5S,6R)-4-[4-(4-Fluorphenyl)-6-tert.-butyldiphenylsilyloxymethyl-2-oxabicyclo[2.2.1]hept-5-yl]-2(Z)-butensäureethylester:

683 mg (1,16 mmol) der in Beispiel 276e dargestellten Diole setzte man in Analogie zu Beispiel 1b um und isolierte nach Aufarbeitung und Reinigung 516 mg (901 µmol, 78%) der Titelverbindung als farbloses Öl.

IR (Film): 3070, 3050, 3010, 2950, 2930, 2860, 1715, 1650, 1605, 1590, 1510, 1465, 1425, 1390, 1365, 1315, 1265, 1230, 1160, 1110, 895, 830, 820, 740 und 705 $cm^{-1}$.

**Beispiel 276e**

(1R,2S,3R,4S)-4-[1-Hydroxymethyl-1-(4-Fluorphenyl)-3-tert.-butyldiphenylsilyloxymethyl-4-hydroxy-cyclopentan-2-yl]-2(E)-butensäureethylester und (1R,2S,3R,4S)-4-[1-Hydroxymethyl-1-(4-fluorphenyl)-3-tert.-butyldiphenylsilyloxymethyl-4-hydroxy-cyclopentan-2-yl]-2(Z)-butensäure-ethylester:

902 mg (1,34 mmol) des in Beispiel 276f dargestellten Estergemisches setzte man in Analogie zu Beispiel 1c um und isolierte nach Aufarbeitung und Reinigung 683 mg (1,16 mmol, 86%) der Titelverbindungen als farbloses Öl.

IR (Film): 3600-3200, 3070, 3050, 2950, 2930, 2850, 1710, 1645, 1605, 1585, 1510, 1465, 1425, 1265, 1235, 1160, 1110, 1045, 825, 740 und 700 $cm^{-1}$.

**Beispiel 276f**

(1R,2S,3R,4S)-4-[1-Hydroxymethyl-1-(4-fluorphenyl)-3-tert.-butyldiphenylsilyloxymethyl-4-(tetrahydropyran-2-yloxy)-cyclopentan-2-yl]-2(E)-butensäureethylester und (1R,2S,3R,4S)-4-[1-Hydroxymethyl-1-(4-fluorphenyl)-3-tert.-butyldiphenylsilyloxymethyl-4-(tetrahydropyran-2-yloxy)-cyclopentan-2-yl]-2(Z)-butensäureethylester:

969 mg (1,60 mmol) (1R,4RS,6S,7R,8S)-1-(4-Fluorphenyl)-4-hydroxy-7-tert.-butyldiphenyls ilyloxy-methyl-8-(tetrahydropyran-2-yloxy)-3-oxabicyclo[4.3.0]nonan, das man in Analogie zu den Beispielen 1f bis 1l aus enantiomerem Corey-Lakton darstellte (vgl. Beispiel 120a), löste man in 3 ml wasserfreiem Toluol, versetzte mit 1,67 g Ethoxycarbonylethyltriphenylphosphoran und erhitzte unter einer Atmosphäre aus trockenem Argon 36 Stunden auf 80°C. Nach dem Erkalten reinigte man direkt durch Chromatographie an ca. 70 ml Kieselgel unter Verwendung eines Gradientensystems aus n-Hexan und Diethylether. Isoliert wurden 902 mg (1,34 mmol, 84%) eines Gemisches der beiden Titelverbindungen als farbloses Öl.

IR (Film): 3600-3400, 3070, 3040, 2940, 2860, 1710, 1645, 1600, 1585, 1510, 1465, 1425, 1365, 1265, 1230, 1200, 1160, 1110, 1070, 1030, 870, 820, 755 und 700 $cm^{-1}$.

**Beispiel 277**

(1S,4R,5S,6R)-7-[4-(4-Fluorphenyl)-6-[2-(4-fluorphenoxy)-ethoxymethyl]-2-oxabicyclo[2.2.1]hept-5-yl]-3-oxa-5(E)-heptensäure:

20,3 mg (50 μmol) des nach Beispiel 276a dargestellten Alkohols setzte man in Analogie zu Beispiel 1 um und isolierte nach Aufarbeitung und Reinigung 9,2 mg (19 μmol, 38%) der Titelverbindung als farbloses Öl.

IR (Film): 3600-2400, 3070, 3050, 2930, 2870, 1735, 1605, 1510, 1455, 1250, 1225, 1210, 1130, 895, 830, und 745 $cm^{-1}$.

**Beispiel 278**

(1S,4R,5S,6R)-7-[4-(4-Fluorphenyl)-6-[2-(4-fluorphenoxy)-ethoxymethyl]-2-oxabicyclo[2.2.1]hept-5-yl]-3-oxa-5(E)-heptensäuremethylester:

3 mg (6 μmol) der in Beispiel 277 dargestellten Säure veresterte man in Analogie zu Beispiel 186 und isolierte nach Aufarbeitung 3 mg der Titelverbindung als farbloses Öl.

IR (Film): 3070, 3050, 2930, 2870, 1745, 1605, 1510, 1455, 1295, 1250, 1225, 1210, 1166, 1130, 1030, 970, 895, 830, und 745 $cm^{-1}$.

**Beispiel 279**

(1S,4R,5S,6R)-7-[4-(4-Fluorphenyl)-6-[2-(4-fluorphenoxy)-ethoxymethyl]-2-oxabicyclo[2.2.1]hept-5-yl]-3-oxa-5(Z)-heptensäure:

17,7 mg (44 μmol) des nach Beispiel 274a dargestellten Alkohols setzte man in Analogie zu Beispiel 1 um und isolierte nach Aufarbeitung und Reinigung 8,7 mg (18 μmol, 41%) der Titelverbindung als farbloses Öl.

IR (Film): 3700-2400, 3070, 3050, 3010, 2920, 2870, 1730, 1605, 1510, 1455, 1295, 1250, 1220, 1210, 1160, 1130, 1100, 1030, 895, 830, und 745 $cm^{-1}$.

**Beispiel 280**

(1S,4R,5S,6R)-7-[4-(4-Fluorphenyl)-6-[2-(4-fluorphenoxy)-ethoxymethyl]-2-oxabicyclo[2.2.1]hept-5-yl]-3-oxa-5(Z)-heptensäuremethylester:

3,1 mg (6 μmol) der in Beispiel 277 dargestellten Säure veresterte man in Analogie zu Beispiel 186 und isolierte nach Aufarbeitung 3 mg der Titelverbindung als farbloses Öl.

IR (Film): 3110, 3070, 3050, 3010, 2930, 2870, 1745, 1605, 1510, 1455, 1425, 1295, 1250, 1225, 1210, 1160, 1130, 1100, 1030, 895, 830 und 745 $cm^{-1}$.

**Beispiel 281**

(1S,4R,5S,6S)-7-[4-(4-Fluorphenyl)-6-[3(S)-hydroxy-1(E)-octenyl]-2-oxabicyclo[2.2.1]hept-5-yl]-3-oxa-5(E)-heptensäure:

12 mg (24 μmol) des nach Beispiel 283 dargestellten unpolaren Esters A verseifte man in Analogie zu Beispiel 2 und isolierte nach Aufarbeitung und Reinigung 9,4 mg (21 μmol, 88%) der Titelver-bindung als farbloses Öl.

IR (Film): 3600-2400, 3040, 2950, 2930, 2870, 2860, 1725, 1605, 1515, 1455, 1425, 1230 1160, 1115, 995, 970, 895, 835 und 755 $cm^{-1}$.

**Beispiel 282**

(1S,4R,5S,6S)-7-[4-(4-Fluorphenyl)-6-[3(R)-hydroxy-1(E)-octenyl]-2-oxabicyclo[2.2.1]hept-5-yl]-3-oxa-5(E)-heptensäure:

13 mg (26 μmol) des nach Beispiel 283 dargestellten polaren Esters B verseifte man in Analogie zu Beispiel 2 und isolierte nach Aufarbeitung und Reinigung 9,5 mg (21 μmol, 82%) der Titelver-bindung als farbloses Öl.

IR (Film): 3600-2400, 3040, 2950, 2930, 2860, 1725, 1610, 1515, 1425, 1235, 1160, 1115, 1000, 970, 895, 835 und 755 $cm^{-1}$.

**Beispiel 283**

(1S,4R,5S,6S)-7-[4-(4-Fluorphenyl)-6-[3(S)-hydroxy-1(E)-octenyl]-2-oxabicyclo[2.2.1]hept-5-yl]-3-oxa-5(E)-heptensäure-t-butylester (A) und (1S,4R,5S,6S)-7-[4-(4-Fluorphenyl)-6-[3(R)-hydroxy-1(E)-octenyl]-2-oxabicyclo [2.2.1]hept-5-yl]-3-oxa-5(E)-heptensäure-t-butylester (B):

26,7 mg (53 μmol) des in Beispiel 283a dargestellten ungesättigten Ketons reduzierte man in Analogie zu Beispiel 23 und isolierte nach Aufarbeitung und Reinigung 26 mg (52 μmol, 98%) eines Gemisches der beiden Titelverbindungen als farbloses Öl.

IR (Film): 3600-3200, 3040, 2970, 2950, 2930, 2860, 1745, 1605, 1510, 1365, 1230, 1160, 1130, 965, 895 und 835 $cm^{-1}$.

Die chromatographische Trennung lieferte 12 mg (24 μmol, 45%) des unpolareren Alkohols, dem man die Struktur A zuordnete, sowie 13 mg (26 μmol, 48%) des polareren Alkohols dem man die Struktur B zuordnete.

**Beispiel 283a**

(1S,4R,5S,6S)-7-[4-(4-Fluorphenyl)-6-[3-oxo-1(E)-octenyl]-2-oxabicyclo[2.2.1]hept-5-yl]-3-oxa-5(E)-heptensäure-t-butylester:

40,9 mg (101 μmol) des in Beispiel 283b dargestellten Aldehyds setzte man in Analogie zu Beispiel 23a unter Verwendung von Dimethyl-(2-oxo-heptyl)-phosphonat um und isolierte nach Aufarbeitung und Reinigung 26,7 mg (53 μmol, 53%) der Titelverbindung als farbloses Öl.

IR (Film): 3040, 2970, 2950, 2930, 2870, 1745, 1690, 1670, 1620, 1510, 1365, 1225, 1160, 1130, 995, 970, 900 und 835 $cm^{-1}$.

**Beispiel 283b**

(1S,4R,5S,6S)-7-[4-(4-Fluorphenyl)-6-formyl-2-oxabicyclo[2.2.1]hept-5-yl]-3-oxa-5(E)-hept ensäure-t-butylester:

83 mg (204 μmol) des in Beispiel 276a dargestellten Alkohols oxidierte man in Analogie zu Beispiel 38b und isolierte nach Aufarbeitung 83 mg (204 μmol, 100%) der Titelverbindung als farbloses Öl, das man ohne Reinigung weiter umsetzte.

**Beispiel 284**

(1S,4R,5S,6S)-7-[4-(4-Fluorphenyl)-6-[3(S)-hydroxy-1(E)-octenyl]-2-oxabicyclo[2.2.1]hept-5-yl]-3-oxa-5(Z)-heptensäure:

18 mg (36 μmol) des nach Beispiel 286 dargestellten unpolaren Esters A verseifte man in Analogie zu Beispiel 2 und isolierte nach Aufarbeitung und Reinigung 12 mg (27 μmol, 75%) der Titelver-bindung als farbloses Öl.

IR (Film): 3700-2400, 3070, 3050, 3010, 2950, 2930, 2870, 2860, 1730, 1610, 1515, 1455, 1425, 1300, 1230, 1160, 1120, 1000, 970, 895 und 835 $cm^{-1}$.

**Beispiel 285**

(1S,4R,5S,6S)-7-[4-(4-Fluorphenyl)-6-[3(R)-hydroxy-1(E)-octenyl]-2-oxabicyclo[2.2.1]hept-5-yl]-3-oxa-5(Z)-heptensäure:

22 mg (44 μmol) des nach Beispiel 286 dargestellten polaren Esters B verseifte man in Analogie zu Beispiel 2 und isolierte nach Aufarbeitung und Reinigung 15 mg (34 μmol, 76%) der Titelver-bindung als farbloses Öl.

IR (Film): 3600-2400, 3070, 3040, 3020, 2950, 2930, 2870, 2860, 1730, 1610, 1515, 1455, 1430, 1300,

1235, 1160, 1120, 1000, 970, 895 und 835 $cm^{-1}$.

**Beispiel 286**

(1S,4R,5S,6S)-7-[4-(4-Fluorphenyl)-6-[3(S)-hydroxy-1(E)-octenyl]-2-oxabicyclo[2.2.1]hept-5-yl]-3-oxa-5(Z)-heptensäure-t-butylester (A) und (1S,4R,5S,6S)-7-[4-(4-Fluorphenyl)-6-[3(R)-hydroxy-1(E)-octenyl]-2-oxabicyclo [2.2.1]hept-5-yl]-3-oxa-5(Z)-heptensäure-t-butylester (B):

42 mg (84 µmol) des in Beispiel 286a dargestellten ungesättigten Ketons reduzierte man in Analogie zu Beispiel 23 und isolierte nach Aufarbeitung und Reinigung 42 mg (84 µmol, 100%) eines Gemisches der beiden Titelverbindungen als farbloses Öl.

IR (Film): 3600-3200, 3050, 2970, 2950, 2930, 2860, 1745, 1605, 1510, 1455, 1365, 1230, 1160, 1125, 995, 970, 940, 895 und 835 $cm^{-1}$.

Die chromatographische Trennung lieferte 18 mg (36 µmol, 43%) des unpolareren Alkohols, dem man die Struktur A zuordnete, sowie 22 mg (44 µmol, 52%) des polareren Alkohols dem man die Struktur B zuordnete.

**Beispiel 286a**

(1S,4R,5S,6S)-7-[4-(4-Fluorphenyl)-6-[3-oxo-1(E)-octenyl]-2-oxabicyclo[2.2.1]hept-5-yl]-3-oxa-5(Z)-heptensäure-t-butylester:

48,6 mg (120 µmol) des in Beispiel 286b dargestellten Aldehyds setzte man in Analogie zu Beispiel 23a unter Verwendung von Dimethyl-(2-oxo-heptyl)-phosphonat um und isolierte nach Aufarbeitung und Reinigung 42 mg (84 µmol, 70%) der Titelverbindung als farbloses Öl.

IR (Film): 3050, 2970, 2950, 2930, 2870, 1745, 1690, 1670, 1625, 1515, 1450, 1370, 1230, 1160, 1130, 1040, 995, 975, 940, 900 und 835 $cm^{-1}$.

**Beispiel 286b**

(1S,4R,5S,6S)-7-[4-(4-Fluorphenyl)-6-formyl-2-oxabicyclo[2.2.1]hept-5-yl]-3-oxa-5(Z)-heptensäure-t-butylester:

186 mg (458 µmol) des in Beispiel 274a dargestellten Alkohols oxidierte man in Analogie zu Beispiel 38b und isolierte nach Aufarbeitung 185 mg (457 µmol, 100%) der Titelverbindung als farbloses Öl, das man ohne Reinigung weiter umsetzte.

**Beispiel 287**

(1S,4R,5S(5E),6S(1E,3S,4S))-7-[4-(4-Fluorphenyl)-6-(3-hydroxy-4-methyl-non-1-en-6-inyl)-2-oxabicyclo[2.2.1]hept-5-yl]-3-oxa-5-heptensäure:

11,2 mg (21 µmol) des nach Beispiel 289 dargestellten unpolaren Esters A verseifte man in Analogie zu Beispiel 2 und isolierte nach Aufarbeitung und Reinigung 9,8 mg (21 µmol, 99%) der Titelver-bindung als farbloses Öl.

IR (Film): 3600-2400, 3040, 2970, 2930, 2880, 1730, 1605, 1515, 1455, 1430, 1230, 1160, 1115, 995, 970, 895, 835 und 760 $cm^{-1}$.

**Beispiel 288**

(1S,4R,5S(5E),6S(1E,3R,4S))-7-[4-(4-Fluorphenyl)-6-(3-hydroxy-4-methyl-non-1-en-6-inyl)-2-oxabicyclo[2.2.1]hept-5-yl]-3-oxa-5-heptensäure:

12,8 mg (24 µmol) des nach Beispiel 289 dargestellten polaren Esters B verseifte man in Analogie zu Beispiel 2 und isolierte nach Aufarbeitung und Reinigung 11 mg (23 µmol, 97%) der Titelver-bindung als farbloses Öl.

IR (Film): 3600-2400, 3070, 3040, 2970, 2920, 2870, 1725, 1605, 1515, 1455, 1430, 1230, 1160, 1110, 995, 970, 895, 835 und 755 $cm^{-1}$.

**Beispiel 289**

(1S,4R,5S(5E),6S(1E,3S,4S))-7-[4-(4-Fluorphenyl)-6-(3-hydroxy-4-methyl-non-1-en-6-inyl)-2-oxabicyclo[2.2.1]hept-5-yl]-3-oxa-5-heptensäure-t-butylester (A) und (1S,4R,5S(5E),6S(1E,3R,4S))-7-[4-(4-Fluor-phenyl)-6-(3-hydroxy-4-methyl-non-1-en-6-inyl)-2-oxabicyclo[2.2.1]hept-5-yl]-3-oxa-5-heptensäure-t-butylester (B):

29 mg (55 µmol) des in Beispiel 289a dargestellten ungesättigten Ketons reduzierte man in Analogie

zu Beispiel 23 und isolierte nach Aufarbeitung und Reinigung 26 mg (49 µmol, 90%) eines Gemisches der beiden Titelverbindungen als farbloses Öl.

IR (Film): 3600-3200, 3050, 2970, 2930, 2870, 1745, 1605, 1510, 1450, 1370, 1230, 1160, 1130, 970, 895 und 835 $cm^{-1}$.

Die chromatographische Trennung lieferte 11,2 mg (21 µmol, 38%) des unpolareren Alkohols, dem man die Struktur A zuordnete, sowie 12,8 mg (24 µmol, 44%) des polareren Alkohols dem man die Struktur B zuordnete.

**Beispiel 289a**

(1S,4R,5S(5E),6S(1E,4S))-7-[4-(4-Fluorphenyl)-6-(3-oxo-4-methyl-non-1-en-6-inyl)-2-oxabicyclo[2.2.1]hept-5-yl]-3-oxa-5-heptensäure-t-butylester:

42,8 mg (106 µmol) des in Beispiel 283b dargestellten Aldehyds setzte man in Analogie zu Beispiel 23a unter Verwendung von Dimethyl-(2-oxo-3(S)-methyl-oct-5-inyl)-phosphonat um und isolierte nach Aufarbeitung und Reinigung 29 mg (55 µmol, 52%%) der Titelverbindung als farbloses Öl.

IR (Film): 3070, 3040, 2970, 2930, 2870, 1745, 1690, 1665, 1620, 1510, 1450, 1365, 1225, 1160, 1130, 1040, 995, 970, 940, 900 und 835 $cm^{-1}$.

**Beispiel 290**

(1S,4R,5S(5Z),6S(1E,3S,4S))-7-[4-(4-Fluorphenyl)-6-(3-hydroxy-4-methyl-non-1-en-6-inyl)-2-oxabicyclo[2.2.1]hept-5-yl]-3-oxa-5-heptensäure:

15 mg (28 µmol) des nach Beispiel 292 dargestellten unpolaren Esters A verseifte man in Analogie zu Beispiel 2 und isolierte nach Aufarbeitung und Reinigung 13,2 mg (28 µmol, 100%) der Titelver-bindung als farbloses Öl.

IR (Film): 3600-2400, 3070, 3050, 3010, 2970, 2930, 2920, 2880, 2240, 1730, 1605, 1515, 1455, 1430, 1410, 1230, 1160, 1115, 995, 970, 940, 910, 895, 830 und 730 $cm^{-1}$.

**Beispiel 291**

(1S,4R,5S(5Z),6S(1E,3R,4S))-7-[4-(4-Fluorphenyl)-6-(3-hydroxy-4-methyl-non-1-en-6-inyl)-2-oxabicyclo[2.2.1]hept-5-yl]-3-oxa-5-heptensäure:

23 mg (44 µmol) des nach Beispiel 292 dargestellten polaren Esters B verseifte man in Analogie zu Beispiel 2 und isolierte nach Aufarbeitung und Reinigung 16,4 mg (35 µmol, 80%) der Titelver-bindung als farbloses Öl.

IR (Film): 3600-2400, 3070, 3040, 3010, 2970, 2930, 2920, 2880, 2240, 1730, 1605, 1510, 1455, 1430, 1230, 1160, 1115, 995, 970, 940, 910, 895, 835 und 735 $cm^{-1}$.

**Beispiel 292**

(1S,4R,5S(5Z),6S(1E,3S,4S))-7-[4-(4-Fluorphenyl)-6-(3-hydroxy-4-methyl-non-1-en-6-inyl)-2-oxabicyclo[2.2.1]hept-5-yl]-3-oxa-5-heptensäure-t-butylester (A) und (1S,4R,5S(5Z),6S(1E,3R,4S))-7-[4-(4-Fluor-phenyl)-6-(3-hydroxy-4-methyl-non-1-en-6-inyl)-2-oxab icyclo[2.2.1]hept-5-yl]-3-oxa-5-heptensäuret-t-butylester (B):

45 mg (86 µmol) des in Beispiel 292a dargestellten ungesättigten Ketons reduzierte man in Analogie zu Beispiel 23 und isolierte nach Aufarbeitung und Reinigung 41 mg (78 µmol, 91%) eines Gemisches der beiden Titelverbindungen als farbloses Öl.

IR (Film): 3600-3200, 3050, 2970, 2930, 2870, 1745, 1605, 1510, 1450, 1370, 1230, 1160, 1125, 970, 940, 895 und 835 $cm^{-1}$.

Die chromatographische Trennung lieferte 15 mg (28 µmol, 33%) des unpolareren Alkohols, dem man die Struktur A zuordnete, sowie 23 mg (44 µmol, 51%) des polareren Alkohols dem man die Struktur B zuordnete.

**Beispiel 292a**

(1S,4R,5S(5Z),6S(1E,4S))-7-[4-(4-Fluorphenyl)-6-(3-oxo-4-methyl-non-1-en-6-inyl)-2-oxabicyclo[2.2.1]hept-5-yl]-3-oxa-5-heptensäure-t-butylester:

43,7 mg (108 µmol) des in Beispiel 286b dargestellten Aldehyds setzte man in Analogie zu Beispiel 23a unter Verwendung von Dimethyl-(2-oxo-3(S)-methyl-oct-5-inyl)-phosphonat um und isolierte nach Aufarbeitung und Reinigung 45 mg (86 µmol, 79%) der Titelverbindung als farbloses Öl.

IR (Film): 3070, 3050, 3010, 2970, 2930, 2870, 1745, 1690, 1665, 1620, 1510, 1455, 1365, 1225, 1160, 1130, 1040, 995, 975, 945, 900 und 835 $cm^{-1}$.

**Beispiel 293**

(1S,4R,5S(5Z),6R)-7-[4-(4-Phenylphenyl)-6-[1-butylaminocarbonyloxymethyl]-2-oxabicyclo[2.2.1]hept-5-yl]-5-heptensäuremethylester:

Die Lösung von 20 mg (48 µmol) des in Beispiel 293a dargestellten Alkohols in 400 µl wasserfreiem Dimethylformamid versetzte man mit 6 mg Kupfer(I)chlorid, 10 µl n-Butylisocyanat und rührte 2 Stunden bei 25°C unter einer Atmosphäre aus trockenem Argon. Man verdünnte mit Diethylether, wusch mit Wasser, trocknete über Magnesiumsulfat und reinigte den nach Filtration und Lösungs-mittelabzug erhaltenen Rückstand durch Chromatographie an zwei analytischen Dünnschichtplatten. Als Laufmittel diente ein Gemisch aus n-Hexan und Ethylacetat, als Elutionsmittel Diethylether. Isoliert wurden 16 mg 31 µmol, 65%) der Titelverbindung als farbloses Öl.

IR (Film): 3340, 3060, 3030, 2950, 2930, 2870, 1720, 1600, 1505, 1485, 1245, 1145, 1115, 1005, 970, 895, 830, 765, 730 und 695 $cm^{-1}$.

**Beispiel 293a**

(1S,4R,5S(5Z),6R)-7-[4-(4-Phenylphenyl)-6-hydroxymethyl-2-oxabicyclo[2.2.1]hept-5-yl]-5-hepten-säuremethylester:

13,7 g (49,6 mmol) enantiomeres Corey-Lakton setzte man in Analogie zu den Beispielen 17a bis 17g und 1h bis 1l um und isolierte 1,44 g (3,41 mmol, 6,9%) der Titelverbindung als farbloses Öl.

IR (Film): 3600-2400, 3080, 3060, 3030, 3000, 2930, 2870, 1730, 1710, 1600, 1505, 1455, 1295, 1250, 1210, 1130, 1095, 895, 830, 765, 750, 730 und 700 $cm^{-1}$.

**Beispiel 294**

(1S,4R,5S(5Z),6R)-7-[4-(4-Phenylphenyl)-6-[1-butylaminocarbonyloxymethyl]-2-oxabicyclo[2.2.1]hept-5-yl]-5-heptensäure:

16 mg (31 µmol) des nach Beispiel 293 dargestellten Esters verseifte man in Analogie zu Beispiel 2 und isolierte nach Aufarbeitung und Reinigung 13,7 mg (27 µmol, 88%) der Titelverbindung als farbloses Öl.

IR (Film): 3600-2400, 3330, 3080, 3050, 3030, 2950, 2930, 2870, 1710, 1600, 1525, 1505, 1490, 1455, 1250, 1225, 1145, 1035, 1020, 1010, 970, 895, 835, 765, 730 und 700 $cm^{-1}$.

**Beispiel 295**

(1S,4R,5S(5Z),6R)-7-[4-(4-Phenylphenyl)-6-[2-(4-fluorphenoxy)-ethoxymethyl]-2-oxabicyclo[2.2.1]hept-5-yl]-5-heptensäuremethyl(bzw.2-(4-fluorphenoxy)-ethyl)ester:

20,6 mg (49 µmol) des in Beispiel 293a dargestellten Alkohols setzte man in Analogie zu Beispiel 1 mit 4-Fluorphenoxy-2-ethylbromid um und isolierte nach Aufarbeitung und Reinigung 15 mg der Titelverbindungen als farbloses Öl.

IR (Film): 3080, 3050, 3030, 3000, 2930, 2870, 1745, 1600, 1510, 1490, 1455, 1295, 1250, 1215, 1125, 1095, 1010, 895, 830, 765, 745, 730 und 700 $cm^{-1}$.

**Beispiel 296**

(1S,4R,5S(5Z),6R)-7-[4-(4-Phenylphenyl)-6-[2-(4-fluorphenoxy)-ethoxymethyl]-2-oxabicyclo[2.2.1]hept-5-yl]-5-heptensäure:

15 mg des nach Beispiel 295 dargestellten Estergemisches verseifte man in Analogie zu Beispiel 2 und isolierte nach Aufarbeitung und Reinigung 10,7 mg (19,6 µmol, 22%, bezogen auf Startmaterial in Beispiel 295) der Titelverbindung als farbloses Öl.

IR (Film): 3600-2500, 3080, 3060, 3030, 3000, 2930, 2870, 1730, 1710, 1600, 1505, 1490, 1455, 1295, 1250, 1210, 1130, 1095, 1010, 895, 830, 765, 750, 730 und 700 $cm^{-1}$.

**Beispiel 297**

(1S,4R,5S(5Z),6R)-7-[4-(4-Phenylphenyl)-6-(benzyloxymethyl)-2-oxabicyclo[2.2.1]hept-5-yl]-5-heptensäuremethyl(bzw.benzyl)ester:

19,4 mg (46 μmol) des in Beispiel 293a dargestellten Alkohols setzt man in Analogie zu Beispiel 1 mit Benzylchlorid um und isolierte nach Aufarbeitung und Reinigung 24 mg der Titelverbindungen als farbloses Öl.

IR (Film): 3090, 3060, 3030, 3010, 2930, 2870, 1745, 1600, 1490, 1450, 1405, 1365, 1300, 1240, 1210, 1115, 1095, 1075, 1010, 895, 835, 765, 730 und 695 $cm^{-1}$.

**Beispiel 298**

(1S,4R,5S(5Z),6R)-7-[4-(4-Phenylphenyl)-6-(benzyloxymethyl)-2-oxabicyclo[2.2.1]hept-5-yl]-5-heptensäure:

24 mg des nach Beispiel 297 dargestellten Estergemisches verseifte man in Analogie zu Beispiel 2 und isolierte nach Aufarbeitung und Reinigung 20,4 mg (41 μmol, 89%, bezogen auf Startmaterial in Beispiel 297) der Titelverbindung als farbloses Öl.

IR (Film): 3600-2400, 3060, 3030, 3000, 2930, 2870, 1730, 1705, 1600, 1490, 1450, 1405, 1365, 1300, 1240, 1205, 1120, 1095, 1070, 1010, 895, 835, 765, 730 und 695 $cm^{-1}$.

**Beispiel 299**

(1S,4R,5S(5Z),6R)-7-[4-(4-Phenylphenyl)-6-(4-cyanobenzyloxymethyl)-2-oxabicyclo[2.2.1]hept-5-yl]-5-heptensäuremethyl(bzw.4-cyanobenzyl)ester:

19,6 mg (47 μmol) des in Beispiel 293a dargestellten Alkohols setzte man in Analogie zu Beispiel 1 mit 4-Cyanobenzylbromid um und isolierte nach Aufarbeitung und Reinigung 16,5 mg der Titelver-bindungen als farbloses Öl.

IR (Film): 3080, 3060, 3030, 3000, 2930, 2870, 2230, 1745, 1610, 1600, 1490, 1450, 1405, 1365, 1240, 1210, 1125, 1110, 1095, 1020, 1010, 970, 895, 835, 820, 765, 730 und 695 $cm^{-1}$.

**Beispiel 300**

(1S,4R,5S(5Z),6R)-7-[4-(4-Phenylphenyl)-6-(4-cyanobenzyloxymethyl)-2-oxabicyclo[2.2.1]hept-5-yl]-5-heptensäure:

16,5 mg des nach Beispiel 299 dargestellten Estergemisches verseifte man in Analogie zu Beispiel 2 und isolierte nach Aufarbeitung und Reinigung 14,7 mg (28 μmol, 61%, bezogen auf Startmaterial in Beispiel 299) der Titelverbindung als farbloses Öl.

IR (Film): 3600-2400, 3060, 3030, 3000, 2930, 2870, 2230, 1725, 1705, 1610, 1600, 1490, 1450, 1405, 1365, 1240, 1210, 1125, 1110, 1095, 1020, 1010, 970, 895, 835, 820, 765, 730 und 700 $cm^{-1}$.

**Beispiel 301**

(1S,4R,5S(5Z),6R)-7-[4-(4-Phenylphenyl)-6-(3-nitrobenzyloxymethyl)-2-oxabicyclo[2.2.1]hept-5-yl]-5-heptensäuremethyl(bzw.3-nitrobenzyl)ester:

19,4 mg (46 μmol) des in Beispiel 293a dargestellten Alkohols setzte man in Analogie zu Beispiel 1 mit 3-Nitrobenzylchlorid um und isolierte nach Aufarbeitung und Reinigung 21 mg der Titelver-bindungen als farbloses Öl.

IR (Film): 3080, 3060, 3030, 3010, 2930, 2870, 1740, 1600, 1580, 1530, 1490, 1450, 1350, 1240, 1210, 1125, 1095, 1010, 895, 835, 805, 765, 730 und 700 $cm^{-1}$.

**Beispiel 302**

(1S,4R,5S(5Z),6R)-7-[4-(4-Phenylphenyl)-6-(3-nitrobenzyloxymethyl)-2-oxabicyclo[2.2.1]hept-5-yl]-5-heptensaure:

21 mg des nach Beispiel 301 dargestellten Estergemisches verseifte man in Analogie zu Beispiel 2 und isolierte nach Aufarbeitung und Reinigung 15,2 mg (28 μmol, 61%, bezogen auf Startmaterial in Beispiel 301) der Titelverbindung als farbloses Öl.

IR (Film): 3600-2400, 3060, 3030, 3000, 2930, 2870, 1730, 1710, 1600, 1580, 1530, 1490, 1450, 1350, 1240, 1210, 1125, 1095, 1010, 895, 835, 810, 765, 730 und 700 $cm^{-1}$.

**Beispiel 303**

(1S,4R,5S(5Z),6R)-7-[4-(4-Phenylphenyl)-6-(4-fluorbenzyloxymethyl)-2-oxabicyclo[2.2.1]hept-5-yl]-5-heptensäuremethyl(bzw.4-fluorbenzyl)ester:

19,5 mg (46 µmol) des in Beispiel 293a dargestellten Alkohols setzte man in Analogie zu Beispiel 1 mit 4-Fluorbenzylchlorid um und isolierte nach Aufarbeitung und Reinigung 26 mg der Titelver-bindungen als farbloses Öl.

IR (Film): 3070, 3050, 3030, 3010, 2930, 2870, 1745, 1600, 1510, 1490, 1445, 1405, 1295, 1220, 1155, 1115, 1090, 1015, 1010, 970, 895, 835, 765, 730 und 700 $cm^{-1}$.

**Beispeil 304**

(1S,4R,5S(5Z),6R)-7-[4-(4-Phenylphenyl)-6-(4-fluorbenzyloxymethyl)-2-oxabicyclo[2.2.1]hept-5-yl]-5-heptensäure:

26 mg des nach Beispiel 303 dargestellten Estergemisches verseifte man in Analogie zu Beispiel 2 und isolierte nach Aufarbeitung und Reinigung 20,7 mg (40 µmol, 87%, bezogen auf Startmaterial in Beispiel 303) der Titelverbindung als farbloses Öl.

IR (Film): 3600-2400, 3050, 3030, 3010, 2930, 2870, 1730, 1705, 1600, 1510, 1490, 1450, 1405, 1295, 1225, 1155, 1115, 1090, 1010, 970, 895, 830, 765, 730 und 700 $cm^{-1}$.

**Beispiel 305**

(1S,4R,5S(5Z),6R)-7-[4-(4-Phenylphenyl)-6-[phenylaminocarbonyloxymethyl)-2-oxabicyclo[2.2.1]hept-5-yl]-5-heptensäuremethylester:

19,6 mg des in Beispiel 293a dargestellten Alkohols setzte man in Analogie zu Beispiel 293 mit Phenylisocyanat um und isolierte nach Aufarbeitung und Reinigung 24 mg (45 µmol, 95%) der Titelverbindung als farbloses Öl.

IR (Film): 3400, 3080, 3050, 3030, 2950, 2930, 2880, 1740, 1600, 1540, 1500, 1485, 1445, 1405, 1315, 1220, 1055, 1030, 895, 835, 765, 735 und 695 $cm^{-1}$.

**Beispiel 306**

(1S,4R,5S(5Z),6R)-7-[4-(4-Phenylphenyl)-6-[phenylaminocarbonyloxymethyl)-2-oxabicyclo[2.2.1]hept-5-yl]-5-heptensäure:

24 mg (45 µmol) des nach Beispiel 305 dargestellten Esters verseifte man in Analogie zu Beispiel 2 und isolierte nach Aufarbeitung und Reinigung 17,7 mg (34 µmol, 72%) der Titelverbindung als farbloses Öl.

IR (Film): 3600-2400, 3050, 3030, 3010, 2930, 2870, 1730, 1705, 1600, 1510, 1490, 1450, 1405, 1295, 1225, 1155, 1115, 1090, 1010, 970, 895, 830, 765, 730 und 700 $cm^{-1}$.

**Beispiel 307**

(1S,4R,5S(5Z),6S)-7-[4-(4-Phenylphenyl)-6-[(1E,3S)-3-hydroxy-3-cyclohexyl-prop-1-enyl]-2-oxa-bicyclo[2.2.1]hept-5-yl]-5-heptensäuremethylester (A) und (1S,4R,5S(5Z),6S)-7-[4-(4-Phenylphenyl)-6-[(1E,3R)-3-hydroxy-3-cyclohexyl-prop-1-enyl]-2-oxabicyclo[2.2.1]hept-5-yl]-5-heptensäuremethylester (B):

42,5 mg (81 µmol) des in Beispiel 307a dargestellten ungesättigten Ketons reduzierte man in Analogie zu Beispiel 23 und isolierte nach Aufarbeitung und Reinigung 34 mg (64 µmol, 79%) eines Gemisches der beiden Titelverbindungen als farbloses Öl.

IR (Film): 3600-3200, 3050, 3030, 2920, 2850, 1735, 1600, 1485, 1455, 1245, 1195, 1005, 995, 970, 895, 835, 765, 730 und 695 $cm^{-1}$.

Die chromatographische Trennung lieferte 17,5 mg (33 µmol, 41%) des unpolareren Alkohols, dem man die Struktur A zuordnete, sowie 12,6 mg (24 µmol, 30%) des polareren Alkohols dem man die Struktur B zuordnete.

**Beispiel 307a**

(1S,4R,5S(5Z),6S)-7-[4-(4-Phenylphenyl)-6-[(1E)-3-oxo-3-cyclohexyl-prop-1-enyl]-2-oxa-bicyclo[2.2.1]hept-5-yl]-5-heptensäuremethylester:

50,4 mg (120 µmol) des in Beispiel 307b dargestellten Aldehyds setzte man in Analogie zu Beispiel 23a unter Verwendung von Dimethyl-(2-oxo-2-cyclohexyl-ethyl)-phosphonat um und isolierte nach Aufarbeitung und Reinigung 42,5 mg (81 µmol, 67%) der Titelverbindung als farbloses Öl.

IR (Film): 3050, 3030, 2920, 2850, 1735, 1685, 1600, 1485, 1455, 1245, 1150, 1000, 970, 895, 835, 765,

730 und 695 $cm^{-1}$.

**Beispiel 307b**

(1S,4R,5S(5Z),6S)-7-[4-(4-Phenylphenyl)-6-formyl-2-oxa-bicyclo[2.2.1]hept-5-yl]-5-heptensäure-methylester:

258 mg (613 μmol) des in Beispiel 293a dargestellten Alkohols oxidierte man in Analogie zu Beispiel 38b und isolierte nach Aufarbeitung 256 mg (612 μmol, 100%) der Titelverbindung als farbloses Öl, das man ohne Reinigung weiter umsetzte.

**Beispiel 308**

(1S,4R,5S(5Z),6S)-7-[4-(4-Phenylphenyl)-6-[(1E,3S)-3-hydroxy-3-cyclohexyl-prop-1-enyl]-2-oxa-bicyclo[2.2.1]hept-5-yl]-5-heptensäure:

17,5 mg (33 μmol) des nach Beispiel 307 dargestellten unpolaren Esters A verseifte man in Analogie zu Beispiel 2 und isolierte nach Aufarbeitung und Reinigung 17 mg (33 μmol, 100%) der Titelver-bindung als farbloses Öl.

IR (Film): 3600-2400, 3080, 3060, 3030, 3000, 2930, 2850, 1710, 1600, 1490, 1450, 1405, 1295, 1240, 1195, 1005, 995, 970, 910, 890, 840, 765, 730 und 695 $cm^{-1}$.

**Beispiel 309**

(1S,4R,5S(5Z),6S)-7-[4-(4-Phenylphenyl)-6-[(1E,3R)-3-hydroxy-3-cyclohexyl-prop-1-enyl]-2-oxa-bicyclo[2.2.1]hept-5-yl]-5-heptensäure:

12,6 mg (24 μmol) des nach Beispiel 307 dargestellten polaren Esters B verseifte man in Analogie zu Beispiel 2 und isolierte nach Aufarbeitung und Reinigung 12 mg (23 μmol, 98%) der Titelver-bindung als farbloses Öl.

IR (Film): 3600-2400, 3080, 3050, 3030, 3000, 2930, 2850, 1715, 1600, 1490, 1445, 1405, 1300, 1240, 1195, 1010, 995, 970, 910, 890, 835, 765, 730 und 700 $cm^{-1}$.

**Beispiel 310:**

(1S,4R,5S(5Z),6S(1E,3S,4S))-7-[4-(4-Phenylphenyl)-6-(3-hydroxy-4-methyl-non-1-en-6-inyl)-2-oxabicyclo[2.2.1]hept-5-yl]-5-heptensäure-methylester (A) und (1S,4R,5S(5Z),6S(1E,3R,4S))-7-[4-(4-Phenylphenyl)-6-(3-hydroxy-4-methyl-non-1-en-6-inyl)-2-oxabicyclo[2.2.1]hept-5-yl]-5-heptensäuremethylester (B):

41,6 mg (77 μmol) des in Beispiel 310a dargestellten ungesättigten Ketons reduzierte man in Analogie zu Beispiel 23 und isolierte nach Aufarbeitung und Reinigung 36 mg (67 μmol, 86%) eines Gemisches der beiden Titelverbindungen als farbloses Öl.

IR (Film): 3600-3200, 3060, 3030, 2970, 2930, 2880, 1745, 1600, 1490, 1450, 1405, 1320, 1295, 1245, 1195, 1110, 995, 970, 895, 835, 765, 730 und 695 $cm^{-1}$.

Die chromatographische Trennung lieferte 15,8 mg (29 μmol, 38%) des unpolareren Alkohols, dem man die Struktur A zuordnete, sowie 17,3 mg (32 μmol, 42%) des polareren Alkohols dem man die Struktur B zuordnete.

**Beispiel 310a**

(1S,4R,5S(5Z),6S(1E,4S))-7-[4-(4-Phenylphenyl)-6-(3-oxo-4-methyl-non-1-en-6-inyl)-2-oxabicyclo[2.2.1]hept-5-yl]-5-heptensäure-methylester:

51,8 mg (124 μmol) des nach Beispiel 307b dargestellten Aldehyds setzte man in Analogie zu Beispiel 23a unter Verwendung von Dimethyl-(2-oxo-3(S)-methyl-oct-5-in)-phosphonat um und isolierte nach Aufarbeitung und Reinigung 41,6 mg (77 μmol, 62%) der Titelverbindung als farbloses Öl.

IR (Film): 3060, 3030, 2970, 2930, 2880, 1735, 1685, 1600, 1490, 1450, 1430, 1245, 1195, 995, 970, 895, 835, 765, 730 und 700 $cm^{-1}$.

**Beispiel 311**

(1S,4R,5S(5Z),6S(1E,3S,4S))-7-[4-(4-Phenylphenyl)-6-(3-hydroxy-4-methyl-non-1-en-6-inyl)-2-oxabicyclo[2.2.1]hept-5-yl]-5-heptensäure:

15,8 mg (29 μmol) des nach Beispiel 310 dargestellten unpolaren Esters A verseifte man in Analogie

zu Beispiel 2 und isolierte nach Aufarbeitung und Reinigung 13,7 mg (26 µmol, 90%) der Titelver-bindung als farbloses Öl.

IR (Film): 3700-2400, 3080, 3060, 3030, 2970, 2930, 2880, 1710, 1600, 1490, 1450, 1405, 1320, 1295, 1245, 1195, 1110, 995, 970, 895, 835, 765, 730 und 700 $cm^{-1}$.

## Beispiel 312

(1S,4R,5S(5Z),6S(1E,3R,4S))-7-[4-(4-Phenylphenyl)-6-(3-hydroxy-4-methyl-non-1-en-6-inyl)-2-oxabicyclo[2.2.1]hept-5-yl]-5-heptensäure:

17,3 mg (32 µmol) des nach Beispiel 310 dargestellten polaren Esters B verseifte man in Analogie zu Beispiel 2 und isolierte nach Aufarbeitung und Reinigung 16,6 mg (32 µmol, 99%) der Titelver-bindung als farbloses Öl.

IR (Film): 3600-2400, 3080, 3050, 3030, 2970, 2930, 2880, 1710, 1600, 1490, 1455, 1405, 1320, 1295, 1245, 1195, 1105, 995, 970, 910, 895, 835, 765, 730 und 695 $cm^{-1}$.

## Beispiel 313

(1S,4R,5S(5Z),6R(1E/Z,3S,4S))-7-[4-(4-Phenylphenyl)-6-(2-brom-3-hydroxy-4-methyl-non-1-en-6-inyl)-2-oxabicyclo[2.2.1]hept-5-yl]-5-heptensäuremethylester (A) und (1S,4R,5S(5Z),6R(1E/Z,3R,4S))-7-[4-(4-Phenylphenyl)-6-(2-brom-3-hydroxy-4-methyl-non-1-en-6-inyl)-2-oxabicyclo[2.2.1]hept-5-yl]-5-heptensäuremethylester (B):

61,3 mg (99 µmol) des in Beispiel 313a dargestellten ungesättigten Ketons reduzierte man in Analogie zu Beispiel 23 und isolierte nach Aufarbeitung und Reinigung 58,7 mg (95 µmol, 96%) eines Gemisches der beiden Titelverbindungen als farbloses Öl.

IR (Film): 3600-3200, 3060, 3030, 2970, 2930, 2870, 1730, 1600, 1485, 1450, 1435, 1315, 1265, 1035, 990, 895, 835, 765, 735 und 700 $cm^{-1}$.

Die chromatographische Trennung lieferte 24,5 mg (40 µmol, 40%) des unpolareren Alkohols, dem man die Struktur A zuordnete, sowie 33 mg (53 µmol, 54%) des polareren Alkohols dem man die Struktur B zuordnete.

## Beispiel 313a

(1S,4R,5S(5Z),6R(1E/Z,4S))-7-[4-(4-Phenylphenyl)-6-(2-brom-3-oxo-4-methyl-non-1-en-6-inyl)-2-oxabicyclo[2.2.1]hept-5-yl]-5-heptensäuremethylester:

67,5 mg (161 µmol) des nach Beispiel 307b dargestellten Aldehyds setzte man in Analogie zu Beispiel 190a unter Verwendung von Dimethyl-(2-oxo-3(S)-methyl-oct-5-in)-phosphonat um und isolierte nach Aufarbeitung und Reinigung 61,3 mg (99 µmol, 62%) der Titelverbindung als farbloses Öl.

IR (Film): 3060, 3030, 2970, 2930, 2870, 1730, 1685, 1600, 1485, 1450, 1435, 1240, 1150, 985, 900, 835, 765, 730 und 695 $cm^{-1}$.

## Beispiel 314

(1S,4R,5S(5Z),6R(1E/Z,3R,4S))-7-[4-(4-Phenylphenyl)-6-(2-brom-3-hydroxy-4-methyl-non-1-en-6-inyl)-2-oxabicyclo[2.2.1]hept-5-yl]-5-heptensäure:

6,3 mg (10 µmol) des nach Beispiel 313 dargestellten polaren Esters B verseifte man in Analogie zu Beispiel 2 und isolierte nach Aufarbeitung und Reinigung 5,5 mg (9 µmol, 88% der Titelverbindung als farbloses Öl.

IR (Film): 3600-2400, 3050, 3030, 2970, 2930, 2870, 1710, 1600, 1485, 1450, 1435, 1315, 1265, 1240, 1195, 1035, 990, 895, 835, 765, 735 und 695 $cm^{-1}$.

## Beispiel 315

(1S,4R,5S(5Z),6R(1E/Z,3S,4S))-7-[4-(4-Phenylphenyl)-6-(2-brom-3-hydroxy-4-methyl-non-1-en-6-inyl)-2-oxabicyclo[2.2.1]hept-5-yl]-5-heptensäure:

5,7 mg (9 µmol) des nach Beispiel 313 dargestellten unpolaren Esters A verseifte man in Analogie zu Beispiel 2 und isolierte nach Aufarbeitung und Reinigung 4,8 mg (8 µmol, 86%) der Titelver-bindung als farbloses Öl.

IR (Film): 3600-2400, 3050, 3030, 2970, 2930, 2870, 1715, 1600, 1485, 1450, 1435, 1315, 1265, 1240, 1195, 1035, 990, 895, 835, 765, 735 und 695 $cm^{-1}$.

**Beispiel 316**

(1S,4R,5S(5Z),6R(3R,4S))-7-[4-(4-Phenylphenyl)-6-(3-hydroxy-4-methyl-nona-1,6-diinyl)-2-oxabicyclo[2.2.1] hept-5-yl]-5-heptensäure:

26 mg (42 µmol) des nach Beispiel 313 dargestellten polaren Alkohols B setzte man in Analogie zu Beispiel 187 um und isolierte nach Aufarbeitung und Reinigung 18 mg (34 µmol, 82%) der Titel-verbindung als farbloses Öl.

IR (Film): 3600-2400, 3050, 3030, 2970, 2930, 2880, 2230, 1705, 1600, 1485, 1450, 1435, 1405, 1320, 1240, 1145, 1020, 1005, 990, 895, 835, 765, 730 und 695 $cm^{-1}$.

**Beispiel 317**

(1S,4R,5S(5Z),6R(3R,4S))-7-[4-(4-Phenylphenyl)-6-(3-hydroxy-4-methyl-nona-1,6-diinyl)-2-oxabicyclo[2.2.1] hept-5-yl]-5-heptensäuremethylester:

2,7 mg (5 µmol) der nach Beispiel 316 dargestellten Säure veresterte man in Analogie zu Beispiel 186 und isolierte nach Lösungsmittelabzug 2,7 mg (5 µmol, 97%) der Titelverbindung als farbloses Öl.

IR (Film): 3600-3200, 3050, 3030, 2970, 2930, 2870, 2230, 1745, 1600, 1485, 1445, 1435, 1405, 1320, 1240, 1145, 1020, 1005, 990, 895, 835, 765, 730 und 700 $cm^{-1}$.

**Beispiel 318**

(1S,4R,5S(5Z),6R(3S,4S))-7-[4-(4-Phenylphenyl)-6-(3-hydroxy-4-methyl-nona-1,6-diinyl)-2-oxabicyclo[2.2.1] hept-5-yl]-5-heptensäure:

18 mg (29 µmol) des nach Beispiel 313 dargestellten unpolaren Alkohols A setzte man in Analogie zu Beispiel 187 um und isolierte nach Aufarbeitung und Reinigung 13,2 mg (25 µmol, 87%) der Titelverbindung als farbloses Öl.

IR (Film): 3600-2400, 3080, 3050, 3030, 2970, 2930, 2880, 2230, 1710, 1600, 1525, 1485, 1450, 1435, 1405, 1320, 1290, 1240, 1145, 1020, 1005, 990, 940, 895, 835, 765, 730 und 695 $cm^{-1}$.

**Beispiel 319**

(1S,4R,5S(5Z),6R(3S,4S))-7-[4-(4-Phenylphenyl)-6-(3-hydroxy-4-methyl-nona-1,6-diinyl)-2-oxabicyclo[2.2.1] hept-5-yl]-5-heptensäuremethylester:

3,1 mg (6 µmol) der nach Beispiel 318 dargestellten Säure veresterte man in Analogie zu Beispiel 186 und isolierte nach Lösungsmittelabzug 3,1 mg (6 µmol, 98%) der Titelverbindung als farbloses Öl.

IR (Film): 3600-3200, 3070, 3050, 3030, 2970, 2930, 2880, 2230, 1740, 1600, 1485, 1450, 1435, 1405, 1320, 1290, 1240, 1145, 1020, 1000, 940, 895, 835, 765, 730 und 695 $cm^{-1}$.

**Beispiel 320**

(1S,4R,5S(5Z),6S)-7-[4-(4-Phenylphenyl)-6-(3(S)-hydroxy-1(E)-octenyl]-2-oxabicyclo[2.2.1]hept-5-yl]-5-heptensäuremethylester (A) und (1S,4R,5S(5Z),6S)-7-[4-(4-Phenylphenyl)-6-[3(R)-hydroxy-1(E)-octenyl]-2-oxabicyclo[2.2.1]hept-5-yl]-5-heptensäuremethylester (B):

41,6 mg (81 µmol) des in Beispiel 320a dargestellten ungesättigten Ketons reduzierte man in Analogie zu Beispiel 23 und isolierte nach Aufarbeitung und Reinigung 35,3 mg (64 µmol, 85%) eines Gemisches der beiden Titelverbindungen als farbloses Öl.

IR (Film): 3600-3200, 3060, 3030, 2950, 2930, 2860, 1735, 1600, 1485, 1450, 1435, 1245, 1195, 995, 970, 895, 835, 765, 730 und 695 $cm^{-1}$.

Die chromatographische Trennung lieferte 18,7 mg (36 µmol, 45%) des unpolareren Alkohols, dem man die Struktur A zuordnete, sowie 14,3 mg (28 µmol, 34%) des polareren Alkohols dem man die Struktur B zuordnete.

**Beispiel 320a**

(1S,4R,5S(5Z),6S)-7-[4-(4-Phenylphenyl)-6-[3-oxo-1(E)-octenyl]-2-oxabicyclo[2.2.1]hept-5-yl]-5-heptensäuremethylester:

49,8 mg (118 µmol) des in Beispiel 307b dargestellten Aldehyds setzte man in Analogie zu Beispiel 23a unter Verwendung von Dimethyl-(2-oxo-heptyl)-phosphonat um und isolierte nach Aufarbeitung und Reinigung

41,4 mg (80 µmol, 68%) der Titelverbindung als farbloses Öl.

IR (Film): 3060, 3030, 2950, 2930, 2860, 1740, 1690, 1600, 1485, 1440, 1245, 1195, 1005, 995, 970, 895, 835, 765, 730 und 695 $cm^{-1}$.

**Beispiel 321**

(1S,4R,5S(5Z),6S)-7-[4-(4-Phenylphenyl)-6-[3(R)-hydroxy-1(E)-octenyl]-2-oxabicyclo[2.2.1]hept-5-yl]-5-heptensäure:

14,3 mg (28 µmol) des nach Beispiel 320 dargestellten polaren Esters B verseifte man in Analogie zu Beispiel 2 und isolierte nach Aufarbeitung und Reinigung 13,8 mg (27 µmol, 96%) der Titelver-bindung als farbloses Öl.

IR (Film): 3600-2400, 3080 3060, 3030, 3010, 2950, 2930, 2870, 2860, 1710, 1600, 1490, 1455, 1405, 1295, 1240, 1195, 1045, 1010, 995, 970, 910, 835, 765 und 700 $cm^{-1}$.

**Beispiel 322**

(1S,4R,5S(5Z),6S)-7-[4-(4-Phenylphenyl)-6-[3(S)-hydroxy-1(E)-octenyl]-2-oxabicyclo[2.2.1]hept-5-yl]-5-heptensäure:

18,7 mg (36 µmol) des nach Beispiel 320 dargestellten unpolaren Esters A verseifte man in Analogie zu Beispiel 2 und isolierte nach Aufarbeitung und Reinigung 18,1 mg (36 µmol, 100%) der Titelver-bindung als farbloses Öl.

IR (Film): 3600-2400, 3060, 3030, 2950, 2930, 2870, 1710, 1600, 1490, 1450, 1405, 1295, 1240, 1195, 1010, 995, 970, 910, 835, 765 und 700 $cm^{-1}$.

**Beispiel 323**

(1S,4R,5S(5Z),6R)-7-[4-(4-Phenylphenyl)-6-(4-fluorbenzyloxyiminomethyl)-2-oxabicyclo[2.2.1]hept-5-yl]-5-heptensäuremethylester:

19,9 mg (48 µmol) des in Beispiel 307b dargestellten Aldehyds setzte man in Analogie zu Beispiel 26 unter Verwendung von p-Fluorphenylmethoxyamin um und isolierte nach Aufarbeitung und Reinigung 17 mg (31 µmol, 66%) der Titelverbindung als farbloses Öl.

IR (Film): 3060, 3030, 2940, 2870, 1730, 1600, 1510, 1485, 1220, 1155, 1045, 1005, 995, 900, 835, 765, 730 und 595 $cm^{-1}$.

**Beispiel 324**

(1S,4R,5S(5Z),6R)-7-[4-(4-Phenylphenyl)-6-(4-fluorbenzyloxyiminomethyl)-2-oxabicyclo[2.2.1]hept-5-yl]-5-heptensäure:

17 mg (31 µmol) des nach Beispiel 323 dargestellten Esters verseifte man in Analogie zu Beispiel 2 und isolierte nach Aufarbeitung und Reinigung 16 mg (30 µmol, 98%) der Titelverbindung als farb-loses Öl.

IR (Film): 3600-2500, 3060, 3030, 3010, 2930, 2880, 1730, 1705, 1600, 1510, 1490, 1445, 1405, 1295, 1220, 1155, 1045, 1005, 995, 895, 835, 765, 730 und 700 $cm^{-1}$.

**Beispiel 325**

(1S,4R,5S(5Z),6R)-7-[4-(4-Phenylphenyl)-6-(cyclohexylmethoxyiminomethyl)-2-oxabicyclo[2.2.1]hept-5-yl]-5-heptensäuremethylester:

18,3 mg (44 µmol) des in Beispiel 307b dargestellten Aldehyds setzte man in Analogie zu Beispiel 26 unter Verwendung von Cyclohexylmethoxyamin um und isolierte nach Aufarbeitung und Reini-gung 18 mg (34 µmol, 78%) der Titelverbindung als farbloses Öl.

IR (Film): 3060, 3030, 3000, 2920, 2850, 1735, 1600, 1485, 1445, 1240, 1190, 1160, 1035, 1000, 900, 835, 765, 730 und 695 $cm^{-1}$.

**Beispiel 326**

(1S,4R,5S(5Z),6R)-7-[4-(4-Phenylphenyl)-6-(cyclohexylmethoxyiminomethyl)-2-oxabicyclo[2.2.1]hept-5-yl]-5-heptensäure:

18 mg (34 µmol) des nach Beispiel 325 dargestellten Esters verseifte man in Analogie zu Beispiel 2 und

isolierte nach Aufarbeitung und Reinigung 17 mg (33 μmol, 97%) der Titelverbindung als farb-loses Öl.
IR (Film): 3600-2400, 3080, 3050, 3030, 3000, 2930, 2850, 1730, 1705, 1600, 1490, 1450, 1405, 1295, 1240, 1190, 1080, 1040, 1025, 1010, 995, 900, 835, 765, 730 und 700 $cm^{-1}$.

**Beispiel 327**

(1S,4R,5S.6R)-7-[4-(4-Fluorphenyl)-6-(4-fluorbenzyloxymethyl)-2-oxabicyclo[2.2.1]hept-5-yl]-3-oxa-heptensäure:
19,5 mg (48 μmol) des in Beispiel 327a dargestellten Alkohols setzte man in Analogie zu Beispiel 1 um und isolierte nach Aufarbeitung und Reinigung 17,5 mg (38 μmol, 80%) der Titelverbindung als farbloses Öl.
IR (Film): 3600-2400, 3070, 3040, 2930, 2860, 1725, 1605, 1510, 1425, 1365, 1225, 1160, 1115, 1025, 900 und 830 $cm^{-1}$.

**Beispiel 327a**

(1S,4R,5S.6R)-7-[4-(4-Fluorphenyl)-6-hydroxymethyl-2-oxabicyclo[2.2.1]hept-5-yl]-3-oxa-heptan-säure-t-butylester:
84 mg (207 μmol) des in Beispiel 276a dargestellten Alkohols löste man in 3 ml Ethylacetat, versetzte mit 30 mg Palladium auf Kohle (10%ig) und hydrierte unter Normaldruck, bis eine deutlich verlangsamte Wasserstoffaufnahme erfolgte. Man filtrierte, engte im Wasserstrahlvakuum ein und reinigte den erhaltenen Rückstand durch Chromatographie an 4 analytischen Dünnschichtplatten. Als Laufmittel diente ein Gemisch aus Trichlormethan und Aceton, als Elutionsmittel ein Gemisch aus Ethanol und Ethylacetat. Isoliert wurden 49 mg (120 μmol, 58%) der Titelverbindung als farbloses Öl.
IR (Film): 3700-3200, 3050, 2980, 2930, 2860, 1740, 1605, 1510, 1365, 1230, 1160, 1135, 1020, 895, 830, 815 und 735 $cm^{-1}$.

**Beispiel 328**

(1S,4R,5S.6R)-7-[4-(4-Fluorphenyl)-6-(4-fluorbenzyloxymethyl)-2-oxabicyclo[2.2.1]hept-5-yl]-3-oxa-heptansäuremethylester:
2,9 mg (6 μmol) der nach Beispiel 327 dargestellten Säure veresterte man in Analogie zu Beispiel 186 und isolierte nach Lösungsmittelabzug 3 mg (6 μmol, 100%) der Titelverbindung als farbloses Öl.
IR (Film): 3070, 3050, 2930, 2860, 1745, 1605, 1515, 1425, 1365, 1225, 1160, 1120, 1100, 1025, 895 und 830 $cm^{-1}$.

**Beispiel 329**

(1S,4R,5S.6R)-7-[4-(4-Fluorphenyl)-6-(4-cyanobenzyloxymethyl)-2-oxabicyclo[2.2.1]hept-5-yl]-3-oxa-heptansäure:
19 mg (47 μmol) des in Beispiel 327a dargestellten Alkohols setzte man in Analogie zu Beispiel 1 um und isolierte nach Aufarbeitung und Reinigung 12,1 mg (26 μmol, 56%) der Titelverbindung als farbloses Öl.
IR (Film): 3600-2400, 3070, 3050, 2930, 2860, 2215, 1725, 1610, 1515, 1415, 1230, 1160, 1125, 1100, 1020, 895, 830 und 815 $cm^{-1}$.

**Beispiel 330**

(1S,4R,5S.6R)-7-[4-(4-Fluorphenyl)-6-(4-cyanobenzyloxymethyl)-2-oxabicyclo[2.2.1]hept-5-yl]-3-oxa-heptansäuremethylester:
3,4 mg (7 μmol) der nach Beispiel 329 dargestellten Säure veresterte man in Analogie zu Beispiel 186 und isolierte nach Lösungsmittelabzug 3,3 mg (7 μmol, 94%) der Titelverbindung als farbloses Öl.
IR (Film): 3070, 3050, 2930, 2860, 2215, 1740, 1610, 1515, 1415, 1230, 1160, 1125, 1110, 1100, 1020, 895, 830 und 820 $cm^{-1}$.

**Beispiel 331**

(1S,4R,5S(5Z),6R(1E/Z,3S,4S))-7-[4-(4-Fluorphenyl)-6-(2-brom-3-hydroxy-4-methyl-non-1-en-6-inyl)-2-oxabicyclo[2.2.1]hept-5-yl]-3-oxa-5-heptensäure-t-butylester (A) und (1S,4R,5S(5Z),6R-(1E/Z,3R,4S))-7-[4-(4-Fluorphenyl)-6-(2-brom-3-hydroxy-4-methyl-non-1-en-6-inyl)-2-oxabicyclo[2.2.1]hept-5-yl]-3-oxa-5-heptensäure-t-butylester (B):

92 mg (151 µmol) des in Beispiel 331a dargestellten ungesättigten Ketons reduzierte man in Analogie zu Beispiel 23 und isolierte nach Aufarbeitung und Reinigung 77 mg (126 µmol, 84%) eines Gemisches der beiden Titelverbindungen als farbloses Öl.

IR (Film): 3600-3200, 3050, 2970, 2930, 2870, 1745, 1605, 1515, 1450, 1370, 1230, 1160, 1125, 1040, 995, 895, 835 und 735 $cm^{-1}$.

Die chromatographische Trennung lieferte 29 mg (48 µmol, 32%) des unpolareren Alkohols, dem man die Struktur A zuordnete, sowie 46 mg (77 µmol, 50%) des polareren Alkohols dem man die Struktur B zuordnete.

**Beispiel 331a**

(1S,4R,5S(5Z),6R(1E/Z,4S))-7-[4-(4-Fluorphenyl)-6-(2-brom-3-oxo-4-methyl-non-1-en-6-in yl)-2-oxabicyclo-[2.2.1]hept-5-yl]-3-oxa-5-heptensäure-t-butylester:

88 mg (218 µmol) des in Beispiel 286b dargestellten Aldehyds setzte man in Analogie zu Beispiel 190a unter Verwendung von Dimethyl-(2-oxo-3(S)-methyl-oct-5-inyl)-phosphonat um und isolierte nach Aufarbeitung und Reinigung 92 mg (151 µmol, 70%) der Titelverbindung als farbloses Öl.

IR (Film): 3050, 2970, 2930, 2870, 1745, 1690, 1600, 1510, 1455, 1365, 1230, 1160, 1130, 1040, 990, 940, 905, 835 und 735 $cm^{-1}$.

**Beispiel 332**

(1S,4R,5S(5Z),6R(1E/Z,3R,4S))-7-[4-(4-Fluorphenyl)-6-(2-brom-3-hydroxy-4-methyl-non-1-en-6-inyl)-2-oxa-bicyclo[2.2.1]hept-5-yl]-3-oxa-5-heptensäure:

8,3 mg (14 µmol) des nach Beispiel 331 dargestellten polaren Esters B verseifte man in Analogie zu Beispiel 2 und isolierte nach Aufarbeitung und Reinigung 6,5 mg (12 µmol, 85%) der Titelver-bindung als farbloses Öl.

IR (Film): 3600-2400, 3050, 2970, 2930, 2870, 1710, 1605, 1510, 1455, 1365, 1230, 1160, 1125, 1040, 995, 940, 895, 835 und 735 $cm^{-1}$.

**Beispiel 333**

(1S,4R,5S(5Z),6R(1E/Z,3S,4S))-7-[4-(4-Fluorphenyl)-6-(2-brom-3-hydroxy-4-methyl-non-1-en-6-inyl)-2-oxa-bicyclo[2.2.1]hept-5-yl]-3-oxa-5-heptensäure:

5,1 mg (8 µmol) des nach Beispiel 331 dargestellten unpolaren Esters A verseifte man in Analogie zu Beispiel 2 und isolierte nach Aufarbeitung und Reinigung 4,2 mg (8 µmol, 96%) der Titelver-bindung als farbloses Öl.

IR (Film): 3600-2400, 3050, 2970, 2930, 2870, 1710, 1605, 1510, 1455, 1370, 1230, 1160, 1125, 1035, 995, 940, 895, 835 und 735 $cm^{-1}$.

**Beispiel 334**

(1S,4R,5S(5Z),6R(3R,4S))-7-[4-(4-Fluorphenyl)-6-(3-hydroxy-4-methyl-nona-1,6-diinyl)-2-oxabicyclo[2.2.1]hept-5-yl]-3-oxa-5-heptensäure:

31 mg (51 µmol) des nach Beispiel 331 dargestellten polaren Alkohols B setzte man in Analogie zu Beispiel 187 um und isolierte nach Aufarbeitung und Reinigung 12 mg (25 µmol, 49%) der Titel-verbindung als farbloses Öl.

IR (Film): 3600-2400, 3050, 3010, 2970, 2930, 2870, 2230, 1710, 1605, 1515, 1450, 1230, 1160, 1125, 1040, 990, 900, 835 und 735 $cm^{-1}$.

**Beispiel 335**

(1S,4R,5S(5Z),6R(3R,4S))-7-[4-(4-Fluorphenyl)-6-(3-hydroxy-4-methyl-nona-1,6-diinyl)-2-oxabicyclo[2.2.1]hept-5-yl]-3-oxa-5-heptensäuremethylester:

4 mg (9 µmol) der nach Beispiel 334 dargestellten Säure veresterte man in Analogie zu Beispiel 186 und isolierte nach Lösungsmittelabzug 4 mg (8 µmol, 97%) der Titelverbindung als farbloses Öl.

IR (Film): 3600-3200, 3050, 3010, 2970, 2930, 2870, 2230, 1740, 1605, 1515, 1450, 1230, 1160, 1130, 1035, 995, 900, 835 und 735 $cm^{-1}$.

**Beispiel 336**

(1S,4R,5S(5Z),6R(3S,4S))-7-[4-(4-Fluorphenyl)-6-(3-hydroxy-4-methyl-nona-1,6-diinyl)-2-oxabicyclo[2.2.1]hept-5-yl]-3-oxa-5-heptensäure:

19,6 mg (32 μmol) des nach Beispiel 331 dargestellten unpolaren Alkohols A setzte man in Analogie zu Beispiel 187 um und isolierte nach Aufarbeitung und Reinigung 8,2 mg (17 μmol, 54%) der Titelverbindung als farbloses Öl.

IR (Film): 3600-2400, 3050, 2970, 2930, 2870, 2230, 1710, 1605, 1510, 1455, 1230, 1160, 1120, 1035, 995, 895, 835 und 735 $cm^{-1}$.

**Beispiel 337**

(1S,4R,5S(5Z),6R(3S,4S))-7-[4-(4-Fluorphenyl)-6-(3-hydroxy-4-methyl-nona-1,6-diinyl)-2-oxabicyclo[2.2.1]hept-5-yl]-3-oxa-5-heptensäuremethylester:

2,7 mg (6 μmol) der nach Beispiel 336 dargestellten Säure veresterte man in Analogie zu Beispiel 186 und isolierte nach Lösungsmittelabzug 2,8 mg (6 μmol, 100%) der Titelverbindung als farbloses Öl.

IR (Film): 3600-3200, 3050, 3010, 2970, 2930, 2870, 2230, 1745, 1605, 1515, 1455, 1230, 1160, 1120, 1040, 995, 895, 835 und 735 $cm^{-1}$.

**Beispiel 338**

(1S,4R,5S(5Z),6R)-7-[4-(4-Fluorphenyl)-6-(cyclohexylmethoxyiminomethyl)-2-oxabicyclo[2.2.1]hept-5-yl]-3-oxa-5-heptensäure-t-butylester:

16,3 mg (40 μmol) des in Beispiel 286b dargestellten Aldehyds setzte man in Analogie zu Beispiel 26 unter Verwendung von Cyclohexylmethoxyamin um und isolierte nach Aufarbeitung und Reini-gung 16,1 mg (31 μmol, 78%) der Titelverbindung als farbloses Öl.

IR (Film): 3070, 3040, 3010, 2930, 2850, 1745, 1610, 1515, 1450, 1430, 1235, 1165, 1115, 1040, 1025, 1000, 945, 900, 835 und 820 $cm^{-1}$.

**Beispiel 339**

(1S,4R,5S(5Z),6R)-7-[4-(4-Fluorphenyl)-6-(cyclohexylmethoxyiminomethyl)-2-oxabicyclo[2.2.1]hept-5-yl]-3-oxa-5-heptensäure:

16,1 mg (31 μmol) des nach Beispiel 338 dargestellten Esters verseifte man in Analogie zu Beispiel 2 und isolierte nach Aufarbeitung und Reinigung 12,8 mg (28 μmol, 90%) der Titelverbindung als farbloses Öl.

IR (Film): 3600-2400, 3070, 3050, 3020, 2930, 2850, 1730, 1610, 1515, 1450, 1235, 1160, 1115, 1040, 1025, 1000, 900, 835 und 820 $cm^{-1}$.

**Beispiel 340**

(1S,4R,5S(5Z),6R)-7-[4-(4-Fluorphenyl)-6-(4-fluorbenzyloxyiminomethyl)-2-oxabicyclo[2.2.1]hept-5-yl]-3-oxa-5-heptensäuremethylester:

16,8 mg (42 μmol) des in Beispiel 286b dargestellten Aldehyds setzte man in Analogie zu Beispiel 26 unter Verwendung von p-Fluorphenylmethoxyamin um und isolierte nach Aufarbeitung und Reinigung 17,6 mg (33 μmol, 80%) der Titelverbindung als farbloses Öl.

IR (Film): 3070, 3050, 3010, 2930, 2880, 1745, 1605, 1515, 1430, 1415, 1365, 1225, 1160, 1120, 1100, 1040, 1015, 945, 900 und 835 $cm^{-1}$.

**Beispiel 341**

(1S,4R,5S(5Z),6R)-7-[4-(4-Fluorphenyl)-6-(4-fluorbenzyloxyiminomethyl)-2-oxabicyclo[2.2.1]hept-5-yl]-3-oxa-5-heptensäure:

17,6 mg (33 μmol) des nach Beispiel 340 dargestellten Esters verseifte man in Analogie zu Beispiel 2 und isolierte nach Aufarbeitung und Reinigung 12,8 mg (27 μmol, 82%) der Titelverbindung als farbloses Öl.

IR (Film): 3600-2400, 3070, 3050, 3020, 2930, 2880, 1730, 1605, 1515, 1430, 1225, 1160, 1120, 1045, 1015, 945, 900 und 835 $cm^{-1}$.

**Beispiel 342**

(1S,4R,5S.6R)-7-[4-(4-Fluorphenyl)-6-(benzyloxymethyl)-2-oxabicyclo[2.2.1]hept-5-yl]-3-oxa-5(Z)-heptensäure:

19 mg (47 µmol) des in Beispiel 274a dargestellten Alkohols setzte man in Analogie zu Beispiel 1 um und isolierte nach Aufarbeitung und Reinigung 14,5 mg (33 µmol, 70%) der Titelverbindung als farbloses Öl.

IR (Film): 3700-2300, 3090, 3060, 3030, 2920, 2870, 1725, 1605, 1515, 1455, 1230, 1160, 1115, 1100, 1030, 900, 835, 740 und 700 $cm^{-1}$.

**Beispiel 343**

(1S,4R,5S.6R)-7-[4-(4-Fluorphenyl)-6-(benzyloxymethyl)-2-oxabicyclo[2.2.1]hept-5-yl]-3-oxa-5(Z)-heptensäuremethylester:

3,1 mg (7 µmol) der in Beispiel 342 dargestellten Säure veresterte man in Analogie zu Beispiel 186 und isolierte nach Lösungsmittelabzug 3,1 mg (7 µmol, 97%) der Titelverbindung als farbloses Öl.

IR (Film): 3090, 3060, 3030, 2970, 2920, 2870, 1745, 1605, 1515, 1455, 1430, 1230, 1160, 1115, 1100, 1030, 975, 900, 835, 740 und 700 $cm^{-1}$.

**Beispiel 344**

(1S,4R,5R(5Z),6S)-7-[4-(4-Phenylphenyl)-6-trifluormethylcarbonylamino-2-oxabicyclo[2.2.1]hept-5-yl]-5-heptensäure:

15 mg (30 µmol) des in Beispiel 344a dargestellten Esters B verseifte man in Analogie zu Beispiel 2 und isolierte nach Aufarbeitung und Reinigung 12 mg (25 µmol, 83%) der Titelverbindung als farbloses Öl.

IR (Film): 3700-2600, 3430, 3310, 3080, 3060, 3030, 2940, 2880, 1710, 1600, 1555, 1490, 1215, 1185, 1115, 1005, 900, 840, 815, 730 und 695 $cm^{-1}$.

**Beispiel 344a**

(1S,4R,5R(5Z),6S)-7-[4-(4-Phenylphenyl)-6-amino-2-oxabicyclo[2.2.1]hept-5-yl]-5-heptensäure-methylester (A) und (1S,4R,5R(5Z),6S)-7-[4-(4-Phenylphenyl)-6-trifluormethylcarbonylamino-2-oxabicyclo[2.2.1]hept-5-yl]-5-heptensäuremethylester (B):

Die auf 0-5°C gekühlte Lösung von 409 mg (903 µmol) des in Beispiel 344b dargestellten Säurechlorids in 1,4 ml Dichlormethan versetzte man mit der Lösung von 71,7 mg Natriumazid in 240 µl Wasser und rührte nach Zugabe von 0,9 mg Tetrabutylammoniumhydrogensulfat 1 Stunde unter Eiskühlung. Die organische Phase wurde abgetrennt, die wässrige mehrfach mit Dichlormethan gewaschen und die vereinigten organischen Extrakte 2 Tage über wasserfreiem Magnesiumsulfat aufbewahrt. Nach Filtration und Einengen des Volumens auf ca. 2 ml versetzte man mit 300 µl Trifluoressigsäure und erhitzte 24 Stunden unter Rückfluß. Den nach Lösungsmittelabzug erhaltenen Rückstand reinigte man durch Chromatographie an ca. 75 ml Kieselgel unter Verwendung eines Gradientensystems aus Dichlormethan und Methanol. Isoliert wurden 277 mg (683 µmol, 76%) der Titelverbindung A sowie 103 mg (205 µmol, 23%) der Titelverbindung B jeweils als farbloses Öl.

IR (Film) von A: 3360, 3290, 3050, 3030, 2930, 2860, 1730, 1605, 1485, 1265, 1075, 1045, 1005, 895, 835, 765, 735 und 700 $cm^{-1}$.

IR (Film) von B: 3300, 3060, 3030, 2940, 2880, 1725, 1705, 1600, 1550, 1485, 1440, 1210, 1180, 1155, 1005, 900, 840, 765, 730 und 695 $cm^{-1}$.

**Beispiel 344b**

(1S,4R,5S(5Z),6S)-7-[4-(4-Phenylphenyl)-6-chlorcarbonyl-2-oxabicyclo[2.2.1]hept-5-yl]-5-hepten-säuremethylester:

Die Lösung von 400 mg (921 µmol) der in Beispiel 344c dargestellten Säure in 10 ml wasserfreiem Dichlormethan kühlte man unter einer Atmosphäre aus trockenem Argon auf 0-5°C, versetzte man mit 160 µl Thionylchlorid, ließ auf 23°C erwärmen und rührte noch 6 Stunden. Nach abzug des Lösungsmittels im Vakuum isolierte man 409 mg (903 µmol, 98%) der Titelverbindung als blassgelbes Öl, das man ohne Reinigung weiter umsetzte.

**Beispiel 344c**

(1S,4R,5S(5Z),6S)-7-[4-(4-Phenylphenyl)-6-hydroxycarbonyl-2-oxabicyclo[2.2.1]hept-5-yl]-5-heptensäuremethylester:

Die Lösung von 540 mg (1,28 mmol) des nach Beispiel 293a dargestellten Alkohols oxidierte man in Analogie zu Beispiel 274m und isolierte nach in Analogie zu Beispiel 1i durchgeführter Aufarbeitung und chromatographisch durchgeführter Reinigung an ca. 70 ml Kieselgel unter Verwendung eines Gradientensystems aus n-Hexan und Aceton 400 mg (921 μmol, 72%) der Titelverbindung als farbloses Öl.

IR (Film): 3700-2500, 3050, 3030, 2950, 2870, 1730, 1700, 1600, 1485, 1440, 1300, 1220, 1170, 1005, 990, 895, 835, 765, 730 und 695 $cm^{-1}$.

## Beispiel 345

(1S,4R,5R(5Z),6S)-7-[4-(4-Phenylphenyl)-6-(3,5-dichlor-2-hydroxy-benzolsulfonamino)-2-oxa-bicyclo[2.2.1]hept-5-yl]-5-heptensäuremethylester:

22 mg (54,2 μmol) des nach Beispiel 544a dargestellten Amins löste man in 800 μl wasserfreiem Dichlormethan, versetzte mit 100 μl Triethylamin, 17 mg 3,5-Dichlor-2-hydroxy-benzolsulfonsäurechlorid und rührte 5 Stunden bei 23°C unter einer Atmosphäre aus trockenem Argon. Die chromatographische Reinigung erfolgte an einer analytischen Dünnschichtplatte. Als Laufmittel diente ein Gemisch aus Chloroform und Aceton, als Elutionsmittel ein Gemisch aus Ethanol und Ethylacetat. Isoliert wurden 11 mg (17 μmol, 31%) der Titelverbindung als farbloses ÖL.

IR (Film): 3450-3150, 3280, 3080, 3060, 3030, 2940, 2870, 1730, 1600, 1580, 1485, 1465, 1265, 1240, 1160, 1070, 1000, 900, 870, 835, 805, 765, 735, 700 und 580 $cm^{-1}$.

## Beispiel 346

(1S,4R,5R(5Z),6S)-7-[4-(4-Phenylphenyl)-6-(3,5-dichlor-2-hydroxy-benzolsulfonamino)-2-oxa-bicyclo[2.2.1]hept-5-yl]-5-heptensäure:

11 mg (17 μmol) des nach Beispiel 345 dargestellten Esters verseifte man in Analogie zu Beispiel 2 und isolierte nach Aufarbeitung und Reinigung 10 mg (16 μmol, 95%) der Titelverbindung als farbloses Öl.

IR (Film): 3700-2400, 3270, 3080, 3060, 3030, 2930, 2870, 1710, 1600, 1580, 1485, 1465, 1415, 1315, 1300, 1240, 1150, 1090, 1075, 1010, 995, 900, 870, 835, 765, 735, 695 und 580 $cm^{-1}$.

## Beispiel 347

(1S,4R,5R(5Z),6S)-7-[4-(4-Phenylphenyl)-6-(4-methyl-benzolsulfonamino)-2-oxa-bicyclo[2.2.1]hept-5-yl]-5-heptensäuremethylester:

25 mg (50 μmol) des nach Beispiel 344a dargestellten Amins setzte man in Analogie zu Beispiel 345 unter Verwendung von 4-Methyl-benzolsulfonsäurechlorid um und isolierte nach Reinigung 15,7 mg (28 μmol, 46%) der Titelverbindung als farbloses ÖL.

IR (Film): 3300, 3060, 3030, 2940, 2880, 1725, 1715, 1600, 1550, 1485, 1435, 1330, 1215, 1180, 1160, 1090, 1005, 900, 835, 765, 735, 700 und 660 $cm^{-1}$.

## Beispiel 348

(1S,4R,5R(5Z),6S)-7-[4-(4-Phenylphenyl)-6-(4-methyl-benzolsulfonamino)-2-oxa-bicyclo[2.2.1]hept-5-yl]-5-heptensäure:

15,7 mg (28 μmol) des nach Beispiel 347 dargestellten Esters verseifte man in Analogie zu Beispiel 2 und isolierte nach Aufarbeitung und Reinigung 12,9 mg (24 μmol, 84%) der Titelverbindung als farbloses Öl.

IR (Film): 3700-2400, 3270, 3050, 3010, 2940, 2880, 1710, 1600, 1555, 1435, 1325, 1230, 1160, 1090, 1005, 905, 835, 815, 765, 735, 700 und 660 $cm^{-1}$.

## Beispiel 349

(1S,4R,5R(5Z),6S)-7-[4-(4-Phenylphenyl)-6-(4-fluor-benzolsulfonamino)-2-oxa-bicyclo[2.2.1]hept-5-yl]-5-heptensäuremethylester:

22,7 mg (56 μmol) des nach Beispiel 344a dargestellten Amins setzte man in Analogie zu Beispiel 345 unter Verwendung von 4-Fluor-benzolsulfonsäurechlorid um und isolierte nach Reinigung 15 mg (27 μmol, 48%) der Titelverbindung als farbloses ÖL.

IR (Film): 3300, 3060, 3030, 2940, 2880, 1725, 1710, 1595, 1550, 1490, 1435, 1330, 1215, 1165, 1155, 1090, 1005, 900, 840, 765, 735, 700 und 660 $cm^{-1}$.

**Beispiel 350**

(1S,4R,5R(5Z),6S)-7-[4-(4-Phenylphenyl)-6-(4-fluor-benzolsulfonamino)-2-oxa-bicyclo[2.2.1]hept-5-yl]-5-heptensäure:

15 mg (27 µmol) des nach Beispiel 349 dargestellten Esters verseifte man in Analogie zu Beispiel 2 und isolierte nach Aufarbeitung und Reinigung 11,4 mg (21 µmol, 77%) der Titelverbindung als farbloses Öl.

IR (Film): 3700-2500, 3300, 3060, 3030, 2940, 2880, 1710, 1600, 1550, 1490, 1435, 1325, 1215, 1160, 1155, 1090, 1005, 900, 835, 765, 735, 700 und 660 $cm^{-1}$.

**Beispiel 351**

(1S,4R,5R(5Z),6S)-7-[4-(4-Phenylphenyl)-6-(naphtalin-2-sulfonamino)-2-oxa-bicyclo[2.2.1]hept-5-yl]-5-heptensäuremethylester:

23,3 mg (57,5 µmol) des nach Beispiel 344a dargestellten Amins setzte man in Analogie zu Beispiel 345 unter Verwendung von Naphtalin-2-sulfonsäurechlorid um und isolierte nach Reinigung 18,4 mg (31 µmol, 54%) der Titelverbindung als farbloses ÖL.

IR (Film): 3280, 3060, 3030, 2940, 2870, 1730, 1600, 1485, 1435, 1330, 1265, 1245, 1160, 1130, 1090, 1075, 1005, 900, 835, 815, 765, 735, 700 und 660 $cm^{-1}$.

**Beispiel 352**

(1S,4R,5R(5Z),6S)-7-[4-(4-Phenylphenyl)-6-(naphtalin-2-sulfonamino)-2-oxa-bicyclo[2.2.1]hept-5-yl]-5-heptensäure:

18,4 mg (31 µmol) des nach Beispiel 351 dargestellten Esters verseifte man in Analogie zu Beispiel 2 und isolierte nach Aufarbeitung und Reinigung 14,9 mg (26 µmol, 83%) der Titelverbindung als farbloses Öl.

IR (Film): 3700-2500, 3280, 3060, 3030, 2940, 2880, 1705, 1600, 1485, 1435, 1330, 1265, 1245, 1160, 1130, 1090, 1075, 1005, 895, 835, 815, 765, 735, 695 und 660 $cm^{-1}$.

**Beispiel 353**

(1S,4R,5R(5Z),6S)-7-[4-Phenylphenyl)-6-(chinolin-8-sulfonamino)-2-oxa-bicyclo[2.2.1]hept-5-yl]-5-heptensäuremethylester:

22,5 mg (55,5 µmol) des nach Beispiel 344a dargestellten Amins setzte man in Analogie zu Beispiel 345 unter Verwendung von Chinolin-8-sulfonsäurechlorid um und isolierte nach Reinigung 20 mg (33,5 µmol, 60%) der Titelverbindung als farbloses ÖL.

IR (Film): 3280, 3060, 3030, 2940, 2870, 1730, 1615, 1595, 1560, 1490, 1370, 1330, 1210, 1170, 1145, 1090, 1070, 1005, 900, 835, 790, 765, 735, 700 und 675 $cm^{-1}$.

**Beispiel 354**

(1S,4R,5R(5Z),6S)-7-[4-(4-Phenylphenyl)-6-(chinolin-8-sulfonamino)-2-oxa-bicyclo[2.2.1]hept-5-yl]-5-heptensäure:

20 mg (33,5 µmol) des nach Beispiel 353 dargestellten Esters verseifte man in Analogie zu Beispiel 2 und isolierte nach Aufarbeitung und Reinigung 17 mg (29 µmol, 87%) der Titelverbindung als farbloses Öl.

IR (Film): 3700-2500, 3420, 3250, 3060, 3030, 3010, 2930, 2870, 1710, 1615, 1595, 1565, 1490, 1450, 1405, 1330, 1215, 1165, 1145, 1090, 1005, 995, 970, 895, 830, 790, 765, 700, 675 und 580 $cm^{-1}$.

**Beispiel 355**

(1S,4R,5R(5Z),6S)-7-[4-(4-Phenylphenyl)-6-(hexylcarbonylamino)-2-oxa-bicyclo[2.2.1]hept-5-yl]-5-heptensäuremethylester:

20,9 mg (51,5 µmol) des nach Beispiel 344a dargestellten Amins setzte man in Analogie zu Beispiel 345 unter Verwendung von n-Heptansäurechlorid um und isolierte nach Reinigung 21,7 mg (41,9 µmol, 81%) der Titelverbindung als farbloses ÖL.

IR (Film): 3290, 3060, 3030, 2950, 2930, 2860, 1735, 1715, 1640, 1540, 1485, 1450, 1435, 1365, 1245, 1215, 1165, 1125, 1050, 1005, 970, 900, 835, 765, 730 und 700 $cm^{-1}$.

**Beispiel 356**

(1S,4R,5R(5Z),6S)-7-[4-(4-Phenylphenyl)-6-(hexylcarbonylamino)-2-oxa-bicyclo[2.2.1]hept-5-yl]-5-heptensäure:

21,7 mg (41,9 μmol) des nach Beispiel 355 dargestellten Esters verseifte man in Analogie zu Beispiel 2 und isolierte nach Aufarbeitung und Reinigung 21 mg (41,7 μmol, 99%) der Titelverbindung als farbloses Öl.

IR (Film): 3700-2500, 3450, 3060, 3030, 3010, 2960, 2930, 2870, 1710, 1645, 1545, 1490, 1455, 1405, 1380, 1300, 1245, 1220, 1125, 1050, 1010, 975, 900, 835, 765, 730 und 695 $cm^{-1}$.

**Beispiel 357**

(1S,4R,5R(5Z),6S)-7-[4-(Phenylphenyl)-6-(4-fluor-phenylcarbonylamino)-2-oxa-bicyclo[2.2.1]hept-5-yl]-5-heptensäuremethylester:

20,2 mg (49.8 μmol) des nach Beispiel 344a dargestellten Amins setzte man in Analogie zu Beispiel 345 unter Verwendung von 4-Fluor-benzoesäurechlorid um und isolierte nach Reinigung 24,7 mg (46,8 μmol, 94%) der Titelverbindung als farbloses ÖL.

IR (Film): 3310, 3060, 3030, 2940, 2870, 1735, 1715, 1655, 1630, 1605, 1535, 1500, 1440, 1315, 1235, 1160, 1095, 1045, 1005, 970, 900, 850, 765, 735 und 700 $cm^{-1}$.

**Beispiel 358**

(1S,4R,5R(5Z),6S)-7-[4-(4-Phenylphenyl)-6-(4-fluor-phenylcarbonylamino)-2-oxa-bicyclo[2.2.1]hept-5-yl]-5-heptensäure:

24,7 mg (46,8 μmol) des nach Beispiel 357 dargestellten Esters verseifte man in Analogie zu Beispiel 2 und isolierte nach Aufarbeitung und Reinigung 18 mg (35 μmol, 75%) der Titelverbindung als farbloses Öl.

IR (Film): 3700-2500, 3330, 3060, 3030, 3010, 2940, 2880, 1710, 1635, 1605, 1540, 1505, 1235, 1160, 1095, 1010, 970, 900, 850, 765, 735 und 700 $cm^{-1}$.

**Beispiel 359**

(1S,4R,5R(5Z),6S)-7-[4-(4-Phenylphenyl)-6-(perfluorbenzolsulfonamino)-2-oxa-bicyclo[2.2.1]hept-5-yl]-5-heptensäuremethylester:

19 mg (46,9 μmol) des nach Beispiel 344a dargestellten Amins setzte man in Analogie zu Beispiel 345 unter Verwendung von Perfluorbenzolsulfonsäurechlorid um und isolierte nach Reinigung 19,5 mg (30,7 μmol, 65%) der Titelverbindung als farbloses ÖL.

IR (Film): 3260, 3060, 3030, 2940, 2880, 1735, 1710, 1645, 1610, 1515, 1495, 1440, 1360, 1295, 1170, 1100, 990, 900, 835, 765, 735, 700, 645 und 605 $cm^{-1}$.

**Beispiel 360**

(1S,4R,5R(5Z),6S)-7-[4-(4-Phenylphenyl)-6-(perfluorbenzolsulfonamino)-2-oxa-bicyclo[2.2.1]hept-5-yl]-5-heptensäure:

19,5 mg (30,7 μmol) des nach Beispiel 359 dargestellten Esters verseifte man in Analogie zu Beispiel 2 und isolierte nach Aufarbeitung und Reinigung 16,8 mg (27 μmol, 88%) der Titelverbindung als farbloses Öl.

IR (Film): 3700-2500, 3260, 3060, 3030, 2940, 2880, 1705, 1640, 1610, 1515, 1495, 1445, 1365, 1295, 1170, 1095, 990, 900, 835, 765, 735, 700, 645 und 605 $cm^{-1}$.

**Beispiel 361**

(1R,4S,5S(5Z),6R)-7-[4-(4-Fluor-phenyl)-6-(4-methylbenzolsulfonamino-2-oxabicyclo[2.2.1]hept-5-yl]-5-heptensäuremethylester:

38 mg (109 μmol) des nach Beispiel 361a dargestellten Amins setzte man in Analogie zu Beispiel 345 unter Verwendung von 4-Methyl-benzolsulfonsäurechlorid um und isolierte nach Reinigung 36,7 mg (73,2 μmol, 67%) der Titelverbindung als farbloses ÖL.

IR (Film): 3270, 3060, 3010, 2940, 2970, 1730, 1710, 1600, 1515, 1435, 1325, 1230, 1160, 1090, 1000, 970, 900, 830, 815, 735 und 665 $cm^{-1}$.

**Beispiel 361a**

(1R,4S,5S(5Z),6R)-7-[4-(4-Fluor-phenyl)-6-amino-2-oxabicyclo[2.2.1]hept-5-yl]-5-heptensäure-methylester (A) und (1R,4S,5S(5Z),6R)-7-[4-(4-Fluor-phenyl)-6-trifluormethylcarbonylamino-2-oxabicyclo[2.2.1]hept-5-yl]-5-heptensäuremethylester (B):

142 mg (392 μmol) des nach Beispiel 17a dargestellten Alkohols setzte man in Analogie zu den Beispielen 344c bis 344a um und isolierte nach Aufarbeitung und Reinigung 38 mg (109 μmol, 28%) der Titelverbindung A sowie 47 mg (102 μmol, 26%) der Titelverbindung B jeweils als farbloses Öl.

IR (Film) von A: 3340, 3040, 3010, 2940, 2870, 1735, 1605, 1510, 1435, 1360, 1230, 1200, 1160, 1100, 1035, 1000, 895, 835 und 720 $cm^{-1}$.

IR (Film) von B: 3310, 3070, 3010, 2950, 2880, 1725, 1705, 1605, 1550, 1515, 1435, 1365, 1300, 1220, 1180, 1160, 1090, 1045, 1005, 970, 945, 900, 835 und 725 $cm^{-1}$.

37 mg (80,5 μmol) der Titelverbindung B löste man in einem Gemisch aus 500 μl Methanol und 200 μl Wasser, versetzte mit 30 mg Kaliumcarbonat und rührte 24 Stunden bei 50°C. Man neutralisierte durch Zugabe einer verdünnten Zitronensäurelösung und reinigte durch Chromatographie an zwei analytischen Dünnschichtplatten. Als Laufmittel diente ein Gemisch aus Chloroform und Aceton, als Elutionsmittel ein Gemisch aus Aceton und Ethylacetat.

Isoliert wurden 22 mg (60,4 μmol, 75%) der Titelverbindung A als farbloses Öl.

**Beispiel 362**

(1R,4S,5S(5Z),6R)-7-[4-(4-Fluor-phenyl)-6-(4-methylbenzolsulfonamino-2-oxabicyclo[2.2.1]hept-5-yl]-5-heptensäure:

36,7 mg (73,2 μmol) des nach Beispiel 361 dargestellten Esters verseifte man in Analogie zu Beispiel 2 und isolierte nach Aufarbeitung und Reinigung 27,8 mg (57 μmol, 78%) der Titelverbindung als farbloses Öl.

IR (Film): 3700-2400, 3270, 3050, 3010, 2940, 2980, 1710, 1600, 1515, 1435, 1410, 1325, 1310, 1235, 1160, 1090, 1000, 975, 905, 835, 815, 735 und 665 $cm^{-1}$.

**Beispiel 363**

(1R,4S,5S(5Z),6R)-7-[4-(4-Fluor-phenyl)-6-(naphtalin-2-sulfonamino)-2-oxabicyclo[2.2.1]hept-5-yl]-5-heptensäuremethylester:

20 mg (57,6 μmol) des nach Beispiel 361a dargestellten Amins setzte man in Analogie zu Beispiel 345 unter Verwendung von Naphtalin-2-sulfonsäurechlorid um und isolierte nach Reinigung 16 mg (30 μmol, 52%) der Titelverbindung als farbloses ÖL.

IR (Film): 3270, 3060, 3010, 2940, 2870, 1730, 1605, 1590, 1510, 1435, 1325, 1230, 1160, 1095, 1075, 1000, 900, 860, 830, 820, 735 und 660 $cm^{-1}$.

**Beispiel 364**

(1R,4S,5S(5Z),6R)-7-[4-(4-Fluor-phenyl)-6-(naphtalin-2-sulfonamino)-2-oxabicyclo[2.2.1]hept-5-yl]-5-heptensäure:

16 mg (30 μmol) des nach Beispiel 363 dargestellten Esters verseifte man in Analogie zu Beispiel 2 und isolierte nach Aufarbeitung und Reinigung 15,3 mg (29 μmol, 97%) der Titelverbindung als farbloses Öl.

IR (Film): 3700-2500, 3270, 3060, 3010, 2930, 2870, 1705, 1605, 1590, 1515, 1455, 1435, 1410, 1325, 1235, 1160, 1130, 1095, 1075, 1000. 975, 900, 865, 830, 815, 750, 660 und 550 $cm^{-1}$.

**Beispiel 365**

(1S,4R,5R(5Z),6S)-7-[4-(4-Phenylphenyl)-6-(3-phenylpropylcarbonylamino)-2-oxa-bicyclo[2.2.1]hept-5-yl]-5-heptensäuremethylester:

19.7 mg (48 μmol) des nach Beispiel 344a dargestellten Amins setzte man in Analogie zu Beispiel 345 unter Verwendung von 4-Phenylbuttersäurechlorid um und isolierte nach Reinigung 22,8 mg (41 μmol, 85%) der Titelverbindung als farbloses ÖL.

IR (Film): 3300, 3080, 3060, 3030, 2940, 2870, 1730, 1635, 1535, 1485, 1450, 1245, 1160, 1005, 900, 835, 765, 730 und 700 $cm^{-1}$.

**Beispiel 366**

(1S,4R,5R(5Z),6S)-7-[4-(4-Phenylphenyl)-6-(3-phenylpropylcarbonylamino)-2-oxa-bicyclo[2.2.1]hept-5-yl]-5-heptensäure:

22,8 mg (41 μmol) des nach Beispiel 365 dargestellten Esters verseifte man in Analogie zu Beispiel 2 und isolierte nach Aufarbeitung und Reinigung 21 mg (39 μmol, 95%) der Titelverbindung als farbloses Öl.

IR (Film): 3430, 3060, 3030, 2930, 2860, 1705, 1635, 1540, 1490, 1450, 1150, 1005, 970, 900, 835, 765, 730 und 695 $cm^{-1}$.

**Beispiel 367**

(1S,4R,5R(5Z),6S)-7-[4-(4-Phenylphenyl)-6-(2-phenoxyethylcarbonylamino)-2-oxa-bicyclo[2.2.1]hept-5-yl]-5-heptensäuremethylester:

17,2 mg (42 μmol) des nach Beispiel 344a dargestellten Amins setzte man in Analogie zu Beispiel 345 unter Verwendung von 3-Phenoxypropylchlorid um und isolierte nach Reinigung 17,7 mg (32 μmol, 76%) der Titelverbindung als farbloses ÖL.

IR (Film): 3300, 3060, 3030, 2940, 2870, 1735, 1640, 1600, 1540, 1490, 1240, 1170, 1040, 1005, 895, 835, 765, 755, 730 und 695 $cm^{-1}$.

**Beispiel 368**

(1S,4R,5R(5Z),6S)-7-[4-(4-Phenylphenyl)-6-(2-phenoxyethylcarbonylamino)-2-oxa-bicyclo[2.2.1]hept-5-yl]-5-heptensäure:

17,7 mg (32 μmol) des nach Beispiel 367 dargestellten Esters verseifte man in Analogie zu Beispiel 2 und isolierte nach Aufarbeitung und Reinigung 14 mg (26 μmol, 81%) der Titelverbindung als farbloses Öl.

IR (Film): 3430, 3060, 3030, 2930, 2870, 1705, 1640, 1600, 1550, 1495, 1490, 1240, 1040, 1005, 900, 835, 765, 755, 730 und 690 $cm^{-1}$.

**Beispiel 369**

(1S,4R,5S(5Z),6S)-7-[4-(4-Phenylphenyl)-6-[3(S)-hydroxy-4(RS)-phenyl-1(E)-pentenyl]-2-oxa-bicyclo[2.2.1]hept-5-yl]-5-heptensäuremethylester (A) und (1S,4R,5S(5Z),6S)-7-[4-(4-Phenylphenyl)-6-[3(R)-hydroxy-4(RS)-phenyl-1(E)-pentenyl]-2-oxa-bicyclo[2.2.1]hept-5-yl]-5-heptensäuremethylester (B):

62 mg (113 μmol) des in Beispiel 369a dargestellten ungesättigten Ketons reduzierte man in Analogie zu Beispiel 23 und isolierte nach Aufarbeitung und Reinigung 54 mg (93 μmol, 87%) eines Gemisches der beiden Titelverbindungen als farbloses Öl.

IR (Film): 3600-3200, 3080, 3060, 3030, 2960, 2870, 1735, 1600, 1485, 1450, 1245, 1195, 1005, 995, 970, 895, 835, 765, 730 und 700 $cm^{-1}$.

Die chromatographische Trennung lieferte 20,1 mg (36 μmol, 32%) des unpolareren Alkohols, dem man die Struktur A zuordnete, sowie 30,1 mg (55 μmol, 48%) des polareren Alkohols dem man die Struktur B zuordnete.

**Beispiel 369a**

(1S,4R,5S(5Z),6S)-7-[4-(4-Phenylphenyl)-6-[3-oxo-4(RS)-phenyl-1(E)-pentenyl]-2-oxa-bicyclo[2.2.1]hept-5-yl]-5-heptensäuremethylester:

54,1 mg (129 μmol) des in Beispiel 307b dargestellten Aldehyds setzte man in Analogie zu Beispiel 23a unter Verwendung von Dimethyl-(2-oxo-3(RS)-phenyl-butyl)-phosphonat um und isolierte nach Aufarbeitung und Reinigung 62 mg (113 μmol, 87%) der Titelverbindung als farbloses Öl.

IR (Film): 3080, 3050, 3030, 2970, 2940, 2870, 1735, 1690, 1670, 1620, 1600, 1485, 1445, 1310, 1240, 1195, 1165, 1075, 1030, 995, 970, 900, 835, 765, 730 und 700 $cm^{-1}$.

**Beispiel 370**

(1S,4R,5S(5Z),6S)-7-[4-(4-Phenylphenyl)-6-[3(R)-hydroxy-4(RS)-phenyl-1(E)-pentenyl]-2-oxa-bicyclo[2.2.1]hept-5-yl]-5-heptensäure:

30,1 mg (55 μmol) des nach Beispiel 369 dargestellten polaren Esters B verseifte man in Analogie zu Beispiel 2 und isolierte nach Aufarbeitung und Reinigung 25,4 mg (47 μmol, 86%) der Titelver-bindung als

wachsartigen Feststoff.

IR (KBr): 3430, 3080, 3060, 3030, 2970, 2930, 2870, 1705, 1600, 1485, 1450, 1400, 1240, 1195, 1005, 995, 970, 895, 835, 765, 730 und 700 $cm^{-1}$.

**Beispiel 371**

(1S,4R,5S(5Z),6S)-7-[4-(4-Phenylphenyl)-6-[3(S)-hydroxy-4(RS)-phenyl-1(E)-pentenyl]-2-oxa-bicyclo[2.2.1]hept-5-yl]-5-heptensäure:

20,1 mg (36 μmol) des nach Beispiel 369 dargestellten unpolaren Esters A verseifte man in Analogie zu Beispiel 2 und isolierte nach Aufarbeitung und Reinigung 19 mg (35 μmol, 98%) der Titelver-bindung als farbloses Öl.

IR (Film): 3430, 3060, 3030, 2950, 2870, 1705, 1600, 1550, 1485, 1400, 1240, 1040, 1005, 995, 970, 895, 835, 765, 730 und 700 $cm^{-1}$.

**Beispiel 372**

(1S,4R,5S(5Z),6S)-7-[4-(4-Phenylphenyl)-6-[3(S)-hydroxy-5-phenyl-1(E)-pentenyl]-2-oxa-bicyclo[2.2.1]hept-5-yl]-5-heptensäuremethylester (A) und (1S,4R,5S(5Z),6S)-7-[4-(4-Phenylphenyl)-6-[3(R)-hydroxy-5-phenyl-1(E)-pentenyl]-2-oxa-bicyclo[2.2.1]hept-5-yl]-5-heptensäuremethylester (B):

65 mg (118 μmol) des in Beispiel 372a dargestellten ungesättigten Ketons reduzierte man in Analogie zu Beispiel 23 und isolierte nach Aufarbeitung und Reinigung 59 mg (107 μmol, 91%) eines Gemisches der beiden Titelverbindungen als farbloses Öl.

IR (Film): 3600-3200, 3080, 3050, 3020, 2940, 2860, 1735, 1600, 1485, 1450, 1435, 1245, 1195, 1165, 1045, 1005, 995, 970, 895, 835, 765, 730 und 700 $cm^{-1}$.

Die chromatographische Trennung lieferte 30 mg (54 μmol, 46%) des unpolareren Alkohols, dem man die Struktur A zuordnete, sowie 26,7 mg (48 μmol, 41%) des polareren Alkohols dem man die Struktur B zuordnete.

**Beispiel 372a**

(1S,4R,5S(5Z),6S)-7-[4-(4-Phenylphenyl)-6-[3-oxo-5-phenyl-1(E)-pentenyl]-2-oxa-bicyclo[2.2.1]hept-5-yl]-5-heptensäuremethylester:

55,5 mg (133 μmol) des in Beispiel 307b dargestellten Aldehyds setzte man in Analogie zu Beispiel 23a unter Verwendung von Dimethyl-(2-oxo-4-phenyl-butyl)-phosphonat um und isolierte nach Aufarbeitung und Reinigung 65 mg (118 μmol, 89%) der Titelverbindung als farbloses Öl.

IR (Film): 3080, 3060, 3030, 2940, 2870, 1735, 1690, 1665, 1620, 1600, 1485, 1450, 1435, 1365, 1240, 1190, 1170, 995, 975, 900, 835, 765, 730 und 700 $cm^{-1}$.

**Beispiel 373**

(1S,4R,5S(5Z),6S)-7-[4-(4-Phenylphenyl)-6-[3(R)-hydroxy-5-phenyl-1(E)-pentenyl]-2-oxa-bicyclo[2.2.1]hept-5-yl]-5-heptensäure:

26,7 mg (48 μmol) des nach Beispiel 372 dargestellten polaren Esters B verseifte man in Analogie zu Beispiel 2 und isolierte nach Aufarbeitung und Reinigung 21,7 mg (40 μmol, 84%) der Titelver-bindung als farbloses Öl.

IR (Film): 3700-2600, 3080, 3060, 3030, 2930, 2870, 1705, 1600, 1490, 1450, 1405, 1195, 1125, 1095, 1005, 995, 970, 890, 765, 730 und 700 $cm^{-1}$.

**Beispiel 374**

(1S,4R,5S(5Z),6S)-7-[4-(4-Phenylphenyl)-6-[3(S)-hydroxy-5-phenyl-1(E)-pentenyl]-2-oxa-bicyclo[2.2.1]hept-5-yl]-5-heptensäure:

30 mg (54 μmol) des nach Beispiel 372 dargestellten unpolaren Esters A verseifte man in Analogie zu Beispiel 2 und isolierte nach Aufarbeitung und Reinigung 24,5 mg (46 μmol, 85%) der Titelver-bindung als farbloses Öl.

IR (Film): 3700-2600, 3080, 3060, 3030, 2930, 2870, 1710, 1600, 1490, 1450, 1405, 1345, 1195, 1125, 1095, 1005, 995, 970, 895, 765, 730 und 700 $cm^{-1}$.

**Beispiel 375**

(1S,4R,5S(5Z),6S)-7-[4-(4-Phenylphenyl)-6-[3(S),5-dihydroxy-4(RS)-phenyl-1(E)-pentenyl]-2-oxa-bicyclo[2.2.1]hept-5-yl]-5-heptensäuremethylester (A) und (1S,4R,5S(5Z),6S)-7-[4-(4-Phenyl phenyl)-6-[3(R),5-dihydroxy-4(RS)-phenyl-1(E)-pentenyl]-2-oxa-bicyclo[2.2.1]hept-5-yl]-5-heptensäuremethylester (B):

33,6 mg (42 µmol) des in Beispiel 375a dargestellten ungesättigten Ketons reduzierte man in Analogie zu Beispiel 23. Die nach Aufarbeitung und chromatographischer Reinigung isolierten diastereoisomeren Alkohole wurden getrennt einer Silyletherspaltung in Analogie zu Beispiel 1a unterworfen. Nach erneuter Aufarbeitung und chromatographischer Reinigung isolierte man 15,3 mg (27 µmol, 64%) des unpolareren Alkohols, dem man die Struktur A zuordnete, sowie 8,2 mg (14 µmol, 34%) des polareren Alkohols dem man die Struktur B zuordnete.

IR (Film) von A: 3600-3200, 3060, 3030, 2940, 2880, 1730, 1600, 1485, 1450, 1425, 1265, 1195, 1110, 995, 970, 835, 765, 735 und 700 $cm^{-1}$.

**Beispiel 375a**

(1S,4R,5S(5Z),6S)-7-[4-(4-Phenylphenyl)-6-[3-oxo-4(RS)-phenyl-5-(tert.-butyldiphenylsilyloxy)-1(E)-pentenyl]-2-oxa-bicyclo[2.2.1]hept-5-yl]-5-heptensäuremethylester:

54 mg (129 µmol) des in Beispiel 307b dargestellten Aldehyds setzte man in Analogie zu Beispiel 23a unter Verwendung von Dimethyl-(2-oxo-3(RS)-phenyl-4-(tert.-butyldiphenylsilyloxy)butyl)-p hosphonat um und isolierte nach Aufarbeitung und Reinigung 33,6 mg (42 µmol, 32%) der Titelver-bindung als farbloses Öl.

IR (Film): 3050, 3030, 2940, 2860, 1735, 1690, 1665, 1620, 1600, 1485, 1450, 1425, 1190, 1110, 995, 970, 900, 820, 765, 735 und 700 $cm^{-1}$.

**Beispiel 376**

(1S,4R,5S(5Z),6S)-7-[4-(4-Phenylphenyl)-6-[3(R),5-dihydroxy-4(RS)-phenyl-1(E)-pentenyl]-2-oxa-bicyclo[2.2.1]hept-5-yl]-5-heptensäure:

8,2 mg (14 µmol) des nach Beispiel 375 dargestellten polaren Esters B verseifte man in Analogie zu Beispiel 2 und isolierte nach Aufarbeitung und Reinigung 6,6 mg (12 µmol, 85%) der Titelver-bindung als farbloses Öl.

IR (Film): 3700-2400, 3080, 3050, 3030, 2930, 2880, 1710, 1600, 1485, 1450, 1405, 1295, 1240, 1195, 1045, 1005, 995, 970, 890, 835, 765, 735 und 700 $cm^{-1}$.

**Beispiel 377**

(1S,4R,5S(5Z),6S)-7-[4-(4-Phenylphenyl)-6-[3(S),5-dihydroxy-4(RS)-phenyl-1(E)-pentenyl]-2-oxa-bicyclo[2.2.1]hept-5-yl]-5-heptensäure:

15,3 mg (27 µmol) des nach Beispiel 375 dargestellten unpolaren Esters A verseifte man in Analogie zu Beispiel 2 und isolierte nach Aufarbeitung und Reinigung 14 mg (25 µmol, 94%) der Titelver-bindung als farbloses Öl.

IR (Film): 3700-2400, 3080, 3060, 3030, 2930, 2880, 1705, 1600, 1490, 1450, 1400, 1240, 1195, 1045, 1005, 995, 970, 890, 835, 765, 730 und 700 $cm^{-1}$.

**Beispiel 378**

(1S,4R,5S,6R)-7-[4-(4-Fluorphenyl)-6-(4-cyanobenzyloxymethyl)-2-oxabicyclo[2.2.1]hept-5-yl]-3-oxa-5(Z)-heptensäure-tert.Butylester:

13,2 mg (32 µmol) des in Beispiel 274a dargestellten Alkohols setzte man in Analogie zu Beispiel 1 mit 4-Cyano-benzylbromid um und isolierte nach Aufarbeitung und Reinigung 13,7 mg (26 µmol, 82%) der Titelverbindung als farbloses Öl.

IR (Film): 3050, 2980, 2930, 2870, 2230, 1745, 1610, 1510, 1365, 1230, 1160, 1125, 1030, 945, 895, 835, 820 und 735 $cm^{-1}$.

**Beispiel 379**

(1S,4R,5S,6R)-7-[4-(4-Fluorphenyl)-6-(4-cyanobenzyloxymethyl)-2-oxabicyclo[2.2.1]hept-5-yl]-3-oxa-5(Z)-heptensäure:

13,7 mg (26 μmol) des nach Beispiel 378 dargestellten Esters verseifte man in Analogie zu Beispiel 2 und isolierte nach Aufarbeitung und Reinigung 10,5 mg (23 μmol, 88%) der Titelverbindung als farbloses Öl.

IR (Film): 3600-2500, 3070, 3040, 2920, 2870, 2230, 1725, 1610, 1510, 1230, 1160, 1115, 1030, 1020, 975, 895, 835 und 820 $cm^{-1}$.

**Beispiel 380**

(1S,4R,5S,6R)-7-[4-(4-Fluorphenyl)-6-(4-cyanobenzyloxymethyl)-2-oxabicyclo[2.2.1]hept-5-yl]-3-oxa-5(E)-heptensäure:

11,2 mg (28 μmol) des in Beispiel 276a dargestellten Alkohols setzte man in Analogie zu Beispiel 1 mit 4-Cyano-benzylbromid um und isolierte nach Aufarbeitung und Reinigung 10 mg (21 μmol, 77%) der Titelverbindung als farbloses Öl.

IR (Film): 3600-2500, 3040, 2920, 2860, 2220, 1720, 1610, 1510, 1420, 1370, 1230, 1160, 1115, 1100, 1030, 970, 895, 830 und 820 $cm^{-1}$.

**Beispiel 381**

(1S,4R,5S,6R)-7-[4-(4-Fluorphenyl)-6-(4-cyanobenzyloxymethyl)-2-oxabicyclo[2.2.1]hept-5-yl]-3-oxa-5(E)-heptensäuremethylester:

3 mg (6 μmol) der nach Beispiel 380 dargestellten Säure veresterte man in Analogie zu Beispiel 186 und isolierte nach Lösungsmittelabzug 3 mg (6 μmol, 99%) der Titelverbindung als farbloses Öl.

IR (Film): 3070, 3040, 2920, 2860, 2220, 1735, 1605, 1510, 1420, 1230, 1160, 1105, 1025, 970, 895, 830 und 820 $cm^{-1}$.

**Beispiel 382**

(1S,4R,5S,6R)-7-[4-(4-Fluorphenyl)-6-(napht-2-ylmethoxymethyl)-2-oxabicyclo[2.2.1]hept-5-yl]-3-oxa-5(E)-heptensäure-tert.Butylester:

11,7 mg (29 μmol) des in Beispiel 276a dargestellten Alkohols setzte man in Analogie zu Beispiel 1 mit 2-Brommethyl-naphtalin um und isolierte nach Aufarbeitung und Reinigung 15,6 mg (28 μmol, 98%) der Titelverbindung als farbloses Öl.

IR (Film): 3050, 2970, 2920, 2860, 1745, 1600, 1510, 1365, 1225, 1160, 1125, 1030, 970, 895, 830, 820 und 750 $cm^{-1}$.

**Beispiel 383**

(1S,4R,5S,6R)-7-[4-(4-Fluorphenyl)-6-(napht-2-ylmethoxymethyl)-2-oxabicyclo[2.2.1]hept-5-yl]-3-oxa-5(E)-heptensäure:

15,6 mg (28 μmol) des nach Beispiel 382 dargestellten Esters verseifte man in Analogie zu Beispiel 2 und isolierte nach Aufarbeitung und Reinigung 10,8 mg (22 μmol, 79%) der Titelverbindung als farbloses Öl.

IR (Film): 3600-2400, 3050, 2920, 2860, 1730, 1605, 1510, 1230, 1160, 1120, 1030, 970, 895, 830, 820 und 750 $cm^{-1}$.

**Beispiel 384**

(1S,4R,5S,6R)-7-[4-(4-Fluorphenyl)-6-(napht-2-ylmethoxymethyl)-2-oxabicyclo[2.2.1]hept-5-yl]-3-oxa-5(Z)-heptensäure-tert.Butylester:

14,3 mg (35 μmol) des in Beispiel 274a dargestellten Alkohols setzte man in Analogie zu Beispiel 1 mit 2-Brommethyl-naphtalin um und isolierte nach Aufarbeitung und Reinigung 18,2 mg (33 μmol, 95%) der Titelverbindung als farbloses Öl.

IR (Film): 3050, 2970, 2920, 2860, 1745, 1600, 1510, 1365, 1230, 1160, 1120, 1030, 945, 895, 830, 820 und 750 $cm^{-1}$.

**Beispiel 385**

(1S,4R,5S,6R)-7-[4-(4-Fluorphenyl)-6-(napht-2-ylmethoxymethyl)-2-oxabicyclo[2.2.1]hept-5-yl]-3-oxa-5(Z)-heptensäure:

18,2 mg (33 μmol) des nach Beispiel 384 dargestellten Esters verseifte man in Analogie zu Beispiel 2

und isolierte nach Aufarbeitung und Reinigung 12,9 mg (26 µmol, 79%) der Titelverbindung als farbloses Öl.

IR (Film): 3700-2500, 3050, 3010, 2920, 2860, 1725, 1605, 1510, 1230, 1160, 1125, 1100, 1030, 975, 950, 895, 830, 820 und 750 $cm^{-1}$.

**Beispiel 386**

(1S,4R,5S,6S)-7-[4-(4-Fluorphenyl)-6-(3(S)-hydroxy-4(RS)-phenyl-1(E)-pentenyl)-2-oxabicyclo[2.2.1]hept-5-yl]-3-oxa-5(Z)-heptansäure-tert.Butylester (A) und (1S,4R,5S,6S)-7-[4-(4-Fluorphenyl)-6-(3(R)-hydroxy-4(RS)-phenyl-1(E)-pentenyl)-2-oxabicyclo[2.2.1]hept-5-yl]-3-oxa-5(Z)-heptensäure-tert.Butylester (B):

39 mg (73 µmol) des in Beispiel 386a dargestellten ungesättigten Ketons reduzierte man in Analogie zu Beispiel 23 und isolierte nach Aufarbeitung und Reinigung 34 mg (63 µmol, 87%) eines Gemisches der beiden Titelverbindungen als farbloses Öl.

IR (Film): 3600-3200, 3060, 3020, 2970, 2930, 2870, 1740, 1600, 1510, 1450, 1370, 1230, 1160, 1125, 1000, 970, 945, 895, 835, 735 und 700 $cm^{-1}$.

Die chromatographische Trennung lieferte 13,1 mg (24 µmol, 33%) des unpolareren Alkohols, dem man die Struktur A zuordnete, sowie 18,7 mg (35 µmol, 48%) des polareren Alkohols dem man die Struktur B zuordnete.

**Beispiel 386a**

(1S,4R,5S,6S)-7-[4-(4-Fluorphenyl)-6-(3-oxo-4(RS)-phenyl-1(E)-pentenyl)-2-oxabicyclo[2.2.1]hept-5-yl]-3-oxa-5(Z)-heptensäure-tert.butylester:

56,8 mg (140 µmol) des in Beispiel 286b dargestellten Aldehyds setzte man in Analogie zu Beispiel 23a unter Verwendung von Dimethyl-(2-oxo-3(RS)-phenyl-butyl)-phosphonat um und isolierte nach Aufarbeitung und Reinigung 39 mg (73 µmol, 52%) der Titelverbindung als farbloses Öl.

IR (Film): 3060, 3020, 2970, 2930, 2870, 1740, 1690, 1665, 1620, 1600, 1510, 1450, 1370, 1230, 1160, 1125, 1040, 1000, 975, 940, 900, 835 und 700 $cm^{-1}$.

**Beispiel 387**

(1S,4R,5S,6S)-7-[4-(4-Fluorphenyl)-6-(3(R)-hydroxy-4(RS)-phenyl)-1(E)-pentenyl)-2-oxabicyclo[2.2.1]hept-5-yl]-3-oxa-5(Z)-heptensäure:

18,7 mg (35 µmol) des nach Beispiel 386 dargestellten polaren Esters B verseifte man in Analogie zu Beispiel 2 und isolierte nach Aufarbeitung und Reinigung 12,8 mg (27 µmol, 76%) der Titelver-bindung als farbloses Öl.

IR (Film): 3600-3200, 3080, 3050, 3020, 2970, 2930, 2870, 1730, 1610, 1510, 1450, 1230, 1160, 1110, 1015, 995, 970, 895, 835 und 700 $cm^{-1}$.

**Beispiel 388**

(1S,4R,5S,6S)-7-[4-(4-Fluorphenyl)-6-(3(S)-hydroxy-4(RS)-phenyl-1(E)-pentenyl)-2-oxabicyclo[2.2.1]hept-5-yl]-3-oxa-5(Z)-heptensäure:

13,1 mg (24 µmol) des nach Beispiel 386 dargestellten unpolaren Esters A verseifte man in Analogie zu Beispiel 2 und isolierte nach Aufarbeitung und Reinigung 8,5 mg (18 µmol, 74%) der Titelver-bindung als farbloses Öl.

IR (Film): 3600-3200, 3080, 3060, 3020, 2970, 2930, 2870, 1725, 1605, 1510, 1450, 1425, 1230, 1160, 1105, 1015, 995, 940, 970, 895, 835 und 700 $cm^{-1}$.

## Patentansprüche

### Patentansprüche für folgende Vertragsstaaten : AT, BE, CH, DE, FR, GB, IT, LI, LU, NL, SE

1. 2-Oxa-bicyclo{2.2.1}heptanderivate der Formel I,

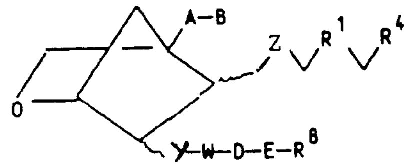

(I),

sowie deren Enantiomere,

worin

A $-(CH_2)_n-$, (E)- oder (Z)-CH=CH-, oder -C≡C-,

n 0-7,

B $C_1$-$C_{10}$-Alkyl, $-OR^2$, Halogen, -C≡N, $-N_3$, $-COOR^3$,

mit der Einschränkung, daß wenn B $OR^2$, halogen oder $N_3$ ist und $A=(CH_2)_n$ ist, n > O Sein muß

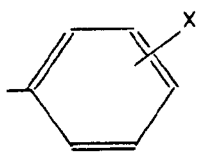

,

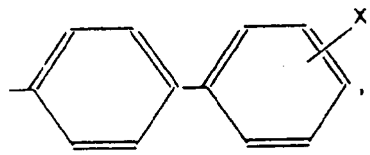

,

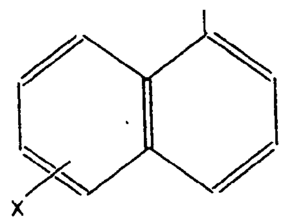

,

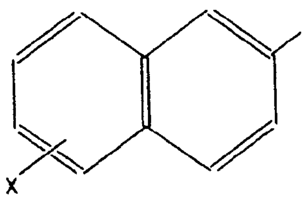

,

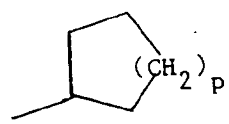

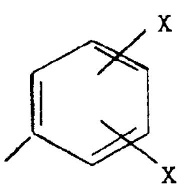

,

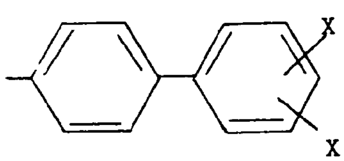

,

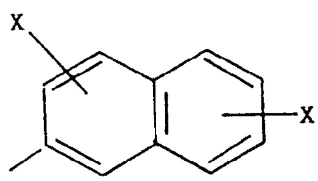

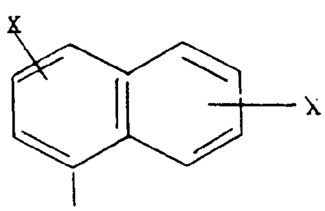

oder

einen 5- oder 6-gliedrigen heterocyclischen Rest mit wenigstens einem N, O-oder S-Atom,

$R^1$ Sauerstoff, eine Direktbindung oder eine $-CH_2$-Gruppe,

$R^2$ Wasserstoff, $C_1$-$C_{10}$-Alkyl, durch X substituiertes $C_6$-$C_{12}$-Aryl oder durch X substituiertes $C_7$-$C_{16}$-Aralkyl,

$R^3$ Wasserstoff, $C_1$-$C_{10}$-Alkyl, $C_5$-$C_6$-Cycloalkyl, $C_6$-$C_{12}$-Aryl oder $C_7$-$C_{16}$-Aralkyl,

X Wasserstoff, $C_1$-$C_5$-Alkyl,

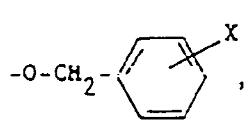

,

$-OR^2$, Halogen, $-C{\equiv}N$, $-N_3$, $-NO_2$, $-COOR^3$, Trifluormethyl, oder Phenyl für den Fall $R_8$ eine Gruppe

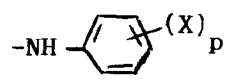

bedeutet,

$R^4$ $-COOR^5$, wobei $R^5$ Wasserstoff oder gegebenenfalls durch Halogen, Phenyl, $C_1$-$C_4$-Alkoxy oder Di-($C_1$-$C_4$)-alkylamino substituiertes $C_1$-$C_{10}$-Alkyl, $C_5$-$C_6$-Cycloalkyl, $C_7$-$C_{16}$-Aralkyl, durch X substituiertes Phenacyl oder $C_6$-$C_{12}$-Aryl oder einen 5- oder 6-gliedrigen heterocyclischen Rest mit wenigstens einem N-, O- oder S-Atom bedeuten kann, oder $-CONHR^6$ mit $R^6$ in der Bedeutung Wasserstoff, $C_1$-$C_{10}$-Alkanoyl oder $C_1$-$C_{10}$-Alkansulfonyl oder $CONR^2R^3$ oder der Rest

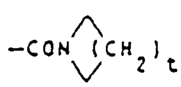

oder den

Rest 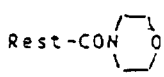

sein kann,

t 2 oder 3,

Z,y unabhängig voneinander $-(CH_2)_p-$, (E)-CH=CH, $-CH=CR^7-$, $-C{\equiv}C$,

p 0 bis 5,

W eine Direktbindung, die Gruppe

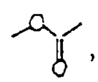,

die Gruppe

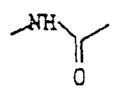,

die Gruppe

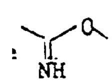,

eine freie oder funktionell abgewandelte Hydroxymethylengruppe oder eine freie oder funktionell abgewandelte

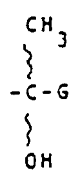 Gruppe.

wobei die OH-Gruppe jeweils α- oder β-ständig sein kann,

D eine Direktbindung, die Gruppe

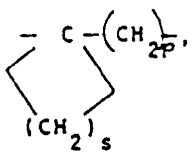

die Gruppe

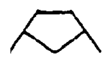

die Gruppe

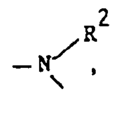

die Gruppe $-NH-SO_2^-$,
eine geradkettige
gesättigte Alkylengruppe mit 1-5 C-Atomen, eine verzweigte gesättigte oder eine geradkettige oder verzweigte ungesättigte Alkylengruppe mit 2-5 C-Atomen, die gegebenenfalls durch Fluoratome substituiert sein können,

s 2 bis 4

E eine Direktbindung, -C≡C- oder $-CH=CR^7-$,

wobei $R^7$ Wasserstoff, $C_1$-$C_5$-Alkyl, Halogen, oder Trifluormethyl,

$R^8$ Wasserstoff, $C_1$-$C_{10}$-Alkyl, $C_3$-$C_{10}$-Cycloalkyl, gegebenenfalls durch X substituiertes $C_7$-$C_{16}$-Aralkyl, gegebenenfalls durch X substituiertes $C_6$-$C_{12}$-Aryl oder einen 5- oder 6-gliedrigen heterocyclischen Rest mit wenigstens einem N-, O- oder S-Atom oder, falls E eine Direktbindung darstellt ,

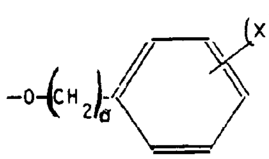 , 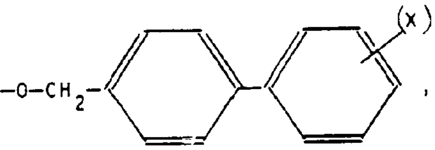 , 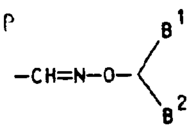 ,

$-O-Si(B^3)_3$,

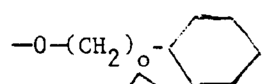 ,

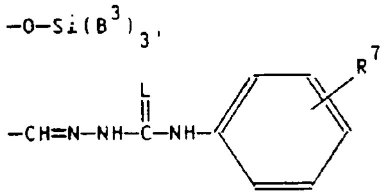 , 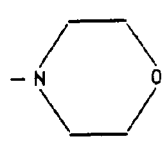 , 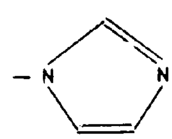 , 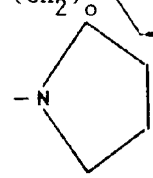 , 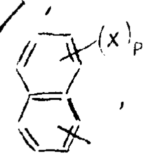 ,

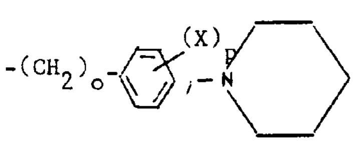 , 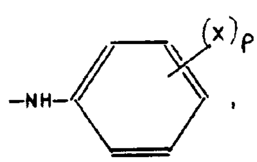 , 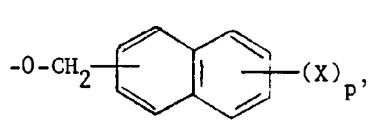

$-O-(C_1-C_8)$-Alkyl, 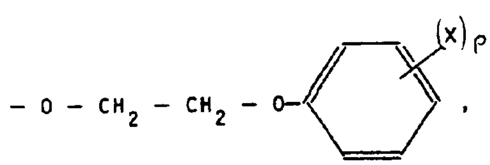 , 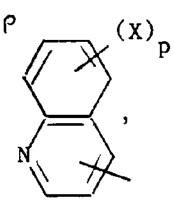 , 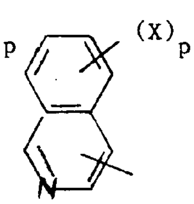 ,

$-O-(CH_2)_S-L-(CH_2)_o-X$, wobei in den Resten $(X)_p$, wenn p=2 oder 3 ist, X gleich oder verschieden sein kann,
o 0 bis 5,
$B^1$ und $B^2$
gleich oder verschieden sind und mit Ausnahme von Halogen $OR^2$,$-C\equiv N$,$N_3$ und $COOR^3$ B bedeuten und $B^3$ gleich oder verschieden sind und $C_1$-$C_4$-Alkyl, Phenyl oder $C_7$-$C_{16}$-Aralkyl bedeuten,
L Sauerstoff oder Schwefel und, falls $R^5$ Wasserstoff bedeutet, deren Salze mit physiologisch verträglichen Basen bedeuten, sowie die α-, ß- oder γ-Cyclodextrinclathrate sowie die mit Liposomen verkapselten Verbindungen der Formel I.

2. Arzneimittel bestehend aus einer oder mehreren Verbindungen des Anspruches 1 und üblichen Hilfs-, Träger- und Zusatzstoffen.

3. Verfahren zur Herstellung von 2-Oxa-bicyclo[2.2.1]heptanderivaten der Formel I, dadurch gekennzeichnet, daß man die 6-Hydroxymethyl-Verbindungen der Formel II,

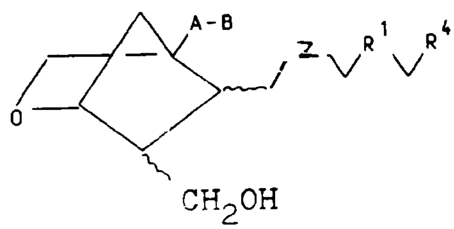

(II),

worin A,B,Z und $R^1$ die oben angegebenen Bedeutungen haben und $R^4$ eine $-COOR^5$-Estergruppe mit $R^5$ in der mit Ausnahme von Wasserstoff oben angegebenen Bedeutung darstellt, mit einer Halogenverbindung der Formel III,

$$Hal-W-D-E-R^8 \quad (III),$$

worin Hal Brom oder Chlor bedeuten und W,D,E und $R^8$ die oben genannten Bedeutungen haben oder nach Oxidation mit Oxalylchlorid/DMSO mit einem Dimethylphosphonat der Formel V,

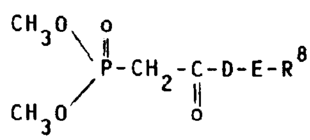

(V)

worin D,E und $R^8$ die oben genannte Bedeutung haben, in Gegenwart von NaH oder NaH/$Br_2$ umsetzt und anschließend reduziert und gegebenenfalls HBr abspaltet oder
das Oxidationsprodukt aus II und Oxalylchlorid/DMSO mit einem Amin der Formel IX,

$$H_2N-R^{10} \quad (IX),$$

worin $R^{10}$ die Reste $-O-CH(C_6H_5)_2$ oder

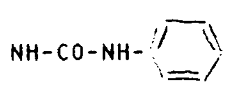

bedeutet oder mit $CBr_4$/Triphenylphosphin umsetzt und anschließend mit einer Verbindung der Formel X,

$$H-R^8 \quad (X),$$

worin $R^8$ die oben angegebene Bedeutung hat, umsetzt oder nach Oxidation und Umsetzung mit $SOCl_2$ und $NaN_3$ das intermediäre Amin mit einer Halogenverbindung der Formel III umsetzt und die erhaltenen Ester verseift, in Salze überführt und in Cyclodextrinclathrate umwandelt oder mit Liposomen verkapselt.

**Patentansprüche für folgende Vertragsstaaten : GR, ES**

1. Verfahren zur Herstellung von 2-Oxa-bicyclo[2.2.1]heptanderivaten der Formel I.

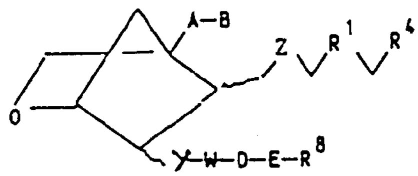

(I),

sowie deren Enantiomeren,

worin

A $-(CH_2)_n-$, (E)- oder (Z)-CH=CH-, oder -C≡C-,

n 0-7,

B $C_1$-$C_{10}$-Alkyl, $-OR^2$, Halogen, -C≡N, $-N_3$, $-COOR^3$,

mit der Einschränkung, daß wenn B $OR^2$, Halogen oder $N_3$ ist und $A=-(CH_2)_n$ ist, n > O Sein muß

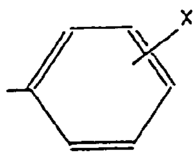

,

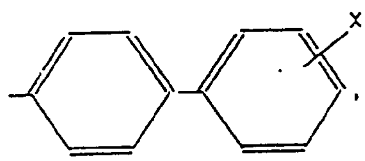

,

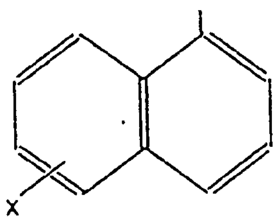

,

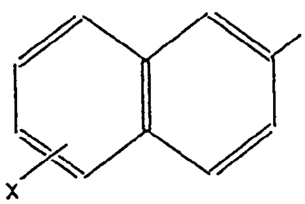

,

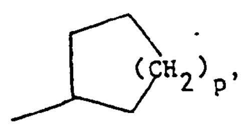

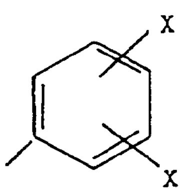

,

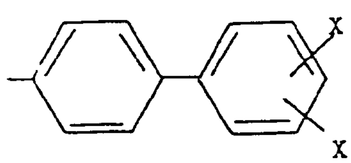

,

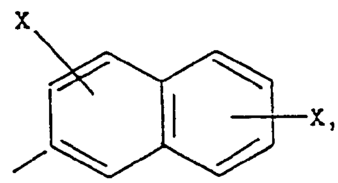

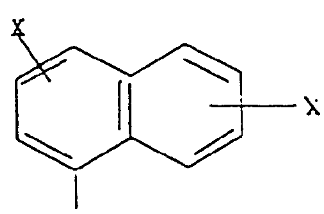

oder

einen 5- oder 6-gliedrigen heterocyclischen Rest mit wenigsten einem N, O-oder S-Atom,

$R^1$ Sauerstoff, eine Direktbindung oder eine $-CH_2$-Gruppe,

$R^2$ Wasserstoff, $C_1$-$C_{10}$-Alkyl, durch X substituiertes $C_6$-$C_{12}$-Aryl oder durch X substituiertes $C_7$-$C_{16}$-Aralkyl,

$R^3$ Wasserstoff, $C_1$-$C_{10}$-Alkyl, $C_5$-$C_6$-Cycloalkyl, $C_6$-$C_{12}$-Aryl oder $C_7$-$C_{16}$-Aralkyl,

X Wasserstoff, $C_1$-$C_5$-Alkyl,

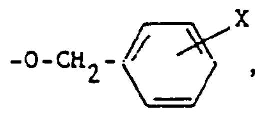 ,

-$OR^2$, Halogen, -C≡N, -$N_3$, -$NO_2$, -$COOR^3$, trifluormethyl oder Phenyl für den Fall $R_8$ eine Gruppe

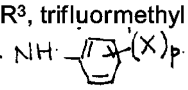

bedeutet,

$R^4$, -$COOR^5$, wobei $R^5$ Wasserstoff oder gegebenenfalls durch Halogen, Phenyl, $C_1$-$C_4$-Alkoxy oder Di-($C_1$-$C_4$)-alkylamino substituiertes $C_1$-$C_{10}$-Alkyl, $C_5$-$C_6$-Cycloalkyl, $C_7$-$C_{16}$-Aralkyl, durch X substituiertes Phenacyl oder $C_6$-$C_{12}$-Aryl oder einen 5- oder 6-gliedrigen heterocyclischen Rest mit wenigstens einem N-, O- oder S-Atom bedeuten kann, oder -$CONHR^6$ mit $R^6$ in der Bedeutung Wasserstoff, $C_1$-$C_{10}$-Alkanoyl oder $C_1$-$C_{10}$-Alkansulfonyl oder $CONR^2R^3$ oder der Rest

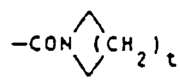

oder den

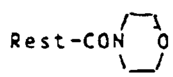

sein kann,

t 2 oder 3,

Z,y unabhängig voneinander -$(CH_2)_p$-, (E)-CH=CH, -CH=$CR^7$-, -C≡C,

p 0 bis 5,

W eine Direktbindung, die Gruppe

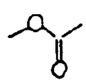 ,

die Gruppe

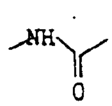 ,

die Gruppe

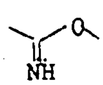 ,

eine freie oder funktionell abgewandelte hydroxymethylengruppe oder eine freie oder funktionell abgewandelte

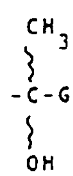 Gruppe,

wobei die OH-Gruppe jeweils α- oder β-ständig sein kann,

D eine Direktbindung, die Gruppe

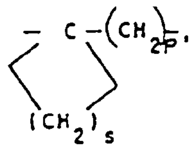

die Gruppe

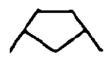 ,

die Gruppe

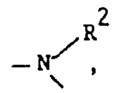

die Gruppe $-NH-SO_2^-$,
eine geradkettige
gesättigte Alkylengruppe mit 1-5 C-Atomen, eine verzweigte gesättigte oder eine geradkettige oder verzweigte ungesättigte Alkylengruppe mit 2-5 C-Atomen, die gegebenenfalls durch Fluoratome substituiert sein können,

s 2 bis 4

E eine Direktbindung, -C≡C- oder $-CH=CR^7-$,

wobei $R^7$ Wasserstoff, $C_1$-$C_5$-Alkyl, Halogen, oder Trifluormethyl,

$R^8$ Wasserstoff, $C_1$-$C_{10}$-Alkyl, $C_3$-$C_{10}$-Cycloalkyl, gegebenenfalls durch X subsituiertes $C_7$-$C_{16}$-Aralkyl, gegebenenfalls durch X substituiertes $C_6$-$C_{12}$-Aryl oder einen 5- oder 6-gliedrigen heterocyclischen Rest mit wenigstens einem N-, O- oder S-Atom oder, falls E eine Direktbindung darstellt,

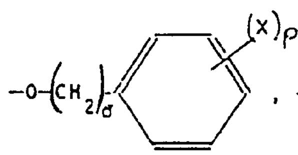 , 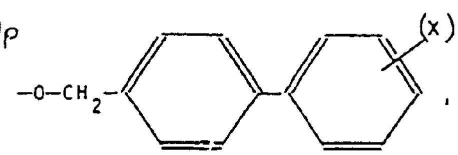 , 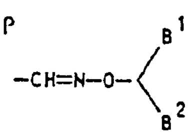 ,

$-O-Si(B^3)_3$, 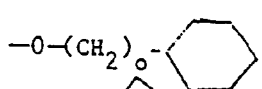 ,

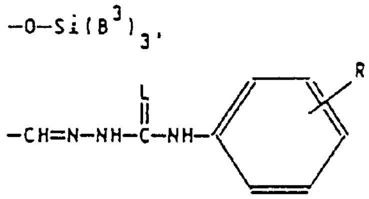 , 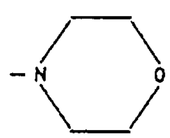 , 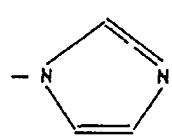 , 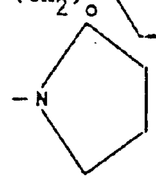 , 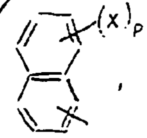 ,

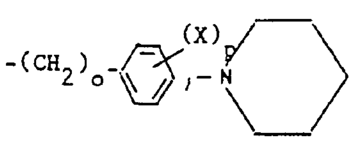 , 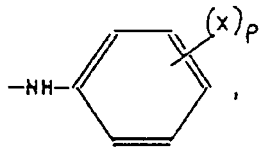 , 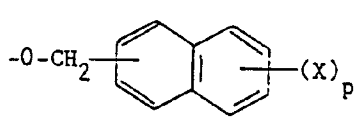

$-O-(C_1-C_8)$-Alkyl, $-O-CH_2-CH_2-O-$ 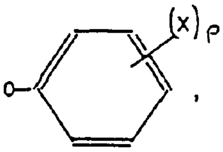 , 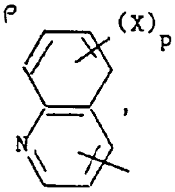 , 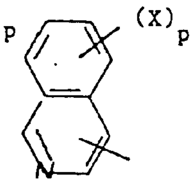 ,

$-O-(CH_2)_S-L-(CH_2)_o-X$, wobei in den Resten $-(X)_p$, wenn p=2 oder 3 ist, X gleicht oder verschieden sein kann,

o 0 bis 5,

$B^1$ und $B^2$

gleich oder verschieden sind und mit Ausnahme von Halogen $OR^2$,$-C{\equiv}N$,$N_3$ und $COOR^3$ B bedeuten und $B^3$ gleich oder verschieden sind und $C_1$-$C_4$-Alkyl, Phenyl oder $C_7$-$C_{16}$-Aralkyl bedeuten,

L Sauerstoff oder Schwefel darstellen,

dadurch gekennzeichnet, daß man die 6-Hydroxymethyl-Verbindungen der Formel II,

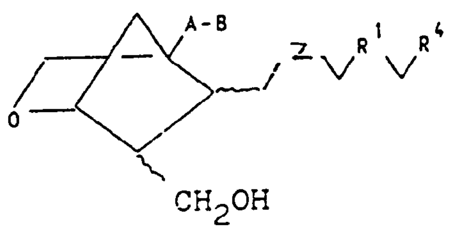 (II),

worin A,B,Z und $R^1$ die oben angegebenen Bedeutungen haben und $R^4$ eine $-COOR^5$-Estergruppe mit $R^5$ in der mit Ausnahme von Wasserstoff oben angegebenen Bedeutung darstellt, mit einer Halogenverbindung der Formel III,

Hal-W-D-E-$R^8$ (III),

worin Hal Brom oder Chlor bedeuten und W, D, E und $R^8$ die oben genannten Bedeutungen haben oder nach Oxidation mit Oxalylchlorid/DMSO mit einem Dimethyphosphonat der Formel V,

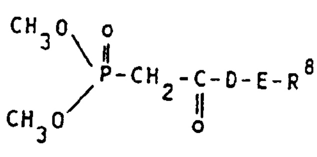 (V)

worin D, E und $R^8$ die oben genannte Bedeutung haben, in Gegenwart von NaH oder NaH/$Br_2$ umsetzt und anschließend reduziert und gegebenenfalls HBr abspaltet oder

das Oxidationsprodukt aus II und Oxalylchlorid/DMSO mit einem Amin der Formel IX,

$H_2N-R^{10}$ (IX),

worin $R^{10}$ die Reste $-O-CH(C_6H_5)_2$ oder

NH-CO-NH- 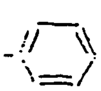

bedeutet oder mit $CBr_4$/Triphenylphosphin umsetzt und anschließend mit einer Verbindung der Formel X,

H-$R^8$ (X),

worin $R^8$ die oben angegebene Bedeutung hat, umsetzt oder nach Oxidation und Umsetzung mit $SOCl_2$ und $NaN_3$ das intermediäte Amin mit einer Halogenverbindung der Formel III umsetzt und die erhaltenen Ester verseift, in Salze überführt und in Cyclodextrinclathrate umwandelt oder mit Liposomen verkapselt.

## Claims

### Claims for the following Contracting States : AT, BE, CH, DE, FR, GB, IT, LI, LU, NL, SE

1. 2-oxabicyclo[2.2.1]heptane derivatives of the formula I

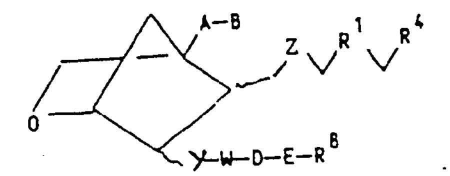

(I),

and the enantiomers thereof,
wherein

A represents $-(CH_2)_n-$, (E)- or (Z)-CH=CH-,or -C≡C-,

n represents 0 to 7,

B represents $C_1$-$C_{10}$-alkyl, $-OR^2$, halogen, -C≡N, $-N_3$ or $-COOR^3$, with the proviso that, when B is $OR^2$, halogen or $N_3$ and A is $-(CH_2)_n-$, n must be greater than zero, or B represents

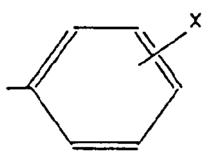 , 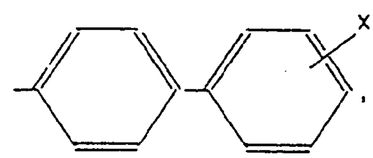 , 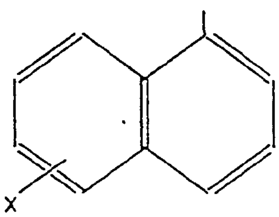 ,

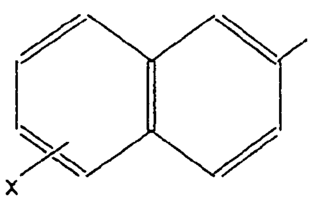 , 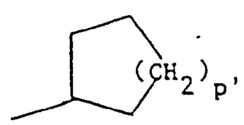

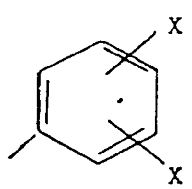 , 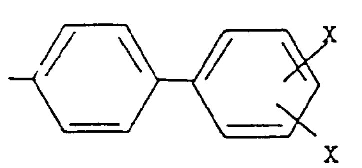 ; 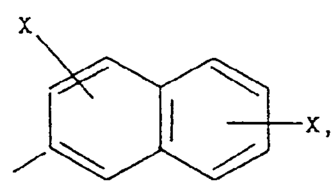

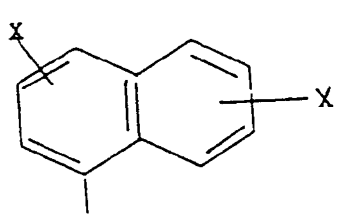

or

a 5- or 6-membered heterocyclic radical having at least one nitrogen, oxygen or sulphur atom,

$R^1$ represents oxygen, a direct bond or a $-CH_2$ group,

$R^2$ represents hydrogen, $C_1$-$C_{10}$-alkyl, $C_6$-$C_{12}$-aryl substituted by X or $C_7$-$C_{16}$-aralkyl substituted by X,

$R^3$ represents hydrogen, $C_1$-$C_{10}$-alkyl, $C_5$-$C_6$-cycloalkyl, $C_6$-$C_{12}$-aryl or $C_7$-$C_{16}$-aralkyl,
X represents hydrogen, $C_1$-$C_5$-alkyl,

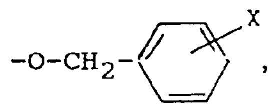

-$OR^2$, halogen -C≡N, -$N_3$, -$NO_2$, -$COOR^3$ trifluoromethyl, or X represents phenyl when $R^8$ represents a group

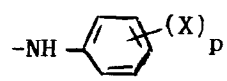

$R^4$ may represent -$COOR^5$, wherein $R^5$ may represent hydrogen or $C_1$-$C_{10}$-alkyl that is optionally substituted by halogen, phenyl, $C_1$-$C_4$-alkoxy or by di-($C_1$-$C_4$)-alkylamino, or $R^5$ may represent $C_5$-$C_6$-cycloalkyl, $C_7$-$C_{16}$-aralkyl, phenacyl substituted by X or $C_6$-$C_{12}$-aryl substituted by X, or a 5- or 6-membered heterocyclic radical having at least one nitrogen, oxygen or sulphur atom, or $R^4$ may represent -$CONHR^6$, wherein $R^6$ represents hydrogen, $C_1$-$C_{10}$-alkanoyl or $C_1$-$C_{10}$-alkanesulphonyl, or $R^4$ may represent $CONR^2R^3$ or the radical

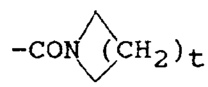

or the radical

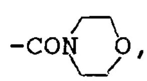

t represents 2 or 3,
each of Z and Y, independently of the other, represents -$(CH_2)_p$-, (E)-CH=CH, -$CH=CR^7$- or -C≡C,
p represents 0 to 5,
W represents a direct bond, the group

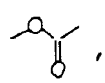 ,

the group

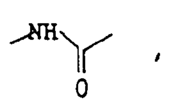 ,

the group

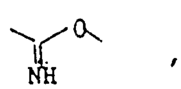 ,

a free or functionally modified hydroxymethylene group or a free or functionally modified

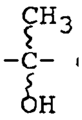

group, wherein the hydroxy group may in each case be in the α- or β- configuration,

D represents a direct bond, the group

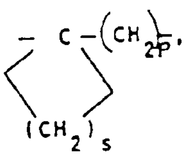

the group

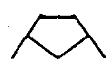 ,

the group

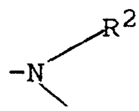 ,

the group $-NH-SO_2-$, or a straight-chain saturated alkylene group having from 1 to 5 carbon atoms or a branched saturated or a straight-chain or branched unsaturated alkylene group having from 2 to 5 carbon atoms, which may optionally be substituted by fluorine atoms,

s represents 2 to 4,

E represents a direct bond, -C≡C- or $-CH=CR^7-$, wherein $R^7$ represents hydrogen, $C_1$-$C_5$-alkyl, halogen or trifluoromethyl,

$R^8$ represents hydrogen, $C_1$-$C_{10}$-alkyl, $C_3$-$C_{10}$-cycloalkyl, $C_7$-$C_{16}$-aralkyl optionally substituted by X, $C_6$-$C_{12}$-aryl optionally substituted by X, or a 5- or 6-membered heterocyclic radical having at least one nitrogen, oxygen or sulphur atom, or, if E is a direct bond,

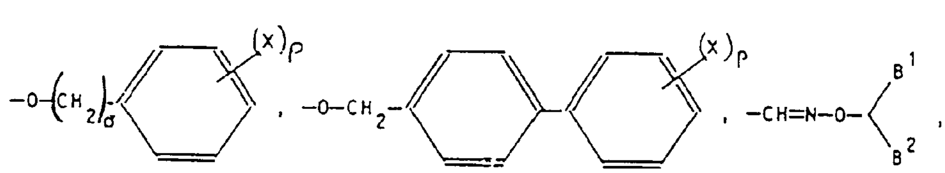

$-O-Si(B^3)_3$,

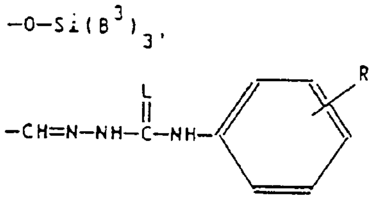 , 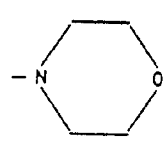 , 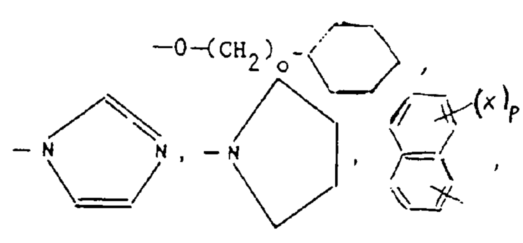

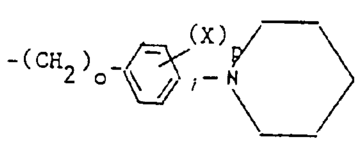, 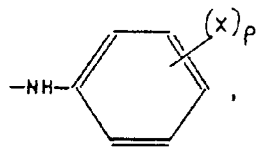, 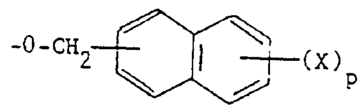,

$-O-(C_1-C_8)$-alkyl, 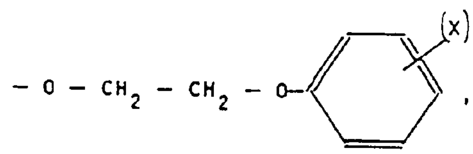, 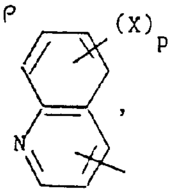, 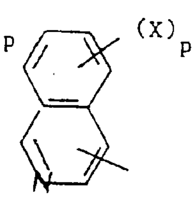,

or $-O-(CH_2)_S-L-(CH_2)_o-X$, wherein, in the radicals $-(X)p$, when p is 2 or 3, X may be identical or different, o represents 0 to 5,

$B^1$ and $B^2$, which may be identical or different, represent B except that they cannot be halogen, $OR^2$, $-C{\equiv}N$, $N_3$ or $COOR^3$, and each $B^3$ is identical to or different from the others and represents $C_1$-$C_4$-alkyl, phenyl or $C_7$-$C_{16}$-aralkyl,

L represents oxygen or sulphur,

and, if $R^5$ represents hydrogen, the salts thereof with physiologically tolerable bases, and the α-, β- or γ-cyclodextrin clathrates, and the compounds of the formula I encapsulated by liposomes.

2. Medicament comprising one or more compounds of claim 1 and customary adjuvants, carriers and additives.

3. Process for the preparation of 2-oxabicyclo[2.2.1]heptane derivatives of the formula I, characterised in that the 6-hydroxymethyl compounds of the formula II

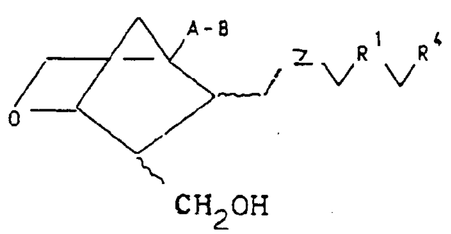

(II),

wherein A, B, Z and $R^1$ are as defined above and $R^4$ represents a $-COOR^5$ ester group wherein $R^5$ is as defined above except that it cannot represent hydrogen, are reacted with a halogen compound of the formula III

$$Hal-W-D-E-R^8 \quad (III),$$

wherein Hal represents bromine or chlorine and W, D, E and $R^8$ are as defined above, or, after oxidation with oxalyl chloride/DMSO, they are reacted with a dimethyl phosphonate of the formula V

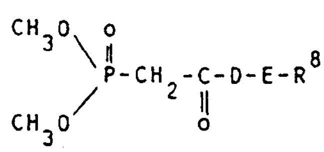

(V),

wherein D, E and $R^8$ are as defined above, in the presence of NaH or NaH/$Br_2$, and then the product is reduced and HBr is, where appropriate, split off, or

the oxidation product of II and oxalyl chloride/DMSO is reacted with an amine of the formula IX

$$H_2N-R^{10} \quad (IX),$$

wherein $R^{10}$ represents the radicals $-O-CH(C_6H_5)_2$ or

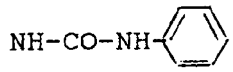

or
with $CBr_4$/triphenylphosphine and then with a compound of the formula X

H-$R^8$ (X),

wherein $R^8$ is as defined above, or, after oxidation and reaction with $SOCl_2$ and $NaN_3$, the intermediate amine is reacted with a halogen compound of the formula III and the resulting esters are hydrolysed, converted into salts and formed into cyclodextrin clathrates or encapsulated by liposomes.

**Claim for the following Contracting States : GR, ES**

1. Process for the preparation of 2-oxabicyclo[2.2.1]heptane derivatives of the formula I

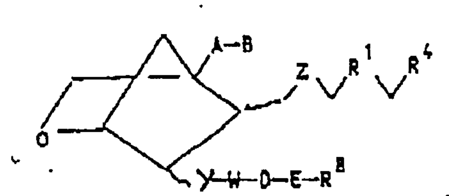

(I),

and the enantiomers thereof,
wherein

A represents $-(CH_2)_n-$, (E)- or (Z)-CH=CH-, or -C≡C-,

n represents 0 to 7,

B represents $C_1$-$C_{10}$-alkyl, or $OR^2$, halogen, -C≡N, $-N_3$ or $-COOR^3$, with the proviso that, when B is $OR^2$, halogen or $N_3$ and A is $-(CH_2)_n-$, n must be greater than zero, or B represents

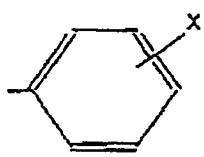
,
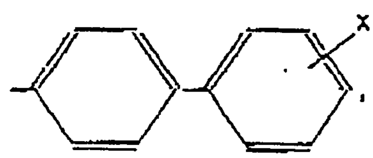
,
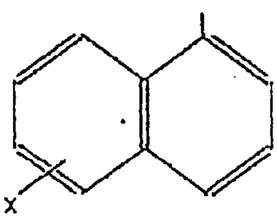
,

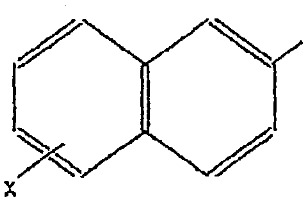
,
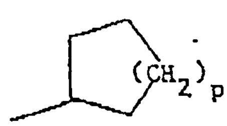

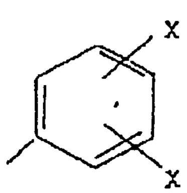
,
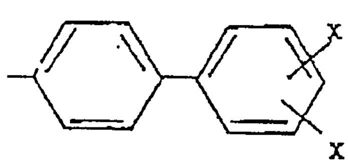
,
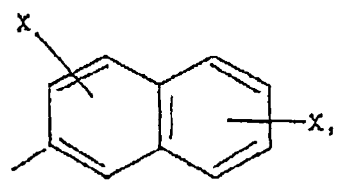

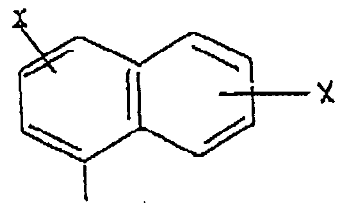

or

a 5- or 6-membered heterocyclic radical having at least one nitrogen, oxygen, or sulphur atom,

$R^1$ represents oxygen, a direct bond or a $-CH_2$ group,

$R^2$ represents hydrogen, $C_1$-$C_{10}$-alkyl, $C_6$-$C_{12}$-aryl substituted by X or $C_7$-$C_{16}$-aralkyl substituted by X,

$R^3$ represents hydrogen, $C_1$-$C_{10}$-alkyl, $C_5$-$C_6$-cycloalkyl, $C_6$-$C_{12}$-aryl or $C_7$-$C_{16}$-aralkyl,

X represents hydrogen, $C_1$-$C_5$-alkyl,

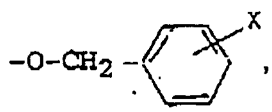 ,

$-OR^2$, halogen, -C≡N, $-N_3$, $-NO_2$, $-COOR^3$, trifluoromethyl, or X represents phenyl when $R^8$ represents a group

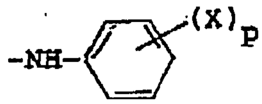

$R^4$ may represent $-COOR^5$, wherein $R^5$ may represent hydrogen or $C_1$-$C_{10}$-alkyl that is optionally substituted by halogen, phenyl, $C_1$-$C_4$-alkoxy or by di-($C_1$-$C_4$)-alkylamino, or $R^5$ may represent $C_5$-$C_6$-cycloalkyl, $C_7$-$C_{16}$-aralkyl, phenacyl substituted by X or $C_6$-$C_{12}$-aryl substituted by X, or a 5- or 6-membered heterocyclic radical having at least one nitrogen, oxygen or sulphur atom, or $R^4$ may represent $-CONHR^6$, wherein $R^6$ represents hydrogen, $C_1$-$C_{10}$-alkanoyl or $C_1$-$C_{10}$-alkanesulphonyl, or $R^4$ may represent $CONR^2R^3$ or the radical

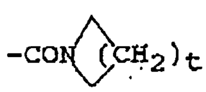

or the radical

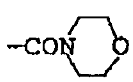

t represents 2 or 3,

each of Z and Y, independently of the other, represents $-(CH_2)_p-$, (E)-CH=CH, $-CH=CR^7-$ or -C≡C,

p represents 0 to 5,

W represents a direct bond, the group

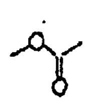 ,

the group

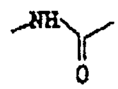 ,

the group

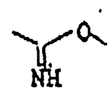 ,

a free or functionally modified hydroxymethylene group or a free or functionally modified

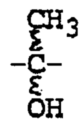

group, wherein the hydroxy group may in each case be in the α- or β-configuration,

D represents a direct bond, the group

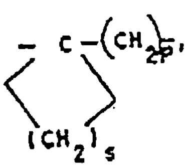

the group

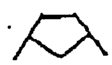 ,

the group

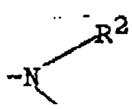 ,

the group $-NH-SO_2-$, or a straight-chain saturated alkylene group having from 1 to 5 carbon atoms or a branched saturated or a straight-chain or branched unsaturated alkylene group having from 2 to 5 carbon atoms, which may optionally be substituted by fluorine atoms,

s represents 2 to 4,

E represents a direct bond, -C≡C- or $-CH=CR^7$, wherein $R^7$ represents hydrogen, $C_1$-$C_5$-alkyl, halogen or trifluoromethyl,

$R^8$ represents hydrogen, $C_1$-$C_{10}$-alkyl, $C_3$-$C_{10}$-cycloalkyl, $C_7$-$C_{16}$-aralkyl optionally substituted by X, $C_6$-$C_{12}$-aryl optionally substituted by X, or a 5- or 6-membered heterocyclic radical having at least one nitrogen, oxygen or sulphur atom, or, if E is a direct bond,

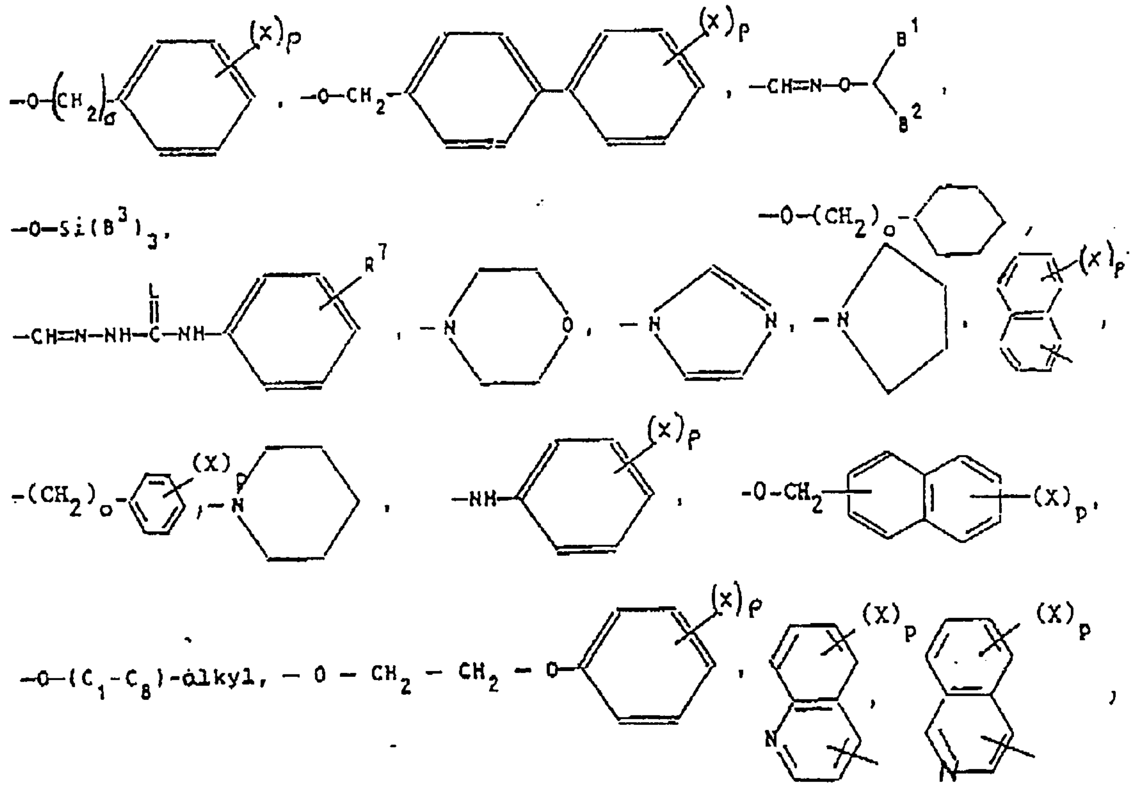

or -O-$(CH_2)_S$-L-$(CH_2)_o$-X, wherein, in the radicals -$(X)_p$, when p is 2 or 3, X may be identical or different, o represents 0 to 5,

$B^1$ and $B^2$, which may be identical or different, represent B except that they cannot be halogen, $OR^2$, -C≡N, $N_3$ or $COOR^3$, and each $B^3$ is identical to or different from the others and represents $C_1$-$C_4$-alkyl, phenyl or $C_7$-$C_{16}$-aralkyl,

L represents oxygen or sulphur,

characterised in that the 6-hydroxymethyl compounds of the formula II

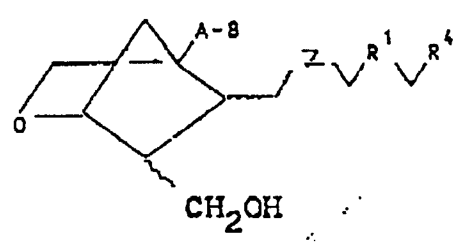

(II),

wherein A, B, Z and $R^1$ are as defined above and $R^4$ represents a -$COOR^5$ ester group wherein $R^5$ is a defined above except that it cannot represent hydrogen, are reacted with a halogen compound of the formula III

Hal-W-D-E-$R^8$ (III),

wherein Hal represents bromine or chlorine and W, D, E and $R^8$ are as defined above, or after oxidation with oxalyl chloride/DMSO, they are reacted with a dimethyl phosphonate of the formula V

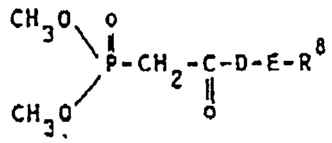 (V),

wherein D, E and $R^8$ are as defined above, in the presence of NaH or NaH/$Br_2$, and then the product is reduced and HBr is, where appropriate, split off, or

the oxidation product of II and oxalyl chloride/DMSO is reacted with an amine of the formula IX

$H_2N-R^{10}$ (IX),

wherein $R^{10}$ represents the radicals $-O-CH(C_6H_5)_2$ or

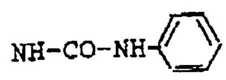

or

with $CBr_4$/triphenylphosphine and then with a compound of the formula X

$H-R^8$ (X),

wherein $R^8$ is a defined above, or, after oxidation and reaction with $SOCl_2$ and $NaN_3$, the intermediate amine is reacted with a halogen compound of the formula III and the resulting esters are hydrolysed, converted into salts and formed into cyclodextrin clathrates or encapsulated by lipsomes.

## Revendications

### Revendications pour les Etats contractants suivants : AT, BE, CH, DE, FR, GB, IT, LI, LU, NL, SE

1. Dérivés du 2-oxa-bicyclo[2.2.1]heptane de formule I :

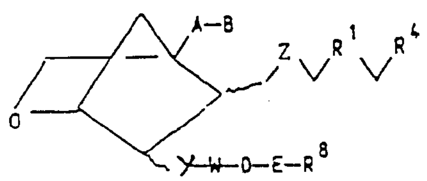 (I), (I)

et leurs énantiomères, formule dans laquelle :

A représente un radical $-(CH_2)_n-$, un radical -CH=CH- E ou Z, ou un radical -C≡C-,

n désigne un nombre de 0 à 7,

B représente un alkyle en $C_1$-$C_{10}$, $-OR^2$, un halogène, -C≡N, $-N_3$, $-COOR^3$, avec la restriction que, lorsque B représente $OR^2$, un halogène ou $-N_3$ et que A représente un radical $-(CH_2)_n-$, l'indice n soit supérieur à 0, ou B représente l'un des radicaux suivants :

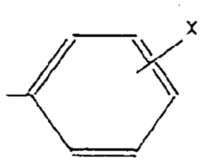 , 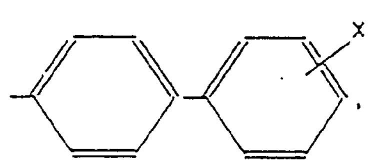 , 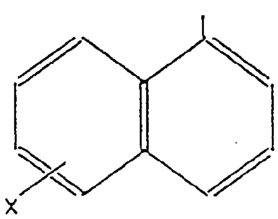 ,

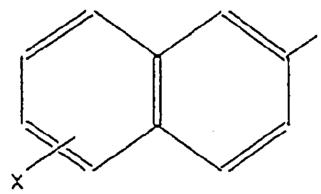

,

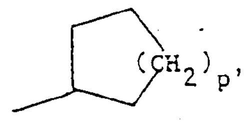

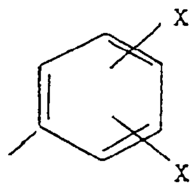

,

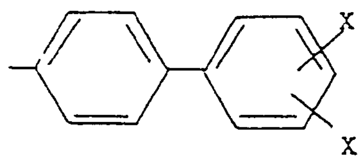

,

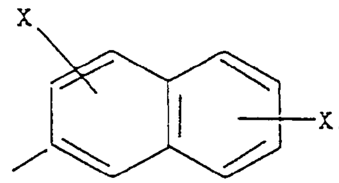

et

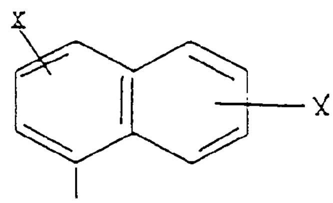

ou un radical hétérocyclique à 5 ou 6 maillons qui contient au moins un atome N, O ou S,

$R^1$ représente l'oxygène, une liaison directe ou un radical $-CH_2-$,

$R^2$ représente l'hydrogène, un alkyle en $C_1$-$C_{10}$, un aryle en $C_6$-$C_{12}$ porteur d'un X ou un aralkyle en $C_7$-$C_{16}$ porteur d'un X,

$R^3$ représente l'hydrogène, un alkyle en $C_1$-$C_{10}$, un cycloalkyle en $C_5$ ou $C_6$, un aryle en $C_6$-$C_{12}$ ou un aralkyle en $C_7$-$C_{16}$,

X représente l'hydrogène, un alkyle en $C_1$-$C_5$,

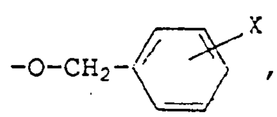

,

$-OR^2$, un halogène, -C≡N, $-N_3$, $-NO_2$, $-COOR^3$ ou un trifluorométhyle, ou encore un phényle si $R^8$ est un groupe

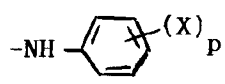

$R^4$ représente :

- un radical $-COOR^5$ dans lequel $R^5$ représente l'hydrogène, un alkyle en $C_1$-$C_{10}$ éventuellement porteur d'un halogène, d'un phényle, d'un alcoxy en $C_1$-$C_4$ ou d'un di-($C_1$-$C_4$)-alkylamino, un cycloalkyle en $C_5$ ou $C_6$, un aralkyle en $C_7$-$C_{16}$, un radical aryle en $C_6$-$C_{12}$ ou phénacyle porteur d'un X, ou un radical hétérocyclique à 5 ou 6 maillons qui renferme au moins un atome N, O ou S,
- un radical $-CONHR^6$ dans lequel $R^6$ représente l'hydrogène, un alcanoyle en $C_1$-$C_{10}$ ou un alcane-sulfonyle en $C_1$-$C_{10}$,
- un radical $-CONR^2R^3$,
- un radical

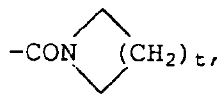

ou

- un radical

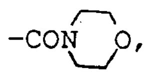

t est égal à 2 ou à 3,

Z et Y représentent chacun, indépendamment l'un de l'autre, un radical $-(CH_2)_p-$, -CH=CH- (E), $-CH=CR^7-$ ou -C≡C-,

p désigne un nombre de 0 à 5,

W représente une liaison directe, un radical

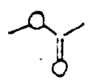 , 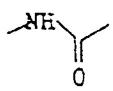

ou

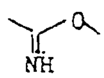 ,

un radical hydroxyméthylène libre ou sous la forme d'un dérivé fonctionnel, ou un radical

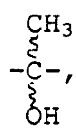

le radical OH pouvant à chaque fois avoir la configuration α ou la configuration β,

D représente une liaison directe, un radical

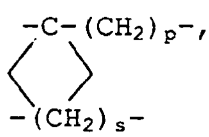

un radical

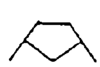 ,

un radical

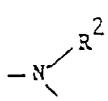 ,

un radical $-NH-SO_2-$, un alkylène saturé linéaire contenant de 1 à 5 atomes de carbone, un alkylène en

$C_2$-$C_5$ saturé ramifié ou un alkylène en $C_2$-$C_5$ insaturé linéaire ou ramifié, qui peuvent éventuellement porter des atomes de fluor,

s désigne un nombre de 2 à 4,

E représente une liaison directe, -C≡C- ou un radical $-CH=CR^7-$, le symbole $R^7$ représentant l'hydrogène, un alkyle en $C_1$-$C_5$, un halogène ou un trifluorométhyle,

$R^8$ représente l'hydrogène, un alkyle en $C_1$-$C_{10}$, un cycloalkyle en $C_3$-$C_{10}$, un aralkyle en $C_7$-$C_{16}$ éventuellement porteur d'un X, un aryle en $C_6$-$C_{12}$ éventuellement porteur d'un X, un radical hétérocyclique à 5 ou 6 maillons qui renferme au moins un atome N, O ou S, ou encore, dans le cas où E représente une liaison directe, $R^8$ peut représenter l'un des radicaux suivants :

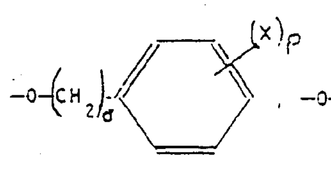 , 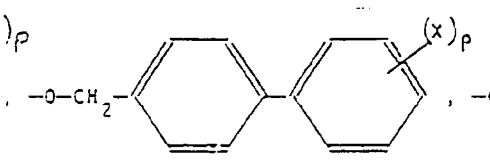 , 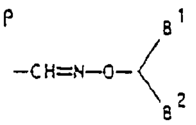 ,

$-O-Si(B^3)_3$,

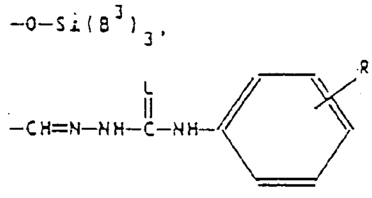 , 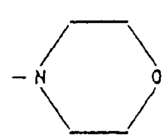 , 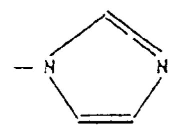 , 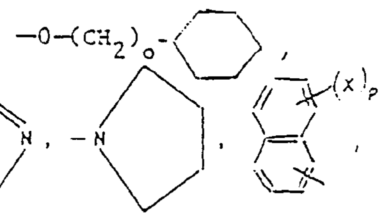 ,

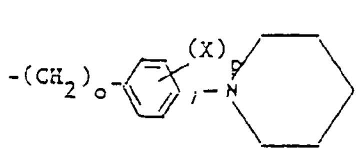 , 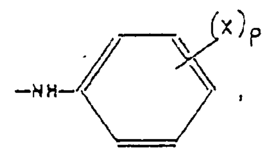 , 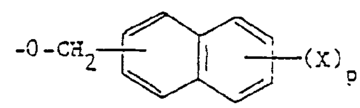 ,

$-O-(C_1-C_8)$-Alkyl, 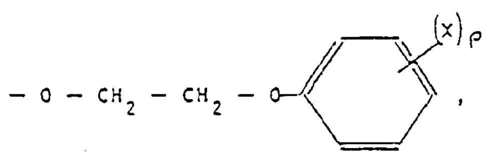 , 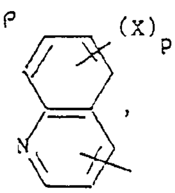 , 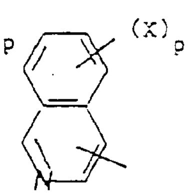 ,

et $-O-(CH_2)_s-L-(CH_2)_o-X$,

(lorsque p est égal à 2 ou à 3 dans les radicaux $-(X)_p$ les X peuvent être identiques ou différents),

o désigne un nombre de 0 à 5,

$B^1$ et $B^2$ sont identiques ou différents et ont les significations de B à l'exclusion des halogènes et des radicaux $-OR^2$, -C≡N, $-N_3$ et $-COOR^3$,

les $B^3$ représentent chacun, indépendamment l'un de l'autre, un alkyle en $C_1$-$C_4$, un phényle ou un aralkyle en $C_7$-$C_{16}$, et

L représente l'oxygène ou le soufre,

et, dans le cas où $R^5$ représente l'hydrogène, les sels que forment ces composés avec des bases acceptables du point de vue physiologique, ainsi que les clathrates d'α-, de β- ou de γ-cyclodextrine, et les composés de formule I encapsulés avec des liposomes.

2. Médicament constitué d'un ou de plusieurs composés selon la revendication 1 et d'adjuvants, excipients et additifs usuels.

3. Procédé pour préparer des dérivés du 2-oxabicyclo[2.2.1]heptane de formule I, procédé caractérisé en ce qu'on fait réagir des composés à radical hydroxyméthyle en 6 qui répondent à la formule II :

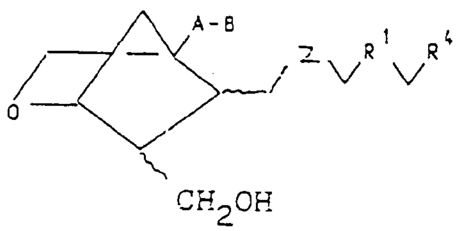

(II)

dans laquelle A, B, Z et $R^1$ ont les significations qui leur ont été données ci-dessus et $R^4$ représente un radical d'ester $-COOR^5$ dont le symbole $R^5$ a les significations indiquées ci-dessus à l'exception de l'hydrogène,
avec un composé halogéné répondant à la formule III :

$$Hal\text{-}W\text{-}D\text{-}E\text{-}R^8 \qquad (III)$$

dans laquelle Hal représente le brome ou le chlore, et W, D, E et $R^8$ ont les significations qui leur ont été données ci-dessus,
ou, après oxydation avec du chlorure d'oxalyle/DMSO, on fait réagir avec un phosphonate de diméthyle répondant à la formule V :

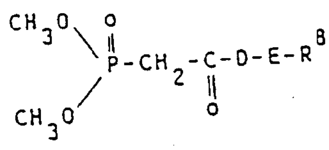

(V)

dans laquelle D, E et $R^8$ ont les significations qui leur ont été données plus haut,
en présence de NaH ou de $NaH/Br_2$, puis on réduit et, éventuellement, on élimine HBr,
ou on fait réagir le produit d'oxydation de II et du chlorur d'oxalyle/DMSO avec une amie répondant à la formule IX :

$$H_2N\text{-}R^{10} \qquad (IX)$$

dans laquelle $R^{10}$ représente un radical $-O-CH(C_6H_5)_2$ ou un radical

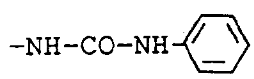 ,

ou avec $CBr_4$/triphénylphosphine, puis avec un composé répondant à la formule X :

$$H\text{-}R^8 \qquad (X)$$

dans laquelle $R^8$ a la signification indiquée ci-dessus,
ou, après oxydation et réaction avec $SOCl_2$ et $NaN_3$, on fait réagir l'amine intermédiaire avec un composé halogéné de formule III et on saponifie l'ester obtenu, on transforme en sels et on convertit en clathrates de cyclodextrine ou on encapsule avec des liposomes.

**Revendication pour les Etats contractants suivants : GR, ES**

1. Procédé de préparation de dérivés du 2-oxabicyclo[2.2.1]heptane de formule I

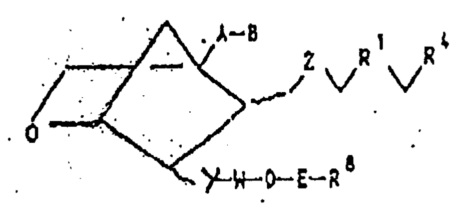

(I)

et de leurs énantiomères, formule dans laquelle :

A représente un radical $-(CH_2)_n-$, un radical -CH=CH- E ou Z, ou un radical -C≡N,
n désigne un nombre de 0 à 7,

B représente un alkyle en $C_1$-$C_{10}$, $-OR^2$, un halogène, -C≡N, $-N_3$, $-COOR^3$, avec la restriction que, lorsque B représente $OR^2$, un halogène ou $-N_3$ et que A représente un radical $-(CH_2)_n-$, l'indice n soit supérieur à 0, ou B représente l'un des radicaux suivants :

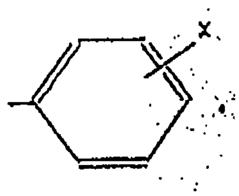

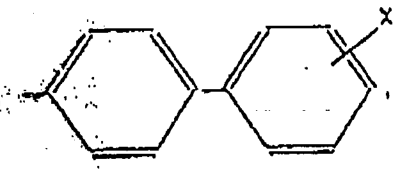,

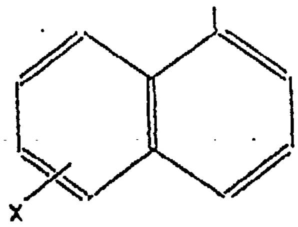,

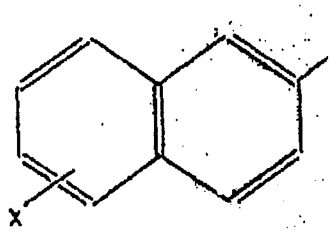,

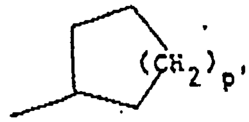

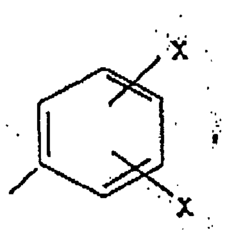

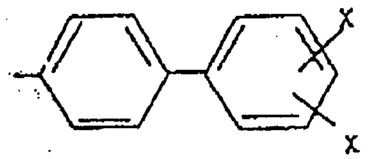

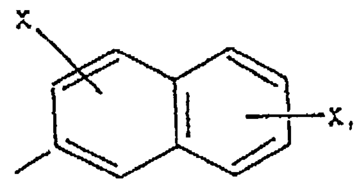,

et

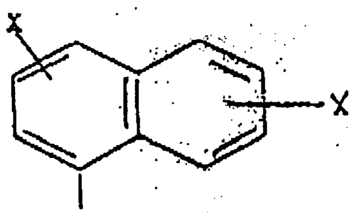

ou un radical hétérocyclique à 5 ou 6 maillons qui contient au moins un atome N, O ou S,

R[1] représente l'oxygène, une liaison directe ou un radical $-CH_2-$,

$R^2$ représente l'hydrogène, un alkyle en $C_1$-$C_{10}$, un aryle en $C_6$-$C_{12}$ porteur d'un X ou un aralkyle en $C_7$-$C_{16}$ porteur d'un X,

$R^3$ représente l'hydrogène, un alkyle en $C_1$-$C_{10}$, un cycloalkyle en $C_5$ ou $C_6$, un aryle en $C_6$-$C_{12}$ ou un aralkyle en $C_7$-$C_{16}$,

X représente l'hydrogène, un alkyle en $C_1$-$C_5$,

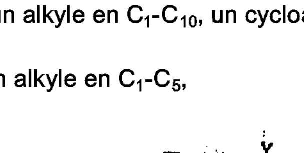

$-OR^2$, un halogène, -C≡N, $-N_3$, $-NO_2$, $-COOR^3$ ou un trifluorométhyle, ou encore un phényle si $R^8$ est un groupe

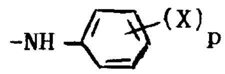

$R^4$ représente :

- un radical $-COOR^5$ dans lequel $R^5$ représente l'hydrogène, un alkyle en $C_1$-$C_{10}$ éventuellement porteur d'un halogène, d'un phényle, d'un alcoxy en $C_1$-$C_4$ ou d'un di-($C_1$-$C_4$)-alkylamino, un cycloalkyle en $C_5$ ou $C_6$, un aralkyle en $C_7$-$C_{16}$, un radical aryle en $C_6$-$C_{12}$ ou phénacyle porteur d'un X, ou un radical hétérocyclique à 5 ou 6 maillons qui renferme au moins un atome N, O ou S,
- un radical $-CONHR^6$ dans lequel $R^6$ représente l'hydrogène, un alcanoyle en $C_1$-$C_{10}$ ou un alcanesulfonyle en $C_1$-$C_{10}$,
- un radical $-CONR^2R^3$,
- un radical

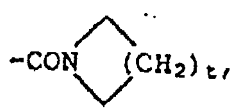

ou
- un radical

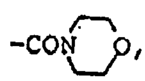

t est égal à 2 ou à 3,

Z et Y représentent chacun, indépendamment l'un de l'autre, un radical $-(CH_2)_p-$, -CH=CH- (E), $-CH=CR^7-$ ou -C≡C-,

p désigne un nombre de 0 à 5,

W représente une liaison directe, un radical

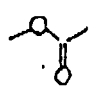 , 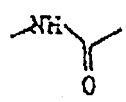

ou

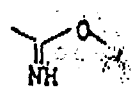

un radical hydroxyméthylène libre ou sous la forme d'un dérivé fonctionnel, ou un radical

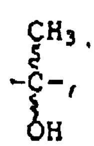

le radical OH pouvant à chaque fois avoir la configuration α ou la configuration β,

D représente une liaison directe, un radical

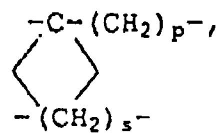

un radical

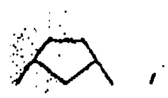

un radical

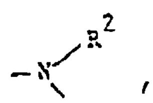

un radical -NH-$SO_2$, un alkylène saturé linéaire contenant de 1 à 5 atomes de carbone, un alkylène en $C_2$-$C_5$ saturé ramifié ou un alkylène en $C_2$-$C_5$ insaturé linéaire ou ramifié, qui peuvent éventuellement porter des atomes de fluor,

s désigne un nombre de 2 à 4,

E représente une liaison directe, -C≡C- ou un radical -CH=$CR^7$-, le symbole $R^7$ représentant l'hydrogène, un alkyle en $C_1$-$C_5$, un halogène ou trifluorométhyle,

$R^8$ représente l'hydrogène, un alkyle en $C_1$-$C_{10}$, un cycloalkyle en $C_3$-$C_{10}$, un aralkyle en $C_7$-$C_{16}$ éventuellement porteur d'un X, un aryle en $C_6$-$C_{12}$ éventuellement porteur d'un X, un radical hétérocyclique à 5 ou 6 maillons qui renferme au moins un atome N, O ou S, ou encore, dans le cas où E représente une liaison directe, $R^8$ peut représenter l'un des radicaux suivants :

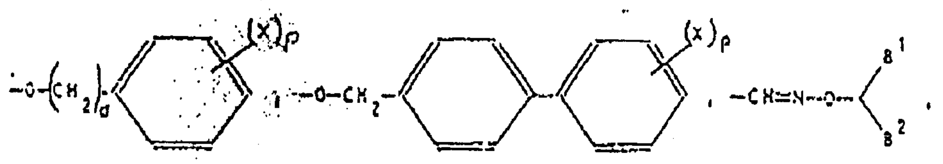

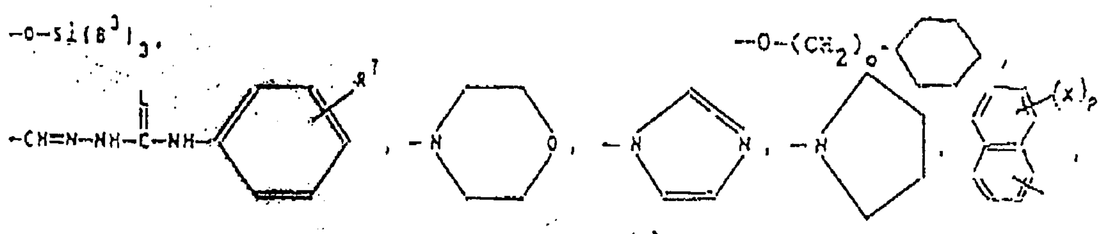

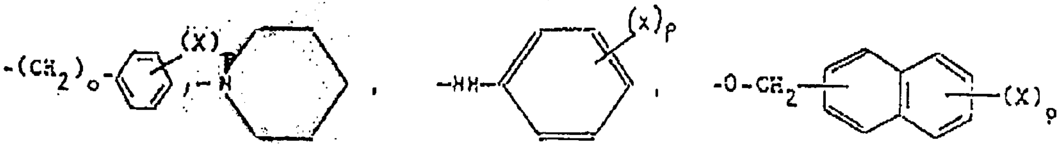

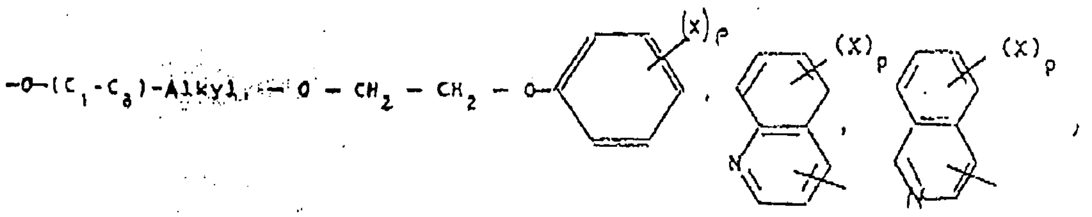

et -O- $(CH_2)_s$-L-$(CH_2)_o$-X,

(lorsque p est égal à 2 ou à 3 dans les radicaux $-(X)_p$ les X peuvent être identiques ou différents),

o désigne un nombre de 0 à 5,

$B^1$ et $B^2$ sont identiques ou différentes et ont les significations de B à l'exclusion des halogènes et des radicaux $-OR^2$, -C≡N, $-N_3$ et $-COOR^3$,

les $B^3$ représentent chacun, indépendamment l'un de l'autre, un alkyle en $C_1$-$C_4$, un phényle ou un aralkyle en $C_7$-$C_{16}$, et

L représente l'oxygène ou le soufre,

procédé caractérisé en ce qu'on fait réagir des composés à radical hydroxy-méthyle en 6 qui répondent à la formule II :

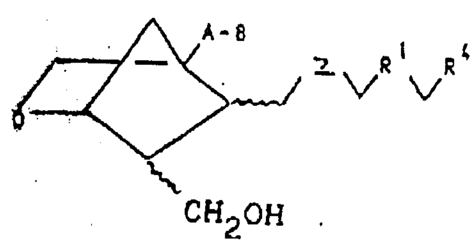

(II)

dans laquelle A, B, Z et $R^1$ ont les significations qui leur ont été données ci-dessus et $R^4$ représente un radical d'ester $-COOR^5$ dont le symbole $R^5$ a les significations indiquées ci-dessus à l'exception de l'hydrogène,

avec un composé halogéné répondant à la formule III :

$$Hal\text{-}W\text{-}D\text{-}E\text{-}R^8 \qquad (III)$$

dans laquelle Hal représente le brome ou le chlore, et W, D, E et $R^8$ ont les significations qui leur ont été données ci-dessus,

ou, après oxydation avec du chlorure d'oxalyle/DMSO, on fait réagir avec un phosphonate de diméthyle répondant à la formule V :

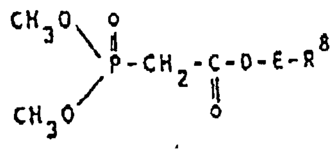

(V)

dans laquelle D, E et $R^8$ ont les significations qui leur ont été données plus haut,

en présence de NaH ou de NaH/$Br_2$, puis on réduit et, éventuellement, on élimine HBr,

ou on fait réagir le produit d'oxidation de II et du chlorure d'oxalyle/DMSO avec une amine répondant à la formule IX :

$$H_2N\text{-}R^{10} \qquad (IX)$$

dans laquelle $R^{10}$ représente un radical $-O-CH(C_6H_5)_2$ ou un radical

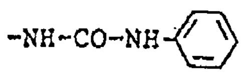

,

ou avec $CBr_4$/triphénylphosphine, puis avec un composé répondant à la formule X :

$$H\text{-}R^8 \qquad (X)$$

dans laquelle $R^8$ a la signification indiquée ci-dessus,

ou, après oxydation et réaction avec $SOCl_2$ et $NaN_3$, on fait réagir l'amine intermédiaire avec un composé halogéné de formule III et on saponifie l'ester obtenu, on transforme en sels et on convertit en clathrates de cyclodextrine ou on encapsule avec des liposomes.